# FASCICULE DE BREVET EUROPEEN

(11) **EP 3 233 848 B1**
(45) Date de publication et mention de la délivrance du brevet: **30.01.2019**
(21) Numéro de dépôt: 15820127.7
(22) Date de dépôt: 18.12.2015
(51) Int. Cl.: C07D 417/06, C07D 309/06, C07D 309/04, C07D 307/93, C07D 261/08, C07D 211/22

(54) **DÉRIVÉS BENZÈNESULFONAMIDES EN TANT QU'AGONISTES INVERSES DU RÉCEPTEUR GAMMA ORPHELIN ASSOCIÉ AUX RÉTINOÏDES ROR GAMMA (T)**
BENZOLSULFONAMIDDERIVATE ALS INVERSE AGONISTEN VON RETINOID-RELATED ORPHAN RECEPTOR GAMMA (ROR GAMMA (T))
BENZENESULFONAMIDE DERIVATIVES AS INVERSE AGONISTS OF RETINOID-RELATED ORPHAN RECEPTOR GAMMA (ROR GAMMA (T))

(30) Priorité: 19.12.2014 FR 1463034; 10.07.2015 FR 1556629
(43) Date de publication de la demande: 25.10.2017
(73) Titulaire: Galderma Research & Development, 06410 Biot (FR)
(72) Inventeur: MUSICKI, Branislav, 06000 Nice (FR); OUVRY, Gilles, 06410 Biot (FR); THOREAU, Etienne, 06460 Saint Vallier de Thiey (FR); BOUIX-PETER, Claire, 06220 Vallauris (FR)
(74) Mandataire: Casalonga
(86) Numéro de dépôt international: PCT/EP2015/080687
(87) Numéro de publication internationale: WO 2016/097389

(56) Documents cités:
- WO-A1-2013/160418
- WO-A1-2014/090712

## Description

La présente invention concerne des dérivés sulfonamides particuliers, leurs sels d'addition pharmaceutiquement acceptables leurs hydrates et/ou leurs solvates ainsi que leur utilisation en tant qu'agoniste inverse du récepteur gamma orphelin associé aux rétinoïdes RORγt.

L'invention est également relative à une composition pharmaceutique comprenant de tels composés ainsi que son utilisation pour le traitement par voie topique et/ou orale des maladies inflammatoires médiées par les récepteurs RORyt, notamment l'acné, la dermatite atopique et/ou le psoriasis.

Les récepteurs nucléaires forment une grande famille (appelée superfamille) de facteurs de transcription qui correspondent à des protéines capables d'être activées par un ligand, de se fixer sur des séquences d'ADN spécifiques et de réguler la transcription de gènes cibles. Ainsi ces récepteurs se retrouvent impliqués dans la régulation d'une grande variété de fonctions biologiques, dont la croissance, le développement, la reproduction, la différenciation et le métabolisme dans une multitude d'organismes vivants.

Les premiers membres de cette superfamille à avoir été identifiés et décrits dans la littérature scientifique sont les récepteurs nucléaires des hormones stéroïdes tels que les récepteurs aux glucocorticoïdes et les récepteurs oestrogènes. Cette superfamille comprend également parmi ses membres de nombreux récepteurs pour lesquels aucun ligand n'a été identifié. Ces récepteurs nucléaires sont appelés « récepteurs orphelins ».

Les récepteurs orphelins associés aux rétinoïdes (*Retinoid-related orphan receptors* en langue anglaise) constituent donc une sous-famille des récepteurs nucléaires. Cette sous-famille est composée de trois membres ayant chacun leur propre profil d'expression : ROR alpha (dénommé RORα), ROR beta (dénommé RORβ) et ROR gamma (dénommé RORγ). Deux isoformes des récepteurs orphelins RORγ ont déjà été identifiés, à savoir RORγ1, qui s'exprime dans une variété de tissus tels que le thymus, les reins, les muscles et le foie, et RORγ2 (aussi nommé RORγt) qui s'exprime exclusivement dans les cellules du système immunitaire.

En particulier, le récepteur RORγt joue un rôle important de régulateur dans la différenciation cellulaire des lymphocytes Th17 qui correspondent à des lymphocytes T auxiliaires ayant pour fonction d'assurer la défense de l'organisme par rapport à un grand nombre d'agents pathogènes extracellulaires tels que les bactéries et les infections fongiques.

Toutefois, il a été démontré que les lymphocytes Th17 sont également impliqués dans une large variété de désordres inflammatoires, tels que l'acné, et de maladies auto-immunes telles que le psoriasis, l'arthrite rhumatoïde ou encore la sclérose en plaques (Peck A, Mellins ED. Precarious balance; Th17 cells in host defense. Infect Immun. 2010 Jan ; 78(1) :32-8; Suarez-Farinas : J. Allergy Clin. Immunol. 2014 ; J. Invest. Dermatol. 2008, 128(11), 2625).

En effet, les lymphocytes Th17 produisent de nombreuses cytokines ayant des profils distincts telles que l'interleukine-17A (IL-17A), l'interleukine-17F (IL-17F), l'interleukine-26 (IL-26), l'interleukine-21 (IL-21), l'interleukine-22 (IL-22) et le TNFα dont leur développement, leur survie et leur prolifération dépendent de l'interleukine-23 (IL-23). Ces cytokines sont capables d'activer différents types de cellules effectrices, telles que les kératinocytes, conduisant ainsi à leur hyperprolifération et à la production supplémentaire de cytokines pro-inflammatoires, de chimiokines et de peptides antimicrobiens, qui à leur tour recrutent et activent d'autres cellules du système immunitaire dans la peau enflammée, ce qui peut conduire à une amplification de la réponse inflammatoire.

Ainsi l'activation des lymphocytes Th17 est responsable du recrutement de cytokines, notamment d'interleukine-17 (IL17), et d'autres types de cellules pro-inflammatoires qui vont conduire à la médiation de désordres inflammatoires tels que l'acné et/ou de maladies auto-immunes telles que le psoriasis.

Des expériences menées sur des souris montrent qu'une diminution au niveau de l'expression du récepteur RORyt conduit à une baisse de l'activité des lymphocytes Th17 ce qui permet, par conséquent, de fortement réduire l'expression d'interleukine-17 (IL-17) (Ivanov II, McKenzie BS, Zhou L, Tadokoro CE, Lepelley A, Lafaille JJ, Cua DJ, Littman DR : Cell 2006, 126, 1121-1133) et de traiter efficacement les désordres inflammatoires et les maladies auto-immunes médiées par ces cytokines, notamment celles pour lesquelles des taux importants en interleukine-17 (IL-17) sont détectés.

A cet effet, la demande de brevet WO 2013/160418 décrit des composés sulfonamides comme agonistes inverses du récepteur RORγt afin de pouvoir traiter les désordres inflammatoires et les maladies auto-immunes. De la même façon, d'autres composés ont également été développés en tant qu'agonistes inverses du récepteur RORγt comme ceux décrits dans les demandes de brevet WO 2014/090712, WO 2014/008214, WO2013/169588, WO2013/160419, WO2013/1002027, WO2013/092939, WO2013/092941, WO2013/085890 et WO2012/ 100732.

Il existe donc un réel besoin de développer de nouveaux composés en tant qu'agonistes inverses du récepteur RORγt afin de pouvoir traiter efficacement les maladies médiées par un tel récepteur, notamment les désordres inflammatoires, tels que l'acné, et/ou les maladies auto-immunes tels que le psoriasis et la dermatite atopique.

Ce but est atteint grâce à la mise en oeuvre de dérivés sulfonamides particuliers tels que décrits ci-après qui permettent de moduler l'activité du récepteur RORγt et de traiter par conséquent efficacement les désordres inflammatoires et les maladies auto-immunes de certaines pathologies.

La présente invention a donc notamment pour objet un ou des composés de formule (I), leurs sels d'addition pharmaceutiquement acceptables, leurs hydrates et/ou leurs solvates:

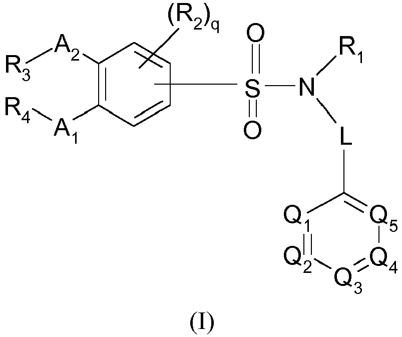

Formule (I) dans laquelle :
- q désigne zéro ou un entier naturel allant de 1 à 3,
- L représente une liaison simple,
- R¹ représente un radical alkyle, linéaire ou ramifié, en C₃-C₅, un radical cycloalkyle en C₃-C₅, un radical alkényle, linéaire ou ramifié, en C₂-C₅, un radical alkyl(C₁)cycloalkyle en C₃-C₅, un radical hétérocycloalkyle en C₄-C₅, un radical alkyl(C₁)hétérocycloalkyle en C₄-C₅,
- R₂ représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement cyano -CN ; les radicaux alkyle, alkényle et alcoxy pouvant être substitués par un ou plusieurs atomes d'halogène,
- R³ représente un radical alkyle en C₁-C₃,
- R⁴ représente un atome d'hydrogène, un groupement (CHR⁵)ₙ-(Z)ₒ-(CHR'⁵)ₚ-R⁶,
- n, o, et p, identiques ou différents, désignent zéro ou un entier naturel variant de 1 à 3,
- Z représente un groupe divalent choisi parmi -CH₂-, -NH- et -O-,
- R⁵ et R'⁵ identiques ou différents, représentent un atome d'hydrogène, un radical méthyle -CH₃, un radical hydroxy -OH, un radical hydroxyalkyle en C₁, un radical carboxylique -COOH,
- R⁶ représente :
   - un atome d'hydrogène ou un atome d'halogène,
   - un radical hétérocycloalkyle éventuellement substitué par un ou plusieurs atomes d'halogène, un ou plusieurs groupements alkyle en C₁-C₃, linéaire ou ramifié, un ou plusieurs groupements - OH, une ou plusieurs fonctions carbonyles =O, un ou plusieurs groupements hydroxyalkyle en C₁-C₄, linéaire ou ramifié, un cycle pyrrolidine un ou plusieurs groupements amino, un ou plusieurs groupements -C(=O)R⁷, un ou plusieurs groupements S(=O)₂R⁷ ; R⁷ représentant un radical alkyle, linéaire ou ramifié, en C₁-C₃, un radical hydroxy -OH, un radical alcoxy en C₁-C₄ linéaire ou ramifié, ou un radical amino N(R^{7a})(R^{7b}); avec R^{7a} et R^{7b}, identiques ou différents, désignent un atome d'hydrogène, un radical alkyle, linéaire ou ramifié, en C₁-C₃ ou un radical cyclopropyle,
   - un radical cycloalkyle en C₃-C₆ éventuellement substitué par un ou plusieurs groupements -OH,
   - un radical aromatique ou hétéroaromatique éventuellement substitué par un ou plusieurs atomes d'halogène, un ou plusieurs groupements alkyle en C₁-C₃, linéaire ou ramifié éventuellement substitué par un ou plusieurs atomes d'halogène, un ou plusieurs groupements alcoxy en C₁-C₃, un ou plusieurs groupements amino -NR¹¹R¹², un ou plusieurs groupements -COR¹¹, une fonction carbonyle (=O), un ou plusieurs groupements -OR¹¹, un ou plusieurs groupements hydroxyalkyle en C₁-C₄, un ou plusieurs groupements - COOR¹¹, un ou plusieurs groupements amido -CONR¹¹R¹², un ou plusieurs groupements -SOR¹¹, un ou plusieurs groupements -SO₂R¹¹, un ou plusieurs groupements -NHCOR¹¹, un ou plusieurs groupements -NHCOOR¹¹, un ou plusieurs groupements -SO₂NR¹¹R¹² ou un ou plusieurs groupements -CN ; R¹¹ et R¹², identiques ou différents, représentant un atome d'hydrogène ou un radical alkyle, linéaire ou ramifié, en C₁-C₃ éventuellement substitué par un ou plusieurs atomes d'halogène ;
- A₁ représente un groupe divalent choisi parmi -NR^{a}-, -O-,-S-, -SO-, -SO₂- -SO(=NH)-, -CH₂-, -C=C-, -CH(R^{a})- ;
- A₂ représente un groupe divalent -SO(=N-R^{b})-,
- R^{a} représente un atome d'hydrogène, un radical alkyle, linéaire ou ramifié, en C₁-C₃, un radical acétyle -C(=O)CH₃,
- R^{b} représente un atome d'hydrogène, un radical alkyle, linéaire ou ramifié, en C₁-C₃, un groupement cyclopropyle,
- Q₁, Q₂, Q₃, Q₄ et Q₅, identiques ou différents, représentent un atome d'azote ou un groupement -CR'₂,
- R'₂ représente un atome d'hydrogène ou un radical alkyle linéaire ou ramifié en C₁-C₅,
- R^{a} et R³ peuvent former ensemble avec les atomes de carbone auxquels ils sont attachés un groupe hétérocycloalkyle pouvant être éventuellement substitué par un ou plusieurs fonctions carbonyle, un ou plusieurs radicaux alkyle en C₁-C₃,
- lorsque A₁ représente -NR^{a}- alors R^{a} et R⁴ peuvent former ensemble avec l'atome d'azote auquel ils sont rattachés un groupe hétérocycloalkyle en C₂-C₁₀ comprenant éventuellement un à trois hétéroatomes choisis parmi un atome de soufre, un atome d'azote et un atome d'oxygène ; ledit groupe hétérocycloalkyle étant éventuellement substitué par au moins un radical R¹⁴,
- R¹⁴ représente un radical alkyle, linéaire ou ramifié, en C₁-C₃, un radical alcoxy, linéaire ou ramifié, en C₁-C₃, un atome d'halogène, un groupement hydroxy -OH, un groupement cyano -CN, un groupement -CONR¹⁵R¹⁶, un groupement -SO₂R¹⁵, un groupement -COR¹⁵ ou un groupement amino -NR¹⁵R¹⁶ ; R¹⁵ et R¹⁶, identiques ou différents, représentant un atome d'hydrogène ou un radical alkyle, linéaire ou ramifié, en C₁-C₃.

Les composés selon l'invention correspondent à des dérivés sulfonamides soufrés qui comportent dans leur structure au moins un groupement sulfonamide SO₂-N et au moins un atome de soufre.

Les composés selon l'invention permettent de moduler, c'est-à-dire d'inhiber, l'activité du récepteur RORγt.

La présente invention a également pour objet le ou les composés tels que définis précédemment en tant que médicament et cosmétique.

Un autre objet de l'invention porte sur le ou les composés tels que définis précédemment pour leur utilisation dans le traitement des maladies médiées par le récepteur RORγt, notamment les désordres inflammatoires et/ou les maladies auto-immunes médiées par le récepteur RORγt.

Par ailleurs, l'invention concerne également une composition pharmaceutique comprenant, dans un milieu pharmaceutiquement acceptable, un ou plusieurs composés de formule (I) telle que définie précédemment, leurs sels d'addition pharmaceutiquement acceptables, leurs hydrates et/ou leurs solvates.

La présente invention a aussi trait à la composition pharmaceutique telle que décrite précédemment pour son utilisation dans le traitement des maladies médiées par le récepteur RORγt, notamment les désordres inflammatoires et/ou les maladies auto-immunes.

Enfin, l'invention est relative à une méthode de traitement des maladies médiées par le récepteur RORγt comprenant l'administration, notamment par voie topique ou orale, d'une quantité thérapeutiquement efficace d'un ou plusieurs composés tels que définis ci-avant à un patient.

D'autres objets et caractéristiques, aspects et avantages de l'invention apparaîtront encore plus clairement à la lecture de la description et des exemples qui suivent.

De préférence, le ou les composés de formule (I) selon l'invention est ou sont choisi(s) parmi le ou les composés de formules (Ia) et/ou (Ib) :

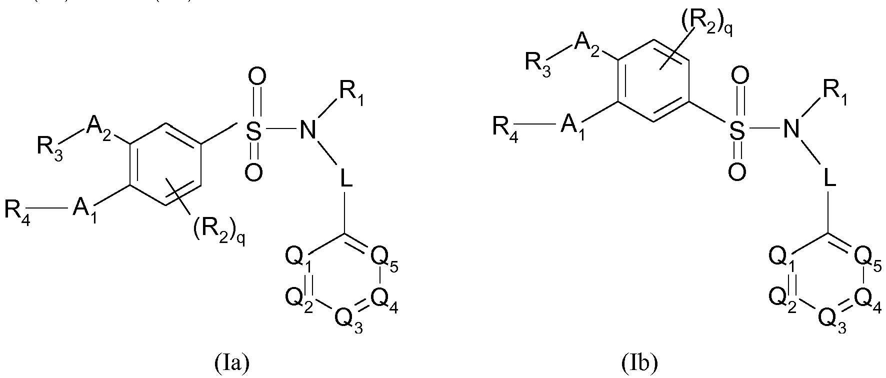

Formules (la) et (Ib) dans lesquelles R¹, R₂, R'₂, R³, R⁴, R⁵, R'⁵, R⁶, R⁷, R^{7a}, R^{7b}, R¹¹, R¹², R¹⁴, R¹⁵, R¹⁶, R^{a}, R^{b}, Z, Q₁, Q₂, Q₃, Q₄, Q₅, A₁, A₂, L et les indices q, n, o et p ont les mêmes significations que dans la formule (I) précédemment décrite.

Dans les formules (I), (Ia) et (Ib), L représente une liaison simple.

Selon un mode de réalisation, dans les formules (I), (Ia) et (Ib), R¹ représente un radical alkyle, linéaire ou ramifié, en C₃-C₅, notamment ramifié en C₄.

Selon un mode de réalisation, dans les formules (I), (Ia) et (Ib), R¹ représente un radical cycloalkyle en C₃-C₅.

Selon un mode de réalisation, dans les formules (I), (Ia) et (Ib), R¹ représente un radical alkényle, linéaire ou ramifié, en C₂-C₅.

Selon un mode de réalisation, dans les formules (I), (Ia) et (Ib), R¹ représente un radical alkyl(C₁)cycloalkyle en C₃-C₅.

Selon un mode de réalisation, dans les formules (I), (Ia) et (Ib), R¹ représente un radical hétérocycloalkyle en C₄-C₅.

Selon un mode de réalisation, dans les formules (I), (Ia) et (Ib), R¹ représente un radical alkyl(C₁)hétérocycloalkyle en C₄-C₅.

Préférentiellement, R¹ représente un radical alkyle, linéaire ou ramifié, en C₃-C₅, notamment ramifié, et encore plus préférentiellement ramifié en C₄.

Dans les formules (I), (Ia) et (Ib), R³ représente un radical alkyle, linéaire ou ramifié en C₁-C₃, notamment en C₁.

Selon un mode de réalisation, dans les formules (I), (Ia) et (Ib), Q₁, Q₂, Q₃, Q₄ et Q₅, identiques ou différents, représentent un groupement -CR'₂, avec R'₂ pouvant représenter un atome d'hydrogène ou un radical alkyle linéaire ou ramifié en C₁-C₅.

De préférence, dans les formules (I), (Ia) et (Ib), Q₃ représente un groupement -CR'₂ avec R'₂ représentant un radical alkyle linéaire ou ramifié en C₁-C₅, notamment en C₂.

De préférence, Q₁, Q₂, Q₄ et Q₅, identiques ou différents, représentent un groupement -CR'₂, avec R'₂ représentant un atome d'hydrogène.

Conformément à un mode préférentiel, Q₃ représente un groupement -CR'₂ avec R'₂ représentant un radical alkyle linéaire ou ramifié en C₁-C₅, notamment en C₂, et Q₁, Q₂, Q₄ et Q₅, identiques ou différents, représentent un groupement -CR'₂, avec R'₂ représentant un atome d'hydrogène.

Selon un mode de réalisation, dans les formules (I), (Ia) et (Ib), l'indice q correspond à zéro.

Dans les formules (I), (Ia) et (Ib), A₂ représente le groupe divalent -SO(=N-R^{b})-.

Préférentiellement, dans les formules (I), (Ia) et (Ib), A₂ représente le groupe divalent -SO(=N-R^{b})- avec R^{b} représentant un atome d'hydrogène.

De préférence, dans les formules (I), (Ia) et (Ib), A₁ représente un groupe divalent choisi parmi les groupes -NR^{a}- et -CH(R^{a})-.

Selon un mode de réalisation, dans les formules (I), (Ia) et (Ib), A₁ représente le groupe divalent -NR^{a}-.

Selon un mode de réalisation, dans les formules (I), (Ia) et (Ib), A₁ représente le groupe divalent -O-.

Selon un mode de réalisation, dans les formules (I), (Ia) et (Ib), A₁ représente le groupe divalent -SO-.

Selon un mode de réalisation, dans les formules (I), (Ia) et (Ib), A₁ représente le groupe divalent -S-.

Selon un mode de réalisation, dans les formules (I), (Ia) et (Ib), A₁ représente le groupe divalent -SO₂-.

Selon un mode de réalisation, dans les formules (I), (Ia) et (Ib), A₁ représente le groupe divalent - SO(=NH)-.

Selon un mode de réalisation, dans les formules (I), (Ia) et (Ib), A₁ représente le groupe divalent -CH₂-.

Selon un mode de réalisation, dans les formules (I), (la) et (Ib), A₁ représente le groupe divalent -C=C-.

Selon un mode de réalisation, dans les formules (I), (la) et (Ib), A₁ représente le groupe divalent -CH(R^{a})-.

Préférentiellement, dans les formules (I), (Ia) et (Ib), A₁ représente le groupe divalent -O-, -S- et -SO-, et encore plus préférentiellement le groupe divalent -O-.

Selon un mode de réalisation, dans les formules (I), (la) et (Ib), les indices n, o, et p, identiques ou différents, désignent zéro.

Selon un mode de réalisation, dans les formules (I), (la) et (Ib), les indices n, o, et p, identiques ou différents, désignent un entier naturel variant de 1 à 3.

Selon un mode de réalisation, les indices n et o désignent 1 et l'indice p désigne zéro.

Selon un mode de réalisation, les indices n et p désignent zéro et l'indice o vaut 1.

Selon un mode de réalisation, dans les formules (I), (la) et (Ib), Z représente un groupe méthylène -CH₂-.

Selon un mode de réalisation, dans les formules (I), (la) et (Ib), Z représente un groupe divalent -O-.

Selon un mode de réalisation, dans les formules (I), (la) et (Ib), Z représente un groupe divalent -NH-.

De préférence, R⁴ est différent d'un atome d'hydrogène.

Selon un mode de réalisation, dans les formules (I), (Ia) et (Ib), R⁴ représente un groupement Z-R⁶, avec Z ayant la signification précédemment décrite.

Selon un mode de réalisation, dans les formules (I), (Ia) et (Ib), R⁴ représente un groupement -CH₂-R⁶.

Selon un mode de réalisation, dans les formules (I), (Ia) et (Ib), R⁴ représente un groupement -O-R⁶.

Selon un mode de réalisation, dans les formules (I), (Ia) et (Ib), R⁴ représente un groupement -NH-R⁶.

Ainsi, dans les formules (I), (Ia) et (Ib), R⁴ est choisi parmi les groupements -CH₂-R⁶, -O-R⁶ ou -NH-R⁶.

Selon un mode de réalisation, dans les formules (I), (la) et (Ib), R⁶ représente un groupe hétérocyclique monocyclique, bicyclique ou bicyclique spiro.

Selon un mode de réalisation, R⁶ représente un radical hétérocycloalkyle, de préférence choisi parmi : 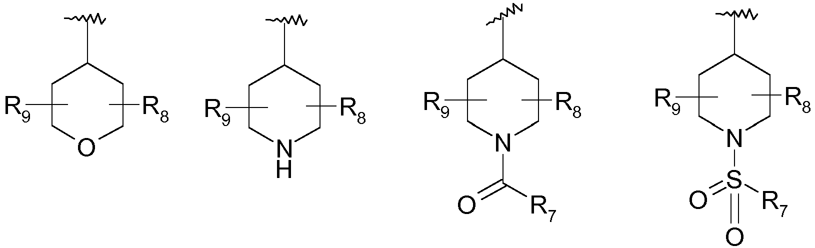 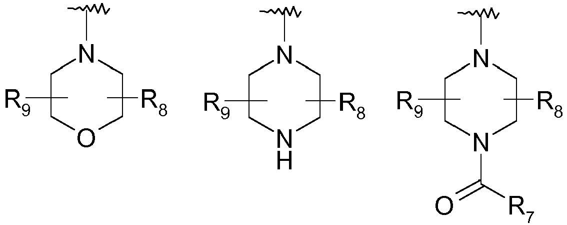 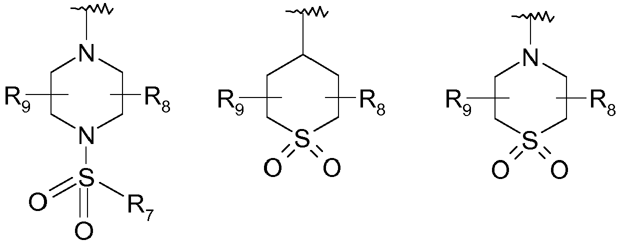 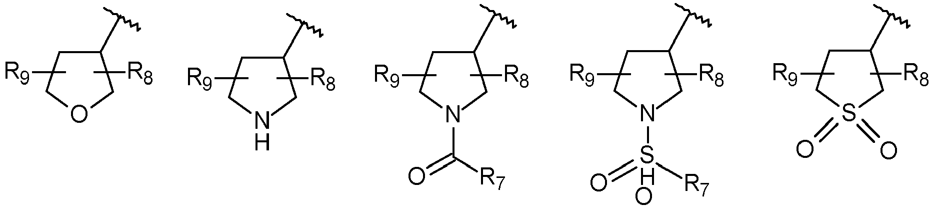 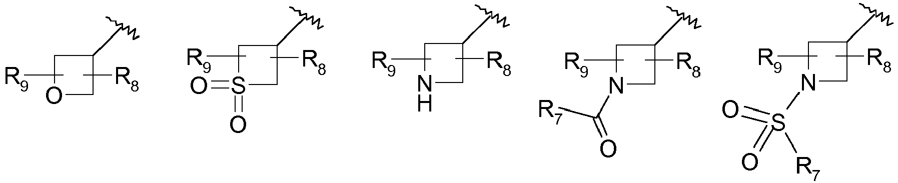 dans lesquels :
- R₇ représente un radical alkyle, linéaire ou ramifié, en C₁-C₃, un radical hydroxy -OH, un radical alcoxy en C₁-C₃ ou un radical amino en N(R^{7a})(R^{7b}),
- R^{7a} et R^{7b}, identiques ou différents, désignent un atome d'hydrogène, un radical alkyle, linéaire ou ramifié, en C₁-C₃ ou un radical cyclopropyle,
- R₈ et R₉, identiques ou différents, représentent un atome d'hydrogène, un radical alkyle, linéaire ou ramifié, en C₁-C₃, un groupement hydroxy -OH, un groupe carbonyle, un radical hydroxyalkyle en C₁ (CH₂OH) un groupe amino -NH₂,
- R₈ et R₉ peuvent former ensemble avec les atomes de carbone auxquels ils sont attachés un cycle carbocyclique comportant de 5 à 7 chaînons.

Selon un mode de réalisation, dans les formules (I), (Ia) et (Ib), R⁶ représente un radical aromatique ou hétéroaromatique choisi de préférence parmi : 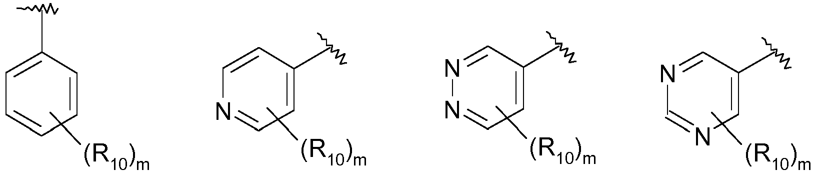 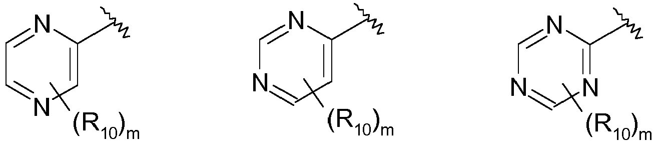 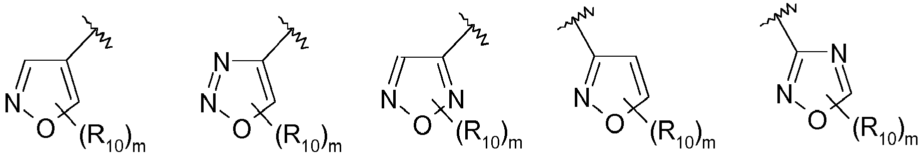 dans lesquels :
- R₁₀ représente un atome d'hydrogène ou un atome d'halogène ; un radical alkyle, linéaire ou ramifié, en C₁-C₃ éventuellement substitué par un ou plusieurs atomes d'halogène ; une fonction carbonyle (=O), un groupe OR¹¹, un groupe hydroxyalkyle en C₁-C₄, un groupe amino -NR¹¹R¹², un groupe -COR¹¹, un groupe-COOR¹¹, un groupe amido -CONR¹¹R¹², un groupe -SOR¹¹, un groupe -SO₂R¹¹, un groupe -NHCOR¹¹, un groupe -NHCOOR¹¹, un groupe-SO₂NR¹¹R¹² ou un groupe cyano -CN,
- R¹¹ et R¹², identiques ou différents, représentent un atome d'hydrogène ou un radical alkyle, linéaire ou ramifié, en C₁-C₃ éventuellement substitué par un ou plusieurs atomes d'halogène
- m désigne zéro ou un entier naturel allant de 1 à 3.

Préférentiellement, R⁶ représente un radical aromatique ou hétéroaromatique tel que défini précédemment éventuellement substitué par un ou plusieurs groupements méthyle -CH₃, un ou plusieurs groupements méthoxy -OCH₃, un ou plusieurs groupements hydroxy -OH, un ou plusieurs groupements amino -NH₂, un ou plusieurs groupements -CH₂OH, un ou plusieurs groupements cyano - CN, un ou plusieurs atomes d'halogène, une ou plusieurs fonctions carbonyles.

Selon un mode de réalisation, R⁶ représente un atome d'hydrogène.

Selon un mode de réalisation, R⁶ représente un radical cycloalkyle en C₃-C₆.

Selon un mode de réalisation, R₈ et R₉ représentent un atome d'hydrogène.

Selon un mode de réalisation, R₈ et R₉ représentent un radical alkyle, linéaire ou ramifié, en C₁-C₃.

Selon un mode de réalisation, dans les formules (I), (la) et (Ib), lorsque A₂ représente un groupe divalent SO(=N-R^{b})- alors A₁ représente un groupe divalent choisi parmi les groupes -NR^{a}- et -CH(R^{a}) - et R^{a} et R₃ forment ensemble avec les atomes de carbone auxquels ils sont attachés un groupe hétérocycloalkyle comportant 5 ou 6 chaînons éventuellement substitué par une ou plusieurs fonctions carbonyles, un ou plusieurs atomes d'halogène ou un ou plusieurs radicaux alkyle en C₁-C₂.

Conformément à ce mode de réalisation, R^{a} et R₃ forment ensemble avec les atomes de carbone auxquels ils sont attachés un groupe hétérocycloalkyle non substitué comportant 5 ou 6 chaînons.

Conformément à ce mode de réalisation, A₂ représente SO(=N-R^{b})- avec R^{b} représentant de préférence un atome d'hydrogène ou un radical alkyle, linéaire ou ramifié, en C₁-C₃.

Selon un autre mode de réalisation, dans les formules (I), (la) et (Ib), lorsque A₂ représente un groupe divalent -SO(=N-R^{b})- alors A₁ représente un groupe divalent choisi parmi les groupes divalents -NR^{a}-, -O-, -CH₂-, -C=C- ou -CH(R^{a}).

Conformément à ce mode de réalisation, R^{a} et R₃ ne forment pas ensemble avec les atomes de carbone auxquels ils sont attachés un groupe hétérocycloalkyle comportant 5 ou 6 chaînons.

Selon un mode de réalisation, lorsque A₁ représente -NR^{a}-alors R^{a} et R⁴ forment ensemble avec l'atome d'azote auquel ils sont rattachés un groupe hétérocycloalkyle en C₂-C₁₀ comprenant éventuellement un à trois hétéroatomes choisis parmi un atome de soufre, un atome d'azote et un atome d'oxygène ; ledit groupe hétérocycloalkyle étant éventuellement substitué par au moins un radical R¹⁴ tel que défini dans la formule (I) précédemment décrite.

En particulier, le groupe hétérocycloalkyle en C₂-C₁₀ peut être monocyclique, bicyclique ou bicyclique spiro.

De préférence, le groupe hétérocycloalkyle est éventuellement substitué par un, deux ou trois radicaux R¹⁴ tels que définis précédemment.

Plus simplement, le ou les composés selon l'invention est ou sont choisis parmi les composés de formule (II) ainsi que leurs sels d'addition pharmaceutiquement acceptables, leurs hydrates et/ou leurs solvates :

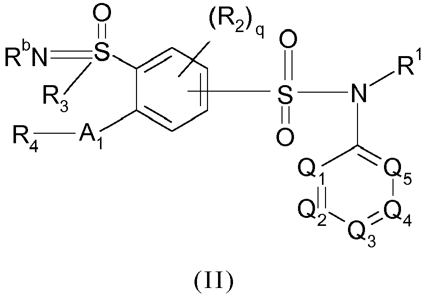

Formule (II) dans laquelle :
- R³ représente un radical alkyle en C₁-C₃,
- R¹, R₂, R'₂, R³, R⁴, R⁵, R'⁵, R⁶, R⁷, R^{7a}, R^{7b}, R₈, R₉, R₁₀, R¹¹, R¹², R^{a}, R^{b}, Z, Q₁, Q₂, Q₃, Q₄, Q₅, A₁, et les indices q, m, n, o et p ont les mêmes significations que celles indiquées précédemment.

De préférence, R^{b} représente un atome d'hydrogène ou un radical alkyle en C₁.

Préférentiellement, R^{b} représente un atome d'hydrogène.

De préférence, R³ représente un radical alkyle en C₁.

De préférence, R¹ représente un radical alkyle ramifié en C₃.

Préférentiellement, R⁴ représente un groupement (CHR⁵)ₙ-(Z)ₒ-(CHR'⁵)ₚ-R⁶ avec R⁶ correspondant de préférence à un radical aromatique ou hétéroaromatique, un radical cycloalkyle ou un radical hétérocyclique tels que définis ci-avant dans la formule (I) ou tel que précédemment.

De préférence, Q¹-Q² et Q⁴-Q⁵ correspondent à un groupement -CR² avec R² désignant un atome d'hydrogène et Q³ correspond à un groupement -CR² avec R² désignant un radical alkyle, linéaire ou ramifié, en C₁-C₅, de préférence en C₂.

De préférence, Q¹ et Q³, identiques ou différents, correspondent à un groupement -CR'₂ avec R'₂ désignant un atome d'hydrogène ou un radical alkyle, linéaire ou ramifié, en C₁-C₅, de préférence en C₂.

En particulier, lorsque le groupe A₁ représente un groupe divalent -NR^{a} alors R^{a} et R₄ ne forment pas ensemble avec l'atome d'azote auquel ils sont rattachés un groupe hétérocycloalkyle en C₂-C₁₀ tel que défini dans la formule (I) précédemment décrite.

Conformément à un mode de réalisation, de préférence, R¹ représente un radical alkyle, linéaire ou ramifié, en C₃-C₅ et R^{b} représente un atome d'hydrogène.

De préférence, le ou les composés selon l'invention est ou sont choisis parmi les composés de formule (III) ainsi que leurs sels d'addition pharmaceutiquement acceptables, leurs hydrates et/ou leurs solvates :

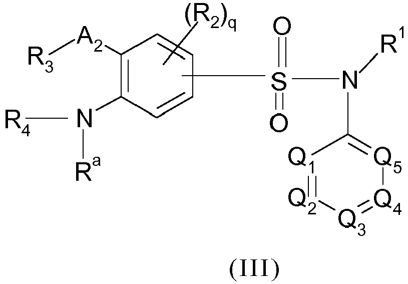

Formule (III) dans laquelle :
- R¹, R₂, R'₂, R³, R^{b}, Q₁, Q₂, Q₃, Q₄, Q₅, A₂ et l'indice q ont les mêmes significations que dans la formule (I) précédemment décrite,
- R^{a} et R₄ forment ensemble avec l'atome d'azote auquel ils sont rattachés un groupe hétérocycloalkyle en C₂-C₁₀ comprenant éventuellement un à trois hétéroatomes choisis parmi un atome de soufre, un atome d'azote et un atome d'oxygène ; ledit groupe hétérocycloalkyle étant éventuellement substitué par au moins un radical R¹⁴,
- R¹⁴ représente un radical alkyle, linéaire ou ramifié, en C₁-C₃, un radical alcoxy, linéaire ou ramifié, en C₁-C₃, un atome d'halogène, un groupement hydroxy -OH, un groupement cyano -CN, un groupement -CONR¹⁵R¹⁶, un groupement -SO₂R¹⁵, un groupement -COR¹⁵ ou un groupement amino -NR¹⁵R¹⁶ ; R¹⁵ et R¹⁶, identiques ou différents, représentant un atome d'hydrogène ou un radical alkyle, linéaire ou ramifié, en C₁-C₃.

En particulier, le groupe hétérocycloalkyle en C₂-C₁₀ peut être monocyclique, bicyclique ou bicyclique spiro.

Les composés de formule (I), (II), (III), (Ia) et (Ib) peuvent se présenter sous la forme de sels pharmaceutiquement acceptables. Des exemples de sels pharmaceutiquement acceptables sont décrits dans Berge et al., 1977, «sels pharmaceutiquement acceptables», J. Pharm. Sci., Vol. 66, pp 1 -19.

En particulier, lorsque que les composés de formule selon l'invention se présentent sous la forme de sels alors l'électroneutralité desdits composés est assurée par un contre ion cationique Y externe pouvant être organique ou minérale.

Y peut être choisi parmi les cations inorganiques appropriés tels que les ions de métaux alcalins, notamment Na⁺, K⁺, les ions métaux alcalino-terreux, notamment Ca²⁺, Mg²⁺, ou encore d'autres cations tels que l'ion aluminium Al³⁺.

Y peut être choisi parmi les cations organiques appropriés tels que l'ion ammonium NH₄⁺, les ions ammonium substitués tels que NH₃R⁺, NHR₂⁺, NR₄⁺ avec R représentant un radical alkyle en C₁-C₄.

En particulier, les ions ammonium substitués sont ceux choisis parmi les dérivés de l'éthylamine, la diéthylamine, la dicyclohexylamine, la triéthylamine, la butylamine, l'étylènediamine, l'éthanolamine, la diéthanolamine, la pipérazine, la benzylamine, la phénylbenzylamine, la choline, la meglumine, et trométhamine, les acides aminés tels que la lysine et l'arginine.

Un exemple d'un ion ammonium quaternaire peut être l'ion N⁺ (CH₃)₄.

Le ou les composés selon l'invention peuvent se présenter sous la forme de leurs solvates.

Au sens de la présente invention, le terme « solvate » signifie un complexe de soluté (c'est-à-dire le composé selon l'invention ou le sel dudit composé) et de solvant.

Si le solvant est l'eau alors le solvate peut être commodément considéré comme un hydrate, par exemple, un semi-hydrate, un monohydrate, un dihydrate, un trihydrate, etc.

Par exemple, les solvates et/ou hydrates peuvent être obtenus directement à la fin du processus de synthèse, le composé cible étant isolé sous la forme d'un hydrate, par exemple un monohydrate ou hémi-hydrate, ou sous la forme d'un solvate du solvant de réaction et/ou du solvant de purification.

Sauf indication contraire, toute référence à un composé selon l'invention inclut également le solvate ou l'hydrate du composé correspondant.

Des procédures typiques pour la préparation et l'identification des hydrates et des solvates sont bien connus de l'homme du métier, voir par exemple, pages 202-209 de KJ Guillory, « Génération of Polymorphs, Hydrates, Solvates, and Amorphous Solids » dans Polymorphism in Pharmaceutical Solids, edition. Harry G. Britain, Vol. 95, Marcel Dekker, Inc., New York, 1999.

Les hydrates et les solvates peuvent être isolés et caractérisés par des méthodes connues dans l'art telles que l'analyse thermogravimétrique (TGA), la spectroscopie TGA-masse, la spectroscopie TGA-infrarouge, la diffraction de poudres aux rayons X, le titrage de Karl Fisher, la diffraction haute résolution aux rayons X et analogue.

De préférence, le ou les composés de formule (I) sont choisis parmi les composés suivants tels que décrits dans les tableaux ci-après, ainsi que leurs sels d'addition pharmaceutiquement acceptables, leurs hydrates et/ou leurs solvates.

Les exemples qui ne tombent pas sous la formule (II) telle que définie dans la revendication 1 ne font pas partie de l'invention.

**Tableau 1 :**

| | | IC50 hRORg | IC50 hCD4/IL17 |
|---|---|---|---|
| 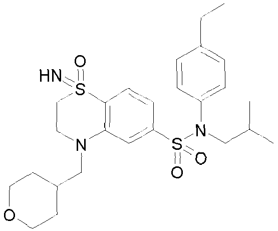 | Acide imino-1-oxo-4-(tétrahydro-pyran-4-ylméthyl)-1,2,3,4-tétrahydro-1λ⁶-benzo[1,4]thiazine-7-sulfonique (4-éthyl-phényl)-isobutyl-amide | C | ND |
| | Composé 1 | | |
| 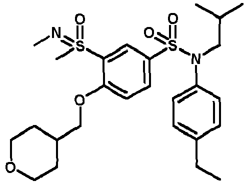 | N-(4-éthyl-phényl)-N-isobutyl-3-méthane-sulfinyl-4-(tétrahydro-pyran-4-ylméthoxy)-benzène-N-méthyl-sulfoximine | B | ND |
| | Composé 2 | | |
| 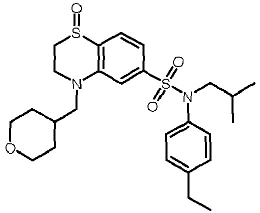 | Acide 1-oxo-4-(tetrahydro-pyran-4-ylmethyl)-1,2,3,4-tetrahydro-1λ⁴-benzo-[1,4]thiazine-7-sulfonique | C | ND |
| | Composé 3 | | |
| 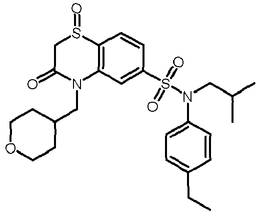 | Acide 1,3-dioxo-4-(tetrahydro-pyran-4-ylméthyl)-1,2,3,4-tetrahydro-1λ⁴-benzo-[1,4]-thiazine-6-sulfonique (4-éthyl-phényl)-isobutyl-amide | C | ND |
| | Composé 4 | | |
| 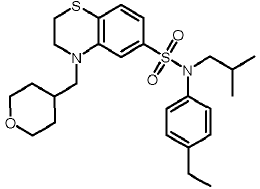 | Acide 4-(tétrahydro-pyran-4-ylméthyl)-3,4-dihydro-2H-benzo-[1,4]-thiazine-6-sulfonique (4-éthyl-phényl)-isobutyl-amide | C | ND |
| | Composé 6 | | |
| 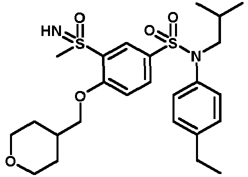 | N-(4-Ethyl-phenyl)-N-isobutyl-3-methane-sulfoximin-4-(tetrahydro-pyran-4-ylmethoxy)-benzenesulfonamide | A | A |
| | Composé 26 | | |
| 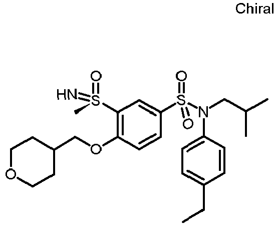 | N-(4-éthyl-phényl)-N-isobutyl-3-méthane-sulfoximin-4-(tétrahydro-pyran-4-ylméthoxy)-benzènesulfonamide | A | A |
| | composé 7 (énantiomère A du composé 26) | | |
| 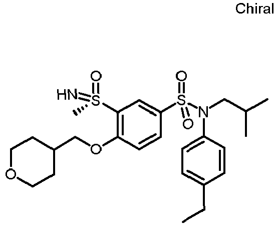 | N-(4-éthyl-phényl)-N-isobutyl-3-méthane-sulfoximin-4-(tétrahydropyran-4-ylméthoxy)-benzènesulfonamide | A | A |
| | composé 8 (énantiomère B du composé 26) | | |
| 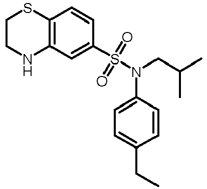 | Acide 3,4-dihydro-2H-benzo[1,4]thiazine-6-sulfonique (4-éthyl-phényl)-isobutyl-amide | C | ND |
| | Composé 9 | | |
| 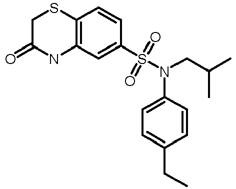 | Acide 3-oxo-3,4-dihydro-2H-benzo[1,4]thiazine-6-sulfonique(4-éthyl-phényl)-isobutyl-amide | C | ND |
| | Composé 10 | | |
| 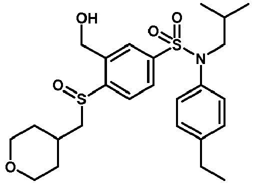 | N-(4-éthyl-phényl)-3-hydroxymethyl-N-isobutyl-4-(tetrahydro-pyran-4-yl-méthane-sulfinyl)-benzenesulfonamide | B | ND |
| | Composé 15 | | |
| 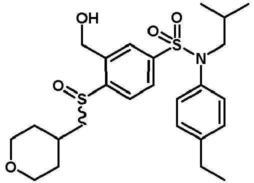 | N-(4-éthyl-phényl)-3-hydroxyméthyl-N-isobutyl-4-(tétrahydro-pyran-4-yl-méthane-sulfinyl)-benzenesulfonamide | B | ND |
| | Composé 11 (énantiomère A du composé 15) | | |
| 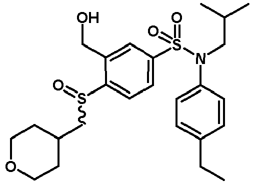 | N-(4-éthyl-phényl)-3-hydroxyméthyl-N-isobutyl-4-(tétrahydro-pyran-4-yl-méthane-sulfinyl)-benzenesulfonamide | B | ND |
| | Composé 12 (énantiomère B du composé 15) | | |
| 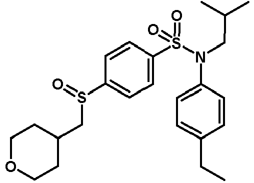 | N-(4-éthyl-phényl)-N-isobutyl-4-(tetrahydro-pyran-4-ylmethanesulfinyl)-benzenesulfonamide | B | B |
| | Composé 29 | | |
| 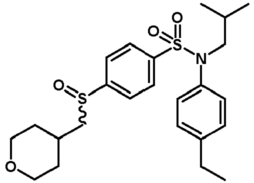 | N-(4-éthyl-phényl)-N-isobutyl-4-(tétrahydro-pyran-4-ylméthanesulfinyl)-benzenesulfonamide | B | B |
| | Composé 13 (énantiomère A du composé 29) | | |
| 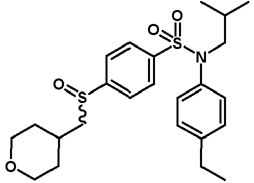 | N-(4-éthyl-phényl)-N-isobutyl-4-(tétrahydro-pyran-4-ylméthanesulfinyl)-benzenesulfonamide | B | B |
| | Composé 14 (énantiomère B du composé 29) | | |
| 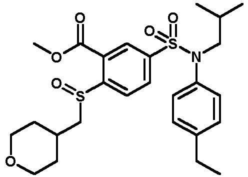 | 5-[(4-éthyl-phényl)-isobutyl-sulfamoyl] -2-(tetrahydro-pyran-4-yl-methanesulfinyl)-benzoate de méthyle | C | N D |
| | Composé 16 | | |
| 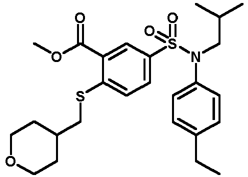 | 5-[(4-éthyl-phényl)-isobutyl-sulfamoyl]-2-(tétrahydro-pyran-4-ylméthylsulfanyl)-benzoate de méthyle | C | ND |
| | Composé 17 | | |
| 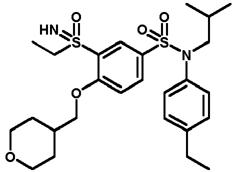 | N-(4-éthyl-phényl)-N-isobutyl-3-éthane-sulfoximin-4-(tétrahydro-pyran-4-ylméthoxy)-benzènesulfonamide | A | A |
| | Composé 18 | | |
| 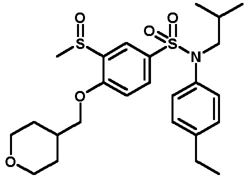 | N-(4-éthyl-phényl)-N-isobutyl-3 -méthane-sulfinyl-4-(tétrahydro-pyran-4-ylméthoxy)-benzènesulfonamide | A | A |
| | Composé 27 | | |
| 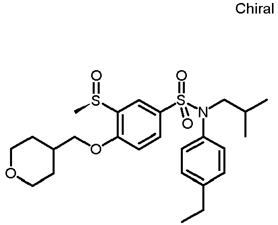 | N-(4-éthyl-phényl)-N-isobutyl-3-méthane-sulfinyl-4-(tétrahydro-pyran-4-ylméthoxy)-benzènesulfonamide | B | A |
| | Composé 19 (énantiomère A du composé 27) | | |
| 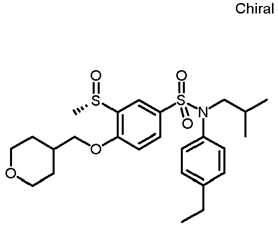 | N-(4-éthyl-phényl)-N-isobutyl-3-méthane-sulfinyl-4-(tétrahydro-pyran-4-ylméthoxy)-benzènesulfonamide | B | B |
| | Composé 20 (énantiomère B du composé 27) | | |
| 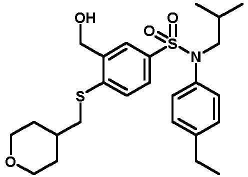 | N-(4-éthyl-phényl)-3-hydroxyméthyl-N-isobutyl-4-(tétrahydro-pyran-4-ylméthyl-sulfanyl)-benzènesulfonamide | B | A |
| | Composé 21 | | |
| 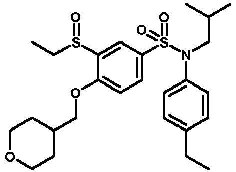 | éthanesulfinyl-N-(4-éthyl-phényl)-N-isobutyl-4-(tétrahydro-pyran-4-ylméthoxy)-benzenesulfonamide | B | B |
| | Composé 22 | | |
| 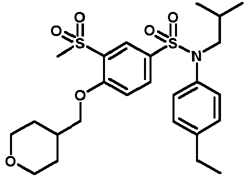 | N-(4-éthyl-phényl)-N-isobutyl-3-méthane-sulfonyl-4-(tétrahydro-pyran-4-ylméthoxy)-benzenesulfonamide | B | A |
| | Composé 24 | | |
| 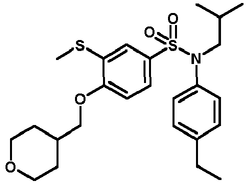 | N-(4-éthyl-phényl)-N-isobutyl-3 -méthylsulfanyl-4-(tétrahydro-pyran-4-ylméthoxy)-benzenesulfonamide | B | B |
| | Composé 25 | | |
| 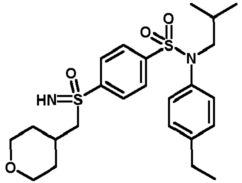 | N-(4-éthyl-phényl)-N-isobutyl-4-(tétrahydro-pyran-4-ylméthanesulfoximinyl)-benzènesulfonamide | C | ND |
| | Composé 28 | | |

**Tableau 2 :**

| | | IC50 hRORg | IC50 hCD4/IL17 |
|---|---|---|---|
| 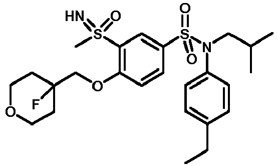 | N-(4-éthylphényl)-4-((4-fluorotétrahydro-2H-pyran-4-yl)méthoxy)-N-isobutyl-3-(S-méthyl-sulfonimidoyl)benzènesulfo namide | A | A |
| | Composé 30 | | |
| 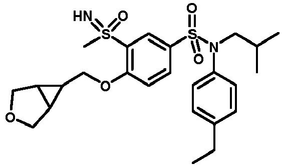 | 4-((3-oxabicyclo-[3.1.0]-hexan-6-yl)méthoxy)-N-(4-éthylphényl)-N-isobutyl-3-(S-méthyl-sulfonimidoyl)benzenesulfonamide | A | ND |
| | Composé 31 | | |
| 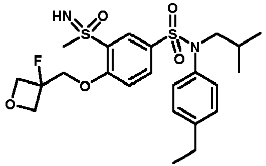 | N-(4-éthylphényl)-4-((3-fluorooxétan-3-yl)-méthoxy)-N-isobutyl-3-(S-méthyl-sulfonimidoyl)-benzènesulfonamide | C | ND |
| | Composé 32 | | |
| 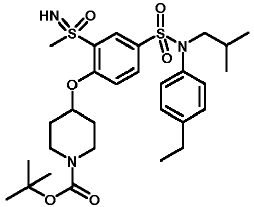 | Tert-butyl 4-(4-(N-(4-éthylphényl)-N-isobutyl-sulfamoyl)-2-(S-méthyl-sulfonimidoyl)-phenoxy)-pipéridine-1-carboxylate | C | ND |
| | Composé 33 | | |
| 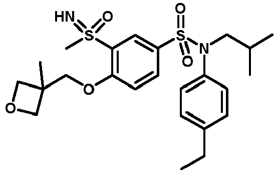 | N-(4-éthylphenyl)-N-isobutyl-4-((3-méthyl-oxétan-3-yl)méthoxy)-3-(S-méthyl-sulfonimidoyl)-benzenesulfonamide | C | ND |
| | Composé 34 | | |
| 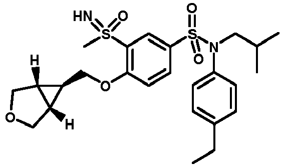 | 4-(((1R,5S,6r)-3-oxabicyclo-[3.1.0]hexan-6-yl)méthoxy)-N-(4-éthylphényl)-N-isobutyl-3-(S-méthylsulfonimidoyl)-benzene-sulfonamide | A | ND |
| | Composé 35 | | |
| 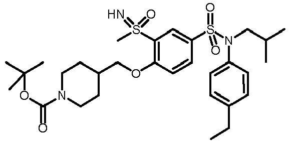 | tert-butyl 4-((4-(N-(4-éthylphényl)-N-isobutyl-sulfamoyl)-2-(S-méthyl-sulfonimidoyl)phénoxy)mét hyl)-piperidine-1-carboxylate | C | ND |
| | Composé 36 | | |
| 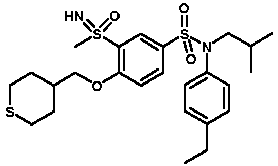 | N-(4-éthylphényl)-N-isobutyl-3-(S-méthyl-sulfonimidoyl)-4-(pyridin-4-ylmethoxy)-benzènesulfonamide | A | ND |
| | Composé 37 | | |
| 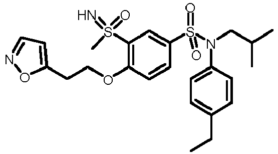 | N-(4-éthylphényl)-N-isobutyl-4-(2-(isoxazol-5-yl)éthoxy)-3-(S-methyl-sulfonimidoyl)benzenesulfo namide | C | ND |
| | Composé 38 | | |
| 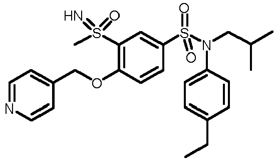 | N-(4-éthylphényl)-N-isobutyl-3-(S-méthyl-sulfonimidoyl)-4-(pyridin-4-ylméthoxy)-benzenesulfonamide | C | ND |
| | Composé 39 | | |
| 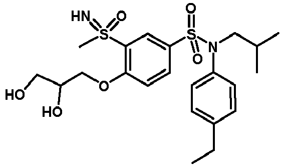 | 4-(2,3-dihydroxypropoxy)-N-(4-éthylphényl)-N-isobutyl-3-(S-methyl-sulfonimidoyl)benzènesulfo namide | C | ND |
| | Composé 40 | | |
| 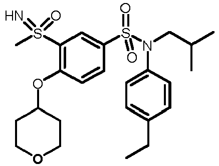 | N-(4-éthylphényl)-N-isobutyl-3-(S-méthylsulfonimidoyl)-4-((tétrahydro-2H-pyran-4-yl)oxy)benzenesulfonamide | C | ND |
| | Composé 42 | | |
| 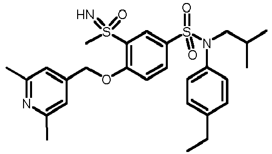 | 4-((2,6-dimethylpyridin-4-yl)méthoxy)-N-(4-éthylphenyl)-N-isobutyl-3-(S-méthylsulfonimidoyl)benzenesulfo namide | C | ND |
| | Composé 43 | | |
| 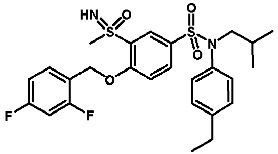 | 4-((2,4-difluorobenzyl)-oxy)-N-(4-ethylphenyl)-N-isobutyl-3-(S-méthyl-sulfonimidoyl)benzenesulfo namide | C | ND |
| | Composé 45 | | |
| 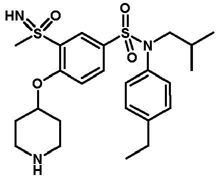 | N-(4-éthyl phényl)-N-isobutyl-3-(S-méthyl-sulfonimidoyl)-4-(piperidin-4-ylméthoxy)-benzènesulfonamide N-(4-éthylphényl)-N-isobutyl-3-(S-méthy-lsulfonimidoyl)-4-(piperidin-4-ylméthoxy)-benzènesulfonamide | C | ND |
| | Composé 46 | | |
| 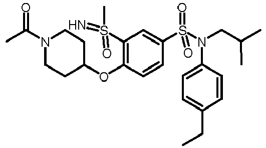 | 4-((1-acétylpiperidin-4-yl)oxy)-N-(4-éthylphényl)-N-isobutyl-3-(S-méthylsulfonimidoyl)benzène sulfonamide | C | ND |
| | Composé 47 | | |
| 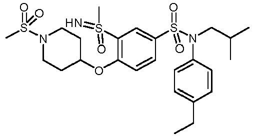 | N-(4-éthylphényl)-N-isobutyl-3-(S-méthylsulfonimidoyl)-4-((1-(méthylsulfonyl)-pipéridin-4-yl)oxy)benzènesulfonamide | C | ND |
| | Composé 48 | | |
| 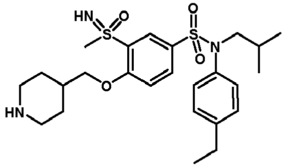 | N-(4-éthylphényl)-N-isobutyl-3-(S-méthyl-sulfonimidoyl)-4-(pipéridin-4-ylméthoxy)-benzenesulfonamide | C | ND |
| | Composé 49 | | |
| 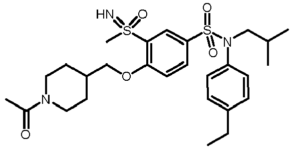 | 4-((1-acétylpiperid in-4-yl)méthoxy)-N-(4-ethylphényl)-N-isobutyl-3-(S-méthyl-sulfonimidoyl)-benzenesulfonamide | C | ND |
| | Composé 50 | | |
| 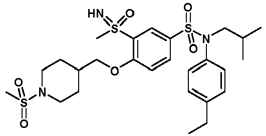 | N-(4-éthylphényl)-N-isobutyl-3-(S-méthylsulfonimidoyl)-4-((1-(methylsulfonyl-pipéridin-4-yl)methoxy)benzene-sulfonamide | C | ND |
| | Composé 51 | | |
| 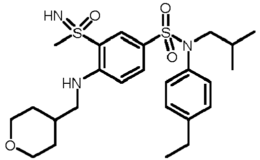 | N-(4-éthylphényl)-N-isobutyl-3-(S-methylsulfonimidoyl)-4-[(tetrahydropyran-4-ylmethyl)-amino]-benzenesulfonamide | A | A |
| | Composé 52 | | |
| 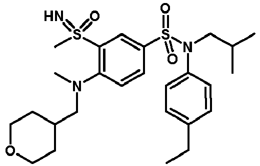 | N-(4-éthylphényl)-N-isobutyl-4-(méthyl-((tétrahydro-2H-pyran-4-yl)méthyl)amino)-3-(S-méthylsulfonimidoyl)-benzenesulfonamide | A | A |
| | Composé 53 | | |
| 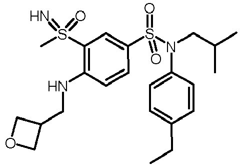 | N-(4-éthylphényl)-N-isobutyl-3-(S-methylsulfonimidoyl)-4-((oxetan-3-ylméthyl)amino)-benzenesulfonamide | B | ND |
| | Composé 54 | | |
| 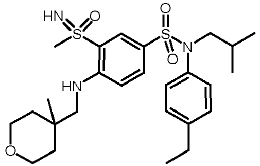 | N-(4-éthylphényl)-N-isobutyl-3-(S-méthylsulfonimidoyl)-4-(((4-methyltetrahydro-2H-pyran-4-yl)methyl)amino)-benzenesulfonamide | A | A |
| | Composé 55 | | |
| 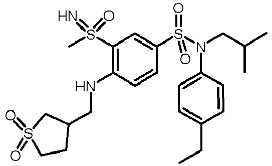 | 4-(((1,1-dioxidotétrahydro-thiophen-3-yl)-methyl)-amino)-N-(4-éthylphényl)-N-isobutyl-3-(S-méthylsulfonimidoyl)benzenesulfonamide | B | ND |
| | Composé 56 | | |
| 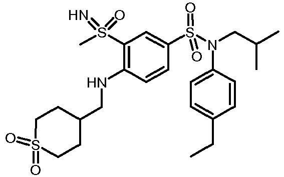 | 4-(((1,1-dioxidotetrahydro-2H-thiopyran-4-yl)-méthyl)amino)-N-(4-éthylphényl)-N-isobutyl-3-(S-methylsulfonimidoyl)-benzenesulfonamide | C | ND |
| | Composé 57 | | |
| 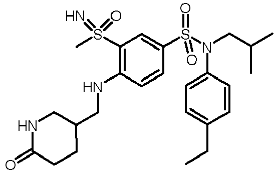 | N-(4-éthylphenyl)-N-isobutyl-3-(S-méthylsulfonimidoyl)-4-(((6-oxopiperidin-3-yl)-méthyl)-amino)benzenesulfonamide | C | ND |
| | Composé 58 | | |
| 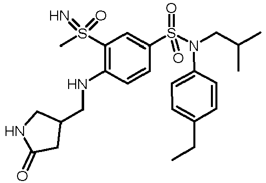 | N-(4-éthylphényl)-N-isobutyl-3-(S-méthylsulfonimidoyl)-4-(((5-oxopyrrolidin-3-yl)-methyl)amino)benzenesulfonamide | B | B |
| | Composé 59 | | |
| 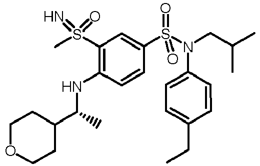 | N-(4-éthyl phényl)-N-isobutyl-3-(S-méthylsulfonimidoyl)-4-(((R)-1-(tétrahydro-2 H-pyran-4-yl)éthyl)amino)benzenesulf onamide | B | ND |
| | Composé 60 | | |
| 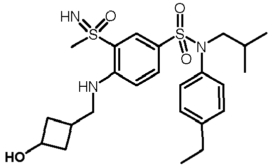 | N-(4-éthylphényl)-4-(((3-hydroxycyclobutyl)méthyl)a mino)-N-isobutyl-3-(S-méthyl-sulfonimidoyl)-benzenesulfonamide | A | A |
| | Composé 61 | | |
| 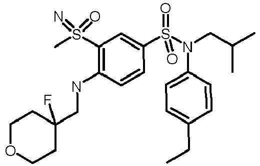 | N-(4-éthylphényl)-4-(((4-fluorotétrahydro-2H-pyran-4-yl)méthyl)amino)- N-isobutyl-3-(S-méthylsulfonimidoyl) benze nesulfonamide | A | A |
| | Composé 62 | | |
| 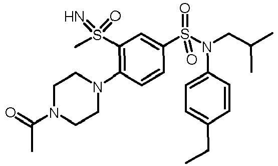 | :4-(4-acetylpiperazin-1-yl)-N-(4-ethylphenyl)-N-isobutyl-3-(S-methylsulfonimidoyl)-benzenesulfonamide | B | C |
| | Composé 63 | | |
| 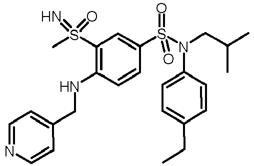 | N-(4-ethylphenyl)-N-isobutyl-3-(S-methylsulfonimidoyl)-4-((pyridin-4-ylmethyl)amino)-benzenesulfonamide | A | A |
| | Composé 64 | | |
| 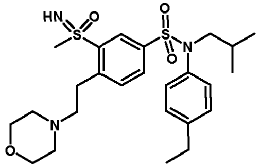 | N-(4-ethylphenyl)-N-isobutyl-3-(S-methylsulfonimidoyl)-4-(2-morpholinoethyl)-benzenesulfonamide | B | B |
| | Composé 65 | | |
| 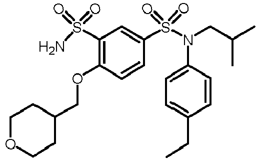 | 4-(tétrahyd ro-pyran-4-ylmethoxy)-benzene-1,3-disulfonic acid 3-amide 1-[(4-ethyl-phenyl)-isobutyl-amide] | A | A |
| | Composé 66 | | |
| 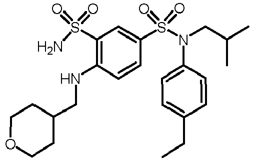 | Acide 4-[(tétrahydro-pyran-4-ylmethyl)-amino]-benzene-1,3-disulfonique 3-amide 1-[(4-éthyl-phényl)-isobutyl-amide] | B | ND |
| | Composé 67 | | |
| 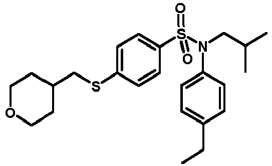 | N-(4-éthyl-phényl)-N-isobutyl-4-(tétrahydropyran-4-ylmethylsulfanyl)-benzenesulfonamide | B | B |
| | Composé 68 | | |
| 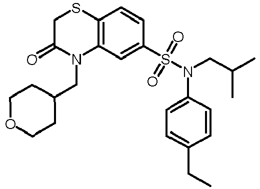 | Acide 3-oxo-4-(tetrahydropyran-4-ylmethyl)-3,4-dihydro-2H-benzo-[1,4]thiazine-7-sulfonique (4-éthyl-phényl)-isobutyl-amide | C | ND |
| | Composé 70 | | |
| 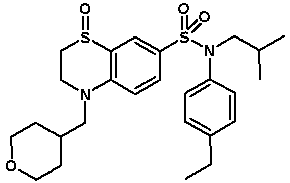 | Acide 1-oxo-4-(tétrahydropyran-4-ylmethyl)-1,2,3,4-tetrahydro-1λ ⁴-benzo-[1,4]-thiazine-7-sulfonique (4-éthyl-phényl)-isobutyl-amide | C | ND |
| | Composé 71 | | |
| 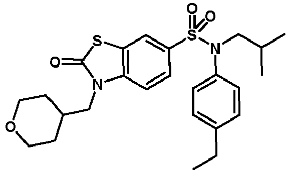 | Acide 3-Oxo-4-(tetrahydropyran-4-ylmethyl)-3,4-dihydro-2H-benzo-[1,4]-thiazine-7-sulfonique (4-éthyl-phényl)-isobutyl-amide | B | ND |
| | Composé 72 | | |
| 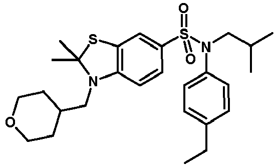 | Acide 2,2-Diméthyl-3-(tetrahyd ro-pyran-4-ylmethyl)-2,3-dihydrobenzothiazole-6-sulfonique (4-éthyl-phényl)-isobutyl-amide | C | ND |
| | Composé 73 | | |
| 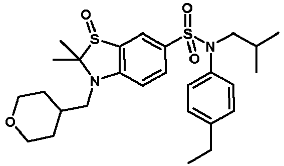 | Acide 2,2-Dimethyl-1-oxo-3-(tetrahydro-pyran-4-ylmethyl)-2,3-dihydro-1H-1 λ⁴-benzothiazole-6-sulfonique(4-éthyl-phényl)-isobutyl-amide | B | ND |
| | Composé 74 | | |
| 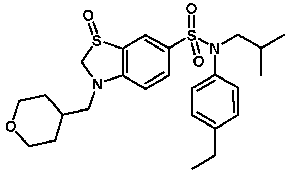 | Acide 1-oxo-3-(tetrahydropyran-4-ylmethyl)-2,3-dihydro-1H-1λ⁴-benzothiazole-6-sulfonique (4-éthyl-phényl)-isobutyl-amide | C | ND |
| | Composé 75 | | |
| 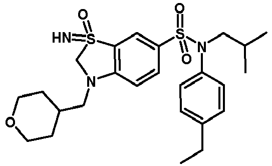 | Acide 1-lmino-1-oxo-3-(tetrahyd ro-pyran-4-ylmethyl)-2,3-dihydro-1H-1 λ⁶-benzothiazole-6-sulfonique(4-éthyl-phényl)-isobutyl-amide | C | ND |
| | Composé 76 | | |
| 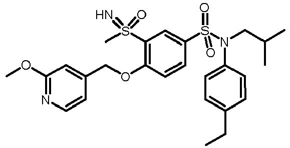 | N-(4-éthylphényl)-N-isobutyl-4-(((2-méthoxy-pyridin-4-yl)methoxy)- 3-(S-méthylsulfonimidoyl)-benzenesulfonamide | C | ND |
| | Composé 77 | | |
| 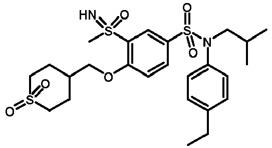 | N-(4-éthylphényl)-N-isobutyl-4-(((2-méthoxypyridin-4-yl)méthoxy)-3-(S-méthylsulfonimidoyl)-benzenesulfonamide | C | ND |
| | Composé 78 | | |
| 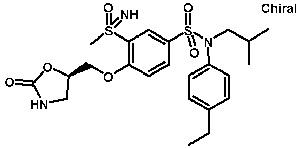 | N-(4-éthylphényl)-N-isobutyl-3-(S-méthylsulfonimidoyl)-4-(((R)-2-oxooxazolidin-5-yl)-méthoxy)benzenesulfonami de | C | ND |
| | Composé 79 | | |
| 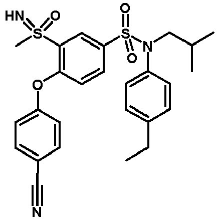 | 4-(4-cyanophénoxy)-N-(4-éthylphényl)-N-isobutyl-3-(S-méthylsulfonimidoyl)-benzenesulfonamide | C | ND |
| | Composé 80 | | |
| 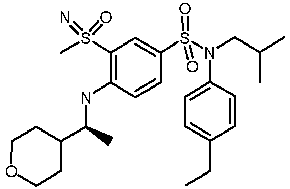 | N-(4-éthylphényl)-N-isobutyl-3-(S-méthylsulfonimidoyl)-4-(((S)-1-(tétrahydro-2H-pyran-4-yl)éthyl)amino)benzenes ulfonamide | B | ND |
| | Composé 81 | | |
| 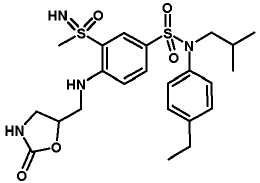 | N-(4-éthylphényl)-N-isobutyl-3-(S-methylsulfonimidoyl)-4-(((2-oxooxazolidin-5-yl)méthyl)amino)benzenesu lfonamide | B | ND |
| | Composé 82 | | |
| 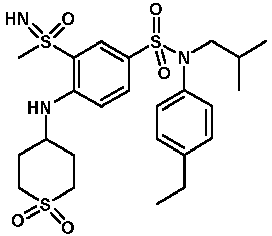 | 4-((1,1-dioxidotétrahydro-2H-thiopyran-4-yl)-amino)-N-(4-éthylphényl)-N-isobutyl-3-(S-méthylsulfonimidoyl)benze nesulfonamide | C | ND |
| | Composé 83 | | |
| 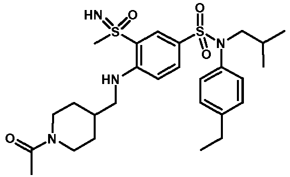 | 4-(((1-acétylpiperidin-4-yl)methyl)amino)-N-(4-éthylphényl)-N-isobutyl-3-(S-méthylsulfonimidoyl)benzenesulfona mide | C | ND |
| | Composé 84 | | |
| 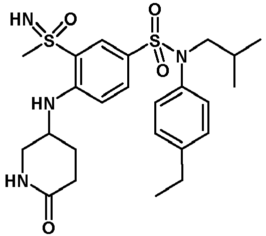 | N-(4-éthylphényl)-N-isobutyl-3-(S-méthylsulfonimidoyl)-4-((6-oxopiperidin-3 -yl)-amino)benzenesulfonam ide | C | ND |
| | Composé 85 | | |
| 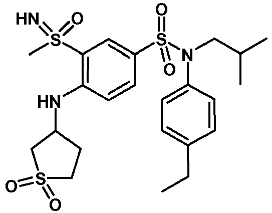 | 4-((1,1-dioxidotetrahydrothioph en-3-yl-)amino)-N-(4-éthylphényl)- N-isobutyl-3-(S-méthylsulfonimidoyl)be nzenesulfonamide | B | ND |
| | Composé 86 | | |
| 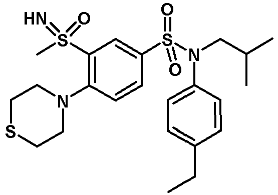 | N-(4-éthylphényl)-N-isobutyl-3-(S-méthylsulfonimidoyl)-4-thiomorpholinobenzene-sulfonamide | A | A |
| | Composé 87 | | |
| 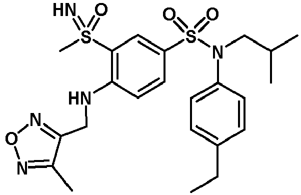 | N-(4-éthylphényl)-N-isobutyl-4-(((4-méthyl-1,2,5-oxadiazol-3-yl)méthyl)amino)-3-(S-méthylsulfonimidoyl)benze nesulfonamide | C | ND |
| | Composé 88 | | |
| 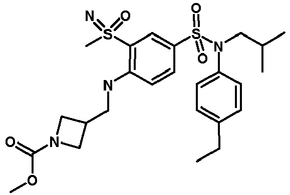 | 3-(((4-(N-(4-éthylphényl)-N-isobutyl-sulfamoyl)-2-(S-méthylsulfonimidoyl)-phényl)amino)méthyl)azeti dine-1-carboxylate de méthyle | C | ND |
| | Composé 89 | | |
| 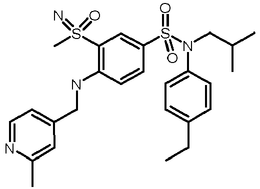 | N-(4-éthylphényl)-N-isobutyl-4-((((2-méthylpyridin-4-yl)méthyl)amino)- 3-(S-méthylsulfonimidoyl)benzenesulfo namide | C | ND |
| | Composé 90 | | |
| 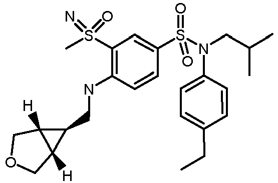 | 4-((((1R,5S,6s)-3-oxabicyclo[3.1.0 ]hexan-6-yl)méthyl)amino)-N-(4-éthylphényl)-N-isobutyl-3-(S-methylsulfonimidoyl)benze ne-sulfonamide | A | A |
| | Composé 91 | | |
| 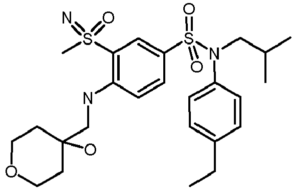 | N-(4-éthylphényl)-4-(((4-hydroxytétrahydro-2H-pyran-4-yl)méthyl)amino)-N-isobutyl-3-(S-méthylsulfonimidoyl)benze nesulfonamide | B | B |
| | Composé 92 | | |
| 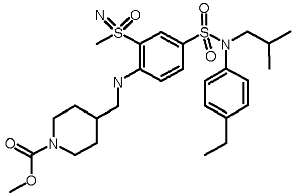 | 4-(((4-(N-(4-éthylphényl)-N-isobutyl-sulfamoyl)-2-(S-méthylsulfonimidoyl)pheny 1)-amino)méthyl)piperidine-1 - carboxylate de méthyle | C | ND |
| | Composé 93 | | |
| 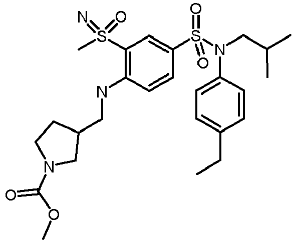 | 3-(((4-(N-(4-éthylphényl)-N-isobutyl-sulfamoyl)-2-(S-méthylsulfonimidoyl)phény 1)-amino)méthyl)pyrrolidine-1 -carboxylate de méthyle | C | ND |
| | Composé 94 | | |
| 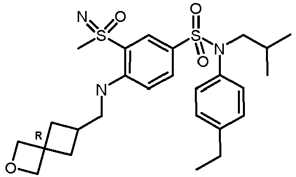 | 4-(((2-oxaspiro[3.3]heptan-6-yl)méthyl)amino)-N-(4-éthylphényl)-N-isobutyl-3-(S-méthylsulfonimidoyl)benzenesulfo namide | B | B |
| | Composé 95 | | |
| 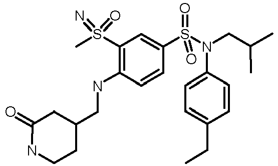 | 4-N-(4-éthylphényl)-N-isobutyl-3-(S-méthylsulfonimidoyl)-4-(((2-oxopiperidin-4-yl)-méthyl)amino)benzenesulfo namide | C | ND |
| | Composé 96 | | |
| 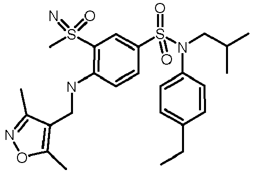 | 4-(((3,5-dimethylisoxazol-4-yl)methyl)amino)-N-(4-ethylphenyl)-N-isobutyl-3-(S-methylsulfonimidoyl)benzenesulfo namide | B | ND |
| | Composé 97 | | |
| 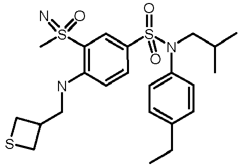 | N-(4-éthylphényl)-N-isobutyl-3-(S-méthysulfonimidoyl)-4-((thietan-3-ylmethyl)amino)-benzenesulfonamide | A | A |
| | Composé 99 | | |
| 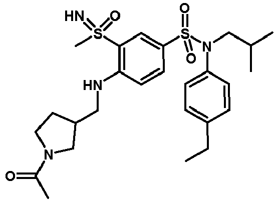 | 4-(((1-acétylpyrrolidin-3-yl)méthyl)amino)-N-(4-éthylphényl)-N-isobutyl-3-(S-méthylsulfonimidoyl)benzenesulfo namide | C | ND |
| | Composé 100 | | |
| 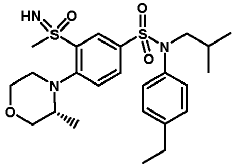 | N-(4-éthylphényl)-N-isobutyl-4-(((R)-3-méthylmorpholino)-3-(S-méthylsulfonimidoyl)benzenesulfo namide | C | C |
| | Composé 103 | | |
| 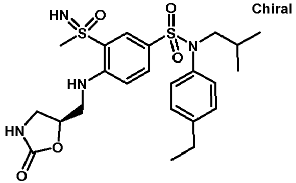 | N-(4-éthylphényl)-N-isobutyl-3-(S-methylsulfonimidoyl)-4-((((R)- 2-oxooxazolidin-5-yl)méthyl)amino)benzenesu lfonamide | C | ND |
| | Composé 105 | | |
| 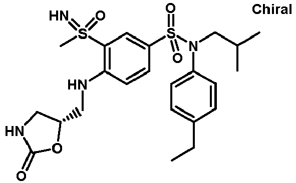 | N-(4-éthylphényl)-N-isobutyl-3-(S-méthylsulfonimidoyl)-4-((((S)-2-oxooxazolidin-5-yl)méthyl)amino)benzenesu lfonamide | C | ND |
| | Composé 106 | | |
| 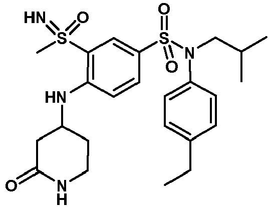 | N-(4-éthylphényl)-N-isobutyl-3-(S-méthylsulfonimidoyl)-4-((2-oxopiperidin-4-yl)amino)benzenesulfonami de | C | ND |
| | Composé 107 | | |
| 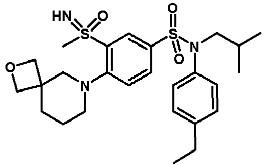 | N-(4-éthylphényl)-N-isobutyl-3-(S-méthylsulfonimidoyl)-4-(2-oxa-6-azaspiro[3.5]nonan-6-yl)benzenesulfonamide | C | ND |
| | Composé 109 | | |
| 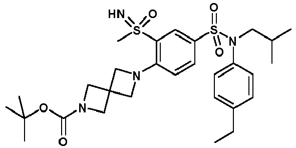 | tert-butyl 6-(4-(N-(4-ethylphenyl)-N-isobutylsulfamoyl)-2-(S-methylsulfonimidoyl)-phenyl)-2,6-diazaspiro[3.3]heptane-2-carboxylate | C | ND |
| | Composé 110 | | |
| 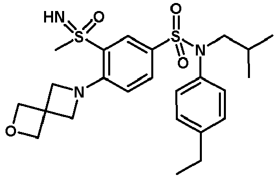 | N-(4-ethylphenyl)-N-isobutyl-3-(S-methylsulfonimidoyl)-4-(2-oxa-6-azaspiro-[3.3]heptan-6-yl)benzenesulfonamide | A | ND |
| | Composé 111 | | |
| 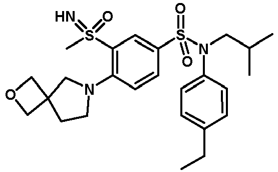 | N-(4-ethylphenyl)-N-isobutyl-3-(S-methylsulfonimidoyl)-4-(2-oxa-6-azaspiro[3.4]octan-6-yl)benzenesulfonamide | B | A |
| | Composé 112 | | |
| 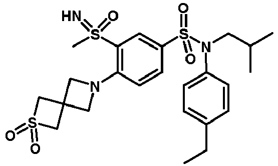 | 4-(2,2-dioxido-2-thia-6-azaspiro[3.3 ]heptan-6-yl)-N-(4-ethylphenyl)-N-isobutyl-3-(S-methylsulfonimidoyl)benzenesulfo namide | B | B |
| | Composé 113 | | |
| 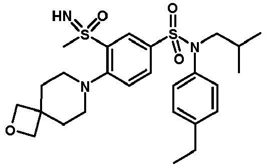 | N-(4-ethylphenyl)-N-isobutyl-3-(S-methylsulfonimidoyl)-4-(2-oxa- 7-azaspiro[3.5]nonan-7-yl)benzenesulfonamide | B | B |
| | Composé 114 | | |
| 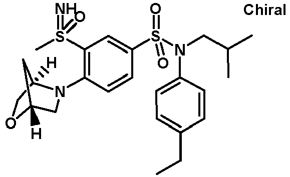 | 4-((1R,4R)-2-oxa-5-azabicyclo[2.2.1]heptan-5-yl)-N-(4-ethylphenyl)-N-isobutyl-3-(S-methylsulfonimidoyl)benzenesulfo namide | B | ND |
| | Composé 115 | | |
| 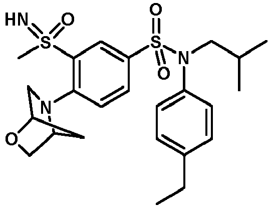 | 4-((1R,4R)-2-oxa-5-azabicyclo[2.2.1]heptan-5-yl)-N-(4-ethylphenyl)-N-isobutyl-3-(S-methylsulfonimidoyl)benzenesulfo namide | B | ND |
| | Composé 117 | | |
| 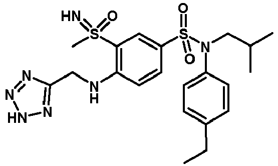 | 4-(((2H-tetrazol-5-yl)methyl)amino)-N-(4-ethylphenyl)-N-isobutyl-3-(S-methylsulfonimidoyl)benzenesulfonamid e | C | ND |
| | Composé 118 | | |
| 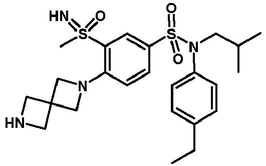 | -(4-éthylphényl)-N-isobutyl-3-(S-méthylsulfonimidoyl)-4-(2,6-diazaspiro[3.3]heptan-2- yl)benzenesulfonamide | C | ND |
| | Composé 121 | | |
| 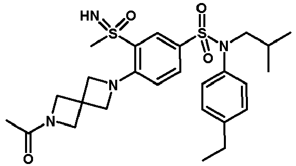 | 4-(6-acétyl-2,6-diazaspiro[3.3]heptan-2-yl)-N-(4-éthylphényl)-N-isobutyl-3-(S-méthylsulfonimidoyl)benze nesulfonamide | C | ND |
| | Composé 122 | | |
| 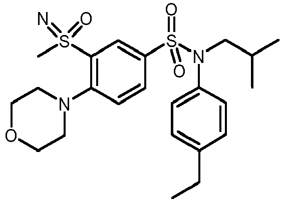 | N-(4-éthylphényl)-N-isobutyl- 3-(S-methylsulfonimidoyl)-4-morpholinobenzenesulfonamide | A | A |
| | Composé 123 | | |
| 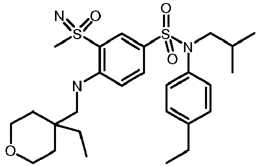 | N-(4-ethylphenyl)-4-(((4-ethyltetrahydro-2H-pyran-4-yl)methyl)amino)- N-isobutyl-3-(S-methylsulfonimidoyl)benze nesulfonamide | A | A |
| | Composé 124 | | |
| 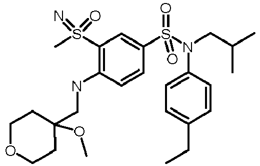 | N-(4-éthylphényl)-N-isobutyl-4-((((4-méthoxytétrahydro-2H-pyran-4-yl)méthyl)amino)-3-(S-méthylsulfonimidoyl)benze nesulfonamide | B | C |
| | Composé 125 | | |
| 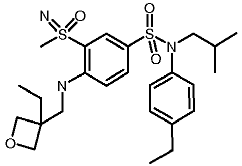 | 4-(((3-ethyloxetan-3-yl)methyl)amino)-N-(4-ethylphenyl)-N-isobutyl-3-(S-methylsulfonimidoyl)benzenesulfonamid e | C | ND |
| | Composé 126 | | |
| 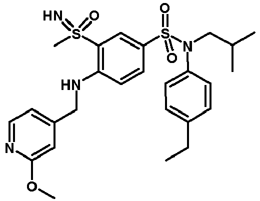 | N-(4-ethylphenyl)-N-isobutyl-4-((((2-methoxypyridin-4-yl)methyl)amino)- 3-(S-methylsulfonimidoyl)benzenesulfo namide | B | B |
| | Composé 127 | | |
| 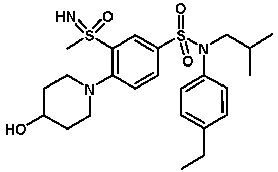 | N-(4-ethylphenyl)-4-(4-hydroxypiperidin-1-yl)-N-isobutyl-3-(S-methylsulfonimidoyl)benze ne-sulfonamide | C | C |
| | Composé 128 | | |
| 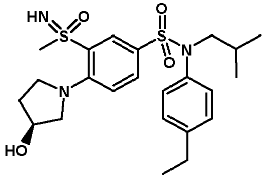 | -(4-éthylphényl)-4-((S)-3-hydroxypyrrolidin-1-yl)-N-isobutyl-3-(S-méthylsulfonimidoyl)-benzenesulfonamide | C | C |
| | Composé 129 | | |
| 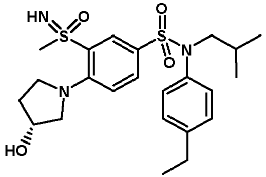 | N-(4-éthylphényl)-4-((R)-3-hydroxypyrrolidin-1-yl)-N-isobutyl-3-(S-methylsulfonimidoyl)-benzenesulfonamide | C | C |
| | Composé 130 | | |
| 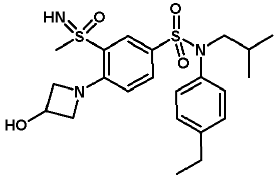 | N-(4-éthylphényl)-4-(3-hydroxyazetidin-1-yl)-N-isobutyl-3-(S-méthylsulfonimidoyl)-benzenesulfonamide | C | C |
| | Composé 131 | | |
| 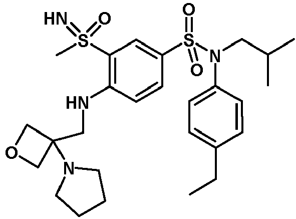 | N-(4-éthylphényl)-N-isobutyl-3-(S-méthylsulfonimidoyl)-4-(((3-(pyrrolidin-1-yl)oxetan-3-yl)méthyl)amino)benzenesu lfonamide | C | ND |
| | Composé 132 | | |
| 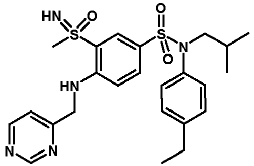 | N-(4-éthylphényl)-N-isobutyl-3-(S-méthylsulfonimidoyl)-4-((pyrimidin-4-ylméthyl)-amino)benzenesulfonamide | A | A |
| | Composé 133 | | |
| 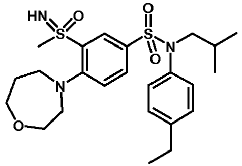 | N-(4-éthylphényl)-N-isobutyl-3-(S-méthylsulfonimidoyl)-4-(1,4-oxazepan-4-yl)benzenesulfonamide | B | B |
| | Composé 137 | | |
| 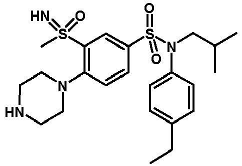 | N-(4-éthylphényl)-N-isobutyl-3-(S-méthylsulfonimidoyl)-4-(pipérazin-1-yl)benzenesulfonamide | C | ND |
| | Composé 140 | | |
| 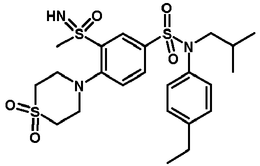 | N-(4-ethylphenyl)-4-(((3-hydroxycyclobutyl)methyl) amino)-N-isobutyl-3-(S-methylsulfonimidoyl)benzenesulfo namide | C | ND |
| | Composé 141 | | |
| 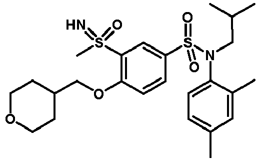 | N-(2,4-diméthylphényl)-N-isobutyl-3-(S-méthylsulfonimidoyl)-4-((tétrahydro-2H-pyran-4-yl)méthoxy)benzenesulfona mide | A | A |
| | Composé 142 | | |
| 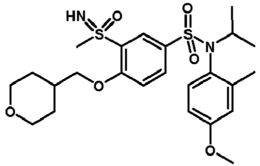 | N-isopropyl-N-(4-méthoxy-2-méthylphényl)-3-(S-méthylsulfonimidoyl)-4-((tétrahydro-2H-pyran-4-yl)méthoxy)benzenesulfona mide | B | B |
| | Composé 143 | | |

| | | | |
|---|---|---|---|
| ND : non déterminé ; A: IC50 <100 nM.; B : IC50 = 100nM-1µM; C : IC50 > 1µM | | | |

Dans les tableaux décrits ci-avant, les concentrations inhibitrices médianes IC₅₀ pour les composés appartenant à la formule (I) selon l'invention ont été données selon les modèles suivants :

### Transactivation GAL4-RORγ

Le modèle de transactivation RORy a été développé à partir de la lignée HG5LN qui est une lignée HeLa exprimant stablement un gène reporter luciférase contrôlé par un pentamère du domaine de reconnaissance GAL4 de levure et d'un promoteur β-globine. La lignée HG5LN a été transfectée stablement par le DNA-binding domain (DBD) (ou domaine de liaison à l'ADN) de GAL4 fusionné au ligand-binding domain (LBD) ROR gamma. Les molécules inhibant l'activité constitutive ROR gamma réduisent l'expression de la luciférase induisant ainsi une baisse de la luminescence émise.

Les cellules sont ensemencées en plaques 384 puits (5 000 cellules dans 45µL/puits de milieu de culture contenant 10% de sérum de veau foetal) et incubées pendant 4 heures à 37°C, 5% CO₂. 5µL des molécules à tester (composés décrits dans les tableaux décrits ci-avant) sont ensuite ajoutés à chaque puits et les plaques sont incubées pendant 18 heures à une température de 37°C sous 5% de CO₂. 20µL du substrat de la luciférase (Promega) sont ajoutés à chaque puits et la luminescence émise est lue par un lecteur de microplaques.

Les unités de luminescence ('RLU') sont normalisées par des contrôles positifs ('POS' contenant une concentration saturante de benzenesulfonamide, N-(2,2,2-trifluoroéthyl)-*N*-[4-[2,2,2-trifluoro-1-hydroxy-1-(trifluorométhyl)éthyl]phényl]) et des contrôles négatifs ('NEG' contenant du DMSO) : % inhibition=((RLU-NEG)*100)/(POS-NEG). Les IC50 sont calculées à partir d'un modèle logistique à 4 paramètres à l'aide du logiciel XLFit (IDBS).

### Sécrétion IL-17A

Ce modèle permet de mesurer l'effet d'inhibiteurs sur la sécrétion d'IL-17A par des cellules CD4+. Les cellules sont des CD4+ congelées (STEMCELL, # 70026), isolées de sang humain périphérique et activées par les anticorps anti-CD3 et anti-CD28. La quantité d'IL-17A sécrété est mesurée par la technologie TR-FRET (kit HTRF® Human Interleukin 17A (Cisbio, #64H17PEC)).

Les cellules sont décongelées rapidement, resuspendues dans leur milieu de culture (RPMI 10% SVF inactivé) supplémenté avec des anticorps anti-CD28 solubles et ensemencées (100 000 cellules/puits) dans des plaques 96 puits préalablement coatées avec des anticorps anti-CD3. Les cellules sont ensuite traitées par les gammes des inhibiteurs à tester (de 1000nM à 0.05nM, 0.1% DMSO). Après 4 jours d'incubation, le signal HTRF est mesuré à l'aide d'un lecteur de microplaque (λexcitation=337nm, λémission=620/665nm). Les ratios obtenus (665/620) sont normalisés par rapport au contrôle positif (cellules activées par anti-CD3 et anti-CD28, 0.1% DMSO). Les IC₅₀ sont calculées à partir d'un modèle logistique à 4 paramètres à l'aide du logiciel XLFit (IDBS).

Dans le tableau ci-après, les concentrations inhibitrices médianes IC₅₀ pour les composés appartenant à la formule (I) selon l'invention ont été données conformément au test hERG.

Le test hERG permet d'étudier un gène qui code pour une protéine nécessaire au fonctionnement des canaux potassium du tissu cardiaque. La méthode du patch clamp sur les cellules CHO-K1 (cellules transfectées avec le gène hERG et qui présente une activité des ions K+ sur les membranes) est utilisée pour prédire in vitro le blocage de hERG (human Ether-a-go-go Related).

La solution extracellulaire (contrôle) est appliquée en premier. Les cellules (cellules ovariennes de Hamster Chinois exprimant le gène Human Ether-a-go-go Related Gene) sont stabilisées avec la solution extracellulaire pendant 5 minutes. Les cellules sont incubées pendant 5 minutes avec les molécules de la plus faible à la plus forte concentration à 0.6 % DMSO final.

La méthode de calcul de l'inhibition pour chaque concentration : % inhibition = 100 x (amplitude Tail current de la molécule incubée - amplitude tail current du véhicule contrôle). Le résultat est exprimé sous la forme d'un IC₅₀ en µM.

Les résultats sont donnés pour les composés suivants :

| Composés | | hERG IC50 |
|---|---|---|
| 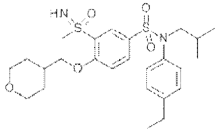 | N-(4-éthyl-phényl)-N-isobutyl-3-méthanesulfoximin-4-(tétrahydropyran-4-ylméthoxy)-benzenesulfonamide | >30 |
| | Composé 26 | |
| 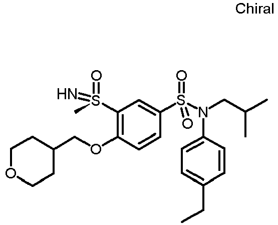 | composé 7, énantiomère A du composé 26 | > 30 |
| énantiomère A | | |
| 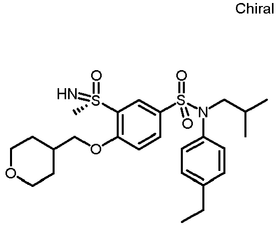 | composé 8, énantiomère B du composé 26 | >30 |
| énantiomère B | | |
| 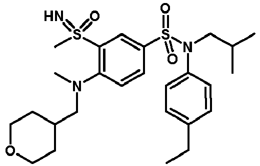 | N-(4-éthylphényl)-N-isobutyl-4-(méthyl-((tétrahydro-2H-pyran-4-yl)méthyl)amino)-3 - (S-méthylsulfonimidoyl)-benzenesulfonamide | 11,8 |
| | Composé 53 | |
| 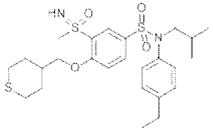 | N-(4-éthylphényl)-N-isobutyl-3-(S-méthyl-sulfonimidoyl)-4-(pyridin-4-ylmethoxy)-benzène-sulfonamide | 11,8 |
| | Composé 37 | |
| 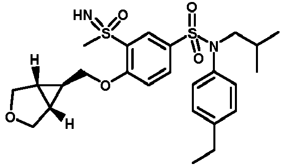 | 4-(((1R,5S,6r)-3-oxabicyclo-[3.1.0]hexan-6-yl)méthoxy)-N-(4-éthylphényl)-N-isobutyl-3-(S-méthylsulfonimidoyl)-benzene-sulfonamide | > 30 |
| | Composé 35 | |
| 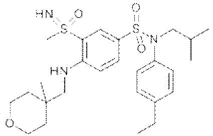 | N-(4-éthylphényl)-N-isobutyl-3-(S-méthyl-sulfonimidoyl)-4-(((4-methyltetrahydro-2H-pyran-4-yl)methyl)amino)-benzenesulfonamide | 14,1 |
| | Composé 55 | |
| 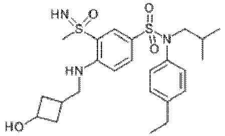 | N-(4-éthylphényl)-4-(((3-hydroxycyclobutyl)méthyl)am ino)-N-isobutyl-3-(S-méthylsulfonimidoyl)-benzenesulfonamide | 25,7 |
| | Composé 61 | |
| 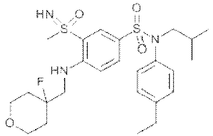 | N-(4-éthylphényl)-4-(((4-fluorotetrahydro-2H-pyran-4-yl)methyl)amino)-N-isobutyl-3-(S-methylsulfonimidoyl)benzenesulfo namide | 16,4 |
| | Composé 62 | |

Préférentiellement, les composés de formule (I) selon l'invention sont choisis parmi les composés suivants :

| Composés | |
|---|---|
| 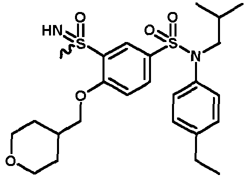 | énantiomère A du composé 7 |
| énantiomère A | |
| 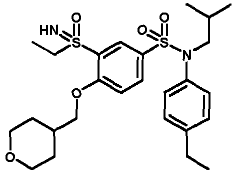 | N-(4-éthyl-phényl)-N-isobutyl-3-éthanesulfoximin-4-(tétrahydro-pyran-4-ylméthoxy)-benzénesulfonamide Composé 18 |
| | |
| 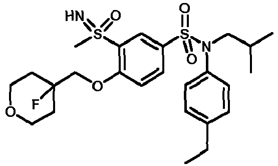 | N-(4-éthylphényl)-4-((4-fluorotétrahydro-2H-pyran-4-yl)méthoxy)-N-isobutyl-3-(S-méthylsulfonimidoyl)benzènesulfonamide |
| | Composé 30 |
| 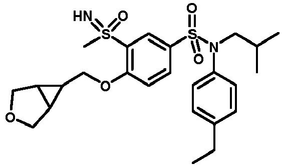 | 4-((3-oxabicyclo-[3.1.0]-hexan-6-yl)méthoxy)-N-(4-éthylphényl)-N-isobutyl-3-(S-méthylsulfonimidoyl)benzene-sulfonamide |
| | Composé 31 |
| 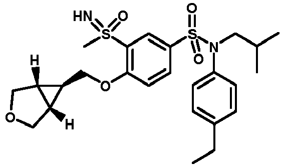 | 4-(((1R,5S,6r)-3-oxabicyclo-[3.1.0]hexan-6-yl)méthoxy)-N-(4-éthylphényl)-N-isobutyl-3-(S-méthylsulfonimidoyl)-benzenesulfonamide |
| | Composé 35 |
| 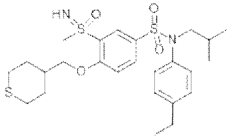 | N-(4-éthylphényl)-N-isobutyl- 3-(S-méthyl-sulfonimidoyl)-4-(pyridin-4-ylmethoxy)-benzène-sulfonamide |
| | Composé 37 |
| 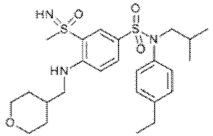 | N-(4-éthylphényl)-N-isobutyl-3-(S-methyl-sulfonimidoyl)-4-[(tetrahydropyran-4-ylmethyl)-amino]-benzenesulfonamide |
| | Composé 52 |
| 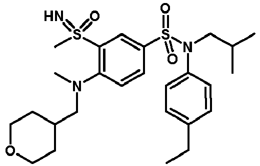 | N-(4-éthylphényl)-N-isobutyl-4-(méthyl-((tétrahydro-2H-pyran-4-yl)méthyl)amino)-3-(S-méthylsulfonimidoyl)-benzenesulfonamide |
| | Composé 53 |
| 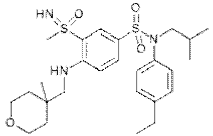 | N-(4-éthylphényl)-N-isobutyl-3-(S-méthyl-sulfonimidoyl)-4-(((4-methyltetrahydro-2H-pyran-4-yl)méthyl)amino)-benzenesulfonamide |
| | Composé 55 |
| 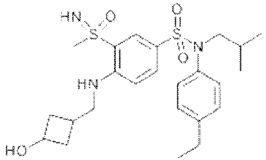 | N-(4-éthylphényl)-4-(((3-hydroxycyclobutyl)methyl)amino)-N-isobutyl-3-(S-methyl-sulfonimidoyl)-benzenesulfonamide |
| | Composé 61 |
| 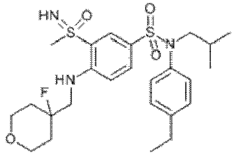 | N-(4-éthylphényl)-4-(((4-fluorotetrahydro-2H-pyran-4-yl)methyl)amino)-N-isobutyl-3-(S-methyl-sulfonimidoyl)benzene sulfonamide |
| | Composé 62 |
| 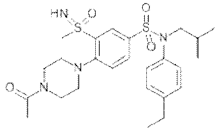 | :4-(4-acetylpiperazin-1-yl)-N-(4-ethylphenyl)-N-isobutyl-3-(S-methylsulfonimidoyl)-benzene-sulfonamide |
| | Composé 63 |
| 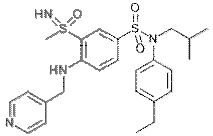 | N-(4-éthylphényl)-N-isobutyl-3-(S-méthyl-sulfonimidoyl)-4-((pyridin-4-ylméthyl)amino)-benzenesulfonamide |
| | Composé 64 |

Le ou les composés préférés selon l'invention présente(nt) l'avantage de disposer d'une forte activité biologique, en particulier une concentration inhibitrice médiane IC50 qui est inférieure à 100 nM conformément au test Transactivation GAL-4 RORγ tel que décrit précédemment.

De plus, le ou les composés préférés selon l'invention présente(nt) l'avantage d'avoir une toxicité faible.

L'invention concerne également le ou les composés tels que décrits précédemment en tant que médicament et cosmétique.

De préférence, l'invention concerne également le ou les composés tels que décrits précédemment en tant que médicament.

En effet, les composés selon l'invention présentent des propriétés pharmacologiques intéressantes étant donné que lesdits composés modulent, c'est-à-dire inhibent, l'activité du récepteur RORγt.

Ainsi ces propriétés rendent le ou les composés de formule (I) telle que décrite précédemment utilisables en tant que médicament dans le traitement des maladies médiées par le récepteur RORγt.

De préférence, le ou les composés selon l'invention sont utilisés dans le traitement des désordres inflammatoires et/ou des maladies auto-immunes médiées par le récepteur RORγt.

Plus préférentiellement, le ou les composés selon l'invention sont utilisés dans le traitement de l'acné, le psoriasis et/ou la dermatite atopique.

Selon un mode de réalisation, les composés (1) à (76) sont utilisés dans le traitement de l'acné, le psoriasis et/ou la dermatite atopique.

De préférence, les composés (7), (8), (18), (26), (30), (31), (35), (37), (52), (53), (55), (61), (62), (63) et (64) sont utilisés dans le traitement de l'acné, le psoriasis et/ou la dermatite atopique.

Selon un autre mode de réalisation, les composés sont utilisés pour le traitement cosmétique de la peau.

Comme indiqué ci-avant, la présente invention est aussi relative à une composition pharmaceutique comprenant, dans un milieu pharmaceutiquement acceptable, un ou plusieurs composés de formule (I) telle que définie précédemment, leurs sels d'addition pharmaceutiquement acceptables, leurs hydrates et/ou leurs solvates.

De préférence, la composition pharmaceutique comprend un ou plusieurs composés de formule (Ia) et/ou (Ib) telles que définies précédemment, leurs sels d'addition pharmaceutiquement acceptables, leurs hydrates et/ou leurs solvates.

Plus préférentiellement, la composition pharmaceutique comprend un ou plusieurs composés de formule (Ia) ou (Ib) choisis parmi les composés (1) à (143) définis précédemment.

Encore plus préférentiellement, la composition pharmaceutique comprend un ou plusieurs composés de formule (Ia) ou (Ib) choisis parmi les composés (7), (8), (18), et (26), (30), (31), (35), (37), (52), (53), (55), (61), (62), (63) et (64).

L'administration de la composition pharmaceutique selon l'invention peut être effectuée par voie orale ou topique.

De préférence, la composition pharmaceutique est conditionnée sous une forme convenant à une application par voie topique.

Par voie orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée.

Par voie topique, la composition pharmaceutique selon l'invention est plus particulièrement destinée au traitement de la peau et des muqueuses et peut se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de suspensions, de sticks, de shampoings, ou de bases lavantes. Elle peut également se présenter sous forme de suspensions de microsphères ou nanosphères ou de vésicules lipidiques ou polymériques ou de patches polymériques ou gélifiés permettant une libération contrôlée.

La composition pharmaceutique est utilisée pour le traitement des désordres inflammatoires et/ou les maladies auto-immunes médiées par le récepteur RORγt.

Plus préférentiellement, la composition pharmaceutique est utilisée dans le traitement de l'acné, le psoriasis ou la dermatite atopique.

L'invention concerne aussi un procédé de traitement des maladies médiées par le récepteur RORγt comprenant l'administration, notamment par voie topique ou orale, d'une quantité thérapeutiquement efficace de la composition pharmaceutique telle que définie ci-avant à un patient.

De préférence, la composition pharmaceutique est appliquée par voie topique.

De préférence, le ou les composés de formule (II) sont choisis parmi le ou les composés de formule (IIa) et (IIb) suivantes :

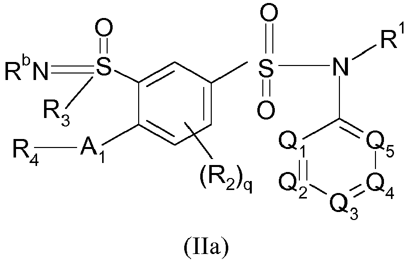

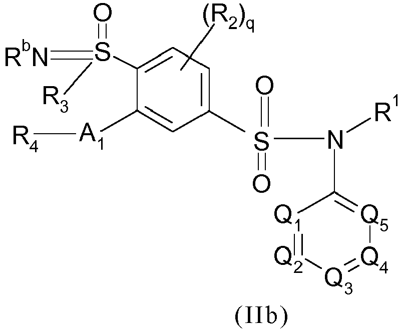

Formules (IIa) et (IIb) dans lesquels R¹, R₂, R'₂, R³, R⁴, R⁵, R'⁵, R⁶, R⁷, R^{7a}, R^{7b}, R₈, R₉, R₁₀, R¹¹, R¹², R^{a}, R^{b}, Z, Q₁, Q₂, Q₃, Q₄, Q₅, A₁, et les indices q, m, n, o et p ont les mêmes significations que dans la formule (II) précédemment décrite.

Préférentiellement, le ou les composés de formule (II) sont choisis parmi le ou les composés de formule (IIa).

L'invention concerne aussi le ou les composés de formules (II), de préférence de formules (IIa) et (IIb), en tant que médicament et cosmétique.

Préférentiellement, l'invention concerne le ou les composés de formule (IIa) en tant que médicament et cosmétique, notamment en tant que médicament.

En particulier, l'invention porte sur le ou les composés de formules (II), de préférence de formule (IIa), en tant que médicament dans le traitement des maladies médiées par le récepteur RORγt, de préférence le traitement des désordres inflammatoires et/ou des maladies auto-immunes médiées par le récepteur RORyt.

Plus préférentiellement, le ou les composés de formules (II) selon l'invention, de préférence de formule (IIa), sont utilisés dans le traitement de l'acné, le psoriasis et/ou la dermatite atopique.

Conformément à ce mode de réalisation, la présente invention est aussi relative à une composition pharmaceutique comprenant, dans un milieu pharmaceutiquement acceptable, un ou plusieurs composés de formule (II) telle que définie précédemment, leurs sels d'addition pharmaceutiquement acceptables, leurs hydrates et/ou leurs solvates.

La composition pharmaceutique est utilisée pour le traitement des désordres inflammatoires et/ou les maladies auto-immunes médiées par le récepteur RORγt, de préférence pour le traitement l'acné, le psoriasis ou la dermatite atopique.

L'invention concerne aussi un procédé de traitement des maladies médiées par le récepteur RORγt comprenant l'administration, notamment par voie topique ou orale, d'une quantité thérapeutiquement efficace de la composition pharmaceutique telle que définie ci-avant à un patient, en particulier par voie topique.

Conformément à un autre mode de réalisation, la présente invention a également pour objet un ou des composés de formule (III) ainsi que son ou leurs sels d'addition pharmaceutiquement acceptables, leurs hydrates et/ou leurs solvates :

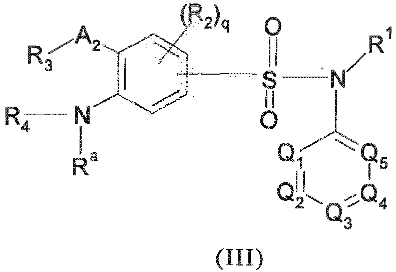

Formule (III) dans laquelle :
- R^{a} et R₄ forment ensemble avec l'atome d'azote auquel ils sont rattachés un groupe hétérocycloalkyle en C₂-C₁₀ comprenant éventuellement un à trois hétéroatomes choisis parmi un atome de soufre, un atome d'azote et un atome d'oxygène ; ledit groupe hétérocycloalkyle étant éventuellement substitué par au moins un radical R¹⁴,
- R¹⁴ représente un radical alkyle, linéaire ou ramifié, en C₁-C₃, un radical alcoxy, linéaire ou ramifié, en C₁-C₃, un atome d'halogène, un groupement hydroxy -OH, un groupement cyano -CN, un groupement -CONR¹⁵R¹⁶, un groupement -SO₂R¹⁵, un groupement -COR¹⁵ ou un groupement amino -NR¹⁵R¹⁶ ; R¹⁵ et R¹⁶, identiques ou différents, représentant un atome d'hydrogène ou un radical alkyle, linéaire ou ramifié, en C₁-C₃,
- R¹, R₂, R'₂, R³, R^{b}, Q₁, Q₂, Q₃, Q₄, Q₅, A₂ et l'indice q ont les mêmes significations que dans la formule (I) précédemment décrite.

Ainsi A₂ ne représente pas le groupe divalent -CH(OH)- et - C(=O)O-.

A₂ représente un groupe divalent -SO(=N-R^{b})-.

En particulier, R^{a} et R₄ forment ensemble avec l'atome d'azote auquel ils sont rattachés un groupe hétérocycloalkyle en C₂-C₁₀ monocyclique, bicyclique ou bicyclique spiro tel que défini précédemment.

De préférence, R^{a} et R₄ forment ensemble avec l'atome d'azote auquel ils sont rattachés un groupe hétérocycloalkyle en C₂-C₁₀ monocyclique ou bicyclique spiro, en particulier monocyclique.

De préférence, R^{a} et R₄ forment ensemble avec l'atome d'azote auquel ils sont rattachés un groupe hétérocycloalkyle en C₂-C₁₀, ledit groupe hétérocycloalkyle étant éventuellement substitué par au moins un radical R¹⁴ tel que défini précédemment.

De préférence, le groupe hétérocycloalkyle est éventuellement substitué par un, deux ou trois radicaux R¹⁴ tels que définis précédemment.

En particulier, le groupe hétérocycloalkyle est substitué par un radical R¹⁴.

De préférence, Q¹-Q² et Q⁴-Q⁵ correspondent à un groupement -CR² avec R² désignant un atome d'hydrogène et Q³ correspond à un groupement -CR'₂ avec R'₂ désignant un radical alkyle, linéaire ou ramifié, en C₁-C₅, de préférence en C₂.

De préférence, Q¹ et Q³, identiques ou différents, correspondent à un groupement -CR'₂ avec R'₂ désignant un atome d'hydrogène ou un radical alkyle, linéaire ou ramifié, en C₁-C₅, de préférence en C₂.

De préférence, l'indice q est égal à zéro.

Selon un cas particulier, A₂ représente un groupe divalent choisi parmi -S-, -SO-, SO₂-.

De préférence, le ou les composés de formule (III) sont choisis parmi le ou les composés de formule (IIIa) et (IIIb) suivantes :

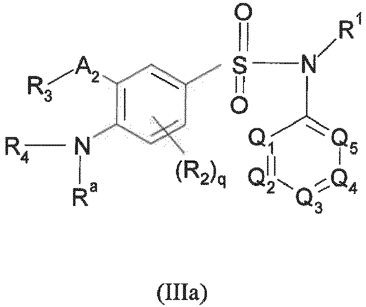

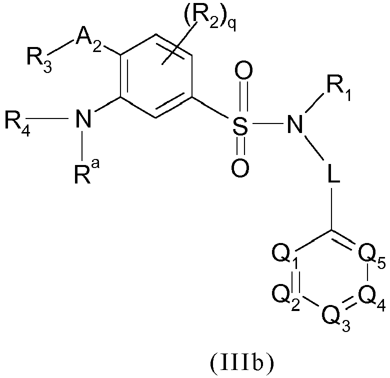

Formules (IIIa) et (IIIb) dans lesquels R¹, R₂, R'₂, R³, R₄, R^{a}, R^{b}, Q₁, Q₂, Q₃, Q₄, Q₅, A₂, et l'indice q ont les mêmes significations que dans la formule (III) précédemment décrite.

Préférentiellement, le ou les composés de formule (III) sont choisis parmi le ou les composés de formule (IIIa).

De préférence, l'invention concerne le ou les composés de formules (III), de préférence de formules (IIIa) et (IIIb) en tant que médicament et cosmétique.

Préférentiellement, l'invention concerne le ou les composés de formule (IIIa) en tant que médicament et cosmétique, notamment en tant que médicament.

En particulier, l'invention porte sur le ou les composés de formules (III), de préférence de formule (IIIa), en tant que médicament dans le traitement des maladies médiées par le récepteur RORyt, de préférence le traitement des désordres inflammatoires et/ou des maladies auto-immunes médiées par le récepteur RORyt.

Plus préférentiellement, le ou les composés de formules (III), de préférence de formule (IIIa), selon l'invention sont utilisés dans le traitement de l'acné, le psoriasis et/ou la dermatite atopique.

Conformément à ce mode de réalisation, la présente invention est aussi relative à une composition pharmaceutique comprenant, dans un milieu pharmaceutiquement acceptable, un ou plusieurs composés de formule (III) telle que définie précédemment, leurs sels d'addition pharmaceutiquement acceptables, leurs hydrates et/ou leurs solvates.

La composition pharmaceutique est utilisée pour le traitement des désordres inflammatoires et/ou les maladies auto-immunes médiées par le récepteur RORγt, de préférence pour le traitement l'acné, le psoriasis ou la dermatite atopique.

L'invention concerne aussi un procédé de traitement des maladies médiées par le récepteur RORγt comprenant l'administration, notamment par voie topique ou orale, d'une quantité thérapeutiquement efficace de la composition pharmaceutique telle que définie ci-avant à un patient, en particulier par voie topique.

Les exemples suivants servent à illustrer l'invention sans toutefois présenter un caractère limitatif.

Les exemples qui ne tombent pas sous la formule (II) telle que définie dans la revendication 1 ne font pas partie de l'invention.

### Exemples :

La méthode standard LCMS pour l'analyse des produits est la suivante : colonne standard BEH C₁₈ (150*2.1mm, 1.8 µm) solvant eau/acétonitrile 0.1% acide formique.

Les purifications par HPLC préparative ont été réalisé sur colonne C₁₈, avec pour éluant : 85% d'acétonitrile dans eau/0.1% d'acide formique.

L'appareil utilisé pour la chromatographie est un appareil pic-solution 10-20, colonne Chiraltechnologie Ic *25x5 micron*, (phase éluante : CO₂ supercritique/méthanol, débit 4ml/minutes).

La méthode standard LCMS pour l'analyse des produits est la suivante : colonne BEH C18 150*2.1mm 1.8µm solvant eau/acétonitrile 0.1% acide formique.

Les purifications par HPLC préparative ont été réalisées sur colonne C18, avec pour éluant : 85% d'acétonitrile dans eau/0.1% d'acide formique.

### Partie I : Synthèse des sulfonamides soufrés à partir du schéma réactionnel 1

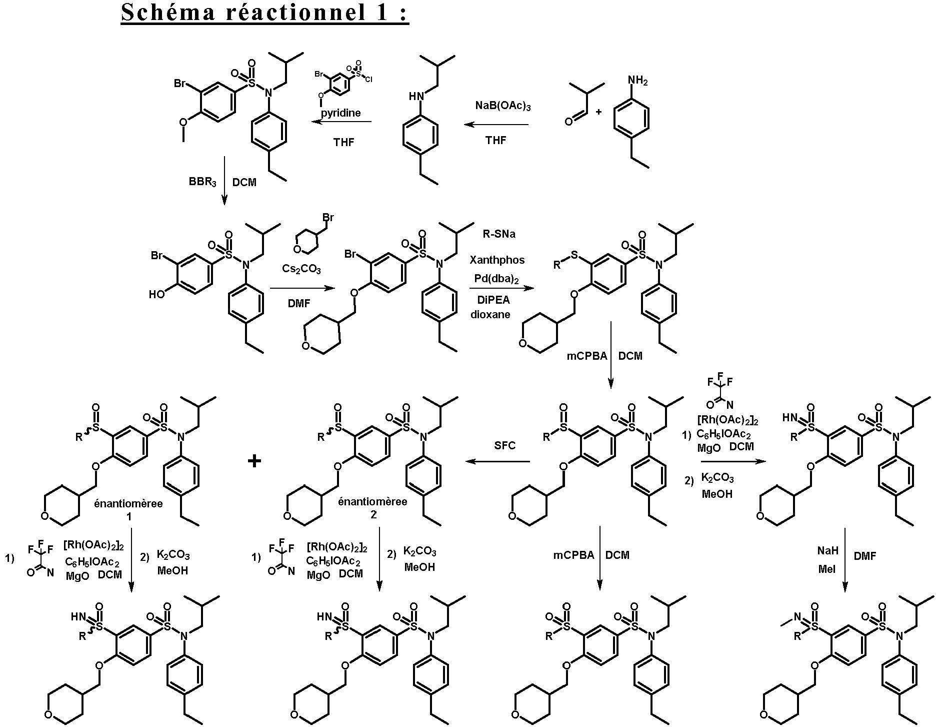

### Exemple 1 : Synthèse du N-(4-éthyl-phényl)-N-isobutyl-3-méthylsulfanyl-4-(tétrahydro-pyran-4-ylméthoxy)-benzenesulfonamide

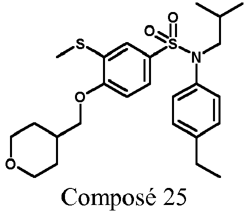

### 1. Synthèse de l'intermédiaire 1.1

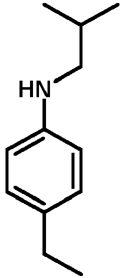

### (4-éthyl-phényl)-isobutyl-amine

A la 4-éthylaniline (9.48 ml; 0.08 mol) est ajouté l'isobutyraldéhyde (6.33 ml; 0.07 mol.) dans le tétrahydrofurane (100ml). Le mélange est agité 2 heures à température ambiante. Puis le triacétoxy-borohydrure de sodium (22.04 g; 0.10 mol) est ajouté. Le mélange est agité une nuit à température ambiante, additionné d'eau (100ml) et extrait à l'acétate d'éthyle (2 x 100ml).

Les phases organiques sont rassemblées, lavées à la saumure (100ml), séchées (Na₂SO₄) et concentrées. Le produit brut est chromatographié sur gel de silice (éluant heptane/dichlorométhane de 0 à 50% de dichlorométhane). La (4-éthyl-phényl)-isobutyl-amine est obtenue sous la forme d'une huile orange avec une RMN¹H conforme.
MS : [M+H] = 179

### 2. Synthèse de l'intermédiaire 1.2

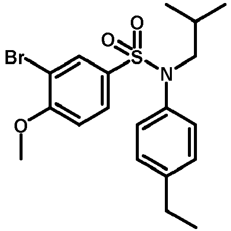

### N-(4-éthyl-phényl)-N-isobutyl-4-méthoxy-benzenesulfonamide

Le chlorure de 3-bromo-4-méthoxy-benzènesulfonyle (3.22 g; 11.28 mmol) est ajouté à la (4-ethyl-phenyl)-isobutyl-amine (2.00 g; 11.28 mmol) et la pyridine (5.5 ml; 67.69 mmol) en solution dans le tétrahydrofurane (40 ml). Le milieu réactionnel est agité pendant 4 heures à température ambiante, hydrolysé et extrait à l'acétate d'éthyle. Les phases organiques sont rassemblées, lavées avec une solution saturée de NH₄Cl puis à la saumure, séchées (Na₂SO₄) et concentrées. Le produit brut est chromatographié sur gel de silice (éluant heptane/acétate d'éthyle de 0 à 20% d'acétate d'éthyle). Le N-(4-éthyl-phényl)-N-isobutyl-4-méthoxy-benzenesulfonamide (1.63 g; 34%) est obtenu sous la forme d'un solide jaune pâle avec une RMN¹H conforme.
MS : [M+H] = 426

### 3. Synthèse de l'intermédiaire 1.3

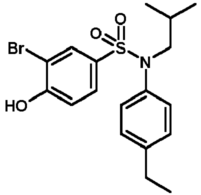

### 3-Bromo-N-(4-éthyl-phényl)-4-hydroxy-N-isobutyl-benzenesulfonamide

Le tribromure de bore, 1M dans le dichlorométhane (5.6 ml; 5.63 mmol) est ajouté lentement à une température de 0°C sur le 3-bromo-N-(4-éthyl-phényl)-N-isobutyl-4-méthoxy-benzenesulfonamide (1.60 g; 3.75 mmol) en solution dans le dichlorométhane (32 ml). Le milieu réactionnel est laissé revenir lentement à température ambiante, agité pendant 16 heures et hydrolysé à une température de 0°C puis extrait au dichlorométhane. Les phases organiques sont rassemblées, lavées à la saumure, séchées (Na₂SO₄) et concentrées.

Le produit brut est chromatographié sur gel de silice (éluant Heptane/Acétate d'éthyle de 0 à 30% d'acétate d'éthyle). Le 3-bromo-N-(4-éthyl-phényl)-4-hydroxy-N-isobutyl-benzenesulfonamide (1.41 g; 91%) est obtenu sous la forme d'un solide beige avec une RMN¹H conforme.
MS : [M+H] = 414

### 4. Synthèse de l'intermédiaire 1.4

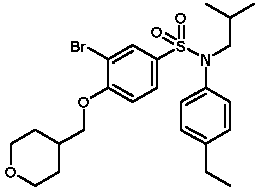

### N-(4-éthyl-phenyl)-N-isobutyl-4-(tétrahydro-pyran-4-ylméthoxy)-benzenesulfon-amide

Le 4-(bromométhyl)-tétrahydropyrane (261 mg; 1.46 mmol) et le carbonate de césium (790 mg; 2.43 mmol) sont ajoutés sur le 3-bromo-N-(4-éthyl-phényl)-4-hydroxy-N-isobutyl-benzenesulfonamide (500 mg; 1.21 mmol) en solution dans le N,N-diméthylformamide (10 ml). Le milieu réactionnel est agité pendant 2 heures à une température de 80°C, hydrolysé et extrait à l'acétate d'éthyle. Les phases organiques sont rassemblées, lavées à la saumure et séchées (Na₂SO₄) et concentrées.

Le produit brut est chromatographié sur gel de silice (éluant : heptane/acétate d'éthyle de 0 à 30% d'acétate d'éthyle). Le N-(4-ethyl-phenyl)-N-isobutyl-4-(tetrahydro-pyran-4-ylmethoxy)-benzenesulfonamide (598 mg; 97 %) est obtenu sous la forme d'un solide blanc avec une RMN¹H conforme.
MS : [M+H] = 512

### Exemple 1 : Synthèse du composé 25 selon l'invention

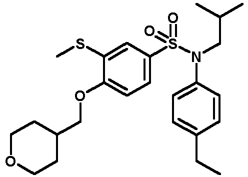

Du bis(dibenzylideneacetone)palladium (0) (216 mg; 0.38 mmol) est ajouté sur une solution dégazée à l'argon pendant 15 minutes de 3-bromo-N-(4-éthyl-phényl)-N-isobutyl-4-(tétrahydro-pyran-4-ylméthoxy)-benzene-sulfonamide (480 mg; 0.94 mmol), de N,N-diisopropyléthylamine (490µl; 2.82 mmol), de 4,5-bis(diphénylphosphino)-9,9-diméthylxanthène (45 mg; 0.08 mmol) et de méthanthiolate de sodium (264 mg; 3.76 mmol) dans du 1,4-dioxanne (5ml). Le milieu réactionnel est agité pendant 3 heures à une température de 110°C, hydrolysé, extrait à l'acétate d'éthyle. Les phases organiques sont rassemblées, lavées à la saumure, séchées (Na₂SO₄) et concentrées.

Le produit brut est chromatographié sur gel de silice (éluant Heptane/Acétate d'éthyle de 0 à 30% d'acétate d'éthyle). Le N-(4-éthyl-phényl)-N-isobutyl-3-methylsulfanyl-4-(tétrahydro-pyran-4-ylméthoxy)-benzenesulfonamide (335 mg; 71%): est obtenu sous la forme d'un solide jaune pâle.
¹H NMR (400 MHz, Chloroforme-d) δ 7.41 (dd, J = 8.4, 2.1 Hz, 1H), 7.19 - 7.11 (m, 2H), 7.08 (d, J = 2.2 Hz, 1H), 7.04 - 6.95 (m, 2H), 6.83 (d, J = 8.5 Hz, 1H), 4.07 (dt, J = 11.5, 2.8 Hz, 2H), 3.95 (d, J = 6.4 Hz, 2H), 3.49 (td, J = 11.9, 2.1 Hz, 2H), 3.27 (d, J = 7.3 Hz, 2H), 2.66 (q, J = 7.6 Hz, 2H), 2.24 - 2.10 (m, 1H), 1.87 - 1.74 (m, 2H), 1.59 (s, 11H), 1.25 (t, J = 7.6 Hz, 4H), 0.93 (d, J = 6.7 Hz, 7H).
MS : [M+H] = 478

### Exemple 2 : Synthèse du N-(4-éthyl-phényl)-N-isobutyl-3-méthanesulfinyl-4-(tétrahydro-pyran-4-ylméthoxy)-benzenesulfonamide

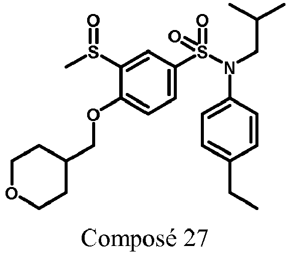

A une solution de N-(4-éthyl-phényl)-N-isobutyl-3-methylsulfanyl-4-(tétrahydro-pyran-4-ylméthoxy)-benzenesulfonamide (0.40 g; 0.84 mmol) dans du dichlorométhane (8 ml) à 0°C est ajouté par portion de l'acide 3-chloroperoxybenzoïque (0.17 g; 0.75 mmol). Le milieu réactionnel est agité 45 minutes, hydrolysé avec une solution aqueuse à 10% de Na₂S₂O₃ et extrait avec du dichlorométhane. La phase organique est lavée à la soude 1N puis est séchée (Na₂SO₄), filtrée et concentrée.

Le produit brut est chromatographié sur gel de silice (éluant heptane/acétate d'éthyle de 50 à 100% d'acétate d'éthyle). Le N-(4-éthyl-phényl)-N-isobutyl-3-méthanesulfinyl-4-(tétrahydro-pyran-4-ylméthoxy)-benzenesulfonamide (0.24 g; 58%) est obtenu sous la forme d'un solide blanc.
¹H NMR (DMSO-d₆) δ: 0.85 (t, J = 6.9 Hz, 6H), 1.18 (t, J = 7.6 Hz, 3H), 1.28 - 1.48 (m, 3H), 1.65 (tdd, J = 11.5, 4.0, 2.1 Hz, 2H), 2.61 (q, J = 7.6 Hz, 2H), 2.74 (s, 3H), 3.21 - 3.40 (m, 5H), 3.90 (ddd, J = 11.5, 4.9, 2.1 Hz, 2H), 4.00 - 4.15 (m, 2H), 6.94 - 7.02 (m, 2H), 7.15 - 7.23 (m, 2H), 7.33 - 7.35 (m, 1H) 7.64 - 7.73 (m, 2H)
MS : [M+H] = 494

Le composé 27 : (550 mg; 1.11 mmol) est chromatographié par SFC chirale pour séparer les 2 énantiomères (composé 19 et composé 20) ci-après :
Conditions super critiques 100bar, 70°C ; colonne CHIRALPAK IC 250x4.6mm 5µ]

### Exemple 3 : N-(4-éthyl-phényl)-N-isobutyl-3-methanesulfinyl-4-(tetrahydro-pyran-4-ylmethoxy)-benzenesulfonamide (composé 19) - énantiomère A du composé 27

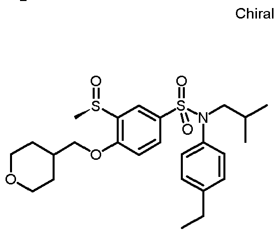

(311 mg; 56 %) sous la forme d'un solide blanc
¹H NMR (Chloroforme-d) δ: 0.86 - 0.96 (m, 6H), 1.23 (t, J = 7.6 Hz, 3H), 1.40 - 1.65 (m, 7H), 1.65 - 1.87 (m, 2H), 2.11 (s, 1H), 2.63 (q, J = 7.6 Hz, 2H), 2.77 (s, 2H), 3.22 - 3.34 (m, 1H), 3.36 - 3.45 (m, 1H), 3.48 (dd, J = 11.9, 2.2 Hz, 1H), 3.89 - 4.09 (m, 4H), 6.88 (d, J = 8.6 Hz, 1H), 6.94 - 7.01 (m, 2H), 7.09 - 7.16 (m, 2H), 7.57 (dd, J = 8.6, 2.4 Hz, 1H), 8.16 (d, J = 2.3 Hz, 1H)
Temps de rétention (SFC chirale) de 6.0 minutes

### Exemple 4: N-(4-éthyl-phényl)-N-isobutyl-3-methanesulfinyl-4-(tetrahydro-pyran-4-ylmethoxy)-benzenesulfonamide (composé 20) - énantiomère B du composé 27

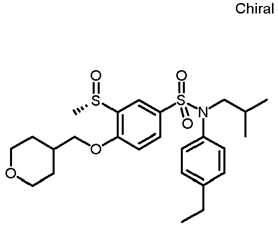

(240 mg; 44 %) sous la forme d'un solide blanc
¹H NMR (Chloroforme-d) δ: 0.91 (dd, J = 13.3, 6.7 Hz, 6H), 1.23 (t, J = 7.6 Hz, 4H), 1.39 - 1.64 (m, 7H), 1.66 - 1.79 (m, 2H), 2.02 - 2.20 (m, 1H), 2.63 (q, J = 7.7 Hz, 2H), 2.77 (s, 3H), 3.27 (dd, J = 12.9, 6.8 Hz, 1H), 3.36 - 3.52 (m, 3H), 3.87 - 4.10 (m, 4H), 6.88 (d, J = 8.6 Hz, 1H), 6.94 - 7.02 (m, 2H), 7.09 - 7.16 (m, 2H), 7.57 (dd, J = 8.6, 2.3 Hz, 1H), 8.16 (d, J = 2.3 Hz, 1H)
Temps de rétention (SFC chirale) de 9.9 minutes

### Exemple 5 : Synthèse du N-(4-éthyl-phényl)-N-isobutyl-3-méthanesulfoximin-4-(tétrahydro-pyran-4-ylméthoxy)-benzenesulfonamide

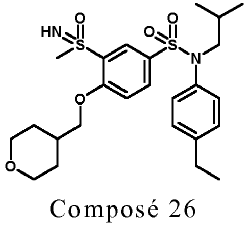

A une solution, préalablement dégazé à l'argon, de N-(4-éthyl-phényl)-N-isobutyl-3-methanesulfinyl-4-(tetrahydro-pyran-4-ylmethoxy)-benzenesulfonamide (0.23 g; 0.47 mmol) dans du dichlorométhane (8ml) sont rajoutés du 2,2,2-trifluoroacétamide (0.13 g; 1.16 mmol), de l'oxyde de magnésium (0.09 g; 2.33 mmol), du rhodium(II) acétate dimère (31mg; 0.07 mmol) et du iodobenzène diacetate (0.29 g; 0.89 mmol). Le milieu réactionnel est agité pendant 4h30, filtré sur célite et concentré.

Le résidu est dilué dans du méthanol (8 ml) et du carbonate de potassium (0.32 g; 2.33 mmol) est ajouté. Le milieu réactionnel est agité pendant 30 minutes, hydrolysé et extrait à l'acétate d'éthyle. Les phases organiques sont réunies, séchées (Na₂SO₄), filtrées et concentrées.

Le produit brut est purifié par HPLC préparative. Le N-(4-éthyl-phényl)-N-isobutyl-3-méthanesulfoximin-4-(tétrahydro-pyran-4-ylméthoxy)-benzenesulfonamide (0.08 g; 34 %) est obtenu sous la forme d'un solide blanc.
¹H NMR (DMSO-d₆) δ: 0.84 (d, J = 4.5 Hz, 3H), 0.86 (d, J = 4.4 Hz, 3H), 1.18 (t, J = 7.6 Hz, 3H), 1.27 - 1.58 (m, 3H), 1.66 - 1.83 (m, 2H), 2.02 - 2.21 (m, 1H), 2.61 (q, J = 7.6 Hz, 2H), 3.19 (d, J = 1.2 Hz, 3H), 3.24-3.39 (m, 4H), 3.83 - 3.96 (m, 2H), 4.11 (dd, J = 6.2, 2.5 Hz, 2H), 4.41 (d, J = 1.5 Hz, 1H), 7.01 (d, J = 8.3 Hz, 2H), 7.21 (d, J = 8.4 Hz, 2H), 7.39 (d, J = 8.8 Hz, 1H), 7.67 (dd, J = 8.8, 2.4 Hz, 1H), 8.00 (d, J = 2.4 Hz, 1H)
MS : [M+H] = 509

Un mode opératoire analogue à celui appliqué au composé 26 pour obtenir les composés 19 et 20 est mis en oeuvre afin d'obtenir les composés 7 et 8 (énantiomères du composé 26).

### Exemple 6 : N-(4-éthyl-phényl)-N-isobutyl-3-(méthanesulfinyl)-4-(tétrahydro-pyran-4-ylméthoxy)-benzenesulfoximine (composé 7) - énantiomère A du composé 26

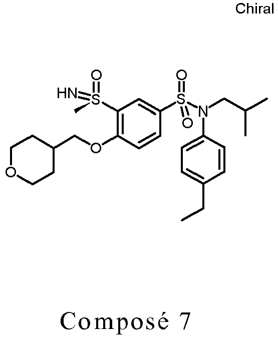

Même mode opératoire que pour l'exemple 3, sur l'exemple 4 (213 mg; 0.43 mmol.). Le N-(4-éthyl-phényl)-N-isobutyl-3-((S)-méthanesulfinyl)-4-(tétrahydro-pyran-4-ylméthoxy)-benzenesulfoximine (20 mg; 9%) est obtenu sous la forme d'un solide beige avec une RMN¹H conforme.
MS : [M+H] = 509

### Exemple 7 : N-(4-éthyl-phényl)-N-isobutyl-3-(méthanesulfinyl)-4-(tétrahydro-pyran-4-ylméthoxy)-benzenesulfoximine (composé 8) - énantiomère B du composé 26

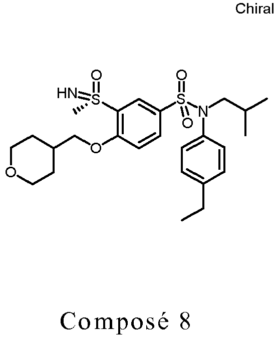

Même mode opératoire que pour l'exemple 3, sur l'exemple 5 (132 mg; 0.27 mmol). Le N-(4-éthyl-phényl)-N-isobutyl-3-((R)-méthanesulfinyl)-4-(tétrahydro-pyran-4-ylméthoxy)-benzenesulfoximine (11 mg; 9 %) est obtenu sous la forme d'un solide blanc-cassé avec une RMN¹H conforme.
MS : [M+H] = 509

### Exemple 8 : Synthèse du N-(4-éthyl-phényl)-N-isobutyl-3-méthanesulfinyl-4-(tétrahydro-pyran-4-ylméthoxy)-benzene-N-méthyl-sulfoximine

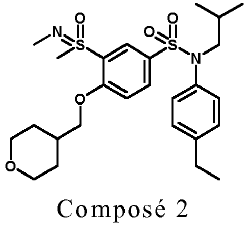

L'hydrure de sodium 60% (9.2 mg; 0.23 mmol) est ajouté par petite portion sur une solution à 0°C de N-(4-éthyl-phényl)-N-isobutyl-3-méthanesulfinyl-4-(tétrahydro-pyran-4-ylméthoxy)-benzenesulfoximine (90 mg; 0.18 mmol) dans du N,N-diméthylformamide (1.8 ml). Le milieu réactionnel est agité pendant 20 minutes à une température de 0°C puis du iodométhane (22 µl; 0.35 mmol) est ajouté goutte à goutte. Le milieu réactionnel est agité pendant 20 heures à température ambiante, hydrolysé et extrait à l'acétate d'éthyle.

Les phases organiques sont rassemblées, lavées à la saumure et séchées (Na₂SO₄) et concentrées et le produit brut est chromatographié sur gel de silice (éluant dichlorométhane/méthanol de 0 à 5% de méthanol).

Le N-(4-éthyl-phényl)-N-isobutyl-3-méthanesulfinyl-4-(tétrahydro-pyran-4-ylméthoxy)-benzene-N-méthyl-sulfoximine (59.3 mg; 64%) est obtenu sous la forme d'un solide blanc.
¹H NMR (DMSO-d6) δ: 0.85 (t, J = 7.1 Hz, 6H), 1.17 (t, J = 7.6 Hz, 3H), 1.30 - 1.51 (m, 3H), 1.62 - 1.71 (m, 1H), 1.80 (ddd, J = 13.0, 4.1, 2.0 Hz, 1H), 2.08 (s, 1H), 2.34 (s, 3H), 2.59 (q, J = 7.6 Hz, 2H), 3.20 (s, 3H), 3.22 - 3.41 (m, 8H), 3.90 (ddd, J = 11.5, 4.6, 1.9 Hz, 2H), 4.04 (dd, J = 9.5, 6.7 Hz, 1H), 4.17 (dd, J = 9.3, 5.6 Hz, 1H), 6.93 - 7.00 (m, 2H), 7.14 - 7.21 (m, 2H), 7.45 (d, J = 8.8 Hz, 1H), 7.74 (d, J = 2.4 Hz, 1H), 7.86 (dd, J = 8.8, 2.5 Hz, 1H).
MS : [M+H] = 523

### Exemple 9 : Synthèse du N-(4-éthyl-phényl)-N-isobutyl-3-méthanesulfonyl-4-(tétrahydro-pyran-4-ylméthoxy)-benzenesulfonamide

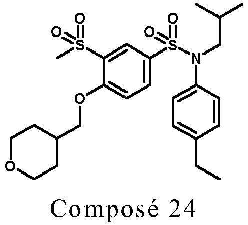

De l'acide 3-chloroperbenzoïque (188 mg; 0.84 mmol) est ajouté par portion à 0°C sur le N-(4-éthyl-phényl)-N-isobutyl-3-methylsulfanyl-4-(tetrahydro-pyran-4-ylmethoxy)-benzenesulfonamide (200 mg; 0.42 mmol) en solution du dichlorométhane (2 ml). Le milieu réactionnel est agité 72h, hydrolysé, extrait à l'acétate d'éthyle. Les phases organiques sont rassemblées, lavées à la saumure, séchées (Na₂SO₄) et concentrées. Le produit brut est chromatographié sur gel de silice (éluant heptane/acétate d'éthyle de 0 à 50% d'acétate d'éthyle). Le N-(4-éthyl-phényl)-N-isobutyl-3-methanesulfonyl-4-(tetrahydro-pyran-4-ylmethoxy)-benzenesulfonamide 150 mg; 71%) est obtenu sous la forme d'un solide blanc.
¹H NMR (400 MHz, DMSO-d6) δ 7.86 - 7.77 (m, 2H), 7.48 (d, J = 8.7 Hz, 1H), 7.24 - 7.17 (m, 2H), 7.05 - 6.96 (m, 2H), 4.16 (d, J = 6.2 Hz, 2H), 3.95 - 3.86 (m, 2H), 3.32 - 3.25 (m, 6H), 2.61 (q, J = 7.6 Hz, 2H), 2.16 - 2.09 (m, 1H), 1.77 - 1.68 (m, 2H), 1.49 - 1.34 (m, 3H), 1.18 (t, J = 7.6 Hz, 3H), 0.85 (d, J = 6.6 Hz, 6H).
MS : [M+H] = 510

### Exemple 10 : Synthèse de l'éthanesulfinyl-N-(4-éthyl-phényl)-N-isobutyl-4-(tetrahydro-pyran-4-ylméthoxy)-benzenesulfonamide

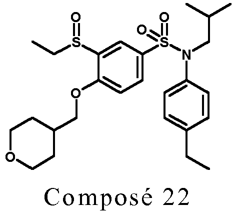

### 1. Synthèse de l'intermédiaire 10.1

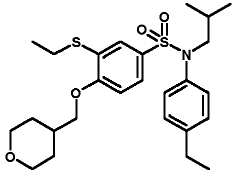

### N-(4-éthyl-phényl)-3-éthylsulfanyl-N-isobutyl-4-(tetrahydro-pyran-4-ylméthoxy)-benzenesulfonamide

Le bis(dibenzylideneacetone)palladium (0) (225 mg; 0.39 mmol) est ajouté à un mélange, dégazé 15mn à l'argon, de 3-bromo-N-(4-éthyl-phényl)-N-isobutyl-4-(tétrahydro-pyran-4-ylméthoxy)-benzenesulfonamide (500 mg; 0.98 mmol), de N,N-diisopropyléthylamine (510 µl; 2.94 mmol), de 4,5-bis(diphenylphosphino)-9,9-dimethylxanthene (45 mg; 0.08 mmol) d'éthanethiolate de sodium (91 mg; 1.08 mmol) en solution dans du 1,4-dioxanne (5 ml). Le milieu réactionnel est agité 1heure à 110°C, hydrolysé et extrait à l'acétate d'éthyle. Les phases organiques sont rassemblées, lavées à la saumure, séchées (Na₂SO₄) et concentrées. Le produit brut est purifié par HPLC préparative (colonne C18, éluant : de 56% à 62% d'acétonitrile dans eau/0.1% d'acide formique). Le N-(4-ethyl-phenyl)-3-ethylsulfanyl-N-isobutyl-4-(tetrahydro-pyran-4-ylmethoxy)-benzenesulfonamide (271 mg; 42%) est obtenu sous la forme d'un solide blanc après trituration dans l'heptane avec une RMN¹H conforme.
MS : [M+H] = 492

### 2. Synthèse du composé 22 selon l'invention

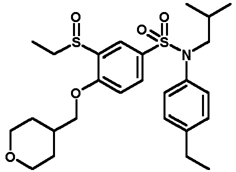

De l'acide 3-chloroperbenzoïque (59mg; 0.26 mmol) est ajouté par portion à une température de 0°C sur le N-(4-éthyl-phényl)-3-éthylsulfanyl-N-isobutyl-4-(tétrahydro-pyran-4-ylméthoxy)-benzenesulfonamide (260 mg; 0.53 mmol) en solution dans du dichlorométhane (5ml). Le milieu réactionnel est agité pendant 1 heure à température ambiante, hydrolysé avec une solution aqueuse à 10% de Na₂S₂O₃ puis extrait au dichlorométhane. Les phases organiques sont rassemblées, lavées avec une solution de soude 0.1N, à la saumure, séchées (Na₂SO₄) et concentrées.

Le produit brut est chromatographié sur gel de silice (éluant heptane/acétate d'éthyle de 0 à 100% d'acétate d'éthyle). Le 3-éthanesulfinyl-N-(4-éthyl-phényl)-N-isobutyl-4-(tétrahydro-pyran-4-ylméthoxy)-benzenesulfonamide (125 mg; 47%) est obtenu sous la forme d'un solide blanc.
¹H NMR (Chloroforme-d) δ: 0.84 (dd, J = 13.3, 6.7 Hz, 6H), 1.10 (t, J = 7.4 Hz, 3H), 1.15 (t, J = 7.6 Hz, 3H), 1.33 - 1.55 (m, 2H), 1.58 - 1.72 (m, 2H), 1.94 - 2.12 (m, 1H), 2.56 (q, J = 7.6 Hz, 2H), 2.73 (dq, J = 13.4, 7.4 Hz, 1H), 2.99 (dq, J = 13.5, 7.4 Hz, 1H), 3.21 (dd, J = 12.8, 6.8 Hz, 1H), 3.28 - 3.45 (m, 3H), 3.84 (dd, J = 9.0, 6.3 Hz, 1H), 3.91 (dd, J = 9.0, 6.4 Hz, 1H), 3.98 (ddd, J = 11.6, 4.6, 1.8 Hz, 2H), 6.81 (d, J = 8.7 Hz, 1H), 6.86 - 6.93 (m, 2H), 7.01 - 7.08 (m, 2H), 7.50 (dd, J = 8.6, 2.4 Hz, 1H), 8.02 (d, J = 2.4 Hz, 1H)
MS : [M+H] = 508

### Exemple 11 : Synthèse du N-(4-éthyl-ohényl)-N-isobutyl-3-ethanesulfoximin-4-(tetrahydro-pyran-4-ylmethoxy)-benzenesulfonamide

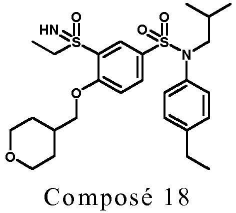

A une solution, préalablement dégazé à l'argon, de 3-éthanesulfinyl-N-(4-éthyl-phényl)-N-isobutyl-4-(tétrahydro-pyran-4-ylméthoxy)-benzenesulfonamide (110 mg; 0.22 mmol) dans du dichlorométhane (8ml) sont ajoutés le 2,2,2-trifluoroacétamide (61 mg; 0.54 mmol), de l'oxyde de magnésium (44 mg; 1.08 mmol), le rhodium(II) acétate (14 mg; 0.03 mmol) et du iodobenzène diacétate (133 mg; 0.41 mmol). Le milieu réactionnel est agité 16 heures à température ambiante, filtré sur célite et concentré. Le résidu obtenu est dilué dans du méthanol (8ml) et du carbonate de potassium (150 mg; 1.08 mmol) est ajouté. Le milieu réactionnel est agité pendant 30 minutes, hydrolysé et extrait à l'acétate d'éthyle. Les phases organiques sont rassemblées, lavées à la saumure, séchées (Na₂SO₄) et concentrées.

Le produit brut est purifié par HPLC préparative (colonne C18, éluant : de 56% à 62% d'acétonitrile dans eau/0.1% d'acide formique). Le N-(4-éthyl-phenyl)-N-isobutyl-3-ethanesulfoximin-4-(tetrahydro-pyran-4-ylmethoxy)-benzenesulfonamide (23 mg; 20%) est obtenu sous la forme d'un solide blanc-cassé.
¹H NMR (Chloroforme-d) δ: 0.93 (dd, J = 8.4, 6.6 Hz, 6H), 1.25 (q, J = 7.4 Hz, 7H), 1.46 - 1.69 (m, 6H), 1.86 (ddq, J = 11.1, 4.5, 2.2 Hz, 2H), 2.14 - 2.30 (m, 1H), 2.67 (q, J = 7.6 Hz, 2H), 2.75 (s, 1H), 3.28 (dd, J = 12.8, 7.0 Hz, 1H), 3.31 - 3.45 (m, 3H), 3.49 (td, J = 11.9, 2.2 Hz, 2H), 4.00 - 4.12 (m, 4H), 6.96 - 7.01 (m, 2H), 7.04 (d, J = 8.8 Hz, 1H), 7.13 - 7.20 (m, 2H), 7.73 (dd, J = 8.7, 2.4 Hz, 1H), 8.12 (d, J = 2.4 Hz, 1H)
MS : [M+H] = 523

### Partie II : Synthèse des sulfonamides soufrés à partir du schéma réactionnel 2

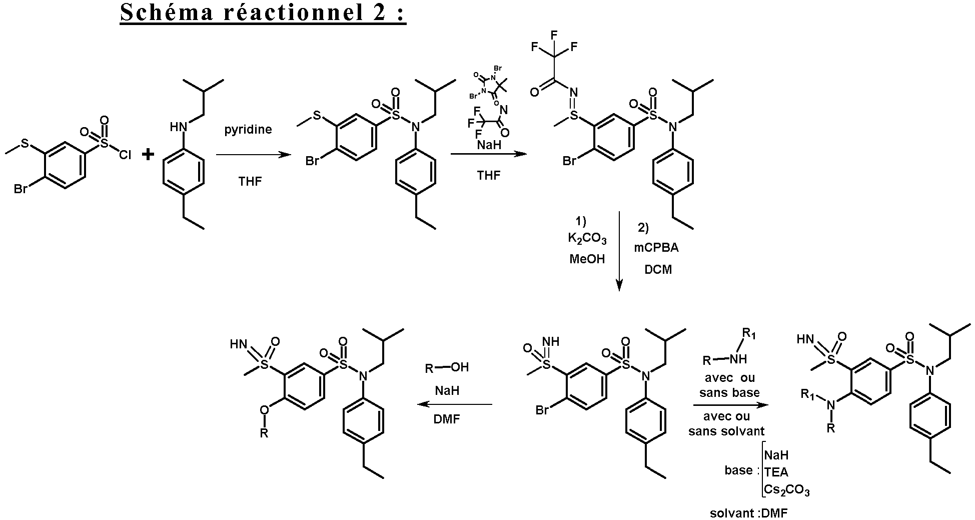

### Exemple 12 : Synthèse du N-(4-éthylphényl)-4-((4-fluorotetrahydro-2H-pyran-4-yl)methoxy)-N-isobutyl-3-(S-methylsulfonimidoyl)benzenesulfonamide

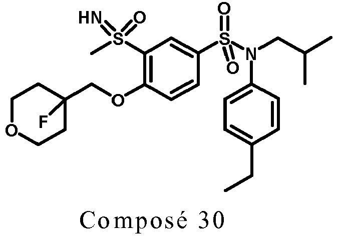

### 1. Synthèse de l'intermédiaire 12.1

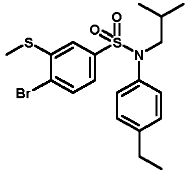

### 4-bromo-N-(4-éthyl-phényl)-N-isobutyl-3-méthylsulfanyl-benzenesulfonamide

Le chlorure de 4-bromo-3-(méthylthio)-benzene-1-sulfonyle (19.63 g; 61.83 mmol) en solution dans le tétrahydrofurane (95ml) est ajouté sur la (4-éthyl-phényl)-isobutyl-amine (10.96 g; 61.83 mmol.) et la pyridine (30 ml; 371 mmol) en solution dans le tétrahydrofurane (370ml). Le milieu réactionnel est agité pendant 16 heures à température ambiant puis hydrolysé et extrait à l'acétate d'éthyle. Les phases organiques sont rassemblées, lavées avec une solution saturée de chlorure d'ammonium puis à la saumure, séchées (Na₂SO₄) et concentrées. Le produit brut est repris dans l'heptane et essoré.

Le 4-bromo-N-(4-ethyl-phenyl)-N-isobutyl-3-methylsulfanyl-benzenesulfonamide (21.31 g; 78%) est obtenu sous la forme d'un solide jaune pâle avec une RMN¹H conforme.
MS : [M+H] = 444

### 2. Synthèse de l'intermédiaire 12.2

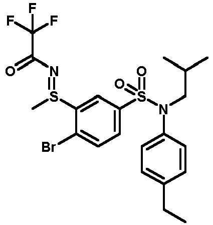

### (E)-N-((2-bromo-5-(N-(4-ethylphenyl)-N-isobutylsulfamoyl)-phenyl)(methyl)-λ⁴-sulfanylidene)-2,2,2-trifluoroacetamide

Le 4-bromo-N-(4-éthyl-phényl)-N-isobutyl-3-méthylsulfanyl-benzenesulfonamide (5.00 g; 11.30 mmol) et le 2,2,2-trifluoroacetamide (1.92 g; 16.95 mmol) en solution dans le tétrahydrofurane (10ml) sont additionnés lentement sur l'hydrure de sodium 60% (0.41 g; 10.17 mmol) en suspension dans le tétrahydrofurane (10ml) à 0-5°C puis la 1,3-dibromo-5,5-dimethylhydantoïne (4.85 g; 16.95 mmol) en solution dans le tétrahydrofurane (25ml) à une température de 0-5°C est ajouté. Le milieu est agité pendant 1 heure à température ambiante. Le milieu réactionnel est hydrolysé avec une solution à 10% d'acide citrique puis extrait à l'acétate d'éthyle.

Les phases organiques sont rassemblées, lavées avec une solution à 25% de sulfite de sodium puis à la saumure, séchées (Na₂SO₄) et concentrées. Le résidu est repris dans l'éther et essoré. Le (E)-N-((2-bromo-5-(N-(4-ethylphenyl)-N-isobutylsulfamoyl)-phenyl)(methyl)-λ⁴-sulfanylidene)-2,2,2-trifluoroacetamide (4.76 g; 76%) est obtenu sous la forme d'une poudre blanche avec une RMN¹H conforme.
MS : [M+H] = 554

### 3. Synthèse de l'intermédiaire 12.3

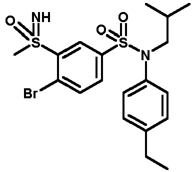

### 2-bromo-N-(4-éthylphényl)-N-isobutyl-3-(S-éthylsulfonimidoyl)-benzene-sulfonamide

Le carbonate de potassium (2.79 g; 20.16 mmol) est ajouté sur le (E)-N-((2-bromo-5-(N-(4-éthylphényl)-N-isobutylsulfamoyl)-phenyl)(methyl)-λ⁴-sulfanylidene)-2,2,2-trifluoroacétamide (3.72 g; 6.72 mmol) en solution dans le méthanol (35ml) puis l'on a ajouté lentement l'acide 3-chloroperoxybenzoique (2.26 g; 10.08 mmol) à une température de 0°C. Le milieu réactionnel est agité pendant 16 heures à température ambiante.

Le milieu réactionnel est hydrolysé puis extrait à l'acétate d'éthyle. Les phases organiques sont rassemblées, lavées à la saumure, séchées (Na₂SO₄) et concentrées.

Le produit brut est chromatographié sur gel de silice (éluant heptane/acétate d'éthyle de 0 à 80% d'acétate d'éthyle). Le 4-bromo-N-(4-ethylphenyl)-N-isobutyl-3-(S-methylsulfonimidoyl)-benzenesulfonamide (1.51 g, 47%) est obtenu sous la forme d'un solide blanc avec une RMN¹H conforme.
MS : [M+H] = 474

### 4. Synthèse du composé 30 selon l'invention

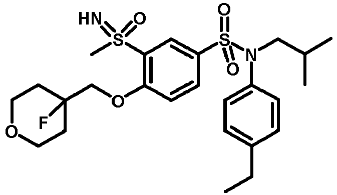

L'hydrure de sodium 60% (9 mg; 0.22 mmol) est ajouté lentement sur le (4-fluoro-tétrahydro-pyran-4-yl)-méthanol (28.33 mg; 0.21 mmol) en solution dans le N,N-diméthylformamide (0.5ml) puis le 4-bromo-N-(4-éthylphényl)-N-isobutyl-3-(S-methylsulfonimidoyl)benzenesulfonamide (50 mg; 0.11 mmol).

Le milieu réactionnel est agité pendant 2 heures à température ambiante. Le milieu réactionnel est hydrolysé à froid puis extrait à l'acétate d'éthyle. Les phases organiques sont rassemblées, lavées à la saumure, séchées (Na₂SO₄) et concentrées.

Le produit brut est purifié par HPLC préparative (colonne C18, éluant : acétonitrile dans eau/0.1% d'acide formique). Le N-(4-éthylphényl)-4-((4-fluorotétra-hydro-2H-pyran-4-yl)-méthoxy)-N-isobutyl-3-(S-methylsulfonimidoyl)benzenesulfonamide (46 mg; 82%) est obtenu sous la forme d'un solide blanc.
¹H NMR (DMSO-d6) δ: 0.86 (dd, J = 6.7, 4.6 Hz, 6H), 1.19 (t, J = 7.6 Hz, 3H), 1.36 - 1.51 (m, 1H), 1.76 - 2.05 (m, 4H), 2.61 (q, J = 7.6 Hz, 2H), 3.19 (d, J = 1.0 Hz, 3H), 3.22 - 3.32 (m, 2H), 3.63 (td, J = 11.2, 3.0 Hz, 2H), 3.80 (dt, J = 11.2, 2.8 Hz, 2H), 4.34 - 4.48 (m, 3H), 6.97 - 7.04 (m, 2H), 7.17 - 7.24 (m, 2H), 7.39 (d, J = 8.8 Hz, 1H), 7.70 (dd, J = 8.7, 2.4 Hz, 1H), 8.01 (d, J = 2.4 Hz, 1H).
MS : [M+H] = 527

Avec un mode opératoire analogue à celui décrit pour l'exemple 12, on obtient :

| | | |
|---|---|---|
| **Exemple 13** | 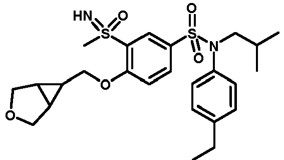 | **4-((3-oxabicyclo[3.1.0]hexan-6-yl)méthoxy)-N-(4-éthylphényl)-N-iso butyl-3-(S-méthylsulfonimidoyl)benzene-sulfonamide** |
| | | ¹H NMR (DMSO-d6) δ: 0.85 (dd, J = 6.7, 4.3 Hz, 6H), 1.12 - 1.23 (m, 4H), 1.43 (dt, J = 13.6, 6.9 Hz, 1H), 1.79 - 1.86 (m, 2H), 2.61 (q, J = 7.6 Hz, 2H), 3.23 (s, 3H), 3.25 - 3.36 (m, 2H), 3.61 (dt, J = 8.3, 1.4 Hz, 2H), 3.78 (d, J = 8.4 Hz, 2H), 4.21 |
| | Composé 31 | (dd, J = 6.9, 1.9 Hz, 2H), 4.41 (d, J = 1.5 Hz, 1H), 6.96 - 7.03 (m, 2H), 7.17 - 7.24 (m, 2H), 7.34 (d, J = 8.8 Hz, 1H), 7.65 (dd, J = 8.7, 2.4 Hz, 1H), 8.00 (d, J = 2.5 Hz, 1H). |
| | | MS : [M+H] = 507 |
| **Exemple 14** | 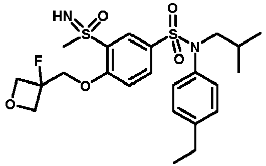 | **N-(4-éthylphényl)-4-((3-fluorooxetan-3-yl)-méthoxy)-N-isobutyl-3-(S-méthylsulfonimidoyl)-benzenesulfonamide** |
| | | ¹H NMR (DMSO-d6) δ: 0.86 (dd, J = 6.6, 4.3 Hz, 6H), 1.19 (t, J = 7.6 Hz, 3H), 1.38 - 1.50 (m, 1H), 2.61 (q, J = 7.6 Hz, 2H), 3.16 (d, J = 1.2 Hz, 3H), 3.22 - 3.41 (m, 2H), 4.46 (d, J = 1.5 Hz, 1H), 4.63 - 4.86 (m, 6H), 6.97 - 7.05 (m, 2H), 7.21 (d, J = 8.3 Hz, 2H), 7.74 (dd, J = 8.7, 2.4 Hz, 1H), 8.00 (d, J = 2.4 Hz, 1H). |
| | Composé 32 | |
| | | MS : [M+H] = 499 |
| **Exemple 15** | 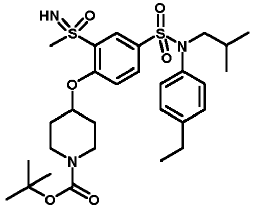 | **4-( 4-(N -(4-éthylphényl)-N -isobutyl-sulfamoyl)-2-(S-méthyl-sulfonimidoyl)-phénoxy)piperidine-1-carboxylate de tert-butyle** |
| | | ¹H NMR (DMSO-d6) δ: 0.85 (dd, J = 6.6, 3.5 Hz, 6H), 1.19 (t, J = 7.6 Hz, 3H), 1.42 (s, 11H), 1.70 - 1.80 (m, 2H), 1.90 (t, J = 10.4 Hz, 2H), 2.61 (q, J = 7.5 Hz, 2H), 3.19 (s, 3H), 3.24 - 3.31 (m, 2H), 3.38 - 3.48 (m, 2H), 3.48 - 3.61 (m, 2H), 4.42 (d, J = 1.4 Hz, 1H), 4.97 - 5.05 (m, 1H), 6.98 - 7.05 (m, 2H), 7.17 - 7.25 (m, 2H), 7.45 (d, J = 9.0 Hz, 1H), 7.64 (dd, J = 8.7, 2.4 Hz, 1H), 8.01 (d, J = 2.3 Hz, 1H). |
| | Composé 33 | |
| | | MS : [M+H] = 594 |
| **Exemple 16** | 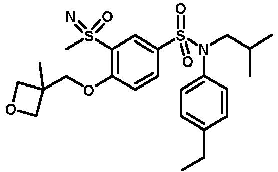 | **N-(4-éthylphényl)-N-isobutyl-4-((3-méthyloxetan-3-yl)méthoxy)-3-(S-methyl-sulfonimidoyl)-benzenesulfonamide** |
| | | ¹H NMR (DMSO-d6) δ: 0.86 (dd, J = 6.6, 4.1 Hz, 6H), 1.19 (t, J = 7.6 Hz, 3H), 1.42 (s, 4H), 2.56 - 2.67 (m, 2H), 3.21 (d, J = 1.2 Hz, 3H), 3.24 - 3.32 (m, 2H), 4.30 (d, J = 3.3 Hz, 2H), 4.36 (d, J = 6.0 Hz, 2H), 4.42 (d, J = 1.4 Hz, 1H), 4.62 (dd, J = 5.9, 3.0 Hz, 2H), 6.98 - 7.05 (m, 2H), 7.18 - 7.25 (m, 2H), 7.43 (d, J = 8.8 Hz, 1H), 7.70 (dd, J = 8.7, 2.5 Hz, 1H), 8.02 (d, J = 2.4 Hz, 1H). |
| | Composé 34 | |
| | | MS : [M+H] = 495 |
| **Exemple 17** | | **4-(((1R,5S,6r)-3-oxabicyclo-[3.1.0]hexan-6-yl)méthoxy)-N-(4-éthylphényl)-N-isobutyl-3-(S-méthylsulfonimidoyl)-benzenesulfonamide** |
| | 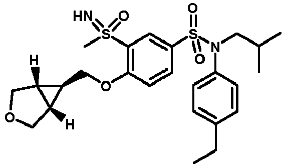 | ¹H NMR (DMSO-d6) δ: 0.85 (dd, J = 6.7, 4.3 Hz, 6H), 1.18 (t, J = 7.6 Hz, 4H), 1.35 - 1.51 (m, 1H), 1.79 - 1.86 (m, 2H), 2.61 (q, J = 7.6 Hz, 2H), 3.23 (d, J = 1.2 Hz, 3H), 3.25 - 3.31 (m, 2H), 3.61 (dt, J = 8.4, 1.3 Hz, 2H), 3.78 (d, J = 8.3 Hz, 2H), 4.21 (dd, J = 7.0, 1.9 Hz, 2H), 4.41 (d, J = 1.4 Hz, 1H), 6.96 - 7.03 (m, 2H), 7.17 - 7.24 (m, 2H), 7.34 (d, J = 8.8 Hz, 1H), 7.65 (dd, J = 8.7, 2.4 Hz, 1H), 8.00 (d, J = 2.5 Hz, 1H). MS : [M+H] = 507 |
| | Composé 35 | |
| **Exemple 18** | 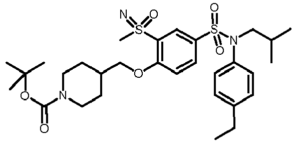 | **4-((4-(N-(4-éthylphényl)-N-isobutyl-sulfamoyl)-2-(S-méthyl-sulfonimidoyl)-phénoxy)méthyl)-piperidine-1-carboxylate de tert-butyle** |
| | | ¹H NMR (DMSO-d6) δ: 0.85 (dd, J = 6.6, 4.4 Hz, 6H), 1.14 - 1.31 (m, 5H), 1.41 (s, 10H), 1.82 (s, 2H), 2.04 (s, 1H), 2.53 - 2.66 (m, 3H), 2.77 (s, 2H), 3.17 (d, J = 1.2 Hz, 3H), 3.21 -3.31 (m, 2H), 3.99 (d, J = 12.8 Hz, 2H), 4.08 - 4.15 (m, 2H), 4.41 (d, J = 1.5 Hz, 1H), 6.97 - 7.04 (m, 2H), 7.17 - 7.24 (m, 2H), 7.37 (d, J = 8.8 Hz, 1H), 7.67 (dd, J = 8.7, 2.5 Hz, 1H), 8.00 (d, J = 2.4 Hz, 1H). |
| | Composé 36 | |
| | | MS : [M+H] = 608 |
| **Exemple 19** | 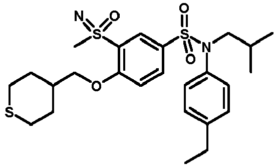 | **N-(4-éthylphényl)-N-isobutyl-3-(S-méthylsulfon-imidoyl)-4-(pyridin-4-ylméthoxy)-benzene-sulfonamide** |
| | | ¹H NMR (DMSO-d6) δ: 0.85 (dd, J = 6.6, 4.5 Hz, 6H), 1.18 (t, J = 7.6 Hz, 3H), 1.35 - 1.57 (m, 3H), 1.82 - 2.01 (m, 1H), 2.11 - 2.20 (m, 2H), 2.52 - 2.76 (m, 7H), 3.19 (d, J = 1.2 Hz, 3H), 3.32 (s, 8H), 4.05 - 4.12 (m, 2H), 4.40 (d, J = 1.5 Hz, 1H), 6.97-7.04 (m, 2H), 7.17-7.24 (m, 2H), 7.37 (d, J = 8.9 Hz, 1H), 7.67 (dd, J = 8.8, 2.5 Hz, 1H), 8.00 (d, J = 2.4 Hz, 1H). |
| | Composé 37 | |
| | | MS : [M+H] = 525 |
| **Exemple 20** | 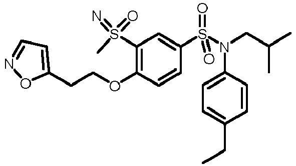 | **N-(4-éthylphényl)-N**-**isobutyl-4-(2-(isoxazol-5-yl)éthoxy)-3-(S-methyl-sulfonimidoyl)benzene-sulfonamide** |
| | | ¹H NMR (DMSO-d6) δ: 0.85 (dd, J = 11.0, 6.7 Hz, 6H), 1.17 (t, J = 7.6 Hz, 3H), 1.43 (t, J = 7.2 Hz, 1H), 2.29 - 2.38 (m, 2H), 2.60 (d, J = 7.6 Hz, 2H), 3.22 - 3.39 (m, 4H), 3.54 (d, J = 2.5 Hz, 3H), 4.64 (s, 1H), 6.83 (s, 1H), 6.94 - 7.04 (m, 2H), 7.22 (d, J = 8.4 Hz, 4H), 7.64 (d, J = 2.3 Hz, 1H), 7.82 (dd, J = 8.9, 2.4 Hz, 1H) |
| | Composé 38 | |
| | | MS : [M+H] = 506 |
| **Exemple 21** | 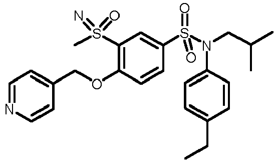 | **N-(4-éthylphényl)-N-isobutyl-3-(S-méthylsulfon-imidoyl)-4-(pyridin-4-ylméthoxy)-benzene-sulfonamide** |
| | | ¹H NMR (DMSO-d6) δ: 0.86 (dd, J = 6.7, 4.3 Hz, 7H), 1.19 (t, J = 7.6 Hz, 3H), 1.36 - 1.51 (m, 1H), 2.61 (q, J = 7.6 Hz, 2H), 3.15 (d, J = 1.2 Hz, 3H), 3.24 - 3.32 (m, 2H), 4.51 (d, J = 1.5 Hz, 1H), 5.47 (d, J = 2.5 Hz, 2H), 6.97 - 7.04 (m, 2H), 7.17 - 7.24 (m, 2H), 7.44 - 7.54 (m, 2H), 7.73 |
| | Composé 39 | (dd, J = 8.7, 2.5 Hz, 1H), 8.00 (dt, J = 7.9, 2.0 Hz, 1H), 8.03 (d, J = 2.4 Hz, 1H), 8.59 (dd, J = 4.8, 1.6 Hz, 1H), 8.79 (d, J = 2.5 Hz, 1H). |
| | | MS : [M+H] = 502 |
| **Exemple 22** | 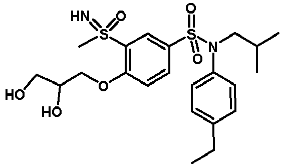 | **4-(2,3-dihydroxypropoxy)-N-(4-éthylphényl)-N-isobutyl-3-(S-méthyl-sulfonimidoyl)benzene-sulfonamide** |
| | | ¹H NMR (DMSO-d6) δ: 0.85 (dd, J = 6.6, 3.5 Hz, 6H), 1.18 (t, J = 7.6 Hz, 3H), 1.37 - 1.49 (m, 1H), 2.61 (q, J = 7.6 Hz, 2H), 3.24 (t, J = 1.4 Hz, 3H), 3.25 - 3.31 (m, 2H), 3.53 (t, J = 5.8 Hz, 2H), 3.89 (dd, J = 10.2, 5.1 Hz, 1H), 4.11 - 4.34 (m, 2H), 4.41 (dd, J = 8.9, 1.4 Hz, 1H), 4.74 (dt, J |
| | Composé 40 | = 7.7, 5.7 Hz, 1H), 5.08 (d, J = 4.9 Hz, 1H), 6.97 - 7.04 (m, 2H), 7.17 - 7.24 (m, 2H), 7.39 (dd, J = 8.9, 2.1 Hz, 1H), 7.64 - 7.73 (m, 1H), 7.95 - 8.00 (m, 1H). |
| | | MS : [M+H] = 485 |
| **Exemple 23** | 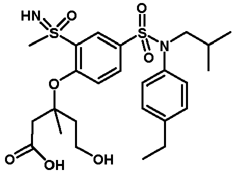 | **Acide 3-(4-(N-(4-éthylphényl)-N-isobutyl-sulfamoyl)-2-(S-methylsulfonimidoyl)-phénoxy)-5-hydroxy-3-methylpentanoique** |
| | | ¹H NMR (DMSO-d6) δ: 0.85 (t, J = 5.4 Hz, 7H), 1.17 (t, J = 7.6 Hz, 5H), 1.36 - 1.48 (m, 1H), 2.60 (q, J = 7.5 Hz, 2H), 3.14 (d, J = 4.9 Hz, 3H), 3.20 - 3.29 (m, 2H), 4.39 - 4.71 (m, 5H), 5.26 (s, 1H), |
| | Composé 41 | 6.99 (d, J = 7.2 Hz, 2H), 7.20 (d, J = 8.0 Hz, 2H), 7.40 (dt, J = 8.5, 4.3 Hz, 1H), 7.65 - 7.76 (m, 1H), 7.99 (dd, J = 14.4, 2.4 Hz, 1H). |
| | | MS : [M+H] = 541 |
| **Exemple 24** | 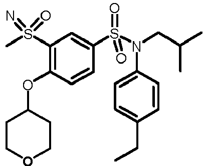 | **N-(4-éthylphényl)-N-isobutyl-3-(S-méthylsulfon-imidoyl)-4-((tétrahydro-2H-pyran-4-yl)oxy)-benzenesulfonamide** |
| | | ¹H NMR (DMSO-d6) δ: 0.85 (dd, J = 6.6, 3.8 Hz, 6H), 1.19 (t, J = 7.6 Hz, 3H), 1.36 - 1.51 (m, 1H), 1.68 - 1.81 (m, 2H), 1.97 - 2.07 (m, 2H), 2.61 (q, J = 7.6 Hz, 2H), 3.21 (d, J = 1.2 Hz, 3H), 3.32 (m,2H, 3.50 - 3.61 (m, 2H), 3.87 - 3.92 (m, 2H), 4.41 (d, J = 1.5 Hz, 1H), 5.00 (dt, J = 7.3, 3.6 Hz, 1H), 6.98 - 7.05 (m, 2H), 7.17 - 7.24 (m, 2H), 7.46 (d, J = 8.9 Hz, 1H), 7.63 (dd, J = 8.8, 2.4 Hz, 1H), 8.02 (d, J = 2.5 Hz, 1H) |
| | Composé 42 | |
| | | MS : [M+H] = 495 |
| **Exemple 25** | 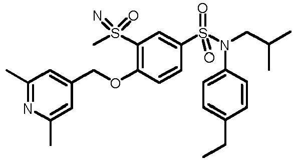 | **4-((2,6-diméthylpyridin-4-yl)méthoxy)-N-(4-éthylphényl)-N**-**isobutyl-3-(S-methylsulfon-imidoyl)benzenesulfonamide** |
| | | 1H NMR (DMSO-d6) δ: 0.85 (dd, J = 6.6, 4.0 Hz, 6H), 1.18 (t, J = 7.6 Hz, 3H), 1.36 - 1.49 (m, 1H), 2.44 (s, 6H), 2.50 - 2.66 (m, 3H), 3.21 (d, J = 1.1 Hz, 3H), 3.32 (m,2H), 4.55 (d, J = 1.4 Hz, 1H), 5.40 (s, 2H), 6.95 - 7.04 (m, 2H), 7.16 - 7.24 (m, 4H), 7.40 (d, J = 8.8 Hz, 1H), 7.71 (dd, J = 8.7, 2.4 Hz, 1H), 8.04 (d, J = 2.4 Hz, 1H) |
| | Composé 43 | |
| | | MS : [M+H] = 530 |
| **Exemple 26** | 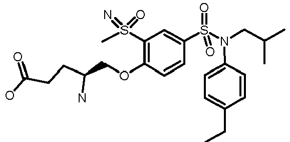 | **Acide ((4S)-4-amino-5-(4-(N-(4-éthylphenyl)-N-iso butylsulfamoyl)-2-(S-méthylsulfonimidoyl)-phénoxy)pentanoique** |
| | | ¹H NMR (DMSO-d6) δ: 0.84 (d, J = 6.4 Hz, 7H), 1.18 (t, J = 7.6 Hz, 3H), 1.42 (dt, J = 13.7, 6.8 Hz, 1H), 1.74 (dd, J = 14.3, 7.3 Hz, 1H), 1.88 (dt, J = 14.3, 6.9 Hz, 1H), 2.30 - 2.41 (m, 2H), 2.61 (q, J = 7.6 Hz, 2H), 3.01 (d, J = 5.9 Hz, 3H), 3.25 (d, J = 7.3 Hz, 3H), 3.50 (s, 2H), 3.68 (s, 1H), 4.67 (d, J = 23.1 Hz, 1H), 4.95 (d, J = 17.8 Hz, 1H), 7.01 (d, J = 8.1 Hz, 3H), 7.20 (d, J = 8.0 Hz, 2H), 7.47 (dd, J = 8.8, 2.9 Hz, 1H), 7.67 -7.81 (m, 2H), 12.12 (s, 1H) |
| | - Composé 44 | |
| | | MS: [M+H] = 526 |
| **Exemple 27** | 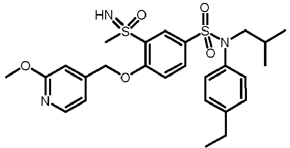 | **N-(4-éthylphényl)-N-isobutyl-4-((2-méthoxy-pyridin-4-yl)methoxy)-3-(S-méthylsulfonimidoyl)-benzenesulfonamide** |
| | | ¹H NMR (DMSO-d6) δ: 0.89 (ddd, J = 10.8, 6.5, 3.9 Hz, 6H), 1.23 (t, J = 7.6 Hz,3H), 1.42 - 1.54 (m, 1H), 2.66 (q, J = 7.7 Hz, 2H), 3.25 (s, 3H), 3.28 - 3.35 (m, 2H), 3.92 (s, 3H), 4.61 (s, 1H), 5.50 (s, 2H), 7.05 (d,J = 8.1 Hz,3H), 7.25 (d, J = 8.0 Hz, 2H), 7.46 (d, J = 8.9 Hz, 1H), 7.57 - 7.73 (m, 2H), 7.76 (dd, J = 8.9, 2.4 Hz, 1H), 8.09 (d, J= 2.6 Hz, 1H), 8.26 (d, J = 5.3 Hz, 1H). |
| | Composé 77 | |
| | | MS : [M+H] = 532 |
| **Exemple 28** | 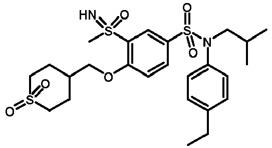 | **N-(4-éthylphényl)-N-isobutyl-4-((2-méthoxy- pyridin-4-yl)méthoxy)-3-(S-méthylsulfonimidoyl)-benzenesulfonamide** |
| | | ¹H NMR (DMSO-d6) δ: 0.85 (dd, J = 6.6, 4.7 Hz, 6H), 1.18 (t, J = 7.6 Hz, 3H), 1.36 - 1.50 (m, 1H), 1.88 (t, J = 12.8 Hz, 2H), 2.15 - 2.30 (m,3H),2.61 (q, J = 7.6Hz, 2H), 3.07 - 3.18 (m, 2H), 3.19 (d, J =1.2 Hz, 3H), 3.20 - 3.32 (m, 4H), 4.18 (dt, J = 6.0,3.6 Hz, 2H), 4.44 (d, J = 1.5 Hz, 1H), 6.97 - 7.04 (m, 2H), 7.17 - 7.24 (m, 2H), 7.38 (d, J = 8.7 Hz, 1H), 7.68 (dd, J = 8.8, 2.4 Hz, 1H), 8.01 (d, J=2.4 Hz, 1H). |
| | Composé 78 | |
| | | MS: [M+H] = 557 |
| **Exemple 29 CD13325** | 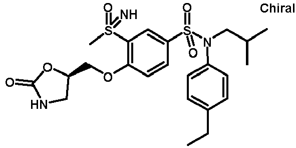 | **N-(4-éthylphényl)-N-isobutyl-3-(S-méthyl-sulfonimidoyl)-4-(((R)-2-oxooxazolidin-5-yl)-méthoxy)benzenesulfonamide** |
| | | ¹H NMR (DMSO-d6) δ: 0.81 - 0.89 (m, 6H), 1.18 (t, J = 7.6 Hz, 3H), 1.35 - 1.50 (m, 1H), 2.61 (q, J = 7.6 Hz, 2H), 3.18 (dd, J = 4.2, 1.2 Hz, 3H), 3.22 - 3.32 (m, 2H), 3.59 (dt, J = 32.3, 8.2 Hz, 2H), 4.30 - 4.45 (m, 2H), 4.46 - 4.57 (m, 1H), 5.01 (dt, J = 4.8,2.3 Hz, 1H), 6.96 - 7.04 (m, 2H), 7.17 - 7.24 (m, 2H), 7.42 (dd, J = 8.8,2.2 Hz, 1H), 7.65 -7.75 (m,2H), 8.00 (dd, J = 10.9, 2.4 Hz, 1H). |
| | Composé 79 | |
| | | MS: [M+H] = 510 |

### Exemple 30 : Synthèse du composé 4-((2,4-difluorobenzyl)oxy)-N-(4-éthylphényl)-N-isobutyl-3-(S-méthyl-sulfonimidoy-benzene-sulfonamide

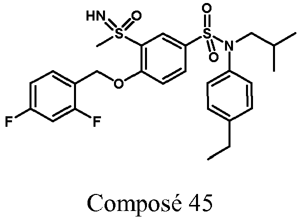

Le 4-bromo-N-(4-éthylphényl)-N-isobutyl-3-(S-méthylsulfonimidoyl)benzenesulfonamide (50.0 mg; 0.11 mmol) est ajouté à un mélange comprenant du 2,4-difluorobenzyl alcool (30.5 mg; 0.21 mmol) et du carbonate de césium (103.2 mg; 0.32 mmol) en solution dans du N,N-diméthylformamide (0.50 ml) après 20 minutes d'agitation. Le milieu réactionnel est agité pendant 20 heures à une température de 80°C, hydrolysé à froid puis extrait à l'acétate d'éthyle. Les phases organiques sont rassemblées, lavées avec une solution saturée de chlorure de sodium, séchées (sulfate de sodium) et concentrées à sec.

Le produit brut est purifié par HPLC préparative (colonne C18, éluant : acétonitrile dans eau/0.1% d'acide formique). Le 4-((2,4-difluoro-benzyl)oxy)-N-(4-ethylphenyl)-N-isobutyl-3-(S-methylsulfonimidoyl)benzenesulfonamide (9.4 mg; 16%) est obtenu sous la forme d'un solide blanc.
¹H NMR (DMSO-d6) δ: 0.86 (dd, J = 6.8, 4.4 Hz, 6H), 1.19 (t, J = 7.6 Hz, 3H), 1.33 - 1.53 (m, 1H), 2.62 (t, J = 7.6 Hz, 2H), 3.12 (s, 3H), 4.47 (s, 1H), 5.42 (s, 2H), 7.01 (d, J = 8.1 Hz, 2H), 7.21 (d, J = 8.0 Hz, 3H), 7.38 (t, J = 9.2 Hz, 1H), 7.55 (d, J = 8.8 Hz, 1H), 7.72 (dd, J = 8.7,2.5 Hz, 1H), 7.80 (q, J = 8.2 Hz, 1H), 8.03 (d, J = 2.5 Hz, 1H).
MS : [M+H] = 537

### Exemple 31 : Synthèse du composé 4-(4-cyanophénoxy)-N-(4-éthylphényl)-N-isobutyl-3-(S-méthylsulfonimidoyl)-benzenesulfonamide

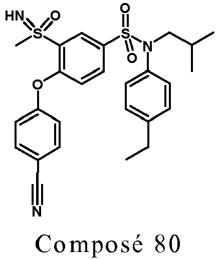

L'hydrure de sodium 60% (6.3 mg; 0.16 mmol) est ajouté à du (1,1-dioxo-hexahydro-1λ⁶-thiopyran-4-yl)-méthanol 4-cyanophénol (13.8 mg; 0.12 mmol) se trouvant en solution dans le N,N-diméthylformamide (1.0 ml). Le milieu réactionnel est agité pendant 20 minutes avant d'ajouter le 4-bromo-N-(4-éthylphényl)-N-isobutyl-3-(S-methylsulfonimidoyl)benzenesulfonamide (50.0 mg; 0.11 mmol).

Le milieu réactionnel est agité pendant 1 heure à température ambiante pendant puis pendant 16 heures à une température de 80°C. Le milieu réactionnel hydrolysé par addition d'eau à froid puis extrait à l'acétate d'éthyle. Les phases organiques sont rassemblées puis lavées à la saumure, séchées (Na₂SO₄) et concentrées.

Le produit brut est chromatographié sur gel de silice, éluant heptane/acétate d'éthyle de 0 à 100% d'acétate d'éthyle. Le 4-(4-cyanophenoxy)-N-(4-ethylphenyl)-N-isobutyl-3-(S-methylsulfonimidoyl)benzenesulfonamide (14.5 mg; 24%) est obtenu sous la forme d'un solide blanc.
¹H NMR (DMSO-d6) δ: 0.87 (dd, J = 6.6, 3.9 Hz, 6H), 1.18 (t, J = 7.6 Hz, 3H), 1.21 -1.31 (m, 2H), 1.39 -1.51 (m, 1H), 2.57 - 2.66 (m, 2H), 3.26 (s, 3H), 3.33 - 3.39 (m, 2H), 4.66 (d, J = 1.5 Hz, 1H), 7.00 - 7.07 (m, 2H), 7.20 - 7.31 (m, 3H), 7.30 -7.38 (m, 2H), 7.72 (dd, J = 8.6, 2.4 Hz, 1H), 7.93 - 8.00 (m, 2H), 8.09 (d, J = 2.4 Hz, 1H).
MS : [M+H] = 512

### Partie III : Synthèse des sulfonamides soufrés à partir du schéma réactionnel 3

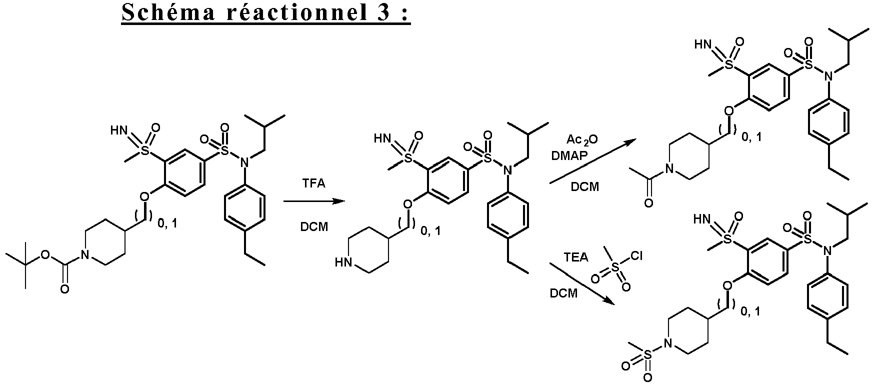

### Exemple 32 : Synthèse du N-(4-éthylphényl)-N-isobutyl-3-(S-méthylsulfonimidoyl)-4-(pipéridin-4-ylméthoxy)-benzenesulfonamide

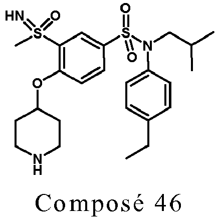

L'acide trifluoroacétique (0.2 ml; 2.61 mmol) est ajouté à du 4-(4-(N-(4-éthylphényl)-N-isobutylsulfamoyl)-2-(S-méthylsulfonimidoyl)phénoxy)piperidine-1-carboxylate de tert-butyle (40.0 mg; 0.07 mmol) en solution dans du dichlorométhane (1.6 ml). Le milieu réactionnel est agité pendant 1 heure à température ambiante, concentré, dilué au dichlorométhane, lavé avec une solution saturée d'hydrogénocarbonate de sodium puis une solution saturée de chlorure de sodium et séchées (Na₂SO₄). Les solvants sont évaporés.

Le produit brut est purifié par HPLC préparative (colonne C18, éluant : acétonitrile dans eau/0.1% d'acide formique). Le N-(4-ethylphenyl)-N-isobutyl-3-(S-methylsulfonimidoyl)-4-(piperidin-4-yloxy)benzenesulfonamide (32.1 mg; 97 %) est obtenu sous la forme d'une poudre blanche.
Mélange de conformères: ¹H NMR (DMSO-d6) δ: 0.86 (dd, J = 6.6, 1.5 Hz, 6H), 1.20 (t, J = 7.6 Hz, 3H), 1.46 - 1.61 (m, 1H), 1.77 - 1.96 (m, 2H), 1.96 - 2.15 (m, 2H), 2.57 - 2.68 (m, 2H), 2.77 - 2.96 (m, 2H), 3.20 (s, 3H), 3.28 - 3.38 (m, 2H), 3.98 - 4.21 (m, 1H), 4.75 - 5.03 (m, 1H), 6.98 - 7.06 (m, 2H), 7.16 - 7.23 (m, 2H), 7.41 (d, J = 8.8 Hz, 1H), 7.64 - 7.72 (m, 1H), 8.05 (d, J = 2.5 Hz, 1H).
MS : [M+H] = 494

### Exemple 33 : Synthèse du 4-((1-acétylpipéridin-4-yl)oxy)-N-(4-éthylphényl)-N-isobutyl-3-(S-méthylsulfonimidoyl)benzenesulfonamide

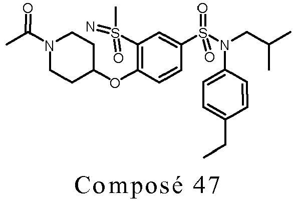

A une solution de N-(4-éthylphényl)-N-isobutyl-3-(S-methylsulfonimidoyl)-4-(piperidin-4-yloxy)benzenesulfonamide (65.0 mg; 0.13 mmol) dans du dichlorométhane (2 ml) refroidi à une température de -10°C sont ajoutés de la 4-diméthylaminopyridine (1.6 mg; 0.01 mmol) et de l'anhydride acétique (11.2 µl; 0.12 mmol).

Le milieu réactionnel est agité pendant 2 heures à température ambiante. Le milieu réactionnel est hydrolysé par ajout d'une solution saturée d'hydrogénocarbonate de sodium puis extrait à l'acétate d'éthyle. Les phases organiques sont rassemblées, lavées avec une solution saturée de chlorure de sodium, séchées (Na₂SO₄ et concentrées à sec.

Le produit brut est purifié par HPLC préparative (colonne C18, éluant : acétonitrile dans eau/0.1% d'acide formique). Le 4-((1-acetylpiperidin-4-yl)oxy)-N-(4-éthylphényl)-N-isobutyl-3-(S-methylsulfonimidoyl)benzenesulfonamide(34.8 mg; 49%) est obtenu sous la forme d'un solide blanc.
¹H NMR (DMSO-d6) δ: 0.86 (dd, J = 6.6, 2.3 Hz, 6H), 1.19 (t, 3H), 1.36 - 1.51 (m, 1H), 1.89 - 2.07 (m, 5H), 2.61 (q, J = 7.6 Hz, 2H), 2.87 (s, 3H), 3.20 (d, J = 1.2 Hz, 3H), 3.23 - 3.31 (m, 5H), 4.47 (d, J = 1.5 Hz, 1H), 5.06 (t, J = 4.1 Hz, 1H), 6.98 - 7.05 (m, 2H), 7.18 - 7.25 (m, 2H), 7.47 (d, J = 9.0 Hz, 1H), 7.67 (dd, J = 8.7, 2.4 Hz, 1H), 8.00 (d, J = 2.4 Hz, 1H).
MS : [M+H] = 536

### Exemple 34 : Synthèse du N-(4-éthylphényl)-N-isobutyl-3-(S-méthylsulfonimidoyl)-4-((1-(méthylsulfonyl)-piperidin-4-yl)oxy)benzenesulfonamide

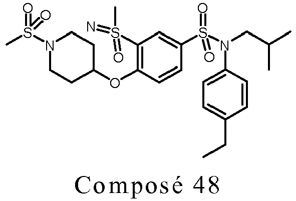

A une solution de N-(4-éthylphényl)-N-isobutyl-3-(S-méthylsulfonimidoyl)-4-(pipéridin-4-ylmethoxy)benzenesulfonamide (65.0 mg; 0.13 mmol) dans du dichlorométhane (1.3ml) sont ajoutés de la triéthylamine (18.3 µl; 0.13 mmol) et du chlorure de méthanesulfonyle (10.2 µl; 0.13 mmol).

Le milieu réactionnel est agité pendant 2 heures à température ambiante, hydrolysé par addition d'une solution saturée d'hydrogénocarbonate de sodium et extrait à l'acétate d'éthyle. Les phases organiques sont rassemblées, lavées avec une solution saturée de chlorure de sodium, séchées sur sulfate de sodium et concentrées à sec.

Le produit brut est purifié par HPLC préparative (colonne C18, éluant : acétonitrile dans eau/0.1% d'acide formique).Le N-(4-ethylphenyl)-N-isobutyl-3-(S-methylsulfonimidoyl)-4-((1-(methylsulfonyl)-piperidin-4-yl)oxy)benzenesulfonamide (24.8 mg; 32%) est obtenu sous la forme d'un solide blanc.
¹H NMR (DMSO-d6) δ: 0.86 (dd, J = 6.7, 3.4 Hz, 6H), 1.19 (t, J = 7.6 Hz, 3H), 1.36 - 1.51 (m, 1H), 1.81 - 1.88 (m, 2H), 1.99 (d, J = 9.4 Hz, 1H), 2.03 (s, 3H), 2.61 (q, J = 7.6 Hz, 2H), 3.19 (d, J = 1.2 Hz, 3H), 3.22 - 3.31 (m, 2H), 3.43 - 3.52 (m, 1H), 3.53 - 3.74 (m, 3H), 4.42 (dd, J = 3.3, 1.5 Hz, 1H), 5.02 - 5.09 (m, 1H), 6.98 - 7.06 (m, 2H), 7.17 - 7.25 (m, 2H), 7.47 (d, J = 8.9 Hz, 1H), 7.65 (dd, J = 8.8,2.4 Hz, 1H), 8.02 (d, J = 2.4 Hz, 1H)).
MS : [M+H] = 572

### Exemple 35 : Synthèse du N-(4-éthylphényl)-N-isobutyl-3-(S-méthylsulfonimidoyl)-4-(pipéridin-4-ylméthoxy)-benzenesulfonamide

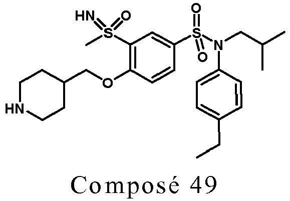

Avec un mode opératoire analogue à celui décrit pour l'exemple 31, le N-(4-éthylphényl)-N-isobutyl-3-(S-méthylsulfonimidoyl)-4-(pipéridin-4-ylméthoxy)benzenesulfonamide (68.2 mg; 100 %) est obtenu sous la forme d'une poudre blanche.
¹H NMR (DMSO-d6) δ: 0.77 (dd, J = 6.6, 4.4 Hz, 6H), 1.11 (t, J = 7.6 Hz, 3H), 1.14 - 1.24 (m, 2H), 1.24 - 1.43 (m, 1H), 1.70 (d, J = 13.3 Hz, 2H), 1.82 - 1.89 (m, 1H), 2.45 - 2.51 (m, 2H), 2.51 - 2.58 (m, 2H), 2.93 (dt, J = 12.1, 3.3 Hz, 2H), 3.11 (s, 3H), 3.18 (dd, J = 13.0, 7.1 Hz, 3H), 3.99 (dd, J = 6.3, 2.3 Hz, 2H), 4.32 (s, 1H), 6.93 (d, J = 8.4 Hz, 2H), 7.13 (d, J = 8.4 Hz, 2H), 7.30 (d, J = 8.8 Hz, 1H), 7.58 (dd, J = 8.8, 2.5 Hz, 1H), 7.92 (d, J = 2.4 Hz, 1H).
MS : [M+H] = 508

### Exemple 36 : Synthèse du 4-((1-acétylpipéridin-4-yl)méthoxy)-N-(4-éthylphényl)-N-isobutyl-3-(S-méthylsulfonimidoyl)benzenesulfonamide

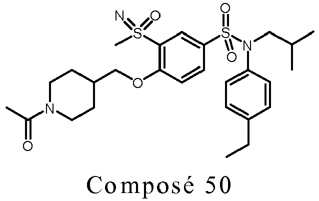

Avec un mode opératoire analogue à celui décrit pour l'exemple 32, le 4-((1-acétylpipéridin-4-yl)méthoxy)-N-(4-éthylphényl)-N-isobutyl-3-(S-méthylsulfonimidoyl)benzenesulfonamide (6.5 mg; 10 %) est obtenu sous la forme d'un solide blanc.
¹H NMR (DMSO-d6) δ: 0.85 (dd, J = 6.7, 4.2 Hz, 6H), 1.08 - 1.23 (m, 5H), 1.22 - 1.35 (m, 2H), 1.43 (dt, J = 13.4, 6.8 Hz, 1H), 1.88 (d, J = 26.7 Hz, 2H), 2.01 (s, 3H), 2.09 - 2.14 (m, 1H), 2.61 (q, J = 7.6 Hz, 3H), 3.08 (t, J = 12.8 Hz, 1H), 3.18 (s, 3H), 3.22 - 3.30 (m, 2H), 3.87 (d, J = 13.6 Hz, 1H), 4.11 (q, J = 4.4 Hz, 2H), 4.42 (d, J = 11.5 Hz, 2H), 7.01 (d, J = 7.9 Hz, 2H), 7.21 (d, J = 8.2 Hz, 2H), 7.38 (d, J = 8.8 Hz, 1H), 7.67 (dd, J = 8.8, 2.4 Hz, 1H), 8.00 (d, J = 2.4 Hz, 1H).
MS : [M+H] = 550

### Exemple 37: Synthèse du N-(4-éthylphényl)-N-isobutyl-3-(S-methylsulfonimidoyl)-4-((1-(methylsulfonyl-piperidin-4-yl)méthoxy)benzenesulfonamide

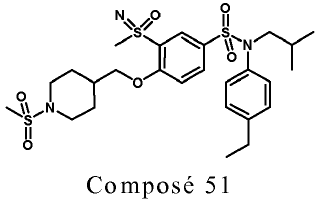

Avec un mode opératoire analogue à celui décrit pour l'exemple 33, le N-(4-éthylphényl)-N-isobutyl-3-(S-méthylsulfonimidoyl)-4-((1-(méthylsulfonyl)pipéridin-4-yl)méthoxy)benzenesulfonamide (17.2 mg; 25 %) est obtenu sous la forme d'un solide blanc.
¹H NMR (DMSO-d6) δ: 0.85 (dd, J = 6.7, 4.3 Hz, 6H), 1.19 (t, J = 7.6 Hz, 3H), 1.37 - 1.51 (m, 3H), 1.89 - 2.04 (m, 3H), 2.61 (q, J = 7.7 Hz, 2H), 2.72 - 2.83 (m, 2H), 2.88 (s, 3H), 3.18 (s, 3H), 3.28 (d, J = 7.8 Hz, 2H), 3.62 (d, J = 11.5 Hz, 2H), 4.09 - 4.20 (m, 2H), 4.42 (s, 1H), 7.01 (d, J = 8.0 Hz, 2H), 7.21 (d, J = 8.1 Hz, 2H), 7.39 (d, J = 8.8 Hz, 1H), 7.68 (dd, J = 8.7, 2.5 Hz, 1H), 8.00 (d, J = 2.5 Hz, 1H).
MS : [M+H] = 586

### Exemple 38 : Synthèse du N-(4-éthylphényl)-N-isobutyl-3-(S-methylsulfonimidoyl)-4-[(tetrahydropyran-4-ylmethyl)-amino]-benzenesulfonamide

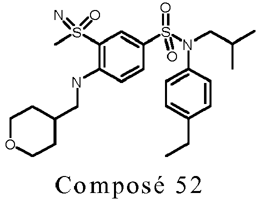

Un mélange de 4-bromo-N-(4-éthylphényl)-N-isobutyl-3-(S-méthylsulfonimidoyl)benzenesulfonamide (15.0 mg; 0.03 mmol) et de 4-aminométhyltetrahydropyran (7.5 µl; 0.06 mmol) est agité pendant une nuit à température ambiante.

Le produit brut est purifié par HPLC préparative (colonne C18, éluant : acétonitrile dans eau/0.1% d'acide formique). Le N-(4-éthylphényl)-N-isobutyl-3-(S-méthylsulfonimidoyl)-4-[(tétrahydropyran-4-ylméthyl)-amino]-benzenesulfonamide (9.0 mg; 56%) est obtenu sous la forme d'un solide blanc.
¹H NMR(DMSO-d6, 400 MHz): δ (ppm) 0.84 (dd, J=6.7, 2.6 Hz, 6H), 1.18 (t, J=7.7 Hz, 3H), 1.28 (dd, J=14.5, 10.3 Hz, 2H), 1.37 - 1.46 (m,1H), 1.66 (d, J=13.3 Hz, 2H), 1.87 (s, 1H), 2.61 (q, J=7.4 Hz, 2H), 3.01 (s, 3H), 3.17 (q, J=6.8, 6.4 Hz, 2H), 3.24 (dd, J=7.4, 3.3 Hz, 2H), 3.89 (dd, J=11.6, 4.0 Hz, 2H), 4.74 (s, 1H), 6.95 (d, J=8.9 Hz, 1H), 7.00 (d, J=7.9 Hz, 2H), 7.20 (d, J=8.1 Hz, 2H), 7.50 (d, J=9.0 Hz, 1H), 7.70 (d, J=2.3 Hz, 1H), 7.84 (t, J=5.5 Hz, 1H).
MS : [M+H] = 508

### Exemple 39 : Synthèse du N-(4-éthylphényl)-N-isobutyl-4-(méthyl((tétrahydro-2H-pyran-4-yl)méthyl)amino)-3-(S-méthylsulfonimidoyl)benzenesulfonamide

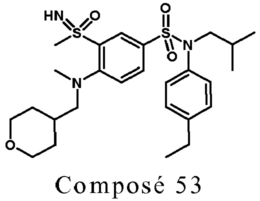

Une solution de 4-bromo-N-(4-éthylphényl)-N-isobutyl-3-(S-méthylsulfonimidoyl)benzenesulfonamide (15.0 mg; 0.03 mmol) dans du N,N-diméthylformamide (0.20 ml) est agité pendant une nuit à une température de 50°C.

Le produit brut est purifié par HPLC préparative (colonne C18, éluant : acétonitrile dans eau/0.1% d'acide formique). Le N-(4-éthylphényl)-N -isobutyl-4-(méthyl((tétrahydro-2H-pyran-4-yl)méthyl)amino)-3-(S-méthylsulfonimidoyl)benzenesulfonamide (9.0 mg; 49%) est obtenu sous la forme d'un film sec incolore.
¹H NMR (DMSO-d6) δ: 0.85 (dd, J = 6.6, 3.2 Hz, 6H), 1.18 (t, J = 7.6 Hz, 3H), 1.44 (dt, J = 13.7, 6.8 Hz, 1H), 1.58 (d, J = 13.4 Hz, 2H), 1.93 (s, 1H), 2.61 (q, J = 7.6 Hz, 2H), 2.82 (s, 3H), 2.93 - 3.10 (m, 2H), 3.26 (d, J = 1.3 Hz, 3H), 3.28 - 3.30 (m, 2H), 3.80 - 3.87 (m, 2H), 4.50 (d, J = 1.5 Hz, 1H), 7.00 (d, J = 8.4 Hz, 2H), 7.20 (d, J = 8.3 Hz, 2H), 7.56 (d, J = 8.6 Hz, 1H), 7.61 (dd, J = 8.6, 2.2 Hz, 1H), 8.13 (d, J = 2.3 Hz, 1H).
MS : [M+H] = 522

### Exemple 40 : Synthèse du N-(4-éthylphényl)-N-isobutyl-3-(S-méthylsulfonimidoyl)-4-((oxetan-3-ylmethyl)amino-benzenesulfonamide

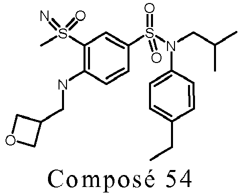

Au 4-bromo-N-(4-éthylphényl)-N-isobutyl-3-(S-méthylsulfonimidoyl)benzenesulfonamide (50.00 mg; 0.11 mmol) en solution dans N,N-diméthylformamide (250µl) sont ajoutés le 3-aminométhyloxetane hydrochlorure (33 mg; 0.26 mmol) et la triéthylamine (51 µl; 0.37 mmol). Le milieu réactionnel est agité une nuit à 50°C.

Le produit brut est purifié directement par HPLC préparative (colonne C18, éluant : acétonitrile dans eau/0.1% d'acide formique). Le N-(4-éthylphényl)-N-isobutyl-3-(S-méthylsulfonimidoyl)-4-((oxetan-3-ylméthyl)amino)benzenesulfonamide (14.0 mg; 26 %) est obtenu sous la forme d'un solide blanc.
¹H NMR (DMSO-d6) δ: 0.84 (dd, J = 6.7, 2.2 Hz, 6H), 1.18 (t, J = 7.6 Hz, 3H), 1.35 - 1.50 (m, 1H), 2.61 (q, J = 7.7 Hz, 2H), 3.00 (s, 3H), 3.25 (d, J = 6.9 Hz, 2H), 3.58 (dt, J = 14.5, 7.1 Hz, 2H), 4.35 (td, J = 6.0, 2.8 Hz, 2H), 4.70 (td, J = 6.4,3.3 Hz, 3H), 6.99 (dd, J = 8.5, 5.0 Hz, 3H), 7.20 (d, J = 7.8 Hz, 2H), 7.51 (dd, J = 8.8, 2.5 Hz, 1H), 7.71 (d, J = 2.5 Hz, 1H), 7.82 (t, J = 5.5 Hz, 1H).
MS : [M+H] = 480

Avec un mode opératoire analogue à celui décrit pour l'exemple 40, on obtient :

| | | |
|---|---|---|
| **Exemple 41** | | **N-(4-ethylphenyl)-N-isobutyl-3-(S-methyl-sulfonimidoyl)-4-(((4-methyltetrahydro-2H-pyran-4-yl)methyl)amino)benzenesulfonamide** |
| | 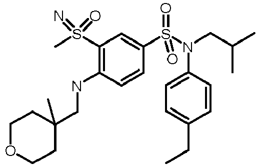 | ¹H NMR (DMSO-d6) δ: 0.84 (dd, J = 6.7,3.3 Hz, 6H), 1.09 (s, 3H), 1.18 (t, J = 7.6 Hz, 3H), 1.31 -1.38 (m, 2H), 1.38 - 1.47 (m, 1H), 1.54 (ddt, J = 14.2, 9.8, 5.0 Hz, 2H), 2.61 (q, J = 7.7 Hz, 2H), 3.01 (s, 3H), 3.07 - 3.21 (m, 2H), 3.24 (p, J = 5.6 Hz, 2H), 3.51 - 3.60 (m, 2H), 3.68 (dt,J = 11.8,4.4 Hz, 2H), 4.84 (s, 1H), 7.00 (d, J = 8.1 Hz, 3H), 7.20 (d, J = 7.9 Hz, 2H), 7.49 (dd, J = 8.9, 2.4 Hz, 1H), 7.69 (d, J = 2.5 Hz, 1H), 7.98 (t, J = 5.1 Hz, 1H). MS: [M+H] = 522 |
| | Composé 55 | |
| **Exemple 42** | | **4-(((1,1-dioxidotetrahydrothiophen-3-yl)-methyl)-amino)-N-(4-ethylphenyl)-N-isobutyl-3-(S-methylsulfonimidoyl)benzene-sulfonamide** |
| | 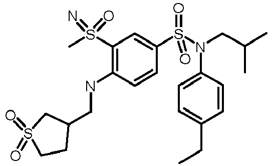 | ¹H NMR (DMSO-d6) δ: 0.84 (dd, J = 6.6, 2.7 Hz, 6H), 1.18 (t, J = 7.6 Hz, 3H), 1.38 - 1.50 (m, 1H), 1.89 (d, J = 22.1 Hz, 1H), 2.61 (q, J = 7.6 Hz, 2H), 2.90 (t, J = 12.1 Hz, 1H), 3.03 (d, J = 1.5 Hz, 3H), 3.25 (dd, J = 7.5, 3.9 Hz, 2H), 3.44 (t, J = 6.3 Hz, 2H), 4.73 (d, J = 6.3 Hz, 1H), 7.01 (dd, J = 8.6, 5.9 Hz, 3H), 7.20 (d, J = 8.3 Hz, 2H), 7.49 (dd, J = 8.9, 2.4 Hz, 1H), 7.72 (d, J = 2.3 Hz, 1H), 7.85 (s, 1H). MS : [M+H] = 542 |
| | Composé 56 | |
| **Exemple 43** | 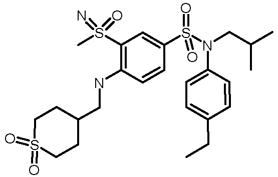 | **4-(((1,1-dioxidotetrahydro-2H - thiopyran-4-yl)-methyl)amino)-N-(4-ethylphenyl)-N-isobutyl-3-(S-methylsulfonimidoyl)benzenesulfona mide** |
| | | ¹H NMR (DMSO-d6) δ: 0.84 (dd, J = 6.7, 2.2 Hz, 6H), 1.18 (t, J = 7.6 Hz, 3H), 1.42 (dt, J = 13.5, 6.8 Hz, 1H), 1.71 (q, J= 12.2 Hz, 2H), 1.91-1.98 (m, 1H), 2.06-2.14 (m, 2H), 2.61 (q, J = 7.6 Hz, 2H), 3.02 (s, 3H), 3.10 (dd, J = 28.9, 13.4 Hz, 2H), 3.23 - 3.28 (m, 4H), 4.74 (d,J= 1.1 Hz, 1H), 6.97-7.02 (m, 3H), 7.20 (d, J = 8.3 Hz, 2H), 7.50 (dd, J = 8.9,2.4 Hz, 1H), 7.71 (d,J = 2.3 Hz, 1H), 7.84 (t, J = 5.8 Hz, 1H). MS: [M+H] = 556 |
| | Composé 57 | |
| **Exemple 44** | | **N-(4-éthylphényl)-N-isobutyl-3-(S-méthyl-sulfonimidoyl)-4-(((6-oxopiperidin-3-yl)-méthyl)amino)benzenesulfonamide** |
| | 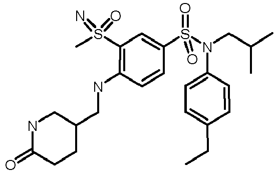 | ¹H NMR (DMSO-d6) δ: 0.84 (dd, J = 6.6, 2.5 Hz, 6H), 1.18 (t, J = 7.6 Hz, 3H), 1.42 (dt, J = 13.6, 6.8 Hz, 1H), 1.47 - 1.59 (m, 1H), 1.83-1.93 (m, 1H), 2.02-2.11 (m, 1H), 2.16-2.25 (m, 2H), 2.60 (q, J = 7.7 Hz, 2H), 2.90 - 2.98 (m, 1H), 3.02 (s, 3H), 3.23 - 3.29 (m, 3H), 4.75 (s, 1H), 6.97 -7.04 (m, 3H), 7.17 - 7.23 (m, 2H), 7.44 - 7.53 (m, 2H), 7.71 (dd, J= 2.3, 1.1 Hz, 1H), 7.83 (s, 1H). MS : [M+H] = 521 |
| | Composé 58 | |
| **Exemple 45** | | **N-(4-éthylphényl)-N-isobutyl-3-(S-méthyl-sulfonimidoyl)-4-(((5-oxopyrrolidin-3-yl)-méthyl)amino)benzenesulfonamide** |
| | 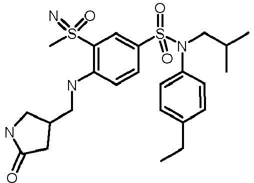 | ¹H NMR (DMSO-d6) δ: 0.84 (dd, J = 6.6, 2.7 Hz, 6H), 1.18 (t, J = 7.6 Hz, 3H), 1.42 (dt, J = 13.5, 6.8 Hz, 1H), 2.00 (ddd, J = 16.6, 6.8, 3.6 Hz, 1H), 2.29 (d, J = 8.8 Hz, 1H), 2.60 (q,J= 7.7 Hz, 2H), 2.68 - 2.78 (m, 1H), 3.02 (s, 3H), 3.24 (dd, J = 7.3,3.6 Hz, 2H), 3.33 - 3.44 (m, 2H), 4.74 (s, 1H), 6.97 (s, 1H), 7.00 (d, J = 8.1 Hz, 2H), 7.16 - 7.23 (m, 2H), 7.50 (dd, J = 8.9, 2.4 Hz, 1H), 7.59 (s, 1H), 7.71 (d, J = 2.3 Hz, 1H), 7.83 (d, J= 5.0 Hz, 1H). MS: [M+H] = 507 |
| | Composé 59 | |
| **Exemple 46** | | **N-(4-éthylphényl)-N-isobutyl-3-(S-méthyl-sulfonimidoyl)-4-(((R)-1-(tétrahydro-2H-pyran-4-yl)éthyl)amino) benzenesulfonamide** |
| | 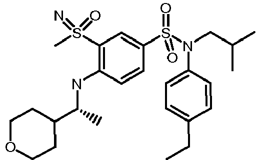 | ¹H NMR (DMSO-d6) δ: 0.84 (dd, J = 6.7, 3.0 Hz, 6H), 1.10 - 1.23 (m, 6H), 1.26 - 1.40 (m, 1H), 1.42 (p, J = 7.0 Hz, 2H), 1.57 (d, J = 13.1 Hz, 2H), 1.62 - 1.79 (m, 1H), 2.61 (q, J = 7.6 Hz, 2H), 2.99 (dd, J = 4.0, 1.0 Hz, 3H), 3.19 - 3.30 (m, 4H), 3.56 - 3.69 (m, 1H), 3.84 - 3.95 (m, 2H), 4.78 (d, J= 4.6 Hz, 1H), 6.94 - 7.02 (m, 1H), 7.01 (d, J = 8.4 Hz, 2H), 7.16 - 7.25 (m, 2H), 7.43 - 7.53 (m,1H), 7.70 (t, J = 2.0 Hz, 1H), 7.80 - 7.90 (m, 1H). MS: [M+H] = 522 |
| | Composé 60 | |
| **Exemple 47** | | **N-(4-éthylphényl)-N-isobutyl-3-(S-méthyl-sulfonimidoyl)-4-(((S)-1-(tétrahydro-2H-pyran-4-yl)éthyl)amino)benzenesulfonamide** |
| | 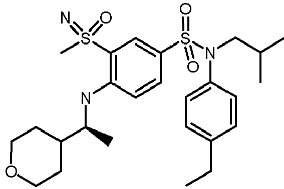 | ¹H NMR (DMSO-d6) δ: 0.84 (dd, J = 6.7, 3.0 Hz, 6H), 1.10 - 1.23 (m, 6H), 1.24 - 1.48 (m, 2H), 1.57 (d, J = 13.0 Hz, 1H), 1.62 -1.77 (m, 3H), 2.61 (q,J = 7.6 Hz, 2H), 2.99 (dd, J = 4.0, 1.0 Hz, 3H), 3.21 - 3.30 (m, 4H), 3.60 (d, J = 20.4 Hz, 1H), 3.84 - 3.97 (m, 2H), 4.75 - 4.82 (m, 1H), 6.94 - 7.01 (m, 1H), 7.01 (d, J = 8.4 Hz, 2H), 7.16 -7.24 (m, 2H), 7.43 - 7.52 (m, 1H), 7.67 - 7.73 (m, 1H), 7.80-7.88 (m, 1H). MS: [M+H] = 522 |
| | Composé 81 | |
| **Exemple 48** | 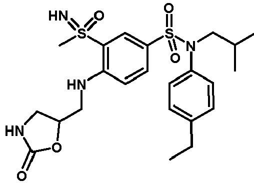 | **N-(4-éthylphényl)-N-isobutyl-3-(S-methylsulfonimidoyl)-4-(((2-oxooxazolidin-5-yl)méthyl)amino)benzenesulfonamide** |
| | | ¹H NMR (DMSO-d6) δ: 0.81 - 0.87 (m, 6H), 1.18 (t, J = 7.6 Hz, 3H), 1.42 (p, J = 6.6 Hz, 1H), 2.60 (q, J = 7.6 Hz, 2H), 3.02 (t, J= 1.1 Hz, 3H), 3.22-3.28 (m, 3H), 3.60 (ddt, J = 11.1,6.9, 3.9 Hz, 2H), 4.55 (s, 1H), 4.73 (d, J = 11.6 Hz, 1H), 4.82 (s, 1H), 6.94 - 7.04 (m, 2H), 7.06 (d,J = 8.9 Hz, 1H), 7.20 (d,J = 8.2 Hz, 2H), 7.51 (dt, J = 8.9, 2.1 Hz, 1H), 7.60 (s, 1H), 7.72 (dd, J = 3.4, 2.3 Hz, 1H), 7.86-8.01 (m,1H). MS: [M+H] = 509 |
| | Composé 82 | |
| **Exemple 49** | | **4-((1,1-dioxidotétrahydro-2H-thiopyran-4-yl)-amino)-N-(4-éthylphényl)-N-isobutyl-3-(S-méthylsulfonimidoyl)benzenesulfona mide** |
| | 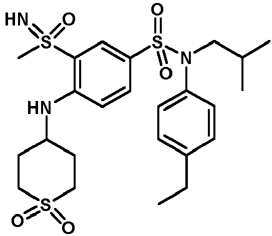 | ¹H NMR (Chloroforme-d) δ: 0.90 (dd, J =9.1, 6.6 Hz, 6H), 1.25 (d,J = 8.2 Hz, 4H), 2.31 (s, 2H), 2.44 (s, 2H), 2.58 - 2.66 (m, 2H), 3.04 (s, 3H), 3.07 - 3.23 (m, 4H), 3.24 - 3.37 (m, 2H), 3.75 (s, 1H), 6.67 (d, J = 8.9 Hz, 1H), 7.00 (d, J = 8.3 Hz, 2H), 7.14 (d, J = 8.0 Hz, 2H), 7.58 (dd, J= 8.8, 2.2 Hz, 1H), 8.03 (d, J =2.2 Hz, 1H). MS : [M+H] = 542 |
| | Composé 83 | |
| **Exemple 50** | | **4-(((1-acétylpiperidin-4-yl)methyl)amino)-N**-(**4-éthylphényl)-**N-**isobutyl-3-(S-méthylsulfonimidoyl)benzenesulfonamide** |
| | 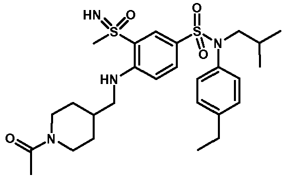 | ¹H NMR (Chloroforme-d) δ: 0.92 (dd, J = 8.9, 6.7 Hz, 6H), 1.22 - 1.28 (m, 4H), 1.59 (hept, J = 6.8 Hz, 1H), 1.90 (dddd, J = 26.2, 18.2, 8.2, 4.9 Hz, 4H), 2.12 (s, 3H), 2.65 (q, J = 7.8 Hz, 2H), 3.04 (s, 3H), 3.09 - 3.17 (m, 4H), 3.24 (dd, J = 12.8, 6.9 Hz, 1H), 3.34 (dd, J = 12.7, 7.7 Hz, 1H), 3.89 (dd, J = 13.2, 3.5 Hz, 1H), 4.66 - 4.76 (m, 1H), 6.68 (d, J = 9.0 Hz, 1H), 7.01 (d, J = 8.0 Hz, 2H), 7.15 (d, J = 8.0 Hz, 2H), 7.55 (dd, J = 8.8, 2.0 Hz, 1H), 7.65 (q, J = 4.4, 3.9 Hz, 1H), 7.99 (d, J = 2.2 Hz, 1H). MS : [M+H] = 549 |
| | Composé 84 | |
| **Exemple 51** | | **N-(4-éthylphényl)-N-isobutyl-3-(S-méthyl-sulfonimidoyl)-4-((6-oxopiperidin-3-yl)-amino) benzenesulfonamide** |
| | 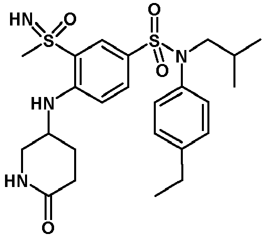 | ¹H NMR (Chloroforme-d) δ: 0.90 (dd, J = 9.2,6.6 Hz, 6H), 1.19 -1.27 (m, 5H), 1.58 (hept, J = 7.0 Hz, 1H), 2.55 (s, 2H), 2.64 (d, J = 7.9 Hz, 2H), 3.03 (s, 3H), 3.29 (ddd, J = 39.3, 12.4, 6.9 Hz, 3H), 3.67 (s, 1H), 3.87 - 3.99 (m, 1H), 6.02 (s, 1H), 6.74 (d, J = 8.4 Hz, 1H), 7.00 (d, J= 7.6 Hz, 2H), 7.14 (d, J= 7.6 Hz, 2H), 7.56 (d,J = 7.6 Hz, 1H), 7.92 (s, 1H), 8.02 (d, J= 5.8 Hz, 1H). MS: [M+H] = 507 |
| | Composé 85 | |
| **Exemple 52** | | **4-((1,1-dioxidotetrahydrothiophen-3-yl-)amino)-N**-(**4-éthylphényl)-N-isobutyl-3-(S-méthylsulfonimidoyl)benzenesulfona mide** |
| | 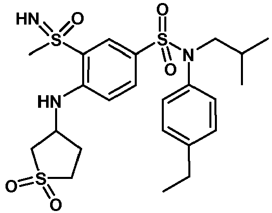 | ¹H NMR (DMSO-d6) δ: 0.84 (ddd, J = 6.6,2.3, 1.0 Hz, 6H), 1.18 (t, J= 7.6 Hz, 3H), 1.42 (dt, J = 13.6, 6.7 Hz, 1H), 2.12 - 2.24 (m,1H), 2.61 (q,J = 7.5 Hz, 2H), 3.04 (d, J = 2.6 Hz, 3H), 3.07 - 3.21 (m, 1H), 3.23 - 3.27 (m, 2H), 3.65 (ddd, J = 20.2, 13.3, 7.0 Hz, 1H), 4.44 - 4.56 (m, 1H), 4.74 (s, 1H), 4.80 (s, 1H), 7.00 (d, J= 8.3 Hz, 2H), 7.20 (d, J = 8.4 Hz, 2H), 7.54 (dt, J = 8.3, 1.4 Hz, 1H), 7.73 (dd, J = 4.0, 2.3 Hz, 1H), 7.98 (d, J = 7.1 Hz, 1H), 8.12 (d, J = 6.6 Hz, 1H). MS: [M+H] = 528 |
| | Composé 86 | |
| **Exemple 53** | 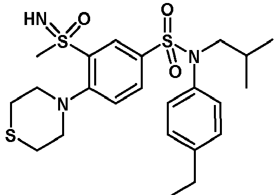 | **N**-(**4-éthylphényl)**-**N-isobutyl-3-(S-méthyl-sulfonimidoyl)-4-thiomorpholinobenzene-sulfonamide** |
| | | ¹H NMR (DMSO-d6) δ: 0.84 (ddd, J = 6.6,2.3, 1.0 Hz, 6H), 1.18 (t, J = 7.6 Hz, 3H), 1.42 (dt, J = 13.6, 6.7 Hz, 1H), 2.12 - 2.24 (m, 1H), 2.61 (q, J = 7.5 Hz, 2H), 3.04 (d, J = 2.6 Hz, 3H), 3.07 - 3.21 (m, 1H), 3.23 - 3.27 (m, 2H), 3.65 (ddd, J = 20.2, 13.3, 7.0 Hz, 1H), 4.44 - 4.56 (m, 1H), 4.74 (s, 1H), 4.80 (s, 1H), 7.00 (d, J = 8.3 Hz, 2H), 7.20 (d, J = 8.4 Hz, 2H), 7.54 (dt, J = 8.3, 1.4 Hz, 1H), 7.73 (dd, J = 4.0, 2.3 Hz, 1H), 7.98 (d, J =7.1 Hz, 1H), 8.12 (d, J = 6.6 Hz, 1H). MS: [M+H] = 528 |
| | Composé 87 | |
| **Exemple 54** | | **N-(4-éthylphényl)-N-isobutyl-4-(((4-méthyl-1,2,5-oxadiazol-3-yl)méthyl)amino)-3-(S-méthylsulfonimidoyl)benzenesulfona mide** |
| | 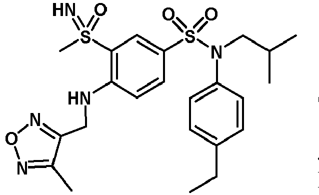 | ¹H NMR (DMSO-d6) δ: 0.85 (dd, J = 6.6, 3.5 Hz, 6H), 1.13 - 1.23 (m, 3H), 1.43 (dt, J = 13.6, 6.8 Hz, 1H), 2.61 (q, J = 7.6 Hz, 2H), 2.80 (dt, J = 6.8, 3.6 Hz, 4H), 3.25 (q, J = 5.9, 5.3 Hz, 4H), 3.31 (s, 3H), 4.53 -4.57 (m, 1H), 6.97-7.05 (m, 2H), 7.17 - 7.26 (m, 2H), 7.63 (d, J = 8.4 Hz, 1H), 7.71 (dd, J = 8.4, 2.3 Hz, 1H), 8.12 (d, J = 2.3 Hz, 1H). MS : [M+H] = 496 |
| | Composé 88 | |

### Exemple 55 : Synthèse du N-(4-éthyphény)-4-(((3-hydroxycylobutyl)méthyl)amino)-N-isobutyl-3-(S-méthyl-sulfonimidoy)benzenesulfonamide

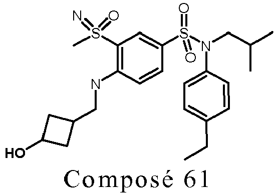

Au 4-bromo-N-(4-éthylphényl)-N-isobutyl-3-(S-méthylsulfonimidoyl)benzenesulfonamide (50.00 mg; 0.11 mmol) en solution dans le N,N-diméthylformamide (150 µl sont ajoutés le 3-aminomethyl-cyclobutanol hydrochlorure (36.33 mg; 0.26 mmol) et le carbonate de césium (120.43 mg; 0.37 mmol).

Le milieu réactionnel est agité pendant un week-end à une température de 50°C.

Le produit brut est purifié directement par HPLC préparative (colonne C18, éluant : acétonitrile dans eau/0.1% d'acide formique). Le N-(4-ethylphenyl)-4-(((3-hydroxycyclobutyl)methyl)amino)-N-isobutyl-3-(S-methylsulfonimidoyl)benzenesulfonamide (20.0 mg; 38%) est obtenu sous la forme d'un solide blanc.
¹H NMR (DMSO-d6) δ: 0.84 (dd, J = 6.7, 2.4 Hz, 6H), 1.18 (t, J = 7.6 Hz, 3H), 1.42 (dt, J = 13.5, 6.7 Hz, 1H), 1.56 (q, J = 8.7 Hz, 1H), 1.94 - 2.07 (m, 2H), 2.27 - 2.36 (m, 2H), 2.60 (q, J = 7.6 Hz, 2H), 2.99 (s, 3H), 3.24 (dd, J = 7.3, 3.5 Hz, 2H), 3.98 (dt, J = 14.2, 7.4 Hz, 1H), 4.70 (d, J = 1.3 Hz, 1H), 5.03 (t, J = 3.1 Hz, 1H), 6.91 (t, J = 8.6 Hz, 1H), 6.99 (dd, J = 8.4, 1.6 Hz, 2H), 7.17 - 7.23 (m, 2H), 7.50 (dt, J = 8.8, 2.9 Hz, 1H), 7.69 (d, J = 2.3 Hz, 1H).
MS : [M+H] = 494

Avec un mode opératoire analogue à celui décrit pour l'exemple 55, on obtient :

| | | |
|---|---|---|
| **Exemple 56** | 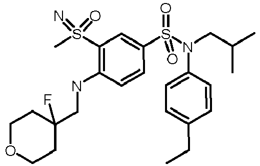 | **N-(4-éthylphényl)-4-(((4-fluorotétrahydro-2H-pyran-4-yl)méthyl)amino)-N-isobutyl-3-(S-méthylsulfonimidoyl)benzenesulfo namide** |
| | | ¹H NMR (DMSO-d6) δ: 0.84 (dd, J = 6.7, 2.4 Hz, 6H), 1.18 (t, J = 7.6 Hz, 3H), 1.42 (dt, J = 13.6, 6.8 Hz, 1H), 1.81 (tq, J = 19.3, 9.1 Hz, 4H), 2.09 (s, 1H), 2.60 (q, J = 7.6 Hz, 2H), 3.02 (d, J = 0.9 Hz, 3H), 3.25 (dd, J = 7.3, 3.1 Hz, 2H), 3.51 - 3.63 (m, 4H), 3.74 - 3.82 (m, 2H), 4.76 - 4.83 (m, 1H), 7.00 (d, J = 8.3 Hz, 2H), 7.06 (d, J = 8.9 Hz, 1H), 7.17 - 7.22 (m, 2H), 7.50 (dd, J = 8.9, 2.3 Hz, 1H), 7.72 (d, J = 2.3 Hz, 1H), 8.01 (t, J = 5.8 Hz, 1H) |
| | Composé 62 | |
| | | MS : [M+H] = 526 |
| **Exemple 57** | 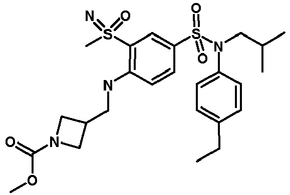 | **3-(((4-(N-(4-éthylphényl)-N-isobutyl-sulfamoyl)-2-(S-méthylsulfonimidoyl)-phényl)amino)méthyl)azetidine-1-carboxylate de méthyle** |
| | | ¹H NMR (DMSO-d6) δ: 0.84 (dd, J = 6.7, 2.3 Hz, 6H), 1.11 - 1.23 (m, 3H), 1.42 (dt, J = 13.6, 6.7 Hz, 1H), 2.55 - 2.66 (m, 2H), 2.99 (s, 3H), 3.25 (ddd, J = 17.8, 9.7, 4.2 Hz, 4H), 3.51 (q, J = 6.5 Hz, 1H), 3.56 (s, 3H), 3.67 (dt, J = 12.1, 6.8 Hz, 2H), 4.00 (p, J = 8.0 Hz, 2H), 4.69 (s, 1H), 6.95 - 7.05 (m, 3H), 7.16 - 7.24 (m, 2H), 7.50 (dd, J = 8.9, 2.3 Hz, 1H), 7.71 (d, J = 2.4 Hz, 1H), 7.81 (t, J = 5.5 Hz, 1H). |
| | Composé 89 | |
| | | MS : [M+H] = 537 |
| **Exemple 58** | 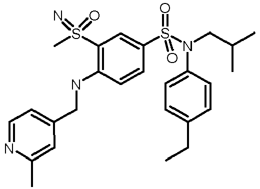 | **N-(4-éthylphényl)-N-isobutyl-4-(((2-méthyl-pyridin-4-yl)méthyl)amino)-3-(S-méthyl-sulfonimidoyl)benzenesulfonamide** |
| | | ¹H NMR (DMSO-d6) δ: 0.83 (dd, J = 6.6, 2.0 Hz, 6H), 1.17 (t, J = 7.6 Hz, 3H), 1.41 (dt, J = 13.6, 6.9 Hz, 1H), 2.45 (s, 3H), 2.59 (q, J = 7.7 Hz, 2H), 3.10 (d, J = 1.0 Hz, 3H), 3.24 (dd, J = 7.4, 2.7 Hz, 2H), 4.58 (d, J = 6.1 Hz, 2H), 4.79 (s, 1H), 6.74 (d, J = 8.9 Hz, 1H), 6.95 - 7.00 (m, 2H), 7.13 - 7.23 (m, 4H), 7.45 (dd, J = 8.8, 2.3 Hz, 1H), 7.76 (d, J = 2.3 Hz, 1H), 8.19 (t, J = 6.0 Hz, 1H), 8.40 (d, J = 5.3 Hz, 1H). |
| | Composé 90 | |
| | | MS : [M+H] = 515 |
| **Exemple 59** | 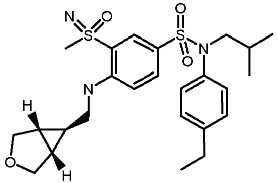 | **4-((((1R,5S,6s)-3-oxabicyclo[3.1.0]hexan-6-yl)méthyl)amino)-N-(4-éthylphényl)-N-isobutyl-3-(S-methylsulfonimidoyl)benzenesulfonamide** |
| | | ¹H NMR (DMSO-d6) δ: 0.84 (dd, **J =** 6.7, 2.1 Hz, 6H), 1.00 (dt, J = 7.0, 3.6 Hz, 1H), 1.18 (t, J = 7.6 Hz, 3H), 1.42 (dt, J = 13.6, 6.8 Hz, 1H), 1.69 (hept, J = 3.5 Hz, 2H), 2.56 - 2.66 (m, 2H), 3.02 (d, J = 0.9 Hz, 3H), 3.25 (dd, J = 7.4, 2.5 Hz, 2H), 3.58 (dd, J = 8.2, 2.5 Hz, 2H), 3.74 (dd, J = 8.3, 1.7 Hz, 2H), 4.71 (d, J = 1.3 Hz, 1H), 6.91 (d, J = 9.0 Hz, 1H), 6.95 - 7.01 (m, 2H), 7.15 - 7.25 (m, 2H), 7.50 (dd, J = 8.8, 2.3 Hz, 1H), 7.70 (d, J = 2.3 Hz, 1H), 7.77 (t, J = 5.1 Hz, 1H). |
| | Composé 91 | |
| | | MS : [M+H] = 506 |
| **Exemple 60** | 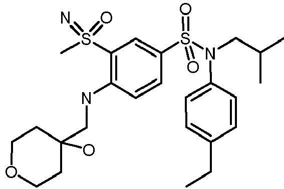 | **N-(4-éthylphényl)-4-(((4-hydroxytétrahydro-2H-pyran-4-yl)méthyl)amino)-N-isobutyl-3-(S-méthylsulfonimidoyl)benzenesulfo namide** |
| | | ¹H NMR (DMSO-d6) δ: 0.84 (dd, J = 6.7, 1.9 Hz, 6H), 1.18 (t, J = 7.6 Hz, 3H), 1.42 (dt, J = 13.6, 6.8 Hz, 1H), 1.51 (d, J = 13.3 Hz, 2H), 1.62 (dt, J = 15.5, 8.6 Hz, 2H), 2.61 (q, J = 7.6 Hz, 2H), 3.02 (d, J = 0.8 Hz, 3H), 3.20 - 3.30 (m, 2H), 3.59 - 3.70 (m, 2H), 4.69 (d, J = 1.1 Hz, 1H), 4.80 (s, 1H), 6.93 - 7.03 (m, 3H), 7.16 - 7.23 (m, 2H), 7.48 (dd, J = 8.9, 2.4 Hz, 1H), 7.72 (d, J = 2.3 Hz, 1H), 7.88 (t, J = 5.1 Hz, 1H). |
| | Composé 92 | |
| | | MS : [M+H] = 524 |
| **Exemple 61** | 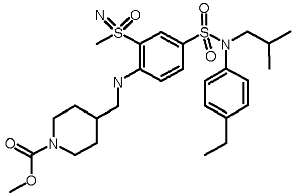 | **4-(((4-(N-(4-éthylphényl)-N-isobutyl-sulfamoyl)-2-(S-méthylsulfonimidoyl)phenyl)-amino)méthyl)piperidine-1-carboxylate de méthyle** |
| | | ¹H NMR (DMSO-d6) δ: 0.84 (dd, J = 6.7, 2.5 Hz, 6H), 1.18 (t, J = 7.6 Hz, 3H), 1.41 (dq, J = 13.8, 6.9 Hz, 1H), 1.73 (d, J = 13.3 Hz, 4H), 1.76 - 1.88 (m, 1H), 2.60 (q, J = 7.6 Hz, 2H), 3.01 (d, J = 1.0 Hz, 3H), 3.13 - 3.20 (m, 2H), 3.24 (dd, J = 7.2, 3.3 Hz, 2H), 3.59 (s, 3H), 3.93 - 4.10 (m, 4H), 4.74 (d, J = 1.3 Hz, 1H), 6.95 (d, J = 8.9 Hz, 1H), 6.97 - 7.02 (m, 2H), 7.17 - 7.23 (m, 2H), 7.50 (dd, J = 8.9, 2.4 Hz, 1H), 7.70 (d, J = 2.3 Hz, 1H), 7.83 (t, J = 5.4 Hz, 1H). |
| | Composé 93 | |
| | | MS : [M+H] = 565 |
| **Exemple 62** | 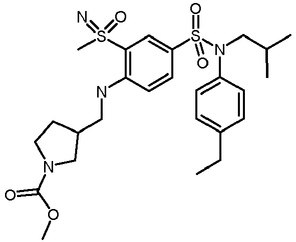 | **3-(((4-(N-(4-éthylphényl)-N-isobutyl-sulfamoyl)-2-(S-méthylsulfonimidoyl)phényl)-amino)méthyl)pyrrolidine-1-carboxylate de méthyle** |
| | | ¹H NMR (DMSO-d6) δ: 0.84 (dd, J = 6.6, 2.5 Hz, 6H), 1.18 (t, J = 7.6 Hz, 3H), 1.41 (dt, J = 13.5, 6.8 Hz, 1H), 1.58 - 1.76 (m, 1H), 1.93 - 2.08 (m, 2H), 2.60 (q, J = 7.6 Hz, 2H), 3.02 (s, 3H), 3.07 (td, J = 8.5, 7.9, 3.9 Hz, 1H), 3.24 (dd, J = 7.3, 3.2 Hz, 4H), 3.43 (td, J = 9.6, 8.2, 4.3 Hz, 1H), 3.50 (ddd, J = 10.2, 7.3, 2.3 Hz, 1H), 3.58 (s, 3H), 4.75 (s, 1H), 6.96 - 7.03 (m, 3H), 7.17 - 7.23 (m, 2H), 7.50 (ddd, J = 8.9, 2.3, 1.2 Hz, 1H), 7.70 (d, J = 2.2 Hz, 1H), 7.83 (s, 1H). |
| | Composé 94 | |
| | | MS : [M+H] = 551 |
| **Exemple 63** | 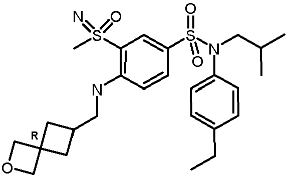 | **4-(((2-oxaspiro[3.3]heptan-6-yl)méthyl)amino)-N-(4-éthylphényl)-N-isobutyl-3-(S-méthyl-sulfonimidoyl)benzenesulfonamide** |
| | | ¹H NMR (DMSO-d6) δ: 0.84 (dd, J = 6.6, 2.4 Hz, 6H), 1.18 (t, J = 7.6 Hz, 3H), 1.40 (dq, J = 13.7, 6.9 Hz, 1H), 1.96 (ddd, J = 10.0, 5.7, 2.0 Hz, 2H), 2.28 - 2.46 (m, 3H), 2.60 (q, J = 7.6 Hz, 2H), 2.99 (d, J = 0.9 Hz, 3H), 3.22 (dtd, J = 14.0, 7.6, 7.0, 5.1 Hz, 4H), 4.49 (s, 2H), 4.59 (s, 2H), 4.71 (d, J = 1.1 Hz, 1H), 6.89 (d, J = 9.0 Hz, 1H), 6.97 - 7.02 (m, 2H), 7.17 - 7.21 (m, 2H), 7.49 (dd, J = 8.9, 2.3 Hz, 1H), 7.69 (d, J = 2.3 Hz, 1H). |
| | Composé 95 | |
| | | MS : [M+H] = 520 |
| **Exemple 64** | 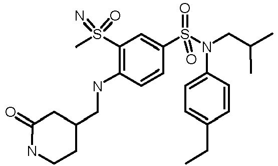 | **4-N-(4-éthylphényl)-N-isobutyl-3-(S-méthyl-sulfonimidoyl)-4-(((2-oxopiperidin-4-yl)-méthyl)amino)benzenesulfonamide** |
| | | ¹H NMR (DMSO-d6) δ: 0.84 (dd, J = 6.6, 2.7 Hz, 6H), 1.18 (t, J = 7.6 Hz, 3H), 1.42 (dq, J = 13.9, 7.0 Hz, 2H), 1.86 (d, J = 13.6 Hz, 1H), 1.96 (ddd, J = 16.8, 10.8, 3.4 Hz, 1H), 2.10 - 2.32 (m, 2H), 2.60 (q, J = 7.6 Hz, 2H), 3.02 (s, 3H), 3.13 (td, J = 12.4, 11.2, 6.5 Hz, 2H), 3.14 - 3.30 (m, 4H), 4.77 (s, 1H), 6.96 - 7.03 (m, 2H), 7.16 - 7.24 (m, 2H), 7.47 - 7.55 (m, 1H), 7.54 - 7.66 (m, 1H), 7.70 (dd, J = 2.4, 1.4 Hz, 1H), 7.86 (d, J = 5.9 Hz, 1H). |
| | Composé 96 | |
| | | MS : [M+H] = 521 |
| **Exemple 65** | 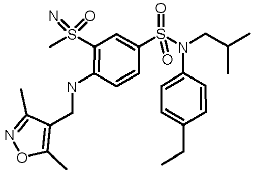 | **4-(((3,5-dimethylisoxazol-4-yl)methyl)amino)-N**-(**4-ethylphenyl)-N-isobutyl-3-(S-methyl-sulfonimidoyl)benzenesulfonamide** |
| | | ¹H NMR (DMSO-d6) δ: 0.84 (dd, J = 6.7, 2.2 Hz, 6H), 1.18 (t, J = 7.6 Hz, 3H), 1.41 (dq, J = 13.7, 6.9 Hz, 1H), 2.23 (s, 3H), 2.42 (s, 3H), 2.61 (q, J = 7.6 Hz, 2H), 3.00 (s, 3H), 3.26 (dd, J = 7.3, 2.9 Hz, 2H), 4.28 (t, J = 5.3 Hz, 2H), 4.75 (s, 1H), 6.95 (d, J = 8.8 Hz, 1H), 6.99 (d, J = 8.2 Hz, 2H), 7.20 (d, J = 8.1 Hz, 2H), 7.59 (dd, J = 8.7, 2.4 Hz, 1H), 7.72 (d, J = 2.3 Hz, 1H), 7.80 (t, J = 5.1 Hz, 1H). |
| | Composé 97 | |
| | | MS : [M+H] = 519 |
| **Exemple 66** | 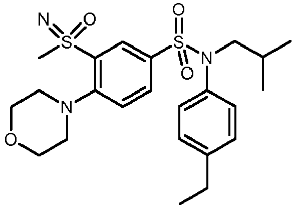 | **N-(4-éthylphényl)-4-(((4-hydroxycyclohexyl)-méthyl)amino)-N-isobutyl-3-(S-méthyl-sulfonimidoyl)benzenesulfonamide** |
| | | ¹H NMR (DMSO-d6) δ: 0.84 (dd, J = 6.7, 2.8 Hz, 6H), 1.01 - 1.10 (m, 2H), 1.18 (t, J = 7.6 Hz, 3H), 1.42 (dt, J = 13.7, 6.8 Hz, 1H), 1.53 (td, J = 18.2, 15.7, 6.5 Hz, 2H), 1.73 - 1.90 (m, 4H), 2.60 (q, J = 7.5 Hz, 2H), 3.00 (s, 3H), 3.08 (d, J = 6.8 Hz, 2H), 3.24 (dd, J = 7.5, 3.6 Hz, 2H), 4.53 (d, J = 4.5 Hz, 1H), 4.74 (s, 1H), 6.91 (d, J = 9.0 Hz, 1H), 7.00 (d, J = 8.1 Hz, 2H), 7.20 (d, J = 8.1 Hz, 2H), 7.49 (dd, J = 8.8, 2.5 Hz, 1H), 7.69 (d, J = 2.2 Hz, 1H), 7.81 (t, J = 5.4 Hz, 1H). |
| | Composé 123 | |
| | | MS : [M+H] = 522 |
| **Exemple 67** | 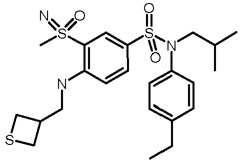 | **N-(4-éthylphényl)-N-isobutyl-3-(S-méthy-sulfonimidoyl)-4-((thietan-3-ylmethyl)amino)-benzenesulfonamide** |
| | | ¹H NMR (Chloroforme-d) δ: 0.93 (dd, J = 9.2, 6.8 Hz, 6H), 1.26 (t, J = 7.6 Hz, 3H), 1.62 (dq, J = 13.6, 7.0 Hz, 1H), 2.67 (q, J = 7.6 Hz, 2H), 3.04 (dd, J = 9.4, 6.0 Hz, 2H), 3.11 (s, 3H), 3.26 (dd, J = 12.9, 7.0 Hz, 1H), 3.32 - 3.44 (m, 2H), 3.49 (d, J = 7.0 Hz, 2H), 3.57 (h, J = 6.8, 6.4 Hz, 1H), 6.74 (d, J = 9.0 Hz, 1H), 7.02 (d, J = 8.1 Hz, 2H), 7.17 (d, J = 7.9 Hz, 2H), 7.58 (dd, J = 8.8, 2.3 Hz, 1H), 8.03 (d, J = 2.2 Hz, 1H). |
| | Composé 99 | |
| | | MS : [M+H] = 496 |
| **Exemple 68** | 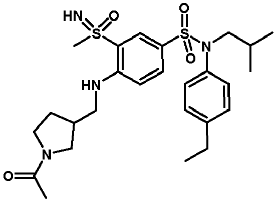 | **4-(((1-acétylpyrrolidin-3-yl)méthyl)amino)-N-(4-éthylphényl)-N-isobutyl-3-(S-méthyl-sulfonimidoyl)benzenesulfonamide** |
| | | ¹H 1H NMR (Chloroform-d) δ: 0.89 (dd, J = 8.8, 6.6 Hz, 6H), 1.23 (t, J = 7.9 Hz, 3H), 1.57 (hept, J = 6.8 Hz, 1H), 1.65 - 1.90 (m, 1H), 2.06 (d, J = 2.9 Hz, 3H), 2.21-2.24 (m, 2H), 2.63 (q, J = 7.7 Hz, 2H), 3.03 (d, J = 4.6 Hz, 3H), 3.27 (dtd, J = 20.1, 12.9, 7.5 Hz, 5H), 3.47 (dt, J = 19.3, 9.3 Hz, 1H), 3.55 - 3.83 (m, 2H), 6.60 - 6.72 (m, 1H), 6.98 (d, J = 7.8 Hz, 2H), 7.13 (d, J = 7.8 Hz, 2H), 7.54 (d, J = 8.3 Hz, 1H), 7.69 (s, 1H), 7.98 (dd, J = 4.5, 2.1 Hz, 1H). |
| | Composé 100 | |
| | | MS : [M+H] = 535 |
| **Exemple 71** | 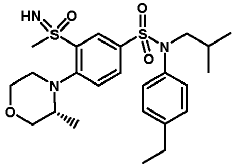 | **N-(4-éthylphényl)-N-isobutyl-4-((R)-3-méthylmorpholino)-3-(S-méthyl-sulfonimidoyl)benzenesulfonamide** |
| | | ¹H NMR (DMSO-d6) δ: 0.77 (dd, J = 6.3, 2.6 Hz, 3H), 0.82 - 0.88 (m, 6H), 1.18 (t, J = 7.6 Hz, 3H), 1.44 (dt, J = 13.7, 6.8 Hz, 1H), 2.61 (ddt, J = 8.7, 6.3, 3.4 Hz, 2H), 3.40 (dd, J = 17.2, 1.2 Hz, 3H), 3.44 - 3.50 (m, 1H), 3.68 (ddd, J = 11.1, 8.0, 3.0 Hz, 1H), 3.78 (ddt, J = 7.9, 5.2, 2.8 Hz, 1H), 3.87 (dd, J = 10.9, 2.4 Hz, 1H), 4.47 (d, J = 1.5 Hz, 1H), 4.62 (d, J = 1.5 Hz, 1H), 6.92 - 7.05 (m, 2H), 7.19 (dd, J = 8.4, 2.2 Hz, 2H), 7.68 (d, J = 1.3 Hz, 1H), 7.70 - 7.72 (m, 1H), 8.09 (d, J = 1.9 Hz, 1H), 8.25 (t, J= 1.3 Hz, 1H). |
| | | MS : [M+H] = 494 |
| **Exemple 73** | 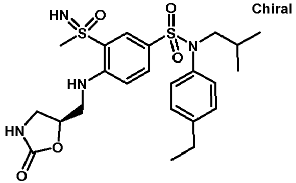 | **N-(4-éthylphényl)-N-isobutyl-3-(S-methyl-sulfonimidoyl)-4-((((R)-2-oxooxazolidin-5-yl)méthyl)amino)benzenesulfonami de** |
| | | ¹H NMR (Chloroform-d) δ: 0.90 (dd, J = 9.3, 6.7 Hz, 6H), 1.23 (t, J = 7.6 Hz, 3H), 1.59 (dq, J = 13.8, 6.8 Hz, 1H), 2.64 (q, J = 7.6 Hz, 2H), 3.06 (d, J = 2.6 Hz, 3H), 3.24 (ddd, J = 12.8, 7.0, 2.1 Hz, 1H), 3.34 (ddd, J = 12.9, 7.8, 3.3 Hz, 1H), 3.44 (ddd, J = 8.6, 5.9, 2.4 Hz, 1H), 3.53 - 3.68 (m, 1H), 3.82 (t, J = 8.7 Hz, 1H), 4.93 (qd, J = 6.0, 3.9 Hz, 1H), 5.07 (s, 1H), 6.75 (dd, J = 8.8, 3.0 Hz, 1H), 6.94 - 7.03 (m, 2H), 7.13 - 7.19 (m, 2H), 7.57 (dt, J = 8.8, 2.4 Hz, 1H), 8.01 (dd, J = 5.5, 2.2 Hz, 1H). |
| | | MS : [M+H] = 509 |
| **Exemple 74** | 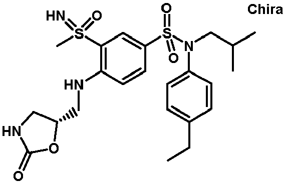 | **N-(4-éthylphényl)-N-isobutyl-3-(S-méthyl-sulfonimidoyl)-4-((((S)-2-oxooxazolidin-5-yl)méthyl)amino)benzenesulfonami de** |
| | | ¹H NMR (DMSO-d6) δ: 0.84 (dd, J = 6.6, 1.9 Hz, 6H), 1.18 (t, J = 7.6 Hz, 3H), 1.42 (dd, J = 13.7, 7.0 Hz, 1H), 1.52 (td, J = 17.8, 15.2, 7.1 Hz, 2H), 1.94 - 2.09 (m, 2H), 2.60 (q, J = 7.6 Hz, 2H), 3.01 (s, 3H), 3.24 (d, J = 7.4 Hz, 2H), 3.37 - 3.46 (m, 2H), 3.78 (d, J = 11.5 Hz, 2H), 4.69 (s, 1H), 6.99 (d, J = 8.2 Hz, 3H), 7.20 (d, J = 8.4 Hz, 2H), 7.47 (dd, J = 8.9, 2.4 Hz, 1H), 7.70 (d, J = 2.3 Hz, 1H), 7.85 (s, 1H). |
| | Composé 106 | |
| | | MS : [M+H] = 509 |
| **Exemple 75** | 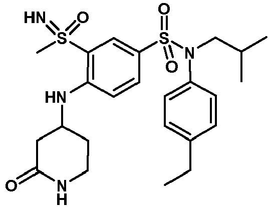 | **N-(4-éthylphényl)-N-isobutyl-3-(S-méthyl-sulfonimidoyl)-4-((2-oxopiperidin-4-yl)amino)benzenesulfonamide** |
| | | ¹H NMR (Chloroform-d) δ: 0.90 (d, J = 7.3 Hz, 6H), 1.17 - 1.27 (m, 3H), 1.45 - 1.68 (m, 2H), 2.57 - 2.68 (m, 2H), 3.01-3.4 (m, 2H), 3.19 - 3.40 (m, 2H), 3.48-3.52 (m, 1H), 3.99 (s, 1H), 6.03 (d, J = 40.9 Hz, 1H), 6.72 (s, 1H), 7.00 (d, J = 7.3 Hz, 2H), 7.15 (d, J = 7.3 Hz, 2H), 7.57 (s, 1H), 8.00 (s, 1H). |
| | Composé 107 | |
| | | MS : [M+H] = 508 |
| **Exemple 77** | 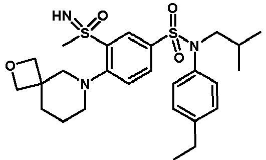 | **N-(4-éthylphényl)-N-isobutyl-3-(S-méthyl-sulfonimidoyl)-4-(2-oxa-6-azaspiro[3.5]nonan-6-yl)benzenesulfonamide** |
| | | ¹H NMR (DMSO-d6) δ: 0.86 (dd, J = 6.7, 4.1 Hz, 6H), 1.19 (t, J = 7.6 Hz, 3H), 1.44 (dt, J = 13.6, 6.8 Hz, 1H), 1.63 (q, J = 5.6 Hz, 2H), 1.83 (s, 2H), 2.61 (q, J = 7.6 Hz, 2H), 2.92 (d, J = 17.9 Hz, 2H), 3.17 (s, 2H), 3.27 (d, J = 1.2 Hz, 3H), 3.33 (s, 2H), 4.29 (d, J = 5.6 Hz, 2H), 4.42 - 4.62 (m, 3H), 6.97 - 7.05 (m, 2H), 7.18 - 7.25 (m, 2H), 7.64 - 7.75 (m, 2H), 8.15 (d, J = 2.0 Hz, 1H). |
| | Composé 109 | |
| | | MS : [M+H] = 520 |
| **Exemple 78** | 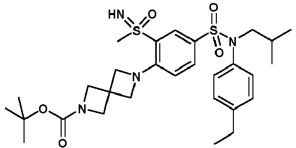 | **tert-butyl 6-(4-(N-(4-ethylphenyl)-N-isobutyl-sulfamoyl)-2-(S-methylsulfonimidoyl)-phenyl)-2,6-diazaspiro[3.3]heptane-2-carboxylate** |
| | | ¹H NMR (DMSO-d6) δ: 0.83 (dd, J = 6.6, 3.4 Hz, 6H), 1.18 (t, J = 7.6 Hz, 3H), 1.39 (s, 9H), 2.60 (q, J = 7.6 Hz, 2H), 3.12 (d, J = 1.5 Hz, 3H), 3.23 (dd, J = 7.3, 5.1 Hz, 2H), 4.05 (s, 4H), 4.20 (d, J = 1.7 Hz, 1H), 4.43 (s, 4H), 6.59 (d, J = 8.9 Hz, 1H), 6.94 - 7.01 (m, 2H), 7.15 - 7.23 (m, 2H), 7.42 (dd, J = 8.8, 2.4 Hz, 1H), 7.94 (d, J = 2.3 Hz, 1H). |
| | Composé 110 | |
| | | MS : [M+H] = 591 |
| **Exemple 79** | 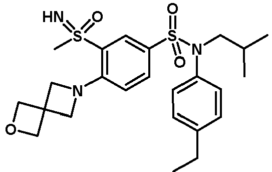 | **N-(4-ethylphenyl)-N-isobutyl-3-(S-methyl-sulfonimidoyl)-4-(2-oxa-6-azaspiro-[3.3]heptan-6-yl)benzenesulfonamide** |
| | | ¹H NMR (DMSO-d6) δ: 0.83 (dd, J = 6.4, 3.3 Hz, 6H), 1.18 (t, J = 7.6 Hz, 3H), 1.33 - 1.47 (m, 1H), 2.60 (q, J = 7.6 Hz, 2H), 3.12 (s, 3H), 3.24 (h, J = 6.7, 5.8 Hz, 3H), 4.20 (s, 1H), 4.48 (s, 4H), 4.73 (s, 4H), 6.61 (d, J = 8.8 Hz, 1H), 6.98 (d, J = 7.9 Hz, 2H), 7.19 (d, J = 8.2 Hz, 2H), 7.42 (dd, J = 8.8, 2.3 Hz, 1H), 7.93 (d, J = 2.4 Hz, 1H). |
| | Composé 111 | |
| | | MS : [M+H] = 493 |
| **Exemple 80** | 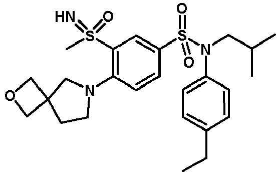 | **N-(4-ethylphenyl)-N-isobutyl-3-(S-methyl-sulfonimidoyl)-4-(2-oxa-6-azaspiro[3.4]octan-6-yl)benzenesulfonamide** |
| | | ¹H 1H NMR (DMSO-d6) δ: 0.90 (dd, J = 6.8, 3.4 Hz, 6H), 1.23 (t, J = 7.6 Hz, 3H), 1.41 - 1.55 (m, 1H), 2.00 (d, J = 5.7 Hz, 4H), 2.66 (q, J = 7.6 Hz, 2H), 2.89 - 3.09 (m, 4H), 3.36 (s, 3H), 4.43 (s, 4H), 4.57 (s, 1H), 7.04 (d, J = 8.0 Hz, 2H), 7.25 (d, J = 8.1 Hz, 2H), 7.62 (d, J = 8.4 Hz, 1H), 7.72 (dd, J = 8.3, 2.3 Hz, 1H), 8.18 (d, J = 2.3 Hz, 1H). |
| | Composé 112 | |
| | | MS : [M+H] = 407 |
| **Exemple 81** | 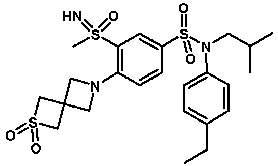 | **4-(2,2-dioxido-2-thia-6-azaspiro[3.3]heptan-6-yl)-N-(4-ethylphenyl)-N-isobutyl-3-(S-methyl-sulfonimidoyl)benzenesulfonamide** |
| | | ¹H NMR (DMSO-d6) δ: 0.84 (dd, J = 6.8, 3.4 Hz, 6H), 1.18 (t, J = 7.6 Hz, 3H), 1.34 - 1.49 (m, 1H), 2.60 (q, J = 7.6 Hz, 2H), 3.13 (s, 3H), 3.25 (dd, J = 7.4, 5.1 Hz, 2H), 4.28 (s, 1H), 4.52 (d, J = 4.5 Hz, 8H), 6.66 (d, J = 8.9 Hz, 1H), 6.98 (d, J = 8.1 Hz, 2H), 7.19 (d, J = 8.0 Hz, 2H), 7.45 (dd, J = 8.8, 2.3 Hz, 1H), 7.96 (d, J = 2.3 Hz, 1H). |
| | Composé 113 | |
| | | MS : [M+H] = 540 |
| **Exemple 82** | 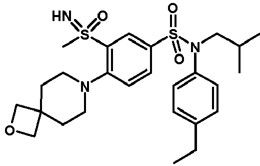 | **N-(4-ethylphenyl)-N-isobutyl-3-(S-methyl-sulfonimidoyl)-4-(2-oxa-7-azaspiro[3.5]nonan-7-yl)benzenesulfonamide** |
| | | ¹H NMR (DMSO-d6) δ: 0.84 (d, J = 6.7 Hz, 6H), 1.18 (t, J = 7.6 Hz, 3H), 1.35 - 1.50 (m, 1H), 2.16 - 2.30 (m, 2H), 2.61 (q, J = 7.5 Hz, 2H), 3.21 (s, 3H), 3.28 (d, J = 7.3 Hz, 2H), 3.42 - 3.62 (m, 2H), 3.68 - 3.83 (m, 2H), 4.21 (s, 1H), 4.54 (dd, J = 6.0, 3.6 Hz, 2H), 4.60 (dd, J = 14.2, 5.9 Hz, 2H), 7.01 (d, J = 7.9 Hz, 2H), 7.19 (dd, J = 8.5, 4.5 Hz, 3H), 7.49 (dd, J = 8.8, 2.4 Hz, 1H), 8.05 (d, J = 2.4 Hz, 1H). |
| | Composé 114 | |
| | | MS : [M+H] = 520 |
| **Exemple 83** | 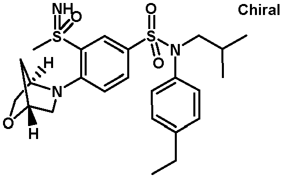 | **4-((1R,4R)-2-oxa-5-azabicyclo[2.2.1] heptan-5-yl)-N-(4-ethylphenyl)-N-isobutyl-3-(S-methyl-sulfonimidoyl)benzenesulfonamide** |
| | | ¹H 1H NMR (DMSO-d6) δ: 0.84 (dd, J = 6.7, 1.5 Hz, 6H), 1.18 (t, J = 7.6 Hz, 3H), 1.36 - 1.48 (m, 1H), 1.82 - 1.97 (m, 2H), 2.53 - 2.66 (m, 3H), 3.18 (dd, J = 7.4, 1.3 Hz, 3H), 3.28 (dd, J = 7.3, 1.7 Hz, 2H), 3.35 - 3.48 (m, 1H), 3.77 (td, J = 7.6, 1.7 Hz, 1H), 3.81 - 3.97 (m, 2H), 4.11 (d, J = 1.7 Hz, 0.5H), 4.46 (d, J = 1.5 Hz, 0.5H), 4.60 - 4.84 (m, 2H), 6.97 - 7.05 (m, 2H), 7.14 - 7.27 (m, 3H), 7.44 (ddd, J = 8.4, 5.7, 2.4 Hz, 1H), 8.07 (dd, J = 14.5, 2.4 Hz, 1H). |
| | Composé 115 | |
| | | MS : [M+H] = 493 |
| **Exemple 85** | 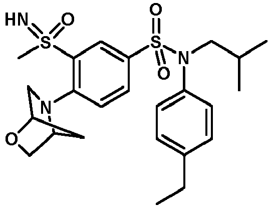 | **4-((1R,4R)-2-oxa-5-azabicyclo[2.2.1] heptan-5-yl)-N-(4-ethylphenyl)-N-isobutyl-3-(S-methyl-sulfonimidoyl)benzenesulfonamide** |
| | | ¹H NMR (DMSO-d6) δ: 0.83 (dt, J = 5.7, 2.8 Hz, 6H), 1.16 (td, J = 7.6, 2.7 Hz, 3H), 1.20 - 1.31 (m, 1H), 1.34 - 1.49 (m, 1H), 1.87 - 1.99 (m, 2H), 2.59 (q, J = 7.6 Hz, 2H), 3.17 - 3.26 (m, 4H), 3.34 (s, 3H), 3.67 - 3.88 (m, 3H), 4.49 (s, 1H), 6.95 - 7.01 (m, 2H), 7.15 - 7.22 (m, 2H), 7.58 - 7.68 (m, 2H), 8.10 (d, J = 2.2 Hz, 1H). |
| | Composé 117 | |
| | | MS : [M+H] = 493 |
| **Exemple 86** | 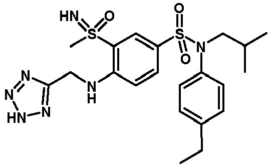 | **4-(((2H-tetrazol-5-yl)methyl)amino)-N-(4-ethylphenyl)-N-isobutyl-3-(S-methylsulfon-imidoyl)benzenesulfonamide** |
| | | ¹H NMR (DMSO-d6) δ: 0.84 (dd, J = 6.7, 1.4 Hz, 7H), 1.18 (t, J = 7.6 Hz, 3H), 1.34 - 1.47 (m, 1H), 2.60 (q, J = 7.6 Hz, 2H), 3.08 (s, 3H), 3.25 (d, J = 7.4 Hz, 4H), 4.86 (d, J = 5.7 Hz, 2H), 6.92 - 7.03 (m, 3H), 7.15 - 7.23 (m, 2H), 7.53 (dd, J = 8.8, 2.3 Hz, 1H), 7.74 (d, J = 2.3 Hz, 1H), 8.20 (t, J = 5.7 Hz, 1H). |
| | Composé 118 | |
| | | MS : [M+H] = 494 |

### Exemple 89 : Synthèse du N-(4-éthylphényl)-N-isobutyl-3-(S-methylsulfonimidoyl)-4-(2,6-diazaspiro[3.3]heptan-2-yl)benzenesulfonamide

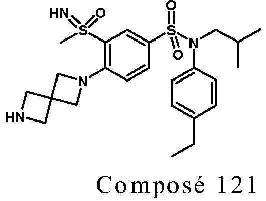

L'acide trifluoroacétique (0.28 ml; 3.59 mmol) est ajouté sur le tert-butyl 6-(4-(N-(4-éthylphényl)-N-isobutylsulfamoyl)-2-(S-methylsulfonimidoyl)phenyl)-2,6-diazaspiro[3.3]heptane-2-carboxylate (55.0 mg; 0.09 mmol) en solution dans le dichlorométhane (2.75 ml). Le milieu réactionnel est agité pendant 4 heures à température ambiante, concentré sous vide, dilué à l'acétate d'éthyle, lavé avec une solution saturée d'hydrogénocarbonate de sodium puis avec une solution saturée de chlorure de sodium, séchées (Na₂SO₄) et concentré. Le résidu est repris dans l'éther et essoré.

Le N-(4-éthylphényl)-N-isobutyl-3-(S-methylsulfonimidoyl)-4-(2,6-diazaspiro[3.3]heptan-2-yl)benzenesulfonamide (44.9 mg; 880 %) est obtenu sous la forme d'un solide blanc.
¹H NMR (DMSO-d6) δ: 0.83 (dd, J = 6.6, 3.3 Hz, 6H), 1.18 (t, J = 7.6 Hz, 3H), 1.25 (s, 1H), 1.34 - 1.47 (m, 1H), 2.60 (q, J = 7.6 Hz, 2H), 3.12 (d, J = 1.8 Hz, 3H), 3.23 (dd, J = 7.2, 4.7 Hz, 2H), 3.79 (s, 2H), 4.21 (d, J = 7.3 Hz, 1H), 4.37 (s, 1H), 4.40 (s, 2H), 6.61 (dd, J = 9.1, 3.7 Hz, 1H), 6.94 - 7.01 (m, 2H), 7.15 - 7.23 (m, 2H), 7.41 (dd, J = 8.8, 2.3 Hz, 1H), 7.93 (dd, J = 4.7, 2.3 Hz, 1H).
MS : [M+H] = 492

### Exemple 90 : Synthèse du 4-(6-acétyl-2,6-diazaspiro[3.3]heptan-2-yl)-N-(4-éthylphényl)-N-isobutyl-3-(S-méthylsulfonimidoyl)benzenesulfonamide

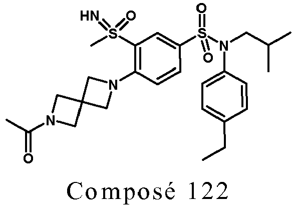

A une solution d'acide 4-Oxo-1-piperidin-4-yl-1,2,3,4-tetrahydro-quinoline-6-sulfonique (4-ethyl-phenyl)-isobutyl-amide (29.0 mg; 0.06 mmol) dans du dichlorométhane (1.45 ml) refroidi à - 10°C sont ajoutés de la 4-diméthylaminopyridine (0.72 mg; 0.01 mmol) et de l'anhydride acétique (5.6 µl; 0.06 mmol). Le milieu réactionnel est agité 30 minutes à température ambiante. Le milieu réactionnel est hydrolysé avec une solution saturée d'hydrogénocarbonate de sodium puis extrait à l'acétate d'éthyle. Les phases organiques sont rassemblées, lavées à la saumure, séchées (Na₂SO₄) et concentrées.

Le produit brut est purifié par HPLC préparative (colonne C18, éluant : acétonitrile dans eau/0.1% d'acide formique). Le 4-(6-acetyl-2,6-diazaspiro[3.3]heptan-2-yl)-N-(4-ethylphenyl)-N-isobutyl-3-(S-methylsulfonimidoyl)benzenesulfonamide (23.10 mg; 73%): est obtenu sous la forme d'un solide blanc.
¹H NMR (DMSO-d6) δ: 0.84 (dd, J = 6.8, 3.4 Hz, 6H), 1.18 (t, J = 7.6 Hz, 3H), 1.34 - 1.47 (m, 1H), 1.76 (s, 3H), 2.60 (q, J = 7.6 Hz, 2H), 3.12 (s, 3H), 3.16 - 3.28 (m, 2H), 4.03 (s, 2H), 4.21 (s, 1H), 4.31 (s, 2H), 4.45 (s, 4H), 6.61 (d, J = 8.9 Hz, 1H), 6.98 (d, J = 8.0 Hz, 2H), 7.19 (d, J = 8.0 Hz, 2H), 7.43 (dd, J = 8.8, 2.3 Hz, 1H), 7.94 (d, J = 2.3 Hz, 1H).
MS : [M+H] = 533

### Exemple 91 : Synthèse du 4-(4-acetylpiperazin-1-yl)-N-(4-ethylphenyl)-N-isobutyl-3-(S-methylsulfonimidoyl)-benzenesulfonamide

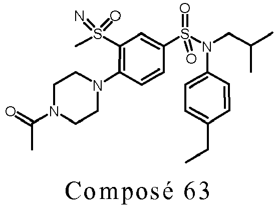

Un mélange de 4-bromo-N-(4-éthylphényl)-N-isobutyl-3-(S-méthylsulfonimidoyl)benzenesulfonamide (50.0 mg; 0.11 mmol) et de 1-piperazin-1-yl-éthanone (67.68 mg; 0.53 mmol) est agité un week-end à 50°C.

Le produit brut est purifié directement par HPLC préparative (colonne C18, éluant : acétonitrile dans eau/0.1% d'acide formique). Le 4-(4-acétylpiperazin-1-yl)-N-(4-éthylphényl)-N-isobutyl-3-(S-méthylsulfonimidoyl)benzenesulfonamide (20.0 mg; 36%) est obtenu sous la forme d'un solide blanc.
¹H NMR (DMSO-d6) δ: 0.85 (dd, J = 6.7, 3.5 Hz, 6H), 1.18 (t, J = 7.6 Hz, 3H), 1.44 (dt, J = 13.7, 6.9 Hz, 1H), 2.61 (q, J = 7.6 Hz, 2H), 3.04 (dq, J = 23.9, 5.3 Hz, 4H), 3.34 (d, J = 1.2 Hz, 3H), 3.61 (d, J = 5.4 Hz, 4H), 4.57 (d, J = 1.4 Hz, 1H), 6.97 - 7.05 (m, 2H), 7.17 - 7.25 (m, 2H), 7.62 (d, J = 8.4 Hz, 1H), 7.71 (dd, J = 8.4, 2.3 Hz, 1H), 8.14 (d, J = 2.3 Hz, 1H).
MS : [M+H] = 521

### Exemple 92 : Synthèse du N-(4-ethylphenyl)-N-isobutyl-3-(S-methylsulfonimidoyl)-4-((pyridin-4-ylméthyl)-amino)benzenesulfonamide

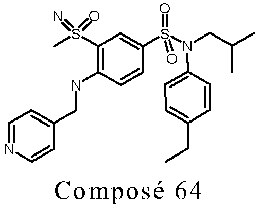

Un mélange de 4-bromo-N-(4-éthylphényl)-N-isobutyl-3-(S-méthylsulfonimidoyl)benzenesulfonamide (50.0 mg; 0.11 mmol)et de 4-picolylamin (53.62 µl; 0.53 mmol) sont agité un week-end à 50°C.

Le produit brut est purifié directement par HPLC préparative (colonne C18, éluant : acétonitrile dans eau/0.1% d'acide formique). Le N-(4-éthylphényl)-N-isobutyl-3-(S-méthylsulfonimidoyl)-4-((pyridin-4-ylmethyl)-amino)benzenesulfonamide (26.0 mg; 49%) est obtenu sous la forme d'un solide blanc.
¹H NMR (DMSO-d6) δ: 0.83 (d, J = 6.7 Hz, 6H), 1.17 (t, J = 7.6 Hz, 3H), 1.41 (hept, J = 6.7 Hz, 1H), 2.59 (q, J = 7.5 Hz, 2H), 3.11 (s, 3H), 3.23 (dd, J = 7.3, 2.5 Hz, 2H), 4.64 (d, J = 5.9 Hz, 2H), 4.79 (s, 1H), 6.75 (d, J = 8.8 Hz, 1H), 6.97 (d, J = 7.9 Hz, 2H), 7.18 (d, J = 8.0 Hz, 2H), 7.36 (d, J = 5.1 Hz, 2H), 7.44 (dd, J = 9.1, 2.4 Hz, 1H), 7.76 (d, J = 2.4 Hz, 1H), 8.21 (t, J = 6.0 Hz, 1H), 8.54 (d, J = 5.2 Hz, 2H).
MS : [M+H] = 501

Avec un mode opératoire analogue à celui décrit pour l'exemple 92, on obtient :

| | | |
|---|---|---|
| **Exemple 94** | 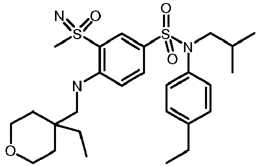 | **N-(4-ethylphenyl)-4-(((4-ethyltetrahydro-2H**-**pyran-4-yl)methyl)amino)-N-isobutyl-3-(S-methylsulfonimidoyl)benzenesulfonami de** |
| | | ¹H NMR (DMSO-d6) δ: 0.81 (t, J = 7.6 Hz, 3H), 0.84 (dd, J = 6.7, 3.4 Hz, 6H), 1.18 (t, J = 7.6 Hz, 3H), 1.38 - 1.57 (m, 7H), 2.61 (q, J = 7.6 Hz, 2H), 3.00 (d, J = 0.8 Hz, 3H), 3.10 - 3.21 (m, 2H), 3.21 - 3.28 (m, 2H), 3.61 (q, J = 6.2 Hz, 4H), 4.85 (s, 1H), 6.99 - 7.04 (m, 3H), 7.16 - 7.24 (m, 2H), 7.51 (dd, J = 8.9, 2.3 Hz, 1H), 7.69 (d, J = 2.3 Hz, 1H), 7.92 (t, J = 4.9 Hz, 1H). MS : [M+H] = 536 |
| | Composé 124 | |
| **Exemple 95** | 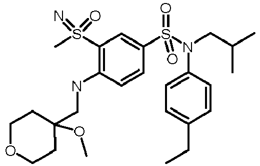 | **N-(4-éthylphényl)-N**-**isobutyl-4-(((4-méthoxy-tétrahydro-2H-pyran-4-yl)méthyl)amino)-3-(S-méthylsulfonimidoyl)benzenesulfonami de** |
| | | ¹H NMR (DMSO-d6) δ: 0.84 (dd, J = 6.6, 2.6 Hz, 6H), 1.18 (t, J = 7.6 Hz, 3H), 1.42 (dt, J = 13.7, 6.9 Hz, 1H), 1.61 (ddd, J = 14.2, 9.0, 4.2 Hz, 2H), 1.75 (t, J = 10.6 Hz, 2H), 2.61 (q, J = 7.6 Hz, 2H), 3.00 (d, J = 0.9 Hz, 3H), 3.13 (s, 3H), 3.19 - 3.32 (m, 4H), 3.57 (ddd, J= 11.1, 8.5, 2.5 Hz, 2H), 3.62 - 3.70 (m, 2H), 4.74 (d, J = 1.1 Hz, 1H), 6.96 (d, J = 9.0 Hz, 1H), 6.99 - 7.04 (m, 2H), 7.16 - 7.22 (m, 2H), 7.51 (dd, J = 8.8, 2.3 Hz, 1H), 7.71 (d, J = 2.2 Hz, 1H), 7.81 (t, J = 4.7 Hz, 1H). |
| | Composé 125 | |
| **Exemple 96** | 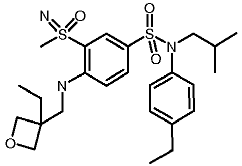 | **4-(((3-ethyloxetan-3-yl)methyl)amino)-N-(4-ethylphenyl)-N-isobutyl-3-(S-methylsulfonimidoyl)benzenesulfonamide** |
| | | ¹H NMR (DMSO-d6) δ: 0.85 (dd, J = 6.6, 3.0 Hz, 6H), 0.91 (t, J = 7.4 Hz, 3H), 1.18 (t, J = 7.6 Hz, 3H), 1.42 (dt, J = 13.7, 6.8 Hz, 1H), 1.70 - 1.81 (m, 2H), 2.61 (q, J = 7.6 Hz, 2H), 3.01 (d, J = 0.9 Hz, 3H), 3.21 - 3.30 (m, 2H), 3.37 - 3.56 (m, 2H), 4.36 (d, J = 1.3 Hz, 2H), 4.38 (d, J = 5.0 Hz, 2H), 4.80 (s, 1H), 6.97 - 7.04 (m, 2H), 7.05 (d, J = 9.0 Hz, 1H), 7.16 - 7.25 (m, 2H), 7.53 (dd, J = 8.8, 2.3 Hz, 1H), 7.71 (d, J = 2.4 Hz, 1H), 7.98 (t, J = 5.1 Hz, 1H). MS : [M+H] = 508 |
| | Composé 126 | |
| **Exemple 97** | 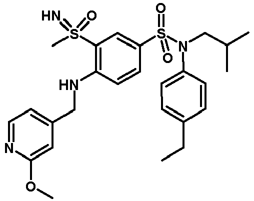 | **N-(4-ethylphenyl)-N-isobutyl-4-(((2-methoxy-pyridin-4-yl)methyl)amino)-3-(S-methyl-sulfonimidoyl)benzenesulfonamide** |
| | | ¹H NMR (DMSO-d6) δ: 0.83 (dd, J = 6.6, 1.9 Hz, 6H), 1.17 (t, J = 7.6 Hz, 3H), 1.42 (dq, J = 13.6, 6.7 Hz, 1H), 2.54-2.64 (m, 2H), 3.09 (d, J = 0.9 Hz, 3H), 3.24 (dd, J = 7.3, 2.6 Hz, 2H), 3.84 (s, 3H), 4.58 (d, J = 6.0 Hz, 2H), 4.79 (s, 1H), 6.73 - 6.77 (m, 2H), 6.95 - 6.99 (m, 3H), 7.15 - 7.20 (m, 2H), 7.44 (dd, J = 8.8, 2.4 Hz, 1H), 7.76 (d, J = 2.3 Hz, 1H), 8.13 (dd, J = 5.3, 0.7 Hz, 1H), 8.17 (t, J = 6.1 Hz, 1H). MS : [M+H] = 531 |
| | Composé 127 | |
| **Exemple 98** | 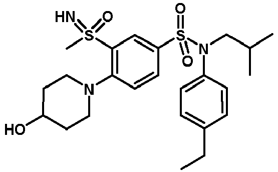 | **N-(4-ethylphenyl)-4-(4-hydroxypiperidin-1-yl)-N-isobutyl-3-(S-methylsulfonimidoyl)benzenesulfonamide** |
| | | ¹H NMR (DMSO-d6) δ: 0.85 (dd, J = 6.7, 3.3 Hz, 6H), 1.18 (t, J = 7.6 Hz, 3H), 1.43 (dt, J = 13.4, 6.6 Hz, 1H), 1.60 (t, J = 9.5 Hz, 2H), 1.82 - 1.91 (m, 2H), 2.61 (q, J = 7.6 Hz, 2H), 2.84 (q, J = 9.2 Hz, 2H), 3.18 - 3.30 (m, 4H), 3.66 (s, 1H), 4.52 (d, J = 1.5 Hz, 1H), 4.71 (d, J = 4.3 Hz, 1H), 6.95 - 7.04 (m, 2H), 7.17 - 7.24 (m, 2H), 7.59 (d, J = 8.4 Hz, 1H), 7.67 (dd, J = 8.4, 2.3 Hz, 1H), 8.12 (d, J = 2.3 Hz, 1H). MS : [M+H] = 494 |
| | Composé 128 | |
| **Exemple 99** | 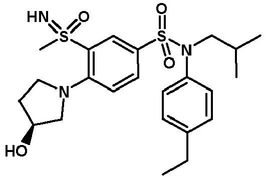 | **N-(4-éthylphényl)-4-((S)-3-hydroxypyrrolidin-1-yl)-N-isobutyl-3-(S-méthylsulfonimidoyl)-benzenesulfonamide** |
| | | ¹H NMR (DMSO-d6) δ: 0.84 (dd, J **=** 6.6, 1.5 Hz, 6H), 1.18 (t, J = 7.6 Hz, 3H), 1.42 (dt, J = 13.6, 6.8 Hz, 1H), 1.86 (d, J = 9.6 Hz, 1H), 2.01 (ddd, J = 14.4, 10.7, 4.8 Hz, 1H), 2.60 (q, J = 7.6 Hz, 2H), 3.21 (dd, J = 12.9, 1.3 Hz, 3H), 3.27 - 3.29 (m, 2H), 3.67 (q, J = 8.2 Hz, 1H), 3.73 **-** 3.85 (m, 1H), 3.91 (dd, J = 10.8, 4.5 Hz, 1H), 4.01 (d, J = 1.8 Hz, 1H), 4.28 (d, J = 1.5 Hz, 1H), 4.39 (s, 1H), 5.01 (dd, J **=** 19.6, 3.4 Hz, 1H), 7.01 (d, J = 8.4 Hz, 1H), 7.12 (dd, J = 12.6, 8.9 Hz, 1H), 7.20 (d, J = 8.4 Hz, 2H), 7.43 (dd, J = 8.9, 2**.**4 Hz, 1H), 8.05 (d, J = 2.4 Hz, 1H). MS : [M+H] = 480 |
| | **Composé 129** | |
| **Exemple 100** | 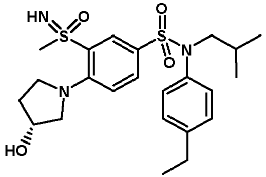 | **N-(4-éthylphényl)-4-((R)-3-hydroxypyrrolidin-1-yl)-N-isobutyl-3-(S-methylsulfonimidoyl)-benzenesulfonamide** |
| | | ¹H NMR (DMSO-d6) δ: 0.84 (dd, J = 6.6, 1.5 Hz, 6H), 1.18 (t, J = 7.6 Hz, 3H), 1.42 (dt, J = 13.6, 6.8 Hz, 1H), 1.86 (d, J = 9.6 Hz, 1H), 2.01 (ddd, J = 14.4, 10.7, 4.8 Hz, 1H), 2.60 (q, J = 7.6 Hz, 2H), 3.21 (dd, J = 12.9, 1.3 Hz, 3H), 3.27 - 3.29 (m, 2H), 3.67 (q, J = 8.2 Hz, 1H), 3.73 - 3.85 (m, 1H), 3.91 (dd, J = 10.8, 4.5 Hz, 1H), 4.01 (d, J = 1.8 Hz, 1H), 4.28 (d, J = 1.5 Hz, 1H), 4.39 (s, 1H), 5.01 (dd, J = 19.6, 3.4 Hz, 1H), 7.01 (d, J = 8.4 Hz, 1H), 7.12 (dd, J = 12.6, 8.9 Hz, 1H), 7.20 (d, J = 8.4 Hz, 2H), 7.43 (dd, J = 8.9, 2.4 Hz, 1H), 8.05 (d, J = 2.4 Hz, 1H). MS : [M+H] = 480 |
| | Composé 130 | |
| **Exemple 101** | 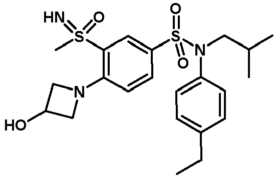 | **N-(4-éthylphényl)-4-(3-hydroxyazetidin-1-yl)-N-isobutyl-3-(S-méthylsulfonimidoyl)-benzenesulfonamide** |
| | | ¹H NMR (DMSO-d6) δ: 0.84 (dd, J = 6.6, 3.1 Hz, 6H), 1.18 (t, J = 7.6 Hz, 3H), 1.41 (dt, J = 14.1, 7.0 Hz, 1H), 2.60 (q,J = 7.7 Hz, 2H), 3.11 (d, J = 1.3 Hz, 3H), 3.24 (dd, J = 7.3, 4.5 Hz, 2H), 4.02 (dd, J = 10.5, 5.7 Hz, 2H), 4.20 (d, J = 1.5 Hz, 1H), 4.50 (s, 2H), 5.71 (s, 1H), 6.63 (d, J = 8.9 Hz, 1H), 6.99 (d, J = 8.4 Hz, 2H), 7.16 - 7.25 (m, 2H), 7.40 (dd, J = 8.8, 2.3 Hz, 1H), 7.94 (d, J = 2.4 Hz, 1H). MS : [M+H] = 466 |
| | Composé 131 | |
| **Exemple 102** | 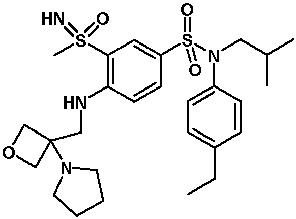 | **N-(4-éthylphényl**)-**N**-**isobutyl-3-(S-méthyl-sulfonimidoyl)-4-(((3-(pyrrolidin-1-yl)oxetan-3-yl)méthyl)amino)benzenesulfonamide** |
| | | ¹H NMR (DMSO-d6) δ: 0.84 (dd, J = 6.6, 2.0 Hz, 6H), 1.18 (t, J = 7.6 Hz, 3H), 1.42 (p, J = 6.9 Hz, 1H), 1.76 (d, J = 6.2 Hz, 4H), 2.61 (q, J = 7.6 Hz, 2H), 2.73 (q, J = 5.9 Hz, 4H), 2.96 (d, J = 0.9 Hz, 3H), 3.25 (dd, J = 7.4, 1.6 Hz, 2H), 3.55 - 3.72 (m, 2H), 4.29 (dd, J = 8.5, 6.6 Hz, 2H), 4.59 (s, 1H), 4.78 (d, J = 6.7 Hz, 2H), 6.97 - 7.02 (m, 3H), 7.17 - 7.23 (m, 2H), 7.53 (dd, J = 8.8, 2.4 Hz, 1H), 7.72 (d, J = 2.3 Hz, 1H), 7.85 (t, J = 4.2 Hz, 1H). MS : [M+H] = 549 |
| | Composé 132 | |

### Exemple 103 : Synthèse du N-(4-éthylphényl)-N-isobutyl-3-(S-méthylsulfonimidoyl)-4-((pyrimidin-4-ylméthyl)-amino)benzenesulfonamide

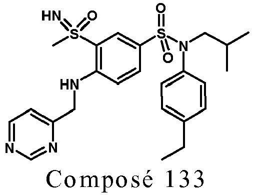

Le 4-bromo-N-(4-éthylphényl)-N-isobutyl-3-(S-méthylsulfonimidoyl)benzenesulfonamide (50.0 mg; 0.11 mmol et le 4-(aminométhyl)pyrimidine (34.6 mg; 0.32 mmol) sont introduits dans un tube à micro-onde. Le milieu réactionnel est agité pendant 30 minutes à 100°C sous micro-ondes.

Le produit brut est purifié par HPLC préparative (colonne C18, éluant : acétonitrile dans eau/0.1% d'acide formique). Le N-(4-ethylphenyl)-N-isobutyl-3-(S-methylsulfonimidoyl)-4-((pyrimidin-4-ylmethyl)amino)benzenesulfonamide 001 (15.0 mg; 28%) est obtenu sous la forme d'un solide beige.
¹H NMR (DMSO-d6) δ: 0.83 (dd, J = 6.6, 1.5 Hz, 6H), 1.17 (t, J = 7.6 Hz, 3H), 1.41 (dt, J = 13.7, 6.8 Hz, 1H), 2.60 (q, J = 7.6 Hz, 2H), 3.11 (d, J = 1.0 Hz, 3H), 3.24 (dd, J = 7.4, 1.4 Hz, 2H), 4.72 (dd, J = 5.7, 1.9 Hz, 2H), 4.75 - 4.79 (m, 1H), 6.81 (d, J = 8.9 Hz, 1H), 6.93 - 7.01 (m, 2H), 7.15 - 7.23 (m, 2H), 7.47 (dd, J = 8.8, 2.3 Hz, 1H), 7.50 (dd, J = 5.2, 1.4 Hz, 1H), 7.77 (d, J = 2.3 Hz, 1H), 8.35 (t, J = 5.9 Hz, 1H), 8.77 (d, J = 5.2 Hz, 1H), 9.17 (d, J = 1.4 Hz, 1H).
MS : [M+H] = 502

### Exemple 107 : Synthèse du N-(4-ethylphenyl)-N-isobutyl-3-(S-methylsulfonimidoyl)-4-(1,4-oxazepan-4-yl)benzenesulfonamide

Sur le 1,4-oxazepane (16.0 mg; 0.16 mmol) et la N,N-diisopropylethylamine (0.11 ml; 0.63 mmol) en solution dans le diméthylsufoxide (2ml) sont ajoutés le 4-bromo-N-(4-éthyl-phényl)-N-isobutyl-3-(S-méthylsulfonimidoyl)benzenesulfonamide (50.0 mg; 0.11 mmol).

Le milieu réactionnel est chauffé à une température de 150°C pendant 20 minutes sous micro-ondes. Le milieu réactionnel est hydrolysé avec une solution d'acide chlorhydrique 1N et dilué puis extrait à l'acétate d'éthyle. Les phases organiques sont rassemblées, lavées à la saumure, séchées (Na₂SO₄) et concentrées sous vide.

Le produit brut est purifié directement par HPLC préparative (colonne C18, éluant : acétonitrile dans eau/0.1% d'acide formique). Le N-(4-éthylphényl)-N-isobutyl-3-(S-méthylsulfonimidoyl)-4-(1,4-oxazepan-4-yl)benzenesulfonamide (23.8 mg; 46 %) est obtenu sous la forme d'un solide blanc.
¹H NMR (Methanol-d4) δ: 0.93 (d, J = 6.7 Hz, 6H), 1.25 (t, J = 7.6 Hz, 3H), 1.49 - 1.64 (m, 1H), 2.09 (p, J = 6.0 Hz, 2H), 2.67 (q, J = 7.6 Hz, 2H), 3.39 (d, J = 7.3 Hz, 2H), 3.48 (s, 3H), 3.90 (dd, J = 6.2, 3.5 Hz, 2H), 3.95 (t, J = 6.1 Hz, 2H), 6.98 - 7.05 (m, 2H), 7.17 - 7.24 (m, 2H), 7.66 (d, J = 8.4 Hz, 1H), 7.74 (dd, J = 8.4, 2.3 Hz, 1H), 8.23 (d, J = 2.2 Hz, 1H).
MS : [M+H] = 494

| | | |
|---|---|---|
| **Exemple 107** | 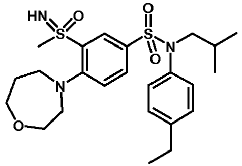 | **N-(4-éthylphényl)-N-isobutyl-3-(S-méthyl-sulfonimidoyl)-4-(1,4-oxazepan-4-yl)benzene-sulfonamide** |
| | | ¹H NMR (Methanol-d4) δ: 0.93 (d, J = 6.7 Hz, 6H), 1.25 (t, J = 7.6 Hz, 3H), 1.49 - 1.64 (m, 1H), 2.09 (p, J = 6.0 Hz, 2H), 2.67 (q, J = 7.6 Hz, 2H), 3.39 (d, J = 7.3 Hz, 2H), 3.48 (s, 3H), 3.90 (dd, J = 6.2, 3.5 Hz, 2H), 3.95 (t, J = 6.1 Hz, 2H), 6.98 - 7.05 (m, 2H), 7.17 - 7.24 (m, 2H), 7.66 (d, J = 8.4 Hz, 1H), 7.74 (dd, J = 8.4, 2.3 Hz, 1H), 8.23 (d, J = 2.2 Hz, 1H). MS : [M+H] = 494 |
| | Composé 137 | |

### Exemple 110 : Synthèse du N-(4-ethylphenyl)-N-isobutyl-3-(S-methylsulfonimidoyl)-4-(piperazin-1-yl)benzene-sulfonamide

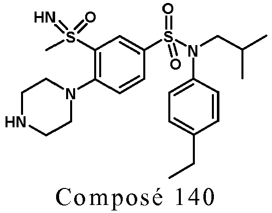

A une solution de 4-bromo-N-(4-ethylphenyl)-N-isobutyl-3-(S-methylsulfonimidoyl)benzenesulfonamide (50.00 mg; 0.11 mmol) dans du N,N-diméthylformamide (200µl) est ajouté de la pipérazine (18.2 mg; 0.21 mmol). Le milieu réactionnel est agité une nuit à 60°C. Le produit brut est purifié par HPLC préparative (colonne C18, éluant : acétonitrile dans eau/0.2% de carbonate d'ammonium). Le N-(4-ethylphenyl)-N-isobutyl-3-(S-methylsulfonimidoyl)-4-(piperazin-1-yl)benzenesulfonamide (30.0 mg; 53 %) est obtenu sous la forme d'un solide blanc.
¹H NMR (DMSO-d6) δ: 0.85 (dd, J = 6.6, 3.5 Hz, 6H), 1.18 (t, J = 7.6 Hz, 3H), 1.43 (dt, J = 13.8, 6.9 Hz, 1H), 2.61 (q, J = 7.6 Hz, 2H), 2.87 (t, J = 4.7 Hz, 4H), 2.99 (d, J = 5.0 Hz, 4H), 3.35 (s, 3H), 4.53 (s, 1H), 6.97 - 7.04 (m, 2H), 7.21 (d, J = 8.4 Hz, 2H), 7.59 (d, J = 8.5 Hz, 1H), 7.69 (dd, J = 8.4, 2.3 Hz, 1H), 8.14 (d, J = 2.3 Hz, 1H).
MS : [M+H] 479

### Exemple 111 : Synthèse du N-(4-éthylphényl)-4-(((3-hydroxycyclobutyl)méthyl)amino)-N-isobutyl-3-(S-methyl-sulfonimidoyl)benzenesulfonamide

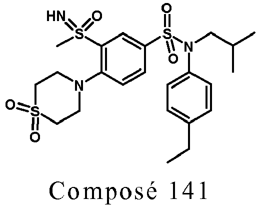

De l'acide 3-chloroperoxybenzoique (24.4 mg; 0.11 mmol) est ajouté à 0°C sur le N-(4-ethylphenyl)-N-isobutyl-3-(S-methylsulfonimidoyl)-4-thiomorpholinobenzenesulfonamide (27.0 mg; 0.05 mmol) en solution dans du dichlorométhane (250µl). Le milieu réactionnel est agité 5 heures à température ambiante, hydrolysé avec une solution d'hydroxyde de sodium 1N et extrait au dichlorométhane. Les phases organiques sont rassemblées, lavées à l'eau, séchées (MgSO₄) et concentrées sous vide.

Le produit brut est purifié par HPLC préparative (colonne C18, éluant : acétonitrile dans eau/0.1% d'acide formique). Le 4-(1,1-dioxidothiomorpholino)-N-(4-ethylphenyl)-N-isobutyl-3-(S-methylsulfonimidoyl)-benzenesulfonamide (8.0 mg; 28%) est obtenu sous la forme d'un solide blanc cassé.
¹H NMR (DMSO-d6) δ: 0.86 (d, J = 6.6 Hz, 6H), 1.18 (t, J = 7.6 Hz, 3H), 1.43 (dt, J = 13.5, 6.8 Hz, 1H), 2.61 (q, J = 7.6 Hz, 2H), 3.31 (d, J = 7.8 Hz, 2H), 3.35-3.40 (m, 7H), 3.49 (dd, J = 6.8, 3.4 Hz, 4H), 6.94 - 7.05 (m, 2H), 7.17 - 7.25 (m, 2H), 7.83 - 7.91 (m, 3H).
MS : [M+H] 529

### Partie III : Synthèse de sulfonamides soufrés à partir du schéma réactionnel 3

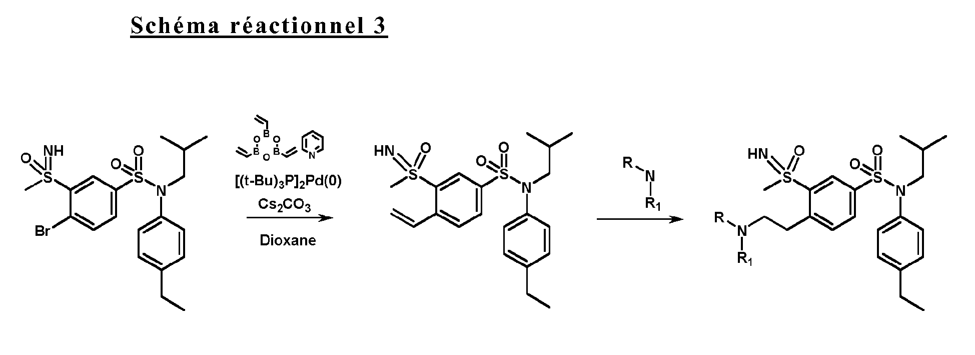

### Exemple 112 : Synthèse du N-(4-ethylphenyl)-N-isobutyl-3-(S-methylsulfonimidoyl)-4-(2-morpholinoethyl)-benzene-sulfonamide

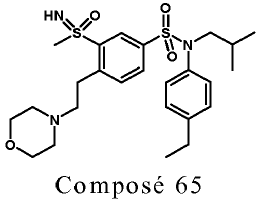

### 1. Synthèse de l'intermédiaire 112.1

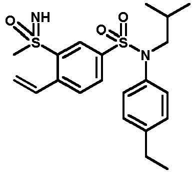

### N-(4-ethylphenyl)-N-isobutyl-3-(S-methylsulfonimidoyl)-4-vinylbenzenesulfonamide

A une solution de 4-bromo-N-(4-éthylphényl)-N-isobutyl-3-(S-methylsulfonimidoyl)benzenesulfonamide (100.0 mg; 0.21 mmol) dans du 1,4-dioxane (1.2 ml) sont ajoutés du carbonate de césium (206.45 mg; 0.63 mmol) du tert-butyl N-(2-oxiranylmethyl)carbamate (101.66 mg; 0.42 mmol) et de l'eau (0.40 ml). Le milieu réactionnel est dégazé sous argon pendant 10min avant d'ajouter le bis(tri-terbutylphosphine)-palladium(0) (10.79 mg; 0.02 mmol; 0.10 eq.). Le milieu réactionnel est agité 2 heures à 90°C, filtré sur célite avec rinçage à l'acétate d'éthyle. La phase organique est lavée avec une solution saturée d'hydrogénocarbonate de sodium puis de l'eau, séchée (MgSO₄), filtrée et concentrée à sec. Le produit brut est purifié par chromatographie sur gel de silice (éluant heptane/acétate d'éthyle de 0 à 100% d'acétate d'éthyle). Le N-(4-ethylphenyl)-N-isobutyl-3-(S-methylsulfonimidoyl)-4-vinylbenzenesulfonamide (70.0 mg; 79%) est obtenu est obtenu sous la forme d'une huile incolore avec une RMN¹H conforme.
MS : [M+H] = 422

### 2. Synthèse du composé N-(4-éthylphényl)-N-isobutyl-3-(S-methylsulfonimidoyl)-4-(2-morpholinoethyl)benzene-sulfonamide

Un mélange de N-(4-ethylphenyl)-N-isobutyl-3-(S-methylsulfonimidoyl)-4-vinylbenzenesulfonamide (70.0 mg; 0.17 mmol; 1.00 eq.) et de la morpholine (1.0 ml; 11.59 mmol) est agité 30min à température ambiante.

Le produit brut est purifié par HPLC préparative (colonne C18, éluant : acétonitrile dans eau/0.1% d'acide formique). Le N-(4-ethylphenyl)-N -isobutyl-3-(S-methylsulfon-imidoyl)-4-(2-morpholinoethyl)benzenesulfonamide (25.00 mg; 29.59 %) est obtenu sous la forme d'une poudre ocre.
¹H NMR (DMSO-d6) δ: 1.10 (d, J = 6.5 Hz, 6H), 1.43 (t, J = 7.6 Hz, 3H), 1.69 (dt, J = 13.4, 6.8 Hz, 1H), 2.71 (d, J = 5.4 Hz, 2H), 2.83 - 2.90 (m, 2H), 3.36 (s, 8H), 3.84 (t, J = 4.5 Hz, 3H), 4.79 (s, 1H), 7.24 (d, J = 8.0 Hz, 2H), 7.45 (d, J = 8.0 Hz, 2H), 7.95 (s, 2H), 8.32 (s, 1H).
MS : [M+H] = 508

### Partie IV : Synthèse de sulfonamides soufrés à partir du schéma réactionnel 4

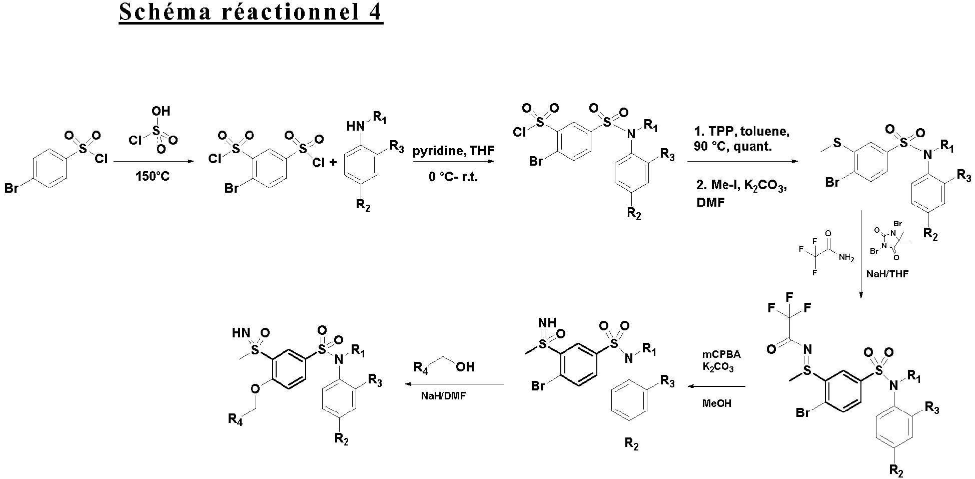

### Exemple 113 : Synthèse du N-(2,4-diméthylphényl)-N-isobutyl-3-(S-méthylsulfonimidoyl)-4-((tétrahydro-2H-pyran-4-yl)méthoxy)benzenesulfonamide

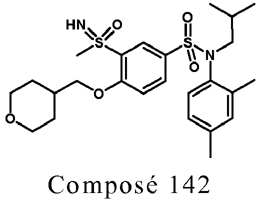

### 1. Synthèse de l'intermédiaire 113.1

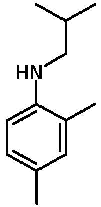

### (2,4-diméthyl-phényl)-isobutyl-amine

Une solution de 2,4-diméthylaniline (30ml; 0.24 mol) et d'isobutyraldéhyde (20ml; 0.22 mol) dans du tétrahydrofurane (320ml) est agitée 30 minutes à température ambiante puis du triacétoxyborohydrure de sodium (70g; 0.33 mol) est ajouté par portions. Le milieu réactionnel est agité pendant 3 heures à température ambiante, hydrolysé et extrait à l'acétate d'éthyle. Les phases organiques sont rassemblées lavées avec une solution saturée de chlorure de sodium et séchée (MgSO₄).

Les solvants sont évaporés. Le produit brut est chromatographié sur gel de silice (éluant heptane/acétate d'éthyle de 0 à 10% d'acétate d'éthyle).

La (2,4-diméthyl-phényl)-isobutyl-amine (29.9 g; 77 %) est obtenue sous la forme d'une huile jaune avec une RMN conforme.
MS : [M+H] = 177

### 2. Synthèse de l'intermédiaire 113.2

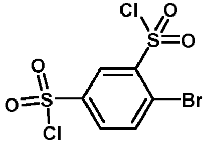

### dichlorure de 4-bromo-benzene-1,3-disulfonyle

Un mélange de chlorure de 4-bromobenzenesulfonyle (50g; 0.20 mol) et d'acide chlorosulfonique (260ml; 3.91 mol) est agité 6 heures à 150°C. Le milieu réactionnel est versé lentement, avec précaution sur un mélange eau et glace et est extrait avec du dichlorométhane. Les phases organiques sont rassemblées, séchées (MgSO₄), filtrées et concentrées. Le dichlorure de 4-bromo-benzene-1,3-disulfonyle (54g ; 78%) est obtenu sous la forme d'une poudre grisâtre avec une RMN conforme.
MS : [M+H] = 177

### 3. Synthèse de l'intermédiaire 113.3

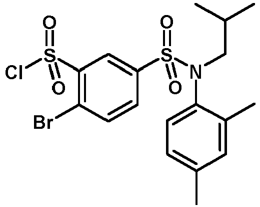

### chlorure de 2-bromo-5-[(2,4-diméthyl-phényl)-isobutyl-sulfamoyl]-benzenesulfonyle

Le dichlorure de 4-bromo-benzene-1,3-disulfonyle (1.0 g; 2.82 mmol) en solution dans le tétrahydrofurane (5ml) est additionné à la (2,4-diméthyl-phényl)-isobutyl-amine (0.50 g; 2.82 mmol) et la pyridine (1.4 ml; 17.0 mmol) en solution dans le tétrahydrofurane (20ml). Le milieu réactionnel est agité pendant 16 heures à température ambiante. Le milieu réactionnel est hydrolysé puis extrait à l'acétate d'éthyle. Les phases organiques sont rassemblées, lavées avec une solution aqueuse d'acide chlorhydrique 1M puis avec une solution saturée de chlorure de sodium, séchées (Na₂SO₄) et concentrées.

Le chlorure de 2-bromo-5-[(2,4-diméthyl-phényl)-isobutyl-sulfamoyl]-benzenesulfonyel (1.23 g; 88%) est obtenu sous la forme d'une huile jaune avec une RMN conforme.

### 4. Synthèse de l'intermédiaire 113.4

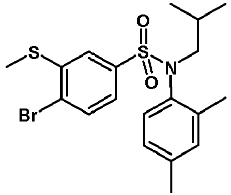

### 4-bromo-N-(4-éthyl-phényl)-N-isobutyl-3-méthylsulfanyl-benzenesulfonamide

Le chlorure de 2-bromo-5-[(2,4-diméthyl-phényl)-isobutyl-sulfamoyl]-benzenesulfonyle (1.67 g; 3.37 mmol.) en solution dans le toluène (8ml) est ajouté lentement sur la triphénylphosphine (2.66 g; 10.12 mmol) en suspension dans le toluène (17ml). Le milieu réactionnel est agité pendant 4 heures à 90°C. Le milieu réactionnel est concentré sous vide et mis en solution dans le N,N-diméthylformamide (14.5ml) sans purification puis du carbonate de potassium (0.51 g; 3.72 mmol) et du iodométhane (0.32 ml; 5.08 mmol) sont ajouté. Le milieu réactionnel est agité pendant 20 minutes à température ambiante, hydrolysé et extrait à l'acétate d'éthyle. Les phases organiques sont rassemblées, lavées avec une solution saturée de chlorure de sodium et séchées (Na₂SO₄).

Les solvants sont évaporés. Le produit brut est chromatographié sur gel de silice (éluant Heptane/Acétate d'éthyle de 0 à 10% d'acétate d'éthyle).

Le 4-bromo-N-(4-ethyl-phenyl)-N-isobutyl-3-methylsulfanyl-benzenesulfonamide (775.30 mg; 52 %) est obtenu sous la forme d'un solide blanc avec une RMN conforme
MS : [M-H] = 441

### 5. Synthèse de l'intermédiaire 113.5

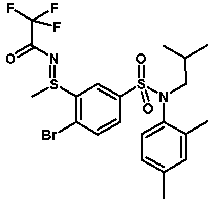

### (E)-N-((2-bromo-5-(N-(2,4-dimethylphenyl)-N-isobutylsulfamoyl)phenyl)(methyl)-λ⁴-sulfanylidene)-2,2,2-trifluoroacetamide

Le 4-bromo-N-(2,4-diméthyl-phényl)-N-isobutyl-3-methylsulfanyl-benzenesulfonamide (755.0 mg; 1.71 mmol) et le 2,2,2-trifluoroacétamide (289.34 mg; 2.56 mmol) en solution dans le tétrahydrofurane (1.51 ml) sont ajoutés lentement à de l'hydrure de sodium 60% (61.43 mg; 1.54 mmol) en suspension dans le tétrahydrofurane (3.78 ml) à 0-5°C. La 1,3-dibromo-5,5-dimethylhydantoine (732 mg; 2.56 mmol) en solution dans le tétrahydrofurane (1.5ml) est ensuite ajoutée.

Le milieu est agité pendant 1 heure à température ambiante, hydrolysé par addition d'une solution d'acide citrique à 10% puis extrait à l'acétate d'éthyle. Les phases organiques sont rassemblées puis lavées avec une solution de sulfite de sodium à 25% puis par 2 fois avec une solution saturée de chlorure de sodium et séchées (Na₂SO₄). Les solvants sont évaporés.

Le produit brut est chromatographié sur gel de silice (éluant Heptane/Acétate d'éthyle de 0 à 50% d'acétate d'éthyle). Le (E)-N-((2-bromo-5-(N-(2,4-dimethylphenyl)-N-isobutylsulfamoyl)phényl)(méthyl)-14-sulfanylidene)-2,2,2-trifluoroacetamide (233.0 mg; 25%) est obtenu sous la forme d'une poudre blanche avec une RMN conforme
MS : [M-H] = 552

### 6. Synthèse de l'intermédiaire 113.6

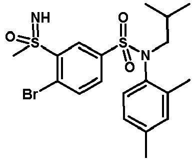

### 4-bromo-N-(2,4-diméthylphényl)-N-isobutyl-3-(S-méthylsulfonimidoyl)-benzenesulfonamide

Le carbonate de potassium (172.30 mg; 1.25 mmol) est ajouté à du (E)-N-((2-bromo-5-(N-(4-éthylphényl)-N-isobutylsulfamoyl)phényl)(méthyl)-14-sulfanylidene)-2,2,2-trifluoroacetamide (230.0 mg; 0.42 mmol) en solution dans le méthanol (2.3ml) puis, à 0°C, l'acide 3-chloroperoxybenzoique (139.7 mg; 0.62 mmol) est ajouté lentement. Le milieu réactionnel est agité pendant 3 jours à température ambiante. Le milieu réactionnel est hydrolysé puis extrait à l'acétate d'éthyle. Les phases organiques sont rassemblées, lavées avec une solution saturée de chlorure de sodium et séchées (Na₂SO₄). Les solvants sont évaporés.

Le produit brut est chromatographié sur gel de silice (éluant Heptane/Acétate d'éthyle de 0 à 80% d'acétate d'éthyle). Le 4-bromo-N-(2,4-diméthylphényl)-N-isobutyl-3-(S-méthylsulfonimidoyl)-benzenesulfonamide (70.8 mg; 36 %) est obtenu sous la forme d'un solide blanc. avec une RMN conforme
MS : [M+H] = 475

### 7. Synthèse du N-(2,4-diméthylphényl)-N-isobutyl-3-(S-méthylsulfonimidoyl)-4-((tétrahydro-2H-pyran-4-yl)méthoxy)benzenesulfonamide

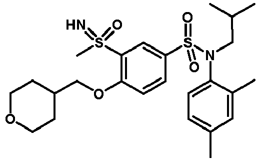

L'hydrure de sodium 60% (8.74 mg; 0.22 mmol) est ajouté lentement à une température de 0°C sur le (tetrahydro-pyran-4-yl)-méthanol (18.62 mg; 0.16 mmol) en solution dans le N,N-diméthylformamide (1.38 ml) puis le 4-bromo-N-(2,4-dimethylphenyl)-N-isobutyl-3-(S-methylsulfonimidoyl)benzenesulfonamide (69.0 mg; 0.15 mmol). Le milieu réactionnel est agité pendant 2 heures à température ambiante puis 1 heure à 80°C. Le milieu réactionnel est hydrolysé à froid puis extrait à l'acétate d'éthyle. Les phases organiques sont rassemblées, lavées avec une solution saturée de chlorure de sodium et séchées (Na₂SO₄). Les solvants sont évaporés.

Le produit brut est purifié par HPLC préparative (colonne C18, éluant : acétonitrile dans eau/0.1% d'acide formique). Le N-(2,4-dimethylphenyl)-N-isobutyl-3-(S-methylsulfonimidoyl)-4-((tetrahydro-2H-pyran-4-yl)méthoxy)benzenesulfonamide (44.81 mg; 59.54 %): est obtenu sous la forme d'un solide blanc.
¹H NMR (DMSO-d6) δ: 0.76 (dd, J = 6.8, 3.2 Hz, 3H), 0.82 - 0.91 (m, 1H), 0.95 (t, J = 6.7 Hz, 3H), 1.25 (q, J = 3.6, 2.6 Hz, 1H), 1.40 (p, J = 4.2 Hz, 2H), 1.43 (s, 1H), 1.75 (d, J = 7.5 Hz, 1H), 2.23 - 2.32 (m, 6H), 3.06 (ddd, J = 21.2, 13.1, 4.6 Hz, 1H), 3.20 (dd, J = 2.7, 1.2 Hz, 3H), 3.35 - 3.44 (m, 2H), 3.87 - 3.95 (m, 2H), 4.12 (dd, J = 6.3, 3.6 Hz, 2H), 4.45 (dd, J = 50.6, 1.5 Hz, 1H), 6.58 (dd, J = 16.3, 8.1 Hz, 1H), 6.94 (dd, J = 8.1, 2.0 Hz, 1H), 7.13 (s, 1H), 7.41 (d, J = 8.9 Hz, 1H), 7.74 (td, J = 8.6, 2.5 Hz, 1H), 8.05 (dd, J = 4.9, 2.4 Hz, 1H)
MS : [M+H] = 509

### Partie V : Synthèse de sulfonamides soufrés à partir du schéma réactionnel 5

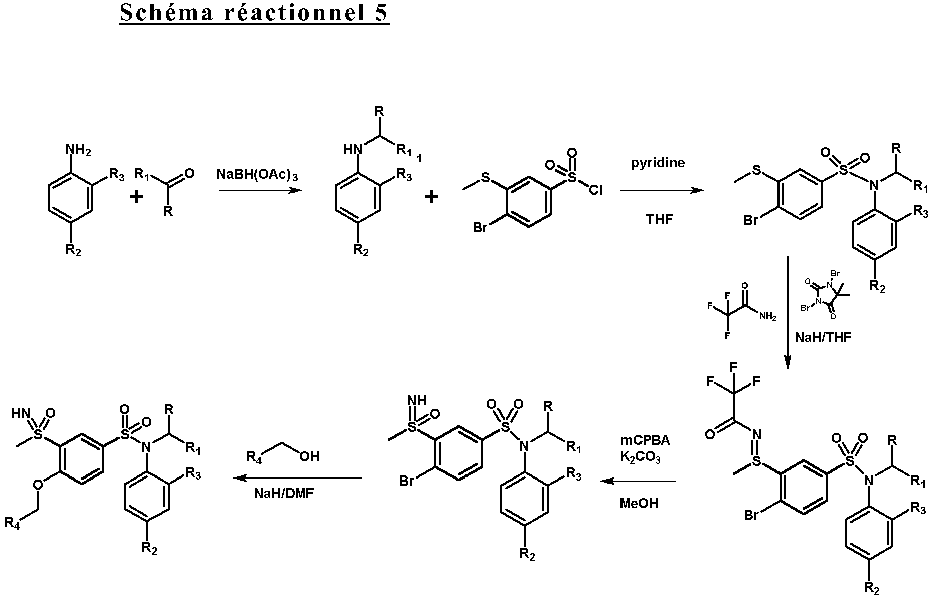

### Exemple 114 : Synthèse du N-isopropyl-N-(4-méthoxy-2-méthylphényl)-3-(S-méthylsulfonimidoyl)-4-((tétrahydro-2H-pyran-4-yl)méthoxy)benzenesulfonamide

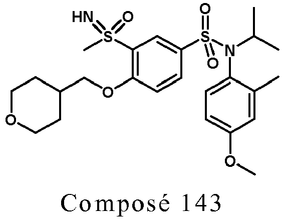

### 1. Synthèse de l'intermédiaire 114.1

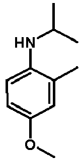

### Isopropyl-(4-méthoxy-2-méthyl-phényl)-amine

Le triacetoxyborohydrure de sodium (4.63 g; 21.9 mmol) est additionné à une solution de 4-méthoxy-2-méthylaniline (2.0 g; 14.6 mmol) dans de l'acétone (20ml). Le milieu réactionnel est chauffé pendant 10 minutes à une température de 70°C sous micro-onde. Le milieu réactionnel est versé sur de la glace et extrait au dichlorométhane. Les phases organiques sont rassemblées, lavées avec une solution saturée de chlorure de sodium, séchées (MgSO₄), filtrées et concentrées. Le produit brut est chromatographié sur gel de silice (éluant Heptane/Acétate d'éthyle de 0 à 15% d'acétate d'éthyle). L'isopropyl-(4-méthoxy-2-méthyl-phényl)-amine (1.43 g; 55%) est obtenue sous forme d'huile jaune avec une RMN conforme.
MS : [M+H] = 180

### 2. Synthèse de l'intermédiaire 114.2

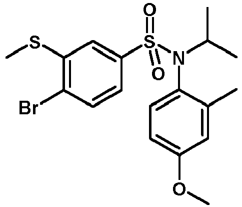

### 4-bromo-N-isopropyl-N-(4-méthoxy-2-méthyl-phényl)-3-méthylsulfanyl-benzenesulfonamide

Le chlorure de 4-bromo-3-(méthylthio)benzène-1-sulfonyle (500 mg; 1.57 mmol) est ajouté sur le isopropyl-(4-méthoxy-2-méthyl-phényl)-amine (290 mg; 1.62 mmol) et la pyridine (2.4ml). Le milieu réactionnel est chauffé pendant 20 minutes à une température de 100°C sous micro-onde hydrolysé et extrait à l'acétate d'éthyle. Les phases organiques sont rassemblées puis lavées avec une solution d'acide chlorhydrique 1N puis avec une solution saturée de chlorure de sodium et séchées (MgSO₄). Les solvants sont évaporés.

Le produit brut est chromatographié sur gel de silice (éluant Heptane/Acétate d'éthyle de 0 à 40% d'acétate d'éthyle). Le 4-bromo-N-isopropyl-N-(4-methoxy-2-methyl-phenyl)-3-methylsulfanyl-benzenesulfonamide (570 mg; 81%) est obtenu sous la forme d'une huile jaune avec une RMN conforme
MS : [M+H] = 444

### 3. Synthèse de l'intermédiaire 114.3

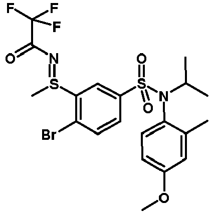

### (E)-N-((2-bromo-5-(N-isopropyl-N-(4-méthoxy-2-methylphenyl)sulfamoyl)phenyl)-(methyl)-λ⁴-sulfanylidene)-2,2,2-trifluoroacetamide

Le 4-bromo-N-isopropyl-N-(4-méthoxy-2-méthyl-phényl)-3-méthylsulfanyl-benzenesulfonamide (565mg; 1.27 mmol) et le 2,2,2-trifluoroacétamide (215.6 mg; 1.91 mmol) en solution dans le tétrahydrofurane (1.1ml) sont ajoutés lentement sur l'hydrure de sodium 60% (45.8 mg; 1.14 mmol) en suspension dans le tétrahydrofurane (2.8ml) à 0-5°C puis la solution de 1,3-dibromo-5,5-diméthylhydantoin (545.3 mg; 1.91 mmol) le tétrahydrofurane (1.13 ml). Le milieu est agité pendant 2 heures à température ambiante, hydrolysé par addition d'une solution d'acide citrique à 10% puis extrait à l'acétate d'éthyle. Les phases organiques sont rassemblées, lavées avec une solution de sulfite de sodium à 25% puis par 2 fois avec une solution saturée de chlorure de sodium et séchées (Na₂SO₄). Les solvants sont évaporés.

Le produit brut est chromatographié sur gel de silice (éluant heptane/acétate d'éthyle de 0 à 60% d'acétate d'éthyle). Le (E)-N-((2-bromo-5-(N-isopropyl-N-(4-methoxy-2-methylphenyl)sulfamoyl)phenyl)(methyl)-14-sulfanylidene)-2,2,2-trifluoroacetamide (706 mg; 100 %) est obtenu sous la forme d'une huile incolore avec une RMN conforme
MS : [M-H] = 557

### 4. Synthèse de l'intermédiaire 114.4

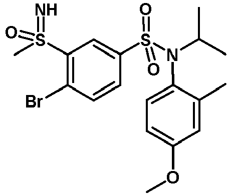

### 4-bromo-N-isopropyl-N-(4-méthoxy-2-méthylphényl)-3-(S-methylsulfonimidoyl)-benzenesulfonamide

Le carbonate de potassium (549 mg; 3.97 mmol) est ajouté sur le (E)-N-((2-bromo-5-(N-isopropyl-N-(4-methoxy-2-méthylphényl)sulfamoyl)phényl)(méthyl)-14-sulfanylidene)-2,2,2-trifluoroacetamide (735 mg; 1.32 mmol) en solution dans le méthanol (7.4ml) puis, à 0°C, l'acide 3-chloroperoxybenzoique (445mg; 1.98 mmol) est ajouté lentement. Le milieu réactionnel est agité pendant 2 heures à température ambiante, hydrolysé puis extrait à l'acétate d'éthyle. Les phases organiques sont rassemblées, lavées avec une solution saturée de chlorure de sodium et séchées (MgSO₄). Les solvants sont évaporés.

Le produit brut est chromatographié sur gel de silice (Heptane/Acétate d'éthyle de 40 à 80% d'acétate d'éthyle). Le 4-bromo-N-isopropyl-N-(4-méthoxy-2-methylphenyl)-3-(S-methylsulfonimidoyl)benzenesulfonamide (211.6 mg; 34 %) est obtenu sous la forme d'un solide blanc avec une RMN conforme
MS : [M+H] = 477

### 5. Synthèse du composé N-isopropyl-N-(4-méthoxy-2-methylphenyn-3-(S-methylsulfonimidoyn-4-((tétrahydro-2H-pyran-4-yl)méthoxy)benzenesulfonamide

L'hydrure de sodium 60% (12.6 mg; 0.32 mmol) est ajouté lentement à 0°C sur le (tétrahydro-pyran-4-yl)-méthanol (26.8 mg; 0.23 mmol) en solution dans le N,N-diméthylformamide (2ml) puis le 4-bromo-N-isopropyl-N-(4-méthoxy-2-methylphenyl)-3-(S-methylsulfonimidoyl)benzenesulfonamide (100mg; 0.21 mmol) est ajouté. Le milieu réactionnel est agité pendant une heure à température ambiante et 90 minutes à une température de 60°C puis hydrolysé et extrait à l'acétate d'éthyle. Les phases organiques sont rassemblées puis lavées avec une solution saturée de chlorure de sodium et séchées (Na₂SO₄). Les solvants sont évaporés.

Le produit est chromatographié sur gel de silice (éluant Dichlorométhane/Méthanol de 0 à 10% de méthanol).

Le N-isopropyl-N-(4-methoxy-2-methylphenyl)-3-(S-methylsulfonimidoyl)-4-((tetrahydro-2H-pyran-4-yl)methoxy)benzenesulfonamide (76.2 mg; 71 %) est obtenu sous la forme d'un solide blanc..
¹H NMR (DMSO-d6) δ: 0.79 - 0.93 (m, 4H), 0.98 (dd, J = 6.8, 2.1 Hz, 3H), 1.26 (dd, J = 10.8, 4.6 Hz, 2H), 1.41 (tdd, J = 12.3, 7.6, 4.4 Hz, 2H), 1.75 (t, J = 11.7 Hz, 2H), 2.10 (d, J = 8.7 Hz, 1H), 2.25 (d, J = 8.3 Hz, 3H), 3.19 (d, J = 1.2 Hz, 3H), 3.33 - 3.43 (m, 2H), 3.82 - 3.99 (m, 2H), 4.02 - 4.19 (m, 2H), 4.34 - 4.55 (m, 2H), 6.57 - 6.76 (m, 2H), 6.92 (t, J = 2.2 Hz, 1H), 7.41 (dd, J = 8.8, 2.9 Hz, 1H), 7.84 (dd, J = 8.7, 2.4 Hz, 1H), 8.14 (t, J = 2.7 Hz, 1H)
MS : [M+H] = 511

### Partie VI : Synthèse de sulfonamides soufrés à partir du schéma réactionnel 6

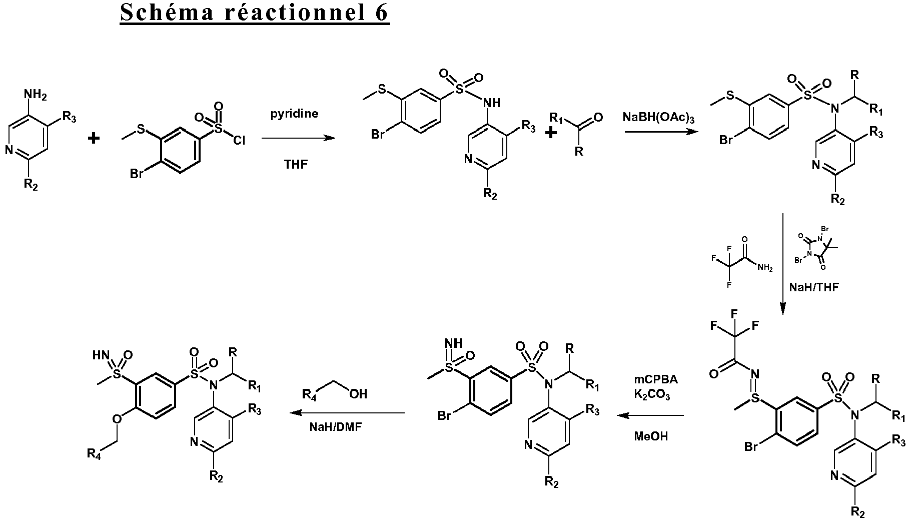

### Exemple 116 : Synthèse du N-(4-éthyl-phényl)-N-isobutyl-4-(tétrahydro-pyran-4-ylméthylsulfanyl)-benzene-sulfonamide

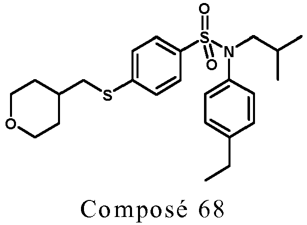

### 1. Synthèse de l'intermédiaire 116.1

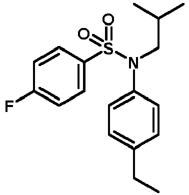

### N-(4-éthyl-phényl)-4-fluoro-N-isobutyl-benzenesulfonamide

Le chlorure de 4-fluoro-benzenesulfonyle (2.78 g; 14.27 mmol) est ajouté sur la (4-éthyl-phényl)-isobutyl-amine (2.3 g; 12.28 mmol) et la diisopropylamine (5.5 ml; 67.69 mmol) en solution dans le dichlorométhane (25 ml). Le milieu réactionnel est agité pendant 16 heures à température ambiante, hydrolysé et extrait à l'acétate d'éthyle. Les phases organiques sont rassemblées, lavées avec une solution saturée de chlorure d'ammonium, à la saumure, séchées (Na₂SO₄) et concentrées.

Le produit brut est chromatographié sur gel de silice (éluant Heptane/Acétate d'éthyle de 0 à 10% d'acétate d'éthyle). Le N-(4-éthyl-phényl)-4-fluoro-N-isobutyl-benzenesulfonamide (2.09 g; 48 %) est obtenu sous la forme d'un solide jaune orangé avec une RMN¹H conforme.
MS : [M+H] = 336

### 2. Synthèse de l'intermédiaire 116.2

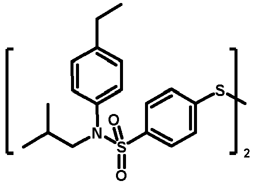

### Bis-[4-[(4-ethyl-phenyl)-isobutyl-sulfamoyl]-thiobenzene]-disulfide

Un mélange de N-(4-éthyl-phényl)-4-fluoro-N-isobutyl-benzenesulfonamide (1.0 g; 2.98 mmol) et d'hydrogénosulfure de sodium (2.1 g; 37.26 mmol) dans de la 1-méthyl-2-pyrrolidinone (4 ml) est agité 2 heures à 80°C puis 16h à température ambiante. Le milieu réactionnel est dilué à l'acétate d'éthyle et acidifié par addition de HCl concentré puis extrait. Les phases organiques sont rassemblées, lavées à l'eau, séchées (MgSO₄), filtrées, concentrées à sec. Le bis-[4-[(4-ethyl-phenyl)-isobutyl-sulfamoyl]-thiobenzene]-disulfide (1.04 g; 50%) obtenu est engagé directement dans la réaction suivante.
MS : [M+H] = 698

### 3. Synthèse du N-(4-éthyl-phényl)-N-isobutyl-4-(tétrahydro-pyran-4-ylmethylsulfanyl)-benzenesulfonamide

A une solution de bis-[4-[(4-Ethyl-phenyl)-isobutyl-sulfamoyl]-thiobenzene]-disulfide (1.03 g; 1.48 mmol) dans du N,N-diméthylformamide (15 ml) est ajouté du carbonate de potassium (0.41 g; 2.96 mmol). Le milieu réactionnel est agité pendant 5 minutes avant d'ajouter le 4-(bromométhyl)-tétrahydropyrane (0.53 g; 2.96 mmol) puis le formaldhyde-sulfoxylate de sodium (0.60 g; 4.44 mmol) et de l'eau (20 µl; 1.10 mmol). Le milieu réactionnel est agité 1heure à température ambiante, hydrolysé et extrait à l'acétate d'éthyle. Les phases organiques sont rassemblées, lavées à la saumure, séchées (MgSO₄), filtrées et concentrées. Le brut est chromatographié sur gel de silice, éluant heptane acétate d'éthyle 5 à 30% d'acétate d'éthyle). Le N-(4-ethyl-phenyl)-N-isobutyl-4-(tetrahydro-pyran-4-ylmethylsulfanyl)-benzenesulfonamide (1.10 g; 83 %) est obtenu sous la forme d'un solide.
¹H NMR (400 MHz, DMSO-d6) δ 7.55 - 7.34 (m, 4H), 7.23 - 7.15 (m, 2H), 7.02 - 6.93 (m, 2H), 3.90 - 3.81 (m, 2H), 3.30 - 3.21 (m, 3H), 3.02 (d, J = 6.6 Hz, 2H), 2.60 (q, J = 7.6 Hz, 2H), 1.79 - 1.68 (m, 2H), 1.48 - 1.36 (m, 1H), 1.36 - 1.22 (m, 1H), 1.18 (t, J = 7.6 Hz, 3H), 0.84 (d, J = 6.7 Hz, 6H).
MS : [M+H] = 448

### Exemple 117 : Synthèse du N-(4-éthyl-phényl)-N-isobutyl-4-(tétrahydro-pyran-4-ylmethanesulfinyl)-benzenesulfonamide

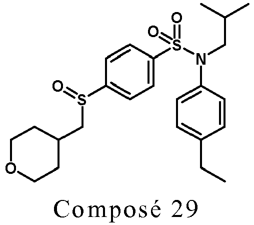

A une solution à 0°C de N-(4-éthyl-phényl)-N-isobutyl-4-(tétrahydro-pyran-4-ylméthylsulfanyl)-benzenesulfonamide (787 mg; 1.76 mmol) dans du dichlorométhane (20 ml) est ajouté par portion de l'acide 3-chloroperbenzoïque (414 mg; 1.85 mmol).

Le milieu est agité pendant 3 heures 30. A 0°C, 13ml d'une solution de soude 1N est ajoutée goutte à goutte puis 13 ml d'eau. Le milieu réactionnel est extrait au dichlorométhane. Les phases organiques sont lavées avec une solution aqueuse de thiosulfate de sodium, séchées sur sulfate de magnésium, filtrées et concentrées. L'huile obtenue est précipitée avec du dichlorométhane et de l'heptane. Le solide est filtré, rincé à l'heptane et séché. Le N-(4-éthyl-phényl)-N-isobutyl-4-(tetrahydro-pyran-4-ylmethanesulfinyl)-benzenesulfonamide (755 mg; 88 %) est obtenu sous la forme d'un solide blanc.
1H NMR (400 MHz, DMSO-d6) δ0.86 (d, J = 6.6 Hz, 7H), 1.18 (t, J = 7.6 Hz, 3H), 1.51 - 1.24 (m, 3H), 1.55 (d, J = 13.3 Hz, 1H), 1.83 (d, J = 13.0 Hz, 1H), 2.16 - 1.98 (m, 1H), 2.60 (q, J = 7.6 Hz, 2H), 2.81 (dd, J = 13.1, 5.0 Hz, 1H), 2.93 (dd, J = 13.2, 8.6 Hz, 1H), 3.38 - 3.28 (m, 4H), 3.95 - 3.70 (m, 2H), 7.08 - 6.85 (m, 2H), 7.27 - 7.14 (m, 2H), 7.80 - 7.66 (m, 2H), 7.95 - 7.81 (m, 2H), MS : [M+H] = 464

Le composé N-(4-éthyl-phényl)-N-isobutyl-4-(tetrahydro-pyran-4-ylmethanesulfinyl)-benzenesulfonamide (450 mg; 1.11 mmol) est chromatographié par SFC chirale pour séparer de les 2 énantiomères (composé 13 et composé 14) ci-dessous :
[Conditions super critiques 100bar, 70°C ; colonne CHIRALPAK IC 250x4.6mm 5µ éluant CO₂/Ethanol : 30% d'éthanol]

### Exemple 118 : N-(4-éthyl-phényl)-N-isobutyl-4-(tétrahydro-pyran-4-ylmethanesulfinyl)-benzene-sulfonamide (composé 13) - énantiomère A du composé 29

(150 mg; 22 %) sous la forme d'un solide cristallin blanc
¹H NMR (400 MHz, DMSO-d6) δ 7.91 - 7.84 (m, 2H), 7.76 - 7.69 (m, 2H), 7.20 (d, J = 8.4 Hz, 2H), 6.98 (d, J = 8.3 Hz, 2H), 3.84 (dd, J = 22.1, 12.3 Hz, 2H), 3.45 - 3.17 (m, 4H), 2.93 (dd, J = 13.2, 8.6 Hz, 1H), 2.81 (dd, J = 13.1, 5.0 Hz, 1H), 2.66 - 2.48 (m, 2H), 2.08 (dddd, J = 19.9, 12.3, 8.7, 4.1 Hz, 1H), 1.89 - 1.79 (m, 1H), 1.60 - 1.25 (m, 4H), 1.18 (t, J = 7.6 Hz, 3H), 0.86 (d, J = 6.7 Hz, 6H).
Temps de rétention (SFC chirale) de 6.92 minutes.

### Exemple 119: N-(4-éthyl-phényl)-N-isobutyl-4-(tetrahydro-pyran-4-ylmethanesulfinyl)-benzene-sulfonamide (composé 14) - énantiomère B du composé 29

(120 mg; 18 %) sous la forme d'un solide blanc
¹H NMR (400 MHz, DMSO-d6) δ 7.92 - 7.80 (m, 2H), 7.80 - 7.64 (m, 2H), 7.28 - 7.11 (m, 2H), 7.04 - 6.92 (m, 2H), 3.85 (ddd, J = 21.3, 10.9, 4.2 Hz, 2H), 3.47 - 3.18 (m, 4H), 2.93 (dd, J = 13.2, 8.6 Hz, 1H), 2.81 (dd, J = 13.1, 5.0 Hz, 1H), 2.61 (q, J = 7.6 Hz, 2H), 2.08 (dtt, J = 19.8, 8.2, 4.1 Hz, 1H), 1.94 - 1.72 (m, 1H), 1.64 - 1.24 (m, 4H), 1.18 (t, J = 7.6 Hz, 3H), 0.86 (d, J = 6.7 Hz, 6H).
Temps de rétention (SFC chirale) de 9.31 minutes

### Exemple 120 : Synthèse du N-(4-éthyl-phényl)-N-isobutyl-4-(tetrahydro-pyran-4-ylmethanesulfoximinyl)-benzenesulfonamide

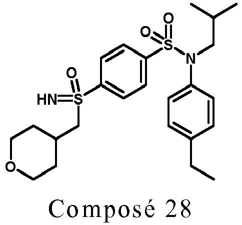

A une solution, préalablement dégazé à l'argon de N-(4-éthyl-phényl)-N-isobutyl-4-(tétrahydro-pyran-4-ylméthanesulfinyl)-benzenesulfonamide (199 mg; 0.43 mmol) dans du dichlorométhane (7 ml), sont ajoutés le 2,2,2- trifluoroacétamide (121 mg; 1.07 mmol), l'oxyde de magnésium (87 mg; 2.15 mmol), le rhodium(II) acétate dimère (28 mg; 0.06 mmol) et l'iodobenzène diacétate (263 mg; 0.82 mmol). Le milieu réactionnel est agité à température ambiante pendant 20 heures, filtré sur célite et concentré. Le résidu obtenu est dilué dans du méthanol (7 ml) et du carbonate de potassium (297 mg; 2.15 mmol) est ajouté.

Le milieu est agité pendant 30 minutes à température ambiante, hydrolysé et extrait à l'acétate d'éthyle. Les phases organiques sont rassemblées, lavées à la saumure, séchées (Na₂SO₄) et concentrées.

Le produit brut est chromatographié sur gel de silice (éluant heptane acétate d'éthyle 10/90). Le N-(4-éthyl-phényl)-N-isobutyl-4-(tetrahydro-pyran-4-ylmethane-sulfoximinyl)-benzenesulfonamide (87 mg; 41%) est obtenu sous la forme d'un solide crème
¹H NMR (400 MHz, DMSO-d6) δ0.86 (dd, J = 6.8, 1.9 Hz, 6H), 1.35 - 1.06 (m, 5H), 1.44 (dt, J = 13.5, 6.9 Hz, 1H), 1.77 - 1.50 (m, 2H), 2.06 (t, J = 9.2 Hz, 1H), 2.61 (d, J = 7.6 Hz, 2H), 3.29 - 3.16 (m, 3H), 3.37 (d, J = 7.2 Hz, 2H), 3.76 (dt, J = 11.7, 3.1 Hz, 2H), 4.55 (s, 1H), 7.07 - 6.84 (m, 2H), 7.19 (dd, J = 8.6, 2.0 Hz, 2H), 7.89 - 7.62 (m, 2H), 8.09 (dd, J = 8.4, 2.0 Hz, 2H). MS : [M+H] = 479

### Partie VII : Synthèse de sulfonamides soufrés à partir du schéma réactionnel 7

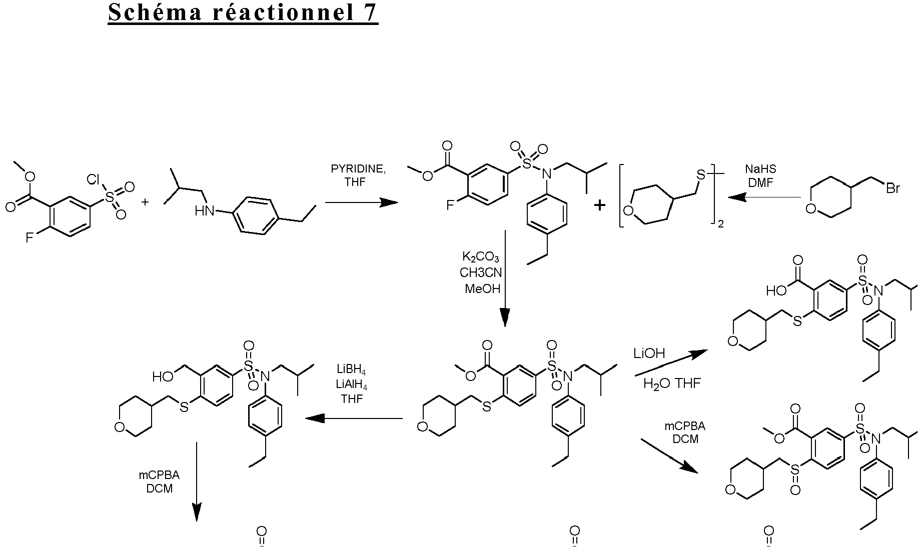

### Exemple 121 : Synthèse du 5-[(4-Ethyl-phenyl)-isobutyl-sulfamoyl]-2-(tetrahydro-pyran-4-ylmethylsulfanyl)-benzoate de méthyle

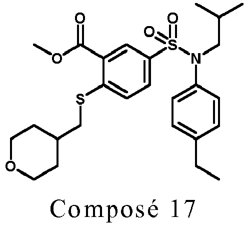

### 1. Synthèse de l'intermédiaire 121.1

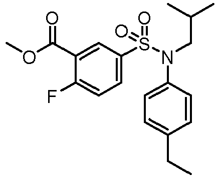

### 5-[(4-éthyl-phényl)-isobutyl-sulfamoyl]-2-fluoro-benzoate de méthyle

Le 5-chlorosulfonyl-2-fluoro-benzoate de méthyle (720 mg; 2.85 mmol) est ajouté sur la (4-éthyl-phényl)-isobutyl-amine (0.95 g; 4.27 mmol) et la pyridine (1.38 ml; 0.02 mol; 6.00 eq.) en solution dans le tétrahydrofurane (16 ml). Le milieu réactionnel est agité à température ambiante pendant 16 heures, hydrolysé et extrait à l'acétate d'éthyle. Les phases organiques sont rassemblées, lavées à la saumure, séchées (Na₂SO₄) et concentré.

Le produit brut est chromatographié sur gel de silice (éluant heptane acétate d'éthyle 5 à 20% d'acétate d'éthyle). Le 5-[(4-éthyl-phényl)-isobutyl-sulfamoyl]-2-fluoro-benzoate de méthyle (800 mg; 71%) est obtenu sous la forme d'un solide beige avec une RMN¹H conforme.
MS : [M+H] = 394

### 2. Synthèse de l'intermédiaire 121.2

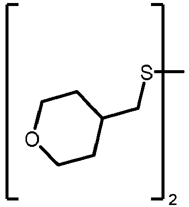

### Bis-[-(Tetrahydro-pyran-4-yl)-methane-bisulfide

Un mélange de 4-(bromométhyl)-tétrahydropyrane (1.0 g; 5.58 mmol) et d'hydrogénosulfure de sodium (0.44 g; 7.82 mmol) dans du diméthylformamide (4 ml) est agité à température ambiante pendant 2 heures. Le milieu réactionnel est dilué à l'éther et acidifié par addition de HCl concentré puis extrait. Les phases organiques sont rassemblées, lavées à l'eau, séchées sur sulfate de magnésium, filtrées, concentrées à sec. Le bis-[-(tetrahydro-pyran-4-yl)-méthane-bisulfide (565.00 mg; 77%) obtenu sous la forme d'une huile claire est engagé directement dans la réaction suivante.
MS : [M+H] = 263

### 3. Synthèse du 5-[(4-éthyl-phényl)-isobutyl-sulfamoyl]-2-(tetrahydro-pyran-4-ylmethylsulfanyl)-benzoate de méthyle

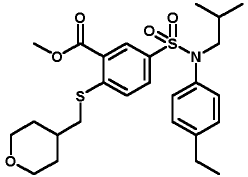

Sur du 5-[(4-éthyl-phényl)-isobutyl-sulfamoyl]-2-fluoro-benzoate de méthyle (200 mg; 0.51 mmol) et du bis-(tetrahydropyran-4-yl)-méthane-bisulfide (133.39 mg; 0.51 mmol; 1.00 eq.) dans de l'acétonitrile (2 ml) est ajouté du carbonate de potassium (81mg; 0.59 mmol). Le milieu réactionnel est agité à température ambiante pendant 16 heures, hydrolysé et extrait à l'acétate d'éthyle. Les phases organiques sont combinées, lavées à la saumure, séchées et concentrées.

Le produit brut est chromatographié sur gel de silice, éluant heptane acétate d'éthyle : 5 à 20% d'acétate d'éthyle. Le 5-[(4-Ethyl-phenyl)-isobutyl-sulfamoyl]-2-(tetrahydro-pyran-4-ylmethylsulfanyl)-benzoate de méthyle (214 mg; 83 %) est obtenu sous la forme d'un solide blanc.
1H NMR (400 MHz, DMSO-d6) δ 0.85 (d, J = 6.6 Hz, 7H), 1.18 (t, J = 7.6 Hz, 3H), 1.37 (dtd, J = 36.3, 12.9, 12.1, 7.3 Hz, 3H), 1.91 - 1.70 (m, 4H), 2.61 (q, J = 7.6 Hz, 2H), 2.98 (d, J = 6.7 Hz, 2H), 3.35 - 3.24 (m, 4H), 3.91 - 3.80 (m, 5H), 7.05 - 6.98 (m, 2H), 7.23 - 7.17 (m, 2H), 7.66 - 7.59 (m, 2H), 7.89 (d, J = 1.9 Hz, 1H).
MS : [M+H] = 506

### Exemple 122 : Synthèse du 5-[(4-Ethyl-phenyl)-isobutyl-sulfamoyl]-2-(tetrahydro-pyran-4-ylmethanesulfinyl)-benzoate de méthyle

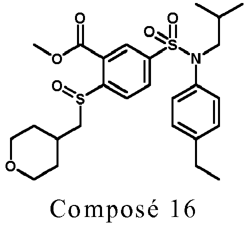

Sur une solution à une température de 0°C de 5-[(4-éthyl-phényl)-isobutyl-sulfamoyl]-2-(tétrahydro-pyran-4-ylméthylsulfanyl)-benzoate de méthyle (150mg; 0.30 mmol) dans du dichlorométhane (5 ml) est ajouté de l'acide 3-perchlorobenzoïque (70 mg; 0.31 mmol). Le milieu réactionnel est agité pendant 2 heures. A 0°C, de la soude 1N est ajoutée goutte à goutte et de l'eau puis le milieu réactionnel est extrait au dichlorométhane.

Les phases organiques sont lavées avec une solution aqueuse de thiosulfate de sodium, séchées sur sulfate de magnésium, filtrées et évaporées.

Le produit brut est chromatographié sur gel de silice, (éluant heptane acétate d'éthyle, 10 à 30% d'éthyle). Le 5-[(4-éthyl-phényl)-isobutyl-sulfamoyl]-2-(tetrahydro-pyran-4-ylmethanesulfinyl)-benzoate de méthyle (140 mg; 90 %) est obtenu sous la forme d'un solide blanc.
1H NMR (400 MHz, DMSO-d6) δ 0.86 (dd, J = 6.7, 4.0 Hz, 6H), 1.19 (t, J = 7.6 Hz, 3H), 1.67 - 1.23 (m, 3H), 1.97 (dd, J = 16.3, 5.1 Hz, 1H), 2.66 - 2.55 (m, 3H), 2.22 (s, 1H), 3.09 (dd, J = 12.8, 9.7 Hz, 1H), 3.44 - 3.31 (m, 4H), 3.89 (s, 5H), 7.07 - 6.97 (m, 2H), 7.22 (d, J = 8.3 Hz, 2H), 8.02 (d, J = 1.9 Hz, 1H), 8.08 (dd, J = 8.3, 2.0 Hz, 1H), 8.30 (d, J = 8.3 Hz, 1H).
MS : [M+H] = 522

### Exemple 123 : Synthèse de l'acide 5-[(4-Ethyl-phenyl)-isobutyl-sulfamoyl]-2-(tetrahydro-pyran-4-ylmethylsulfanyl)-benzoïque

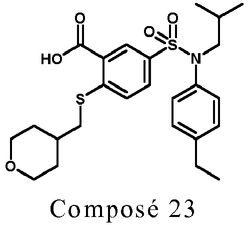

Un mélange 5-[(4-éthyl-phényl)-isobutyl-sulfamoyl]-2-(tetrahydro-pyran-4-ylméthylsulfanyl)-tetrahydro-pyran-4-yl-benzoate de méthyle (277 mg; 0.47 mmol) et d'hydroxyde de lithium (0.70 ml; 1.00 M; 0.70 mmol) dans du tétrahydrofurane (6.93 ml) est agité à une température de 60°C pendant 24 heures. Le milieu réactionnel est hydrolysé avec de la soude 1N, extrait à l'acétate d'éthyle. Les phases organiques sont réunies, lavées à la soude 1N.

Les phases aqueuse sont réunies, acidifiées avec HCl et extrait à l'acétate d'éthyle. Les phases organiques sont rassemblées, lavées à la saumure, séchées sur sulfate de magnésium, filtrées et concentrées.

Le produit brut est chromatographié sur gel de silice, éluant heptane acétate d'éthyle +1%AcOH, 10 à 50% d'acétate d'éthyle. L'acide 5-[(4-éthyl-phényl)-isobutyl-sulfamoyl]-2-(tétrahydro-pyran-4-ylméthylsulfanyl)-benzoique (87.00 mg; 36%) est obtenu sous la forme d'un solide après cristallisation dans un mélange méthanol, dichlorométhane.
1H NMR (400 MHz,DMSO-d6) δ 0.85 (d, J =6.6 Hz, 6H), 1.18 (t,J = 7.6 Hz, 3H), 1.50 - 1.24 (m, 3H), 1.89 - 1.67 (m, 3H), 2.66 - 2.55 (m,2H), 2.94 (d, J = 6.6Hz, 2H), 3.30 (d, J =7.1 Hz, 3H), 4.03 -3.71 (m, 2H), 7.04 - 6.97 (m, 2H), 7.25 - 7.16 (m, 2H), 7.59 (s,2H), 8.10 - 7.74 (m, 1H), 13.46 (s, 1H).
MS : [M+H] = 492

### Exemple 124 : Synthèse N-(4-éthyl-ohényl)-3-hydroxymethyl-N-isobutyl-4-(tétrahydro-pyran-4-ylmethyl-sulfanyl)-benzenesulfonamide

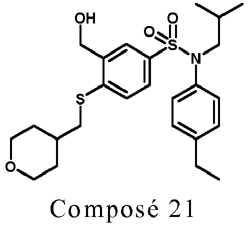

Au 5-[(4-éthyl-phényl)-isobutyl-sulfamoyl]-2-(tétrahydropyran-4-ylméthyl-sulfanyl)-benzoate de méthyle (113 mg; 0.22 mmol) dans le tétrahydrofurane (3 ml) est ajouté du borohydrure de Lithium (9 mg; 0.40 mmol). Le milieu réactionnel est agité à température ambiante pendant 16 heures, hydrolysé avec de l'acide citrique 5% pendant 1 heure, extrait à l'acétate d'éthyle. Les phases organiques sont rassemblées, lavées à la saumure, séchées sur sulfate de magnésium, filtrées et évaporées.

Le produit brut est chromatographié sur gel de silice (éluant heptane acétate d'éthyle 80/20). Le N-(4-éthyl-phényl)-3-hydroxyméthyl-N-isobutyl-4-(tetrahydro-pyran-4-ylméthylsulfanyl)-benzenesulfonamide (106mg, 96%) est obtenu sous la forme d'un solide blanc.
¹H NMR (Chloroforme-d) δ: 0.84 (d, J = 6.7 Hz, 6H), 1.16 (t, J = 7.6 Hz, 3H), 1.35 (qd, J = 13.1, 12.4, 3.6 Hz, 2H), 1.50 (hept, J = 6.7 Hz, 1H), 1.73 (d, J = 12.3 Hz, 1H), 2.51 - 2.62 (m, 2H), 2.64 (d, J = 1.7 Hz, 2H), 2.87 (d, J = 6.5 Hz, 2H), 3.22 (d, J = 7.4 Hz, 2H), 3.31 (td, J = 11.8, 1.9 Hz, 2H), 3.92 (ddd, J = 12.5, 4.6, 1.5 Hz, 2H), 4.65 (s, 2H), 6.87 - 6.94 (m, 2H), 7.06 (d, J = 8.3 Hz, 2H), 7.19 (d, J = 8.2 Hz, 1H), 7.33 (dd, J = 8.3, 2.2 Hz, 1H), 7.56 (d, J = 2.1 Hz, 1H)
MS : [M+H] = 478

### Exemple 125 : Synthèse du N-(4-éthyl-phényl)-3-hydroxymethyl-N-isobutyl-4-(tetrahydro-pyran-4-ylmethane-sulfinyl)-benzenesulfonamide

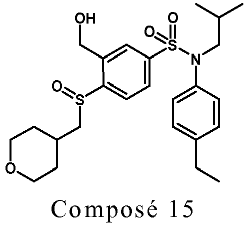

Sur une solution de N-(4-éthyl-phényl)-3-hydroxyméthyl-N-isobutyl-4-(tétrahydro-pyran-4-ylméthyl-sulfanyl)-benzenesulfonamide (614.00 mg; 1.29 mmol) dans du dichlorométhane (20.00 ml) est ajouté de l'acide 3-chloroperoxybenzoïque (302.47 mg; 1.35 mmol). Le milieu est agité à température ambiante pendant 4 heures. A une température de 0°C, le milieu réactionnel est ajouté à de l'eau et extrait à l'acétate d'éthyle. Les phases organiques sont lavées à la saumure, séchées sur sulfate de magnésium, filtrées et évaporées.

Le produit brut est chromatographié sur gel de silice, éluant heptane acétate d'éthyle 50 à 100% d'acétate d'éthyle). Le N-(4-ethyl-phenyl)-3 -hydroxymethyl-N-isobutyl-4-(tetrahydro-pyran-4-ylmethanesulfinyl)-benzenesulfonamide (455 mg, 71%) est obtenu sous la forme d'un solide blanc.
¹H NMR (DMSO-d₆) δ 0.84 (d, J = 6.7 Hz, 6H), 1.16 (t, J = 7.6 Hz, 3H), 1.19 - 1.50 (m, 3H), 1.47 - 1.63 (m, 1H), 1.86 (ddd, J = 13.1, 3.9, 2.1 Hz, 1H), 2.12 (dq, J = 15.5, 5.7, 4.0 Hz, 2H), 2.55 - 2.69 (m, 3H), 2.94 (dd, J = 13.2, 9.4 Hz, 1H), 3.33 (d, J = 3.1 Hz, 3H), 3.74 - 3.95 (m, 2H), 4.60 (dd, J = 5.3, 3.3 Hz, 2H), 5.65 (dd, J = 5.9, 4.8 Hz, 1H), 6.86- 7.07 (m, 2H), 7.10 - 7.30 (m, 2H), 7.57 - 7.76 (m, 2H), 8.01 (d, J = 8.1 Hz, 1H).
MS : [M+H] = 494

Le composé 15 (377 mg; 0.76 mmol) est chromatographié par SFC chirale pour séparer de les 2 énantiomères ci-dessous
[Conditions super critiques 100bar, 70°C ; colonne CHIRALPAK IC 250x4.6mm 5µ éluant CO₂/méthanol : 45% de méthanol]

### Exemple 126: N-(4-éthyl-phenyl)-3-hydroxymethyl-N-isobutyl-4-(tetrahydro-pyran-4-ylmethane-sulfinyl)-benzenesulfonamide (composé 11) -énantiomère A du composé 15

(146 mg; 39 %) sous la forme d'un solide blanc
¹H NMR (400 MHz, DMSO-d6) δ 8.01 (d, J = 8.1 Hz, 1H), 7.76 - 7.57 (m, 2H), 7.30 - 7.10 (m, 2H), 7.07 - 6.86 (m, 2H), 5.65 (dd, J = 5.9, 4.8 Hz, 1H), 4.60 (dd, J = 5.3, 3.3 Hz, 2H), 3.95 - 3.74 (m, 1H), 3.33 (d, J = 3.1 Hz, 2H), 2.94 (dd, J = 13.2, 9.4 Hz, 1H), 2.69 - 2.55 (m, 2H), 2.12 (dq, J = 15.5, 5.7, 4.0 Hz, 1H), 1.86 (ddd, J = 13.1, 3.9, 2.1 Hz, 1H), 1.63 - 1.47 (m, 1H), 1.50 - 1.19 (m, 2H), 1.16 (t, J = 7.6 Hz, 3H), 0.84 (d, J = 6.7 Hz, 6H).
Temps de rétention (SFC chirale) de 2.49 minutes

### Exemple 127: N-(4-éthyl-phényl)-3-hydroxymethyl-N-isobutyl-4-(tetrahydro-pyran-4-ylmethane-sulfinyl)-benzenesulfonamide (composé 12) - énantiomère B du composé 15

(134 mg; 36 %) sous la forme d'un solide blanc
¹H NMR (400 MHz, DMSO-d6) δ 8.01 (d, J = 8.1 Hz, 1H), 7.76 - 7.57 (m, 2H), 7.30 - 7.10 (m, 2H), 7.07 - 6.86 (m, 2H), 5.65 (dd, J = 5.9, 4.8 Hz, 1H), 4.60 (dd, J = 5.3, 3.3 Hz, 2H), 3.95 - 3.74 (m, 1H), 3.33 (d, J = 3.1 Hz, 2H), 2.94 (dd, J = 13.2, 9.4 Hz, 1H), 2.69 - 2.55 (m, 2H), 2.12 (dq, J = 15.5, 5.7, 4.0 Hz, 1H), 1.86 (ddd, J = 13.1, 3.9, 2.1 Hz, 1H), 1.63 - 1.47 (m, 1H), 1.50 - 1.19 (m, 2H), 1.16 (t, J = 7.6 Hz, 3H), 0.84 (d, J = 6.7 Hz, 6H).
Temps de rétention (SFC chirale) de 2.92 minutes

### Partie VIII : Synthèse de sulfonamides soufrés à partir du schéma réactionnel 8

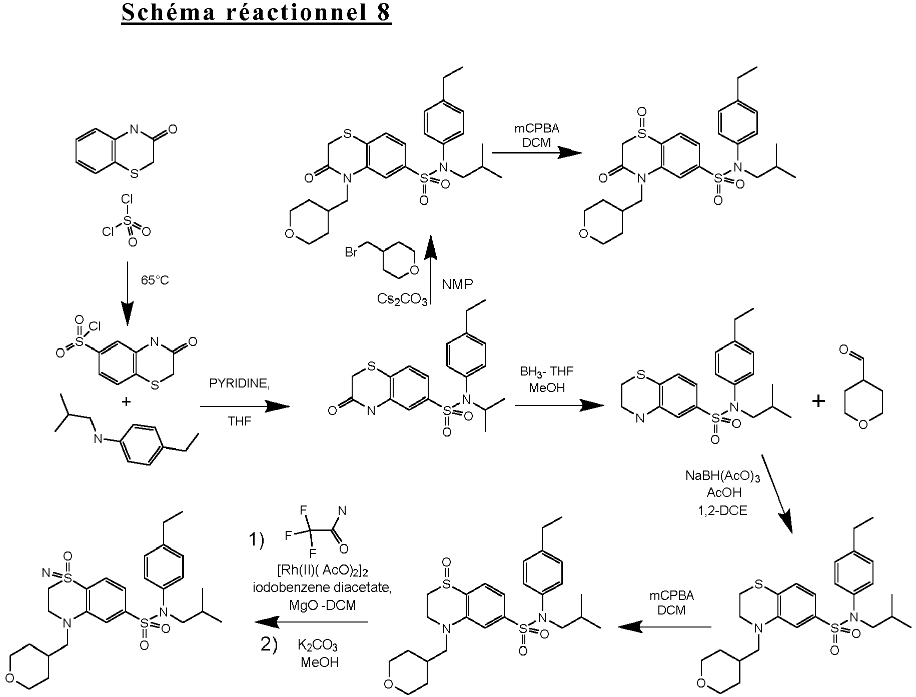

### Exemple 128 : Synthèse de l'acide 3-oxo-3,4-dihydro-2H-benzo[1,4]thiazine-6-sulfonique(4-éthyl-phényl)-isobutyl-amide

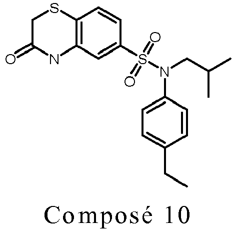

### 1. Synthèse de l'intermédiaire 128.1

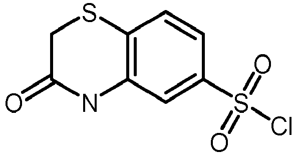

### Chlorure de 3-oxo-3,4-dihydro-2H-benzo-[1,4]thiazine-6-sulfonyle

Le 4H-benzo[1,4]thiazin-3-one (4.0 g; 24.21 mmol est ajouté lentement à de l'acide chlorosulfonique (6.5 ml; 96.84 mmol) refroidi à 10°C. La température est maintenue inférieure à 20°C. Le milieu réactionnel est agité à température ambiante pendant 1 heure puis chauffé à une température de 65°C, versé lentement sur de la glace puis extrait à l'acétate d'éthyle. Les phases organiques sont rassemblées, lavées avec de la saumure, séchées sur sulfate de sodium et concentrées. Le résidu est repris dans l'éther et essoré.

Le chlorure de 3-oxo-3,4-dihydro-2H-benzo-[1,4]-thiazine-6-sulfonyle (4.88 g; 76 %) est obtenu sous la forme d'une poudre ocre.
MS : [M+H] = 262

### 2. Synthèse du composé 10 selon l'invention

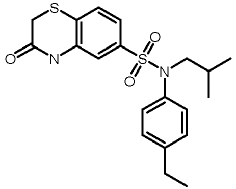

Le chlorure 3-oxo-3,4-dihydro-2H-benzo-[1,4]-thiazine-6-sulfonyl (3.27 g; 12.41 mmol) est ajouté à de la (4-éthyl-phényl)-isobutyl-amine: (2 g; 11.28 mmol) et de la pyridine (40 ml; 495.56 mmol) en solution dans le tétrahydrofuranne (5.4 ml). Le milieu réactionnel est agité à température ambiante pendant 2 heures, hydrolysé et extrait à l'acétate d'éthyle. Les phases organiques sont rassemblées, lavées avec une solution d'acide chlorhydrique 1N, avec de la saumure, séchées sur sulfate de sodium et concentrées.

Le produit brut est chromatographié sur gel de silice (éluant Heptane/Acétate d'éthyle de 0 à 50% d'acétate d'éthyle). L'acide 3-oxo-3,4-dihydro-2H-benzo-[1,4]-thiazine-6-sulfonique (4-éthyl-phényl)-isobutyl-amide (2.53 g; 55 %) est obtenu sous la forme d'un solide jaune
¹H NMR (Chloroforme-d) δ: 0.93 (d, J = 6.7 Hz, 7H), 1.25 (td, J = 7.6, 4.3 Hz, 4H), 1.53 - 1.67 (m, 2H), 2.67 (q, J = 7.6 Hz, 2H), 3.32 (d, J = 7.4 Hz, 2H), 3.50 (s, 2H), 6.94 - 7.04 (m, 2H), 7.07 (d, J = 1.9 Hz, 1H), 7.12 - 7.20 (m, 2H), 7.22 (dd, J = 8.2, 1.8 Hz, 1H), 7.41 (d, J = 8.2 Hz, 1H), 8.28 (s, 1H).
MS : [M+H] = 405

### Exemple 129 : Synthèse de l'acide 3,4-dihydro-2H-benzo[1,4]thiazine-6-sulfonique (4-éthyl-phényl)-isobutyl-amide

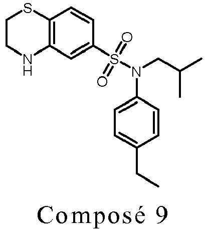

L'acide 3-oxo-3,4-dihydro-2H-benzo-[1,4]-thiazine-6-sulfonique (4-éthyl-phényl)-isobutyl-amide (500mg; 1.28 mmol) est mis en solution dans le complexe de borane tétrahydrofuranne 1M avec 5mmol NaBH₄ (35 ml). Le milieu réactionnel est chauffé à reflux pendant 30 minutes puis est refroidi à une température de 0°C et versé lentement sur du méthanol (35 ml).

Les solvants sont concentrés et le résidu est chromatographié sur gel de silice (éluant heptane/acétate d'éthyle de 0 à 40% d'acétate d'éthyle).

L'acide 3,4-dihydro-2H-benzo[1,4]thiazine-6-sulfonique (4-éthyl-phényl)-isobutyl-amide (437 mg; 86%) est obtenu sous la forme d'un solide blanc cristallin après recristallisation dans un mélange acétate d'éthyle/Heptane.
¹H NMR (DMSO-d6) δ: 0.82 (d, J = 6.7 Hz, 6H), 1.17 (t, J = 7.6 Hz, 3H), 1.31 - 1.47 (m, 1H), 2.59 (q, J = 7.6 Hz, 2H), 2.97 - 3.05 (m, 2H), 3.25 (d, J = 7.3 Hz, 2H), 3.48 (dt, J = 7.0, 3.0 Hz, 2H), 6.48 - 6.56 (m, 2H), 6.75 (d, J = 2.0 Hz, 1H), 6.95 - 7.04 (m, 3H), 7.14 - 7.21 (m, 2H).
MS : [M+H] = 391

### Exemple 130 : Synthèse de l'acide 4-(tétrahydro-pyran-4-ylméthyl)-3,4-dihydro-2H-benzo-[1,4]-thiazine-6-sulfonique (4-éthyl-phényl)-isobutyl-amide

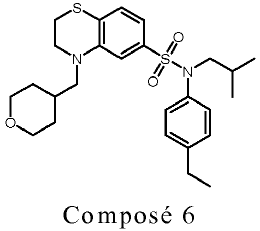

Le triacétoxyborohydrure de sodium (33 mg; 0.15 mmol) est ajouté à une température de 0°C à de l'acide 3,4-dihydro-2H-benzo[1,4]thiazine-6-sulfonique (4-éthyl-phényl)-isobutyl-amide (20 mg; 0.05 mmol), le 4-formyltétrahydropyranne (29 mg; 0.26 mmol) et de l'acide acétique (0.15 µl) en solution dans du 1,2-dichloroéthane. Le milieu réactionnel est agité à température ambiante pendant une durée de 24 heures, ajouté à de l'eau et extrait à l'acétate d'éthyle. Les phases organiques sont rassemblées, lavées à la saumure, séchées sur sulfate de sodium et concentrées.

Le produit brut est chromatographié sur gel de silice (éluant heptane/acétate d'éthyle de 0 à 50% d'acétate d'éthyle). L'acide 4-(tetrahydro-pyran-4-ylmethyl)-3,4-dihydro-2H-benzo-[1,4]-thiazine-6-sulfonique (4-éthyl-phényl)-isobutyl-amide (15 mg; 59 %) est obtenu sous la forme d'un solide beige.
1H NMR (DMSO-d6) δ: 0.84 (d, J = 6.6 Hz, 7H), 1.06 - 1.27 (m, 6H), 1.40 - 1.48 (m, 3H), 1.68 - 1.79 (m, 1H), 2.55 - 2.66 (m, 2H), 3.01 (d, J = 6.9 Hz, 2H), 3.08 (t, J = 4.8 Hz, 2H), 3.19 (t, J = 11.5 Hz, 2H), 3.26 (d, J = 7.3 Hz, 2H), 3.62 (t, J = 4.8 Hz, 2H), 3.82 (dd, J = 11.0, 4.2 Hz, 2H), 6.54 (s, 1H), 6.71 (d, J = 7.9 Hz, 1H), 7.02 (d, J = 8.0 Hz, 2H), 7.13 (d, J = 8.1 Hz, 1H), 7.21 (d, J = 7.9 Hz, 2H). MS : [M+H] = 489

### Exemple 131 : Synthèse de l'acide 1-oxo-4-(tetrahydro-pyran-4-ylméthyl)-1,2,3,4-tetrahydro-1λ⁴-benzo[1,4]thiazine-7-sulfonique (4-éthyl-phényl)-isobutyl-amide

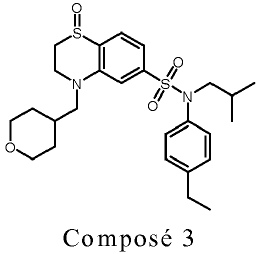

L'acide 3-chloroperbenzoïque (124 mg; 0.55 mmol) est ajouté, à une température de 0°C, sur l'acide 4-(tétrahydro-pyran-4-ylméthyl)-3,4-dihydro-2H-benzo[1,4]thiazine-7-sulfonique (4-éthyl-phényl)-isobutyl-amide (300 mg; 0.61 mmol) en solution du dichlorométhane (6ml). Le milieu réactionnel est agité à température ambiante pendant 30 minutes, hydrolysé avec une solution aqueuse à 10% de Na₂S₂O₃ et extrait au dichlorométhane. Les phases organiques sont rassemblées, lavées avec une solution de soude 0.1N, à la saumure, séchées sur sulfate de sodium et concentrées.

Le produit brut est chromatographié sur gel de silice (éluant dichlorométhane/méthanol de 0 à 10% de méthanol). L'acide 1-oxo-4-(tetrahydro-pyran-4-ylmethyl)-1,2,3,4-tetrahydro-1λ⁴-benzo[1,4]thiazine-7-sulfonique (4-éthyl-phényl)-isobutyl-amide (274 mg; 88 %) est obtenu sous la forme d'un solide blanc par cristallisation dans un mélange Eau/Acétone.
1H NMR (DMSO-d6) δ: 0.85 (dd, J = 6.6, 1.3 Hz, 6H), 1.12 - 1.28 (m, 5H), 1.44 (dt, J = 12.8, 9.6 Hz, 3H), 1.71 - 1.88 (m, 1H), 2.61 (q, J = 7.6 Hz, 2H), 2.85 (td, J = 13.6, 3.4 Hz, 1H), 3.09 - 3.36 (m, 12H), 3.64 (dt, J = 14.0, 3.8 Hz, 1H), 3.78 - 3.90 (m, 3H), 6.81 (d, J = 7.3 Hz, 2H), 7.01 - 7.08 (m, 2H), 7.19 - 7.26 (m, 2H), 7.70 (d, J = 8.2 Hz, 1H).
MS : [M+H] = 505

### Exemple 132 : Synthèse de l'acide 1-Imino-1-oxo-4-(tetrahydro-pyran-4-ylmethyl)-1,2,3,4-tetrahvdro-1λ⁶-benzo-[1,4]thiazine-6-sulfonique (4-éthyl-phényl)-isobutyl-amide

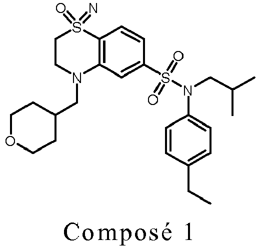

A une solution, préalablement dégazé à l'argon, d'acide 1-oxo-4-(tetrahydro-pyran-4-ylmethyl)-1,2,3,4-tetrahydro-1λ⁴-benzo[1,4]thiazine-6-sulfonique (4-éthyl-phényl)-isobutyl-amide (195.00 mg; 0.39 mmol) dans du dichlorométhane (10 ml) sont ajoutés du 2,2,2-trifluoroacétamide (109 mg; 0.97 mmol), du rhodium(II) acétate (26 mg; 0.06 mmol), de l'oxyde de magnésium (78 mg; 1.93 mmol) et du iodobenzène diacétate (249 mg; 0.77 mmol). Le milieu réactionnel est agité à température ambiante pendant 16 heures, filtré sur célite et concentré. Le résidu obtenu est dilué dans du méthanol (10 ml) et u carbonate de potassium (267 mg; 1.93 mmol) est ajouté.

Le milieu réactionnel est agité pendant 30 minutes puis hydrolysé et extrait à l'acétate d'éthyle. Les phases organiques sont rassemblées, lavées à la saumure et séchées sur sulfate de sodium. Les solvants sont évaporés. Le produit brut est chromatographié sur gel de silice (éluant dichlorométhane/méthanol de 0 à 5% de méthanol).

L'acide 1-imino-1-oxo-4-(tetrahydro-pyran-4-ylméthyl)-1,2,3,4-tétrahydro-1λ⁶-benzo[1,4]thiazine-6-sulfonique (4-éthyl-phényl)-isobutyl-amide (44.50 mg; 21.97 %) est obtenu sous la forme d'un solide blanc.
1H NMR (DMSO-d6) δ: 0.86 (t, J = 4.9 Hz, 8H), 1.09 - 1.33 (m, 9H), 1.36 - 1.49 (m, 3H), 1.75 (qd, J = 8.6, 7.9, 4.2 Hz, 1H), 2.61 (q, J = 7.6 Hz, 2H), 3.17 (dd, J = 15.8, 9.0 Hz, 4H), 3.34 - 3.46 (m, 3H), 3.85 (ddd, J = 23.1, 9.5, 4.1 Hz, 4H), 4.74 (s, 1H), 6.68 (s, 1H), 6.88 (d, J = 8.1 Hz, 1H), 7.05 (d, J = 8.2 Hz, 2H), 7.23 (d, J = 7.9 Hz, 2H), 7.84 (d, J = 8.1 Hz, 1H).
MS : [M+H] = 519

### Exemple 133 : Synthèse de l'acide 3-oxo-4-(tétrahydro-pyran-4-ylmethyl)-3,4-dihydro-2H-benzo[1,4]thiazine-7-sulfonique (4-éthyl-phényl)-isobutyl-amide

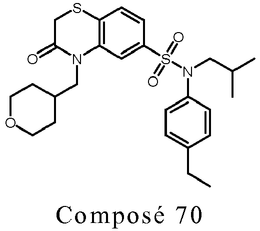

Le 4-(bromométhyl)tétrahydropyrane (18 mg; 0.10 mmol) est ajouté à de l'acide 3-oxo-3,4-dihydro-2H-benzo[1,4]thiazine-7-sulfonique (4-éthyl-phényl)-isobutyl-amide (20 mg; 0.05 mmol) et de le carbonate de césium (24 mg; 0.07 mmol) en solution dans la 1-méthyl-2-pyrrolidone (0.4 ml).

Le milieu réactionnel est chauffé à 80°C pendant 24 heures, hydrolysé puis extrait à l'acétate d'éthyle. Les phases organiques sont rassemblées, lavées à la saumure et séchées sur sulfate de sodium.

Les solvants sont évaporés et le produit brut est chromatographié sur gel de silice (éluant heptane/acétate d'éthyle de 0 à 50% d'acétate d'éthyle).

L'acide 3-oxo-4-(tétrahydro-pyran-4-ylmethyl)-3,4-dihydro-2H-benzo[1,4]thiazine-7-sulfonique (4-éthyl-phényl)-isobutyl-amide (10.4 mg; 40%) est obtenu sous la forme d'un solide beige.
¹H NMR (DMSO-d6) δ: 0.85 (d, J = 6.8 Hz, 7H), 1.17 (t, J = 7.6 Hz, 3H), 1.25 (d, J = 6.5 Hz, 2H), 1.37 - 1.67 (m, 10H), 1.68 - 1.77 (m, 5H), 2.44 (dt, J = 11.1, 4.0 Hz, 10H), 2.60 (q, J = 7.6 Hz, 2H), 3.17 (s, 1H), 3.33 - 3.38 (m, 5H), 3.80 (dt, J = 11.3, 3.7 Hz, 7H), 3.92 (s, 1H), 7.02 (d, J = 7.8 Hz, 2H), 7.18 (dd, J = 18.5, 8.4 Hz, 3H), 7.42 (d, J = 8.4 Hz, 1H), 7.60 (d, J = 2.1 Hz, 1H), 12.17 (s, 2H)
MS : [M+H] = 503

### Exemple 134 : Synthèse de l'acide 1,3-dioxo-4-(tétrahydro-pyran-4-ylméthyl)-1,2,3,4-tétrahydro-1λ⁴-benzo-[1,4]-thiazine-6-sulfonique (4-éthyl-phényl)-isobutyl-amide

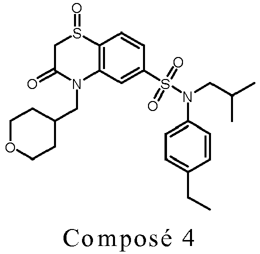

L'acide 3-chloroperbenzoïque (161 mg; 0.72 mmol) est ajouté à 0°C sur l'acide 3-oxo-4-(tetrahydro-pyran-4-ylmethyl)-3,4-dihydro-2H-benzo[1,4]thiazine-6-sulfonique (4-ethyl-phenyl)-isobutyl-amide (400 mg; 0.80 mmol) en solution dans du dichlorométhane (8 ml).

Le milieu réactionnel est agité à température ambiante pendant 1 heure, hydrolysé avec une solution aqueuse à 10% de Na₂S₂O₃ et extrait au dichlorométhane. Les phases organiques sont rassemblées, lavées avec une solution de soude 0.1N puis à la saumure et séchées sur sulfate de sodium. Les solvants sont concentrés.et le produit brut est chromatographié sur gel de silice (éluant Heptane/Acétate d'éthyle de 0 à 80% d'acétate d'éthyle). L'acide 1,3-dioxo-4-(tetrahydro-pyran-4-ylmethyl)-1,2,3,4-tetrahydro-1λ⁴-benzo[1,4]thiazine-6-sulfonique (4-éthyl-phényl)-isobutyl-amide (358 mg; 87%) est obtenu sous la forme d'un solide blanc
1H NMR (DMSO-d6) δ: 0.87 (d, J = 6.6 Hz, 6H), 1.01 - 1.23 (m, 5H), 1.37 - 1.53 (m, 3H), 1.62 - 1.83 (m, 1H), 2.62 (q, J = 7.6 Hz, 2H), 3.05 - 3.22 (m, 2H), 3.33 - 3.49 (m, 2H), 3.72 - 3.89 (m, 3H), 4.04 (dd, J = 14.8, 8.5 Hz, 1H), 4.29 - 4.39 (m, 2H), 7.01 - 7.08 (m, 2H), 7.21 - 7.28 (m, 2H), 7.43 (dd, J = 7.8, 1.5 Hz, 1H), 7.50 (d, J = 1.6 Hz, 1H), 8.08 (d, J = 7.8 Hz, 1H).
MS : [M+H] = 519

### Partie IX : Synthèse de sulfonamides soufrés à partir du schéma réactionnel 9

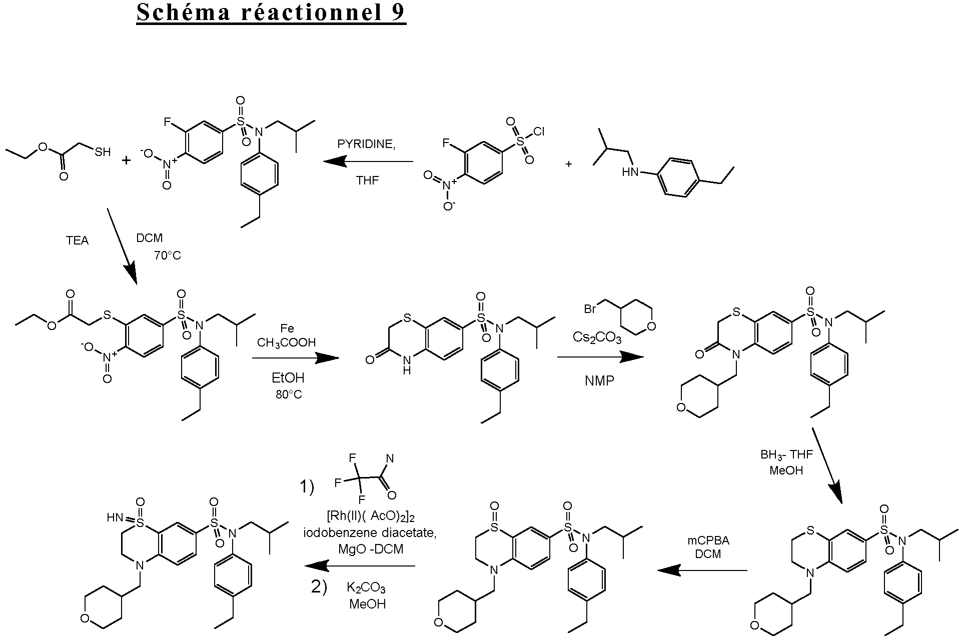

### Exemple 135 : Synthèse de l'acide 1-oxo-4-(tétrahydro-pyran-4-ylméthyl)-1,2,3,4-tetrahydro-1λ⁴-benzo-[1,4]-thiazine-7-sulfonique (4-éthyl-phényl)-isobutyl-amide

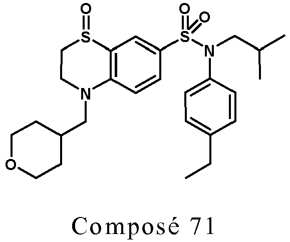

### 1. Synthèse de l'intermédiaire 135.1

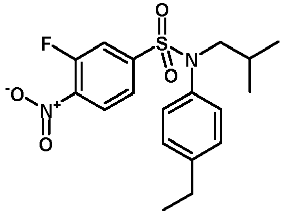

### 3-bromo-N-(4-éthyl-phényl)-N-isobutyl-4-méthoxy-benzenesulfonamide

Le chlorure de 3-fluoro-4-nitro-benzenesulfonyle (5.57 g; 22.56 mmol) est ajouté à une solution de (4-éthyl-phényl)-isobutyl-amine (4.0 g; 22.56 mmol) et de pyridine (11 ml; 135.37 mmol) dans du tétrahydrofurane (80ml). Le milieu réactionnel est agité pendant 16 heures à température ambiante, hydrolysé et extrait à l'acétate d'éthyle. Les phases organiques sont rassemblées, lavées avec une solution saturée de NH₄Cl, à la saumure, séchées (Na₂SO₄) et concentrées.

Le produit brut est chromatographié sur gel de silice (éluant Heptane/Acétate d'éthyle de 0 à 10% d'acétate d'éthyle). Le 3-bromo-N-(4-éthyl-phényl)-N-isobutyl-4-méthoxy-benzenesulfonamide (7.02 g; 82 %) est obtenu sous la forme d'un solide blanc floconneux avec une RMN¹H conforme.
MS : [M+H] = 381

### 2. Synthèse de l'intermédiaire 135.2

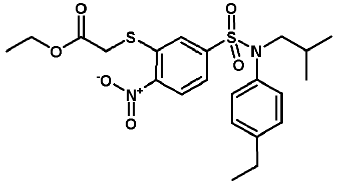

### 5-[(4-éthyl-phényl)-isobutyl-sulfamoyl]-2-nitro-phénylsulfanyl acetate d'éthyle

Le thioglycolate d'éthyle (0.86 ml; 7.89 mmol) est ajouté lentement sur une solution de N-(4-éthyl-phenyl)-3-fluoro-N-isobutyl-4-nitro-benzenesulfonamide (3.0 g; 7.89 mmol) et de triéthylamine (1.31 ml; 9.46 mmol) dans le dichlorométhane (75ml). Le milieu réactionnel est agité pendant 16 heures à température ambiante.

Le produit brut est chromatographié sur gel de silice (éluant heptane/acétate d'éthyle de 0 à 30% d'acétate d'éthyle).

Le {5-[(4-éthyl-phényl)-isobutyl-sulfamoyl]-2-nitro-phénylsulfanyl}-acetate d'éthyle (3.49 g ; 92%) est obtenu sous la forme d'un solide jaune vif avec une RMN¹H conforme.
MS : [M+H] = 481.

### 3. Synthèse de l'intermédiaire 135.3

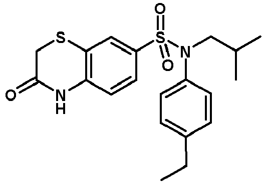

### Acide 3-oxo-3,4-dihydro-2H-benzo[1,4]thiazine-7-sulfonique (4-éthyl-phényl)-isobutyl-amide

La poudre de fer (1.16 g; 20.81 mmol) est ajouté sur une solution de {5-[(4-éthyl-phényl)-isobutyl-sulfamoyl]-2-nitro-phénylsulfanyl}-acetate d'éthyle (2.00 g; 4.16 mmol) dans de l'éthanol (20ml) et l'acide acétique (5ml).

Le milieu réactionnel est agité pendant 2 heures à une température de 80°C, ramené à température ambiante, dilué à l'acétate d'éthyle puis filtré sur célite. Le filtrat est lavé avec une solution saturée de NaHCO₃ puis à la saumure, séché (Na₂SO₄), concentré.

Le produit brut est chromatographié sur gel de silice (éluant heptane/acétate d'éthyle de 0 à 40% d'acétate d'éthyle). L'acide 3-oxo-3,4-dihydro-2H-benzo[1,4]thiazine-7-sulfonique (4-éthyl-phényl)-isobutyl-amide (0.98 g; 58%) est obtenu sous la forme d'une poudre blanche avec une RMN¹H conforme.
MS : [M+H] = 405

### 4. Synthèse de l'intermédiaire 135.4

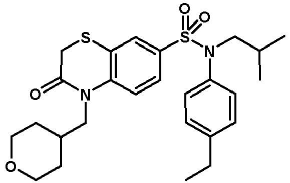

### Acide 3-oxo-4-(tetrahydro-pyran-4-ylmethyl)-3,4-dihydro-2H-benzo-[1,4]thiazine-7-sulfonique (4-éthyl-phényl)-isobutyl-amide

Le 4-(bromométhyl)-tétrahydropyrane (797 mg; 4.45 mmol) est ajouté sur un mélange d'acide 3-oxo-3,4-dihydro-2H-benzo[1,4]thiazine-7-sulfonique (4-éthyl-phényl)-isobutyl-amide (900 mg; 2.22 mmol) et de carbonate de césium (1.09 g; 3.34 mmol) dans la 1-méthyl-2-pyrrolidone (20 ml).

Le milieu réactionnel est agité pendant 4 heures à une température de 110°C, hydrolysé et extrait à l'acétate d'éthyle. Les phases organiques sont rassemblées, lavées à la saumure, séchées (Na₂SO₄), concentrées.

Le produit brut est chromatographié sur gel de silice (éluant heptane/acétate d'éthyle de 0 à 60% d'acétate d'éthyle). L'acide 3-oxo-4-(tétrahydro-pyran-4-ylméthyl)-3,4-dihydro-2H-benzo-[1,4]thiazine-7-sulfonique (4-éthyl-phényl)-isobutyl-amide (1.11 g; 99%) est obtenu sous la forme d'un solide blanc avec une RMN¹H conforme.
MS : [M+H] = 503

### 5. Synthèse de l'intermédiaire 135.5

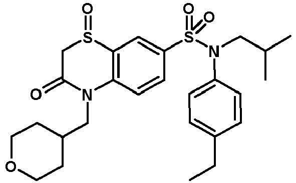

### Acide 1,3-dioxo-4-(tétrahydro-pyran-4-ylméthyl)-1,2,3,4-tetrahydro-1λ⁴-benzo-[1,4]-thiazine-7-sulfonique (4-éthyl-phényl)-isobutyl-amide

L'acide 3-chloroperoxybenzoïque (223 mg; 0.99 mmol) est ajouté à une température de 0°C sur l'acide 3-oxo-4-(tétrahydro-pyran-4-ylméthyl)-3,4-dihydro-2H-benzo-[1,4-]-thiazine-7-sulfonique (4-éthyl-phényl)-isobutyl-amide: (500mg; 0.99 mmol) en solution dans du dichlorométhane (10ml). Le milieu réactionnel est agité pendant 30 minutes à température ambiante, hydrolysé avec une solution aqueuse à 10% de Na₂S₂O₃ et extrait au dichlorométhane. Les phases organiques sont rassemblées puis lavées avec une solution de soude 0.1N puis à la saumure, séchées (Na₂SO₄), concentrées.

Le produit brut est chromatographié sur gel de silice (éluant heptane/acétate d'éthyle de 0 à 80% d'acétate d'éthyle). L'acide 1,3-dioxo-4-(tetrahydro-pyran-4-ylmethyl)-1,2,3,4-tetrahydro-1λ⁴-benzo[1,4]thiazine-7-sulfonique (4-éthyl-phényl)-isobutyl-amide (310 mg ;60%) est obtenu sous la forme d'une poudre blanche cristalline après cristallisation dans un mélange éthanol-heptane.
MS : [M+H] = 519

### 6. Synthèse de l'intermédiaire 135.6

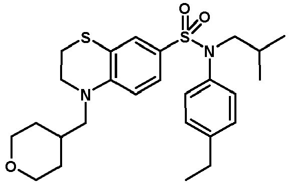

### Acide 4-(tétrahydro-pyran-4-ylméthyl)-3,4-dihydro-2H-benzo[1,4]thiazine-7-sulfonique (4-éthyl-phényl)-isobutyl-amide

L'acide 3-oxo-4-(tétrahydro-pyran-4-ylmethyl)-3,4-dihydro-2H-benzo[1,4]thiazine-7-sulfonique (4-éthyl-phényl)-isobutyl-amide (500 mg; 0.99 mmol) est ajouté à une solution 1M de complexe stabilisé de borane/tétrahydrofurane dans du tétrahydrofurane (35ml). Le milieu réactionnel est agité à reflux, refroidi et versé à 0°C sur du méthanol (35ml). Les solvants sont concentrés.

Le produit brut est chromatographié sur gel de silice (éluant Heptane/Acétate d'éthyle de 0 à 50% d'acétate d'éthyle). L'acide 4-(tetrahydro-pyran-4-ylmethyl)-3,4-dihydro-2H-benzo[1,4]thiazine-7-sulfonique (4-éthyl-phényl)-isobutyl-amide (4-éthyl-phényl)-isobutyl-amide (466 mg; 95%) est obtenu sous la forme d'un solide blanc cristallin après recristallisation dans l'éther avec une RMN¹H conforme.
MS : [M+H] = 489

### 7. Synthèse du composé 71 selon l'invention

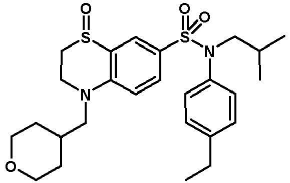

L'acide 3-chloroperoxybenzoïque (175.4 mg; 0.78 mmol) est ajouté, à une température de 0°C, à de l'acide 4-(tetrahydro-pyran-4-ylmethyl)-3,4-dihydro-2H-benzo[1,4]thiazine-7-sulfonique (4-éthyl-phényl)-isobutyl-amide (425.0 mg; 0.87 mmol) en solution dans le dichlorométhane (8.5 ml). Le milieu réactionnel est agité pendant 15 minutes à température ambiante, hydrolysé avec une solution aqueuse à 10% de Na₂S₂O₃ puis extrait au dichlorométhane. Les phases organiques sont rassemblées, lavées avec une solution de soude 0.1N puis à la saumure, séchées (Na₂SO₄), concentrées.

Le produit brut est chromatographié sur gel de silice (éluant heptane/acétate d'éthyle de 0 à 100% d'acétate d'éthyle puis dichlorométhane /méthanol de 0à 10% de méthanol). L'acide 1-oxo-4-(tétrahydro-pyran-4-ylméthyl)-1,2,3,4-tetrahydro-1λ⁴-benzo-[1,4]thiazine-7-sulfonique (4-éthyl-phényl)-isobutyl-amide (379 mg; 86%) est obtenu sous la forme d'une poudre blanche cristalline, après cristallisation dans un mélange éther/heptane.
1H NMR (DMSO-d6) δ: 0.84 (d, J = 6.7 Hz, 6H), 1.19 (t, J = 7.6 Hz, 3H), 1.35 (dddd, J = 34.9, 26.9, 14.1, 8.5 Hz, 3H), 1.53 - 1.61 (m, 2H), 2.02 (ddd, J = 11.6, 7.6, 3.7 Hz, 1H), 2.61 (q, J = 7.6 Hz, 2H), 2.87 (td, J = 13.7, 3.4 Hz, 1H), 3.14 (ddd, J = 13.9, 4.1, 2.3 Hz, 1H), 3.20 - 3.33 (m, 8H), 3.42 - 3.50 (m, 2H), 3.69 (dt, J = 14.1, 3.7 Hz, 1H), 3.82 - 3.98 (m, 3H), 6.97 - 7.04 (m, 2H), 7.09 (d, J = 9.1 Hz, 1H), 7.16 - 7.23 (m, 2H), 7.35 (dd, J = 9.1, 2.3 Hz, 1H), 7.54 (d, J = 2.3 Hz, 1H)
MS : [M+H] = 505

### Exemple 136 : Synthèse de l'acide 1-Imino-1-oxo-4-(tetrahydro-pyran-4-ylmethyl)-1,2,3,4-tetrahydro-1λ⁶-benzo[1,4]thiazine-7-sulfonique (4-éthyl-phényl)-isobutyl-amide

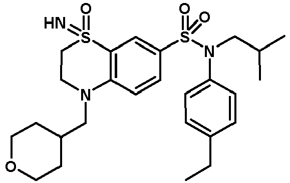

A une solution, préalablement dégazée à l'argon, d'acide 1-oxo-4-(tetrahydro-pyran-4-ylmethyl)-1,2,3,4-tetrahydro-1λ⁴-benzo[1,4]thiazine-7-sulfonique (4-éthyl-phényl)-isobutyl-amide (330 mg; 0.65 mmol) dans du dichlorométhane (16.5 ml) sont ajoutés du 2,2,2-trifluoroacétamide (185mg; 1.63 mmol), de l'acétate de rhodium(II) dimère (44 mg; 0.10 mmol), de l'oxyde de magnésium (132 mg; 3.27 mmol) et du diacétate d'iodobenzène (421 mg; 1.31 mmol).

Le milieu réactionnel est agité pendant 16 heures à température ambiante, filtré sur célite et concentré. Le résidu est dilué dans du méthanol (16.50 ml) et du carbonate de potassium (452 mg; 3.27 mmol) est ajouté. Le milieu réactionnel est agité pendant 30 minutes, hydrolysé et extrait à l'acétate d'éthyle. Les phases organiques sont rassemblées, lavées à la saumure, séchées (Na₂SO₄), concentrées.

Le produit brut est purifié par HPLC préparative (colonne C18, éluant : acétonitrile dans eau/0.1% d'acide formique).

L'acide 1-imino-1-oxo-4-(tétrahydro-pyran-4-ylméthyl)-1,2,3,4-tetrahydro-1λ⁶-benzo[1,4]thiazine-7-sulfonique (4-éthyl-phényl)-isobutyl-amide (44.5 mg; 13%) est obtenu sous la forme d'une poudre crème cristalline après recristallisation dans un mélange heptane/dichlorométhane.
1H NMR (DMSO-d6) δ: 0.73 - 0.94 (m, 7H), 1.19 (t, J = 7.6 Hz, 3H), 1.22 - 1.36 (m, 4H), 1.42 (dt, J = 13.6, 6.8 Hz, 1H), 1.58 (d, J = 13.1 Hz, 2H), 1.85 - 2.10 (m, 1H), 2.61 (q, J = 7.6 Hz, 2H), 3.20 - 3.32 (m, 4H), 3.34 - 3.51 (m, 3H), 3.86 (dd, J = 11.4, 4.0 Hz, 2H), 3.94 (p, J = 3.7 Hz, 2H), 4.68 (s, 1H), 7.00 (dd, J = 15.4, 8.7 Hz, 3H), 7.20 (d, J = 8.1 Hz, 2H), 7.27 (dd, J = 9.2, 2.4 Hz, 1H), 7.86 (d, J = 2.4 Hz, 1H).
MS : [M+H] = 520

### Partie X : Synthèse de sulfonamides soufrés à partir du schéma réactionnel 10

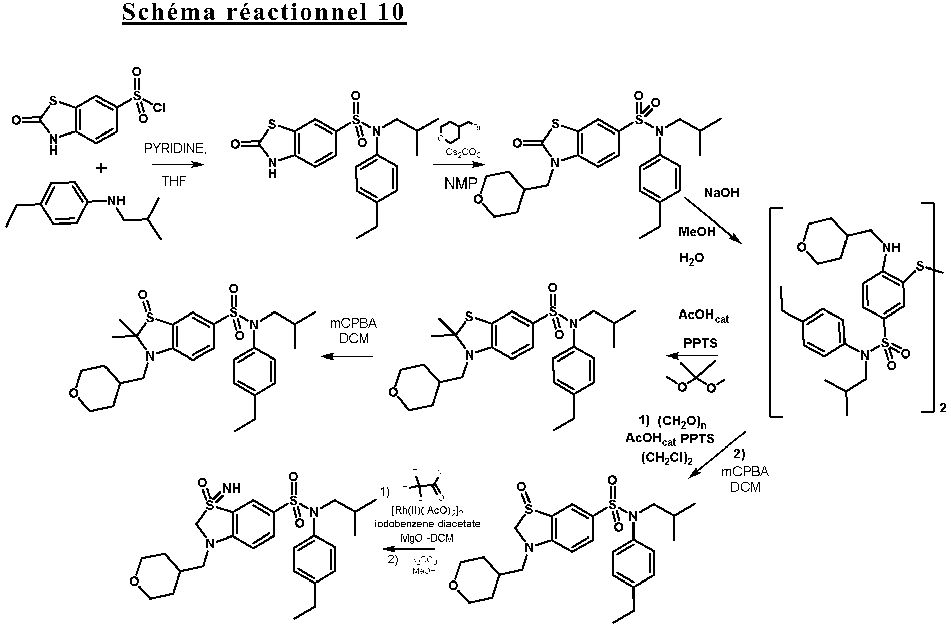

### Exemple 137 : Synthèse de l'acide 3-oxo-4-(tetrahydro-pyran-4-ylmethyl)-3,4-dihydro-2H-benzo-[1,4]-thiazine-7-sulfonique (4-éthyl-phényl)-isobutyl-amide

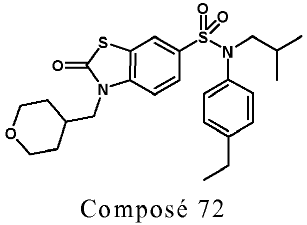

### 1. Synthèse de l'intermédiaire 137.1

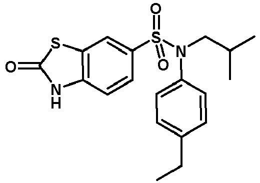

### Acide 2-oxo-2,3-dihydro-benzothiazole-6-sulfonique (4-éthyl-phényl)-isobutyl-amide

Le chlorure de 2-oxo-2,3-dihydro-benzothiazole-6-sulfonyle (2.97 g; 11.28 mmol) est ajouté à la (4-éthyl-phényl)-isobutyl-amine (2.00 g; 11.28 mmol) et la pyridine (5.5 ml; 67.69 mmol) en solution dans le tétrahydrofurane (40ml). Le milieu réactionnel est agité pendant 16 heures à température ambiante, hydrolysé et extrait à l'acétate d'éthyle. Les phases organiques sont rassemblées puis lavées avec une solution saturée de NH₄Cl, à la saumure, séchées (MgSO₄) et concentrées.

Le produit brut est chromatographié sur gel de silice (éluant Heptane/Acétate d'éthyle de 0 à 10% d'acétate d'éthyle). Le acide 2-Oxo-2,3-dihydro-benzothiazole-6-sulfonique (4-éthyl-phényl)-isobutyl-amide (2.67 g ; 61%) est obtenu sous la forme d'un solide blanc floconneux avec une RMN¹H conforme.
MS : [M+H] = 391

### 2. Synthèse du composé 72 selon l'invention

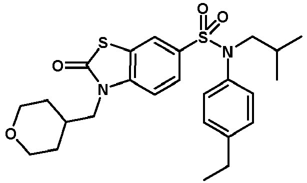

Le 4-(bromométhyl)-tétrahydropyrane (2.25 g; 12.55 mmol) est ajouté sur l'acide 2-oxo-2,3-dihydro-benzothiazole-6-sulfonique (4-éthyl-phényl)-isobutyl-amide: (2.45 g; 6.27 mmol) et le carbonate de césium (3.07 g; 9.41 mmol) en solution dans la 1-méthyl-2-pyrrolidone (50ml). Le milieu réactionnel est agité pendant 4 heures à 90°C, hydrolysé et extrait à l'acétate d'éthyle. Les phases organiques sont rassemblées puis lavées à la saumure et séchées (Na₂SO₄) et concentrées.

Le produit brut est chromatographié sur gel de silice (éluant heptane/acétate d'éthyle de 0 à 60% d'acétate d'éthyle). L'acide 3-oxo-4-(tétrahydro-pyran-4-ylméthyl)-3,4-dihydro-2H-benzo[1,4]thiazine-7-sulfonique (4-éthyl-phényl)-isobutyl- (2.19 g; 72 %) est obtenu sous la forme d'un solide blanc cristallin
1H NMR (DMSO-d6) δ: 0.85 (d, J = 6.6 Hz, 7H), 1.13 - 1.22 (m, 4H), 1.24 - 1.55 (m, 6H), 2.03 (ddt, J = 10.8, 6.9, 3.4 Hz, 1H), 2.50 - 2.66 (m, 3H), 3.23 (td, J = 11.6, 2.1 Hz, 2H), 3.34 (s, 1H), 3.83 (ddd, J = 11.3, 4.4, 1.9 Hz, 2H), 3.90 (d, J = 7.3 Hz, 2H), 6.96 - 7.04 (m, 2H), 7.14 - 7.22 (m, 2H), 7.42 (dd, J = 8.6, 1.9 Hz, 1H), 7.57 (s, 1H), 8.05 (d, J = 1.9 Hz, 1H)
MS : [M+H] = 489

### Exemple 138 : Synthèse de l'acide 2,2-dimethyl-3-(tetrahydro-pyran-4-ylmethyl)-2,3-dihydro-benzothiazole-6-sulfonique (4-éthyl-phényl)-isobutyl-amide

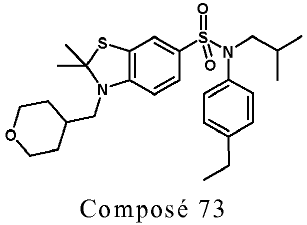

### 1. Synthèse de l'intermédiaire 138.1

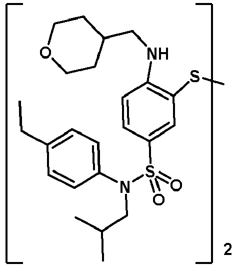

### 3,3'-disulfanediyl-bis-(N-(4-éthylphényl)-N-isobutyl-4-(((tétrahydro-2H-pyran-4-yl)méthyl)amino)benzenesulfonamide

Le mélange d'acide 2-oxo-3-(tétrahydro-pyran-4-ylméthyl)-2,3-dihydro-benzothiazole-6-sulfonique (4-éthyl-phényl)-isobutyl-amide (500 mg; 1.02 mmol) et d'hydroxyde de sodium (410 mg; 10.23 mmol) de méthanol (6ml) et d'eau (100µl) est agité pendant 16 heures à une température de 80°. Le milieu réactionnel est dilué avec 20mL d'acétate d'éthyle.

La phase organique est lavée avec 20mL d'une solution saturée de NH₄Cl, 20mL d'une solution saturée de NaHCO₃ et 20mL d'eau, séchée (MgSO₄), filtrée et concentrée à sec. Le 3,3'-disulfanediyl-bis-(N-(4-ethylphenyl)-N-isobutyl-4-(((tetrahydro-2H-pyran-4-yl)methyl)amino)benzenesulfonamide) (500 mg; 53%)est obtenu sous la forme d'une huile jaune avec une RMN¹H conforme.
MS : [M+H] = 923

### 2. Synthèse du composé 73 selon l'invention

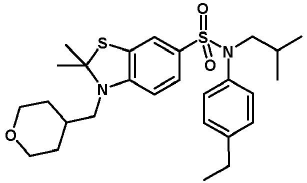

### Acide 2,2-diméthyl-3-(tétrahydro-pyran-4-ylméthyl)-2,3-dihydro-benzothiazole-6-sulfonique (4-éthyl-phényl)-isobutyl-amide

Au 3,3'-disulfandiyl bis-(N-(4-éthylphényl)-N-isobutyl-4-(((tetrahydro-2H-pyran-4-yl)-methyl)amino)-benzenesulfonamide) (300mg; 0.32 mmol) sont ajoutés le 2,2-diméthoxypropane (1.0 ml; 8.35 mmol) et le p-toluènesulfonate de pyridinium (245 mg; 0.97 mmol). Le milieu réactionnel est agité pendant 16 heures à une température de 80°C. 3 gouttes d'acide acétique sont ensuite ajoutées et le milieu réactionnel est agité pendant 2 heures à 80°C.

Le produit brut est purifié par HPLC préparative (colonne C18, éluant : acétonitrile dans eau/0.1% d'acide formique). L'acide 2,2-dimethyl-3-(tetrahydro-pyran-4-ylmethyl)-2,3-dihydro-benzothiazole-6-sulfonique (4-éthyl-phényl)-isobutyl-amide (70 mg; 40%) est obtenu sous la forme d'une huile jaune claire.
1H NMR (DMSO-d6) δ: 0.83 (d, J = 6.7 Hz, 6H), 1.18 (t, J = 7.6 Hz, 3H), 1.21 - 1.33 (m, 2H), 1.31 - 1.47 (m, 1H), 1.63 (s, 8H), 1.80 (s, 1H), 2.60 (q, J = 7.6 Hz, 2H), 3.08 (d, J = 7.2 Hz, 2H), 3.22 - 3.30 (m, 4H), 3.87 (dd, J = 11.5, 3.8 Hz, 2H), 6.47 (d, J = 8.4 Hz, 1H), 6.99 - 7.03 (m, 2H), 7.07 (dq, J = 8.3, 2.1 Hz, 1H), 7.12 (d, J = 2.0 Hz, 1H), 7.16 - 7.21 (m, 2H)
.MS : [M+H] = 503

### Exemple 139 : Synthèse de l'acide 2,2-dimethyl-1-oxo-3-(tetrahydro-pyran-4-ylméthyl)-2,3-dihydro-1H-1λ⁴-benzothiazole-6-sulfonique (4-éthyl-phényl)-isobutyl-amide

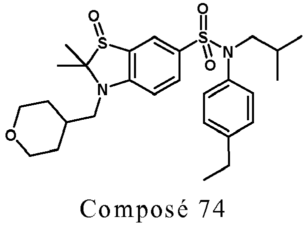

A une solution d'acide 2,2-diméthyl-3-(tétrahydro-pyran-4-ylméthyl)-2,3-dihydro-benzothiazole-6-sulfonique (4-éthyl-phényl)-isobutyl-amide (70mg; 0.14 mmol) le dichlorométhane (2 ml) est ajouté l'acide 3-chloroperoxybenzoïque (30m g; 0.15 mmol). Le milieu réactionnel est agité 45 minutes à température ambiante, dilué avec du dichlorométhane (10ml) et de l'eau (5ml) et extrait.

Les phases organiques sont rassemblées, séchées (MgSO₄), filtrées et concentrées.

Le produit brut est purifié par HPLC préparative (colonne C18, éluant : acétonitrile dans eau/0.1% d'acide formique). L'acide 2,2-dimethyl-1-oxo-3-(tetrahydro-pyran-4-ylmethyl)-2,3-dihydro-1H-1λ⁴-benzothiazole-6-sulfonique (4-éthyl-phényl)-isobutyl-amide (20mg; 28 %) est obtenu sous la forme d'un solide jaune pâle.
1H NMR (DMSO-d6) δ: 0.85 (d, J = 6.7 Hz, 6H), 1.19 (dd, J = 15.7, 8.1 Hz, 6H), 1.25 - 1.49 (m, 3H), 1.56 (d, J = 12.1 Hz, 2H), 1.64 (s, 3H), 1.90 (s, 1H), 2.60 (q, J = 7.6 Hz, 2H), 3.18 - 3.28 (m, 2H), 3.87 (dt, J = 10.6, 4.9 Hz, 2H), 7.00 (t, J = 8.9 Hz, 3H), 7.19 (d, J = 8.1 Hz, 2H), 7.46 (dd, J = 8.5, 2.1 Hz, 1H), 8.00 (d, J = 2.0 Hz, 1H)
.MS : [M+H] = 519

### Exemple 140 : Synthèse du composé 1-oxo-3-(tetrahydro-pyran-4-ylmethyl)-2,3-dihydro-1H-1λ⁴-benzothiazole-6-sulfonique acid (4-ethyl-phenyl)-isobutyl-amide

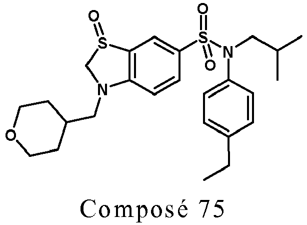

Au 3,3'-disulfane-diyl-bis(N-(4-éthylphényl)-N-isobutyl-4-(((tetrahydro-2H-pyran-4-yl)méthyl)amino)-benzenesulfonamide) (200mg; 0.22 mmol) sont ajoutés le paraformaldéhyde (473 mg; 1.08 mmol), le p-toluènesulfonate de pyridinium (163 mg; 0.65 mmol) et le 1,2-dichloroéthane (3ml).

Le milieu réactionnel est agité pendant 16 heures à 80°C, filtré, dilué avec du dichlorométhane, séché (MgSO₄) et filtrée. De l'acide 3-chloroperoxybenzoïque (107 mg; 0.48 mmol) est ajouté au filtrat. Le milieu réactionnel est agité pendant 30 minutes à température ambiante, dilué avec du dichlorométhane (20ml) et de l'eau (10ml) et extrait. La phase organique est lavée avec 'une solution de sulfite de sodium (20ml), de l'eau (20ml), séchée (MgSO₄), filtrée et concentrée.

Le produit brut est purifié par HPLC préparative (colonne C18, éluant : acétonitrile dans eau/0.1% d'acide formique). L'acide 1-oxo-3-(tétrahydro-pyran-4-ylméthyl)-2,3-dihydro-1H-1λ⁴-benzothiazole-6-sulfonique (4-éthyl-phényl)-isobutyl-amide (70 mg; 66 %) est obtenu sous la forme d'un solide blanc.
1H NMR (DMSO-d6) δ: 0.85 (dd, J = 6.7, 3.5 Hz, 6H), 1.18 (t, J = 7.6 Hz, 3H), 1.22 - 1.34 (m, 2H), 1.42 (dt, J = 13.6, 6.9 Hz, 1H), 1.55 (t, J = 13.0 Hz, 2H), 1.87 - 2.09 (m, 1H), 2.61 (q, J = 7.5 Hz, 2H), 3.27 (ddt, J = 12.1, 9.7, 6.3 Hz, 4H), 3.40 (dd, J = 14.5, 7.4 Hz, 1H), 3.54 (dd, J = 14.5, 7.2 Hz, 1H), 3.81 - 3.89 (m, 2H), 4.49 (d, J = 13.6 Hz, 1H), 4.76 (d, J = 13.6 Hz, 1H), 6.95 - 7.04 (m, 2H), 7.11 (d, J = 9.0 Hz, 1H), 7.19 (d, J = 8.3 Hz, 2H), 7.43 (dd, J = 8.8, 2.0 Hz, 1H), 8.02 (d, J = 2.0 Hz, 1H).MS : [M+H] = 491

### Exemple 141 : Synthèse de l'acide 1-Imino-1-oxo-3-(tetrahydro-pyran-4-ylmethyl)-2,3-dihydro-1H-1λ⁶-benzothiazole-6-sulfonique (4-ethyl-phenyl)-isobutyl-amide

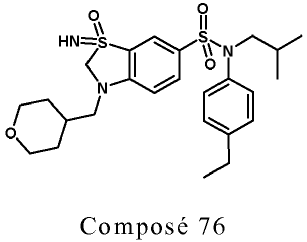

A une solution, préalablement dégazée à l'argon, de l'acide 1-oxo-3-(tétrahydro-pyran-4-ylméthyl)-2,3-dihydro-1H-1λ⁴-benzothiazole-6-sulfonique (4-éthyl-phényl)-isobutyl-amide (70 mg; 0.14 mmol) dans du dichlorométhane (2 ml) sont ajoutés du 2,2,2-trifluoroacétamide (40.3 mg; 0.36 mmol), de l'acétate de rhodium(II) dimère (9.5 mg; 0.02 mmol), de l'oxyde de magnésium (30 mg; 0.71 mmol) et du d'acétate de iodobenzène (92 mg; 0.29 mmol). Le milieu réactionnel est agité 3jours à température ambiante, filtré sur célite et concentré à sec. Le résidu est repris dans du méthanol (1ml) auquel est ajouté du carbonate de potassium (100 mg; 0.71 mol). Le milieu réactionnel est agité pendant 1 heure, dilué avec de l'acétate d'éthyle (20ml) et extrait.

La phase organique est lavée avec une solution saturée (NH₄Cl, 20ml), une solution saturée (NaHCCO₃, 20ml) et de l'eau (20ml, séchée (MgSO₄), filtrée et concentrée.

Le produit brut est purifié par HPLC préparative (colonne C18, éluant : acétonitrile dans eau/0.1% d'acide formique). L'acide 1-imino-1-oxo-3-(tetrahydro-pyran-4-ylmethyl)-2,3-dihydro-1H-1λ⁶-benzothiazole-6-sulfonique (4-éthyl-phényl)-isobutyl-amide (25mg; 33.34%) est obtenu sous la forme d'un solide beige.
1H NMR (DMSO-d6) δ: 0.84 (d, J = 6.4 Hz, 6H), 1.18 (t, J = 7.6 Hz, 3H), 1.29 (ddd, J = 18.9, 11.8, 7.0 Hz, 2H), 1.42 (p, J = 6.9 Hz, 1H), 1.48 - 1.65 (m, 2H), 1.93 (d, J = 11.7 Hz, 1H), 2.61 (q, J = 7.5 Hz, 2H), 3.25 (m, 4H), 3.46 (t, J = 7.0 Hz, 2H), 3.86 (d, J = 9.7 Hz, 2H), 4.49 (t, J = 4.3 Hz, 2H), 7.01 (d, J = 8.0 Hz, 2H), 7.08 - 7.24 (m, 4H), 7.37 (dd, J = 9.4, 2.3 Hz, 1H), 7.50 (d, J = 2.4 Hz, 1H), 8.23 (d, J = 4.9 Hz, 1H)
.MS : [M+H] = 506

## Revendications

1. Composé de formule (II) ainsi que ses sels d'addition pharmaceutiquement acceptables, ses hydrates et/ou ses solvates 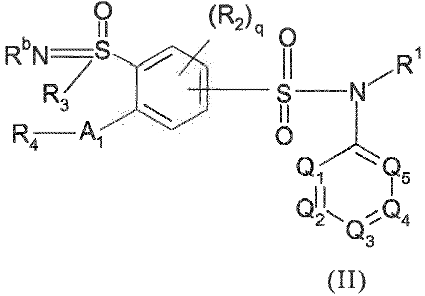 Formule (II) dans laquelle :
• q désigne zéro ou un entier naturel allant de 1 à 3,
• R¹ représente un radical alkyle, linéaire ou ramifié, en C₃-C₅, un radical cycloalkyle en C₃-C₅, un radical alkényle, linéaire ou ramifié, en C₂-C₅, un radical alkyl(C₁)cycloalkyle en C₃-C₅, un radical hétérocycloalkyle en C₄-C₅, un radical alkyl(C₁)hétérocycloalkyle en C₄-C₅,
• R₂ représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement cyano -CN ; les radicaux alkyle, alkényle et alcoxy pouvant être substitués par un ou plusieurs atomes d'halogène,
• R³ représente un radical alkyle en C₁-C₃,
• R⁴ représente un atome d'hydrogène, un groupement (CHR⁵)ₙ-(Z)ₒ-(CHR'⁵)ₚ-R⁶,
• n, o, et p, identiques ou différents, désignent zéro ou un entier naturel variant de 1 à 3,
• Z représente un groupe divalent choisi parmi -CH₂-, -NH-et -O-,
• R⁵ et R'⁵, identiques ou différents, représentent un atome d'hydrogène, un radical méthyle -CH₃, un radical hydroxy -OH, un radical hydroxyalkyle en C₁, un radical carboxylique -COOH,
• R⁶ représente :
- un atome d'hydrogène ou un atome d'halogène,
- un radical hétérocycloalkyle éventuellement substitué par un ou plusieurs atomes d'halogène, un ou plusieurs groupements alkyle en C₁-C₃, linéaire ou ramifié, un ou plusieurs groupements - OH, une ou plusieurs fonctions carbonyles =O, un ou plusieurs groupements hydroxyalkyle en C₁-C₄, linéaire ou ramifié, un cycle pyrrolidine, un ou plusieurs groupements amino, un ou plusieurs groupements -C(=O)R⁷, un ou plusieurs groupements S(=O)₂R⁷ ; R⁷ représentant un radical alkyle, linéaire ou ramifié, en C₁-C₃, un radical hydroxy -OH, un radical alcoxy en C₁-C₄ linéaire ou ramifié, ou un radical amino N(R^{7a})(R^{7b}); avec R^{7a} et R^{7b}, identiques ou différents, désignent un atome d'hydrogène, un radical alkyle, linéaire ou ramifié, en C₁-C₃ ou un radical cyclopropyle,
- un radical cycloalkyle en C₃-C₆ éventuellement substitué par un ou plusieurs groupements -OH,
- un radical aromatique ou hétéroaromatique éventuellement substitué par un ou plusieurs atomes d'halogène, un ou plusieurs groupements alkyle en C₁-C₃, linéaire ou ramifié éventuellement substitué par un ou plusieurs atomes d'halogène, un ou plusieurs groupements alcoxy en C₁-C₃, un ou plusieurs groupements amino -NR¹¹R¹², un ou plusieurs groupements -COR¹¹, une fonction carbonyle (=O), un ou plusieurs groupements -OR¹¹, un ou plusieurs groupements hydroxyalkyle en C₁-C₄, un ou plusieurs groupements - COOR¹¹, un ou plusieurs groupements amido -CONR¹¹R¹², un ou plusieurs groupements -SOR¹¹, un ou plusieurs groupements -SO₂R¹¹, un ou plusieurs groupements -NHCOR¹¹, un ou plusieurs groupements -NHCOOR¹¹, un ou plusieurs groupements -SO₂NR¹¹R¹² ou un ou plusieurs groupements -CN ; R¹¹ et R¹², identiques ou différents, représentant un atome d'hydrogène ou un radical alkyle, linéaire ou ramifié, en C₁-C₃ éventuellement substitué par un ou plusieurs atomes d'halogène ;
• A₁ représente un groupe divalent choisi parmi -NR^{a}-, -O-, - S-, -SO-, -SO₂-, -SO(=NH)-, -CH₂-, -C=C-, -CH(R^{a})- ;
• R^{a} représente un atome d'hydrogène, un radical alkyle, linéaire ou ramifié, en C₁-C₃, un radical acétyle -C(=O)CH₃,
• R^{b} représente un atome d'hydrogène, un radical alkyle, linéaire ou ramifié, en C₁-C₃, un groupement cyclopropyle,
• Q₁, Q₂, Q₃, Q₄ et Q₅, identiques ou différents, représentent un atome d'azote ou un groupement -CR'₂,
• R'₂ représente un atome d'hydrogène ou un radical alkyle linéaire ou ramifié en C₁-C₅,
• R^{a} et R³ peuvent former ensemble avec les atomes de carbone auxquels ils sont attachés un groupe hétérocycloalkyle pouvant être éventuellement substitué par un ou plusieurs fonctions carbonyle, un ou plusieurs radicaux alkyle en C₁-C₃,
• lorsque Ai représente -NR^{a}- alors R^{a} et R⁴ peuvent former ensemble avec l'atome d'azote auquel ils sont rattachés un groupe hétérocycloalkyle en C₂-C₁₀ comprenant éventuellement un à trois hétéroatomes choisis parmi un atome de soufre, un atome d'azote et un atome d'oxygène ; ledit groupe hétérocycloalkyle étant éventuellement substitué par au moins un radical R¹⁴,
• R¹⁴ représente un radical alkyle, linéaire ou ramifié, en Ci-C₃, un radical alcoxy, linéaire ou ramifié, en C₁-C₃, un atome d'halogène, un groupement hydroxy -OH, un groupement cyano -CN, un groupement -CONR¹⁵R¹⁶, un groupement =SO₂R¹⁵, un groupement -COR¹⁵ ou un groupement amino -NR¹⁵R¹⁶ ; R¹⁵ et R¹⁶, identiques ou différents, représentant un atome d'hydrogène ou un radical alkyle, linéaire ou ramifié, en C₁-C₃.

2. Composé de formule (II) selon la revendication 1, **caractérisé en ce que** R⁶ représente un radical hétérocyclique choisi parmi les hétérocycles suivants : 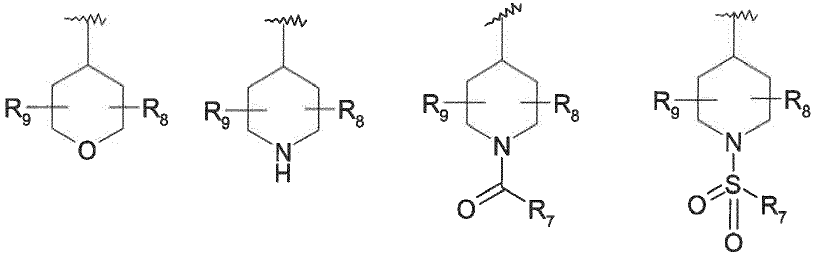 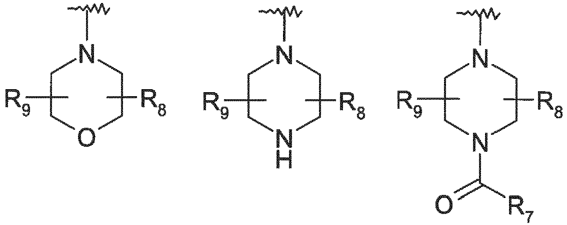 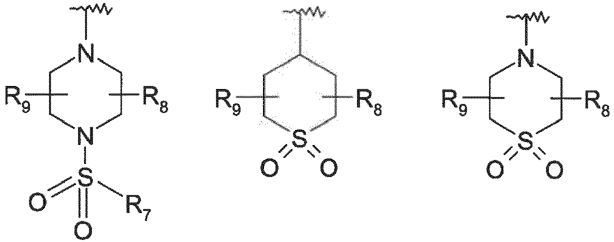 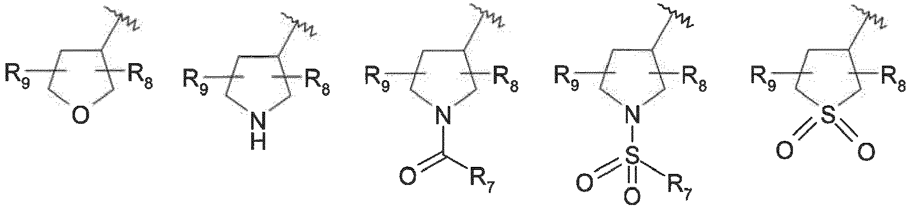 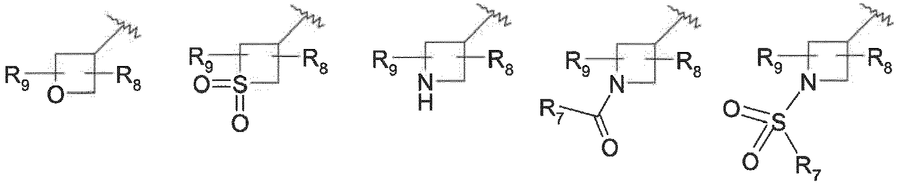 dans lesquels :
- R₇ représente un radical alkyle, linéaire ou ramifié, en Ci-C₃, un radical hydroxy -OH, un radical alcoxy en C₁-C₃ ou un radical amino en N(R^{7a})(R^{7b}),
- R^{7a} et R^{7b}, identiques ou différents, désignent un atome d'hydrogène, un radical alkyle, linéaire ou ramifié, en C₁-C₃ ou un radical cyclopropyle,
- R₈ et R₉, identiques ou différents, représentent un atome d'hydrogène, un radical alkyle, linéaire ou ramifié, en C₁-C₃, un groupement hydroxy -OH, un groupe carbonyle, un radical hydroxyalkyle en C₁ (CH₂OH) un groupe amino -NH₂,
- R₈ et R₉ peuvent former ensemble avec les atomes de carbone auxquels ils sont attachés un cycle carbocyclique comportant de 5 à 7 chaînons,

3. Composé de formule (II) selon la revendication 1, **caractérisé en ce que** R⁶ représente un radical aromatique ou hétéroaromatique choisi parmi : 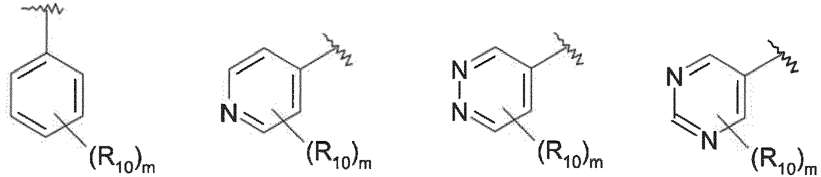 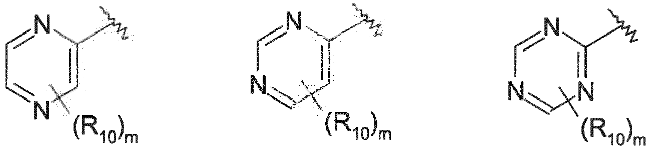 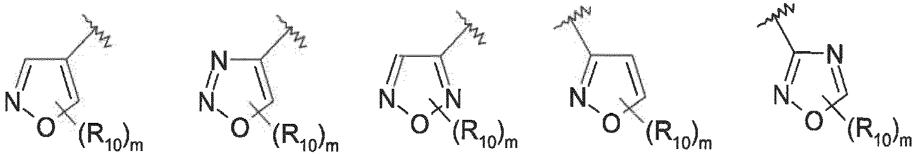 dans lesquels :
- R₁₀ représente un atome d'hydrogène ou un atome d'halogène ; un radical alkyle, linéaire ou ramifié, en C₁-C₃ éventuellement substitué par un ou plusieurs atomes d'halogène ; une fonction carbonyle (=O), un groupe OR¹¹, un groupe hydroxyalkyle en C₁-C₄, un groupe amino -NR¹¹R¹², un groupe -COR¹¹, un groupe - COOR¹¹, un groupe amido -CONR¹¹R¹², un groupe -SOR¹¹, un groupe -SO₂R¹¹, un groupe -NHCOR¹¹, un groupe -NHCOOR¹¹, un groupe - SO₂NR¹¹R¹² ou un groupe cyano -CN,
- R¹¹ et R¹², identiques ou différents, représentent un atome d'hydrogène ou un radical alkyle, linéaire ou ramifié, en C₁-C₃ éventuellement substitué par un ou plusieurs atomes d'halogène
- m désigne zéro ou un entier naturel allant de 1 à 3.

4. Composé selon l'une quelconque des revendications précédentes, **caractérisé en ce qu'**il est choisi parmi les composés suivants ainsi que leurs sels d'addition pharmaceutiquement acceptables, hydrates et/ou leurs solvates :
| | |
|---|---|
| 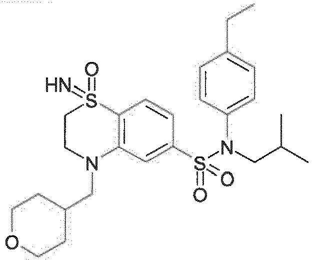 | Acide imino-1-oxo-4-(tétrahydro-pyran-4-ylméthyl)-1,2,3,4-tétrahydro-1λ⁶-benzo[1,4]thiazine-7-sulfonique (4-éthyl-phényl)-isobutyl-amide |
| | Composé 1 |
| 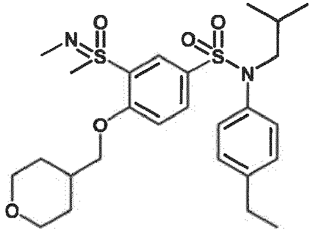 | N-(4-éthyl-phényl)-N-isobutyl-3-méthane-sulfinyl-4-(tétrahydro-pyran-4-ylméthoxy)-benzène- N-méthyl-sulfoximine |
| | Composé 2 |
| 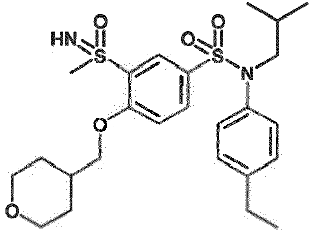 | N-(4-éthyl-phényl)-N-isobutyl-3-methane-sulfoximin-4-(tetrahydro-pyran-4-ylmethoxy)-benzenesulfonamide |
| | Composé 26 |
| 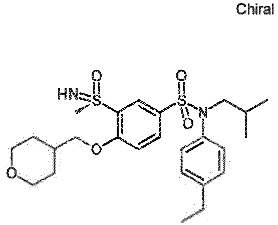 | N-(4-éthyl-phényl)-N-isobutyl-3-méthane-sulfoximin-4-(tétrahydro-pyran-4-ylméthoxy)-benzènesulfonamide |
| | composé 7 (énantiomère A) |
| 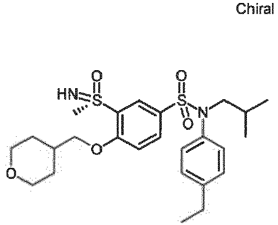 | N-(4-éthyl-phényl)-N-isobutyl-3-méthane-sulfoximin-4-(tétrahydro-pyran-4-ylméthoxy)-benzènesulfonamide |
| | composé 8 (énantiomère B) |
| 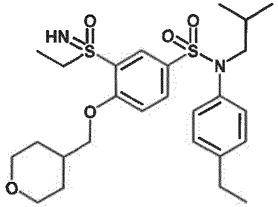 | N-(4-éthyl-phényl)-N-isobutyl-3-éthane-sulfoximin-4-(tétrahydro-pyran-4-ylméthoxy)-benzènesulfonamide |
| | Composé 18 |
| 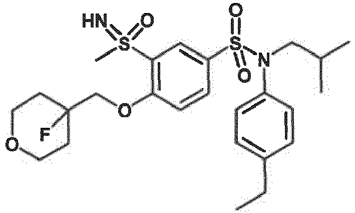 | N-(4-éthylphényl)-4-(4-fluorotétrahydro-2H-pyran-4-yl)méthoxy)-N-isobutyl-3-(S-méthyl-sulfonimidoyl)benzènesulfonamide |
| | Composé 30 |
| 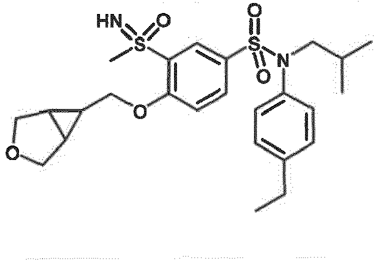 | 4-((3-oxabicyclo-[3.1.0]-hexan-6-yl)méthoxy)-N-(4-éthylphényl)-N-isobutyl-3-(S-méthyl-sulfonimidoyl)benzene-sulfonamide |
| | Composé 31 |
| 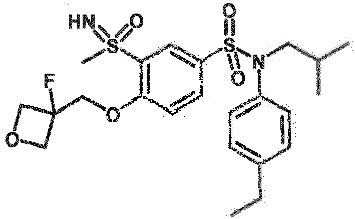 | N-(4-éthylphényl)-4-((3-fluorooxétan-3-yl)-méthoxy)-N-isobutyl-3-(S-méthyl-sulfonimidoyl)-benzènesulfonamide |
| | Composé 32 |
| 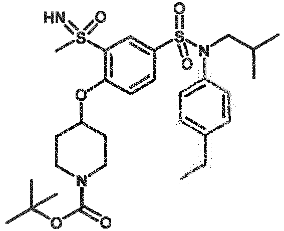 | Tert-butyl 4-(4-(N-(4-éthylphényl)-N-isobutyl-sulfamoyl)-2-(S-méthyl-sulfonimidoyl)-phenoxy)-pipéridine-1-carboxylate |
| | Composé 33 |
| 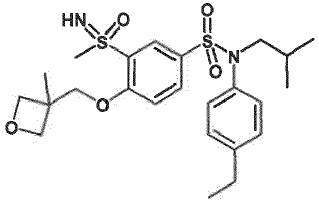 | N-(4-éthylphenyl)-N-isobutyl-4-((3-méthyl-oxétan-3-yl)méthoxy)-3-(S-méthyl-sulfonimidoyl)-benzenesulfonamide |
| | Composé 34 |
| 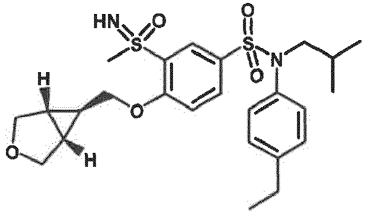 | 4-(((1R,5S,6r)-3-oxabicyclo-[3.1.0]hexan-6-yl)méthoxy)-N-(4-éthylphényl)-N-isobutyl-3-(S-méthylsulfonimidoyl)-benzenesulfonamide |
| | Composé 35 |
| 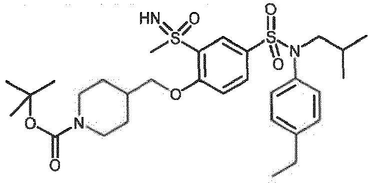 | tert-butyl 4-((4-(N-(4-éthylphényl)-N-isobutyl-sulfamoyl)-2-(S-méthyl-sulfonimidoyl)phénoxy)méthyl)-piperidine-1-carboxylate |
| | Composé 36 |
| 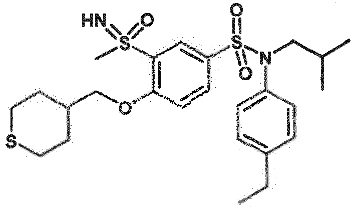 | N-(4-éthylphényl)-N-isobutyl-3-(S-méthyl-sulfonimidoyl)-4-(pyridin-4-ylmethoxy)-benzène-sulfonamide |
| | Composé 37 |
| 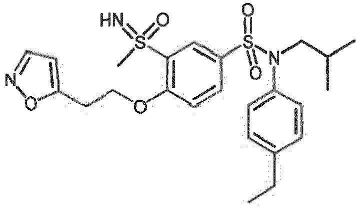 | N-(4-éthylphényl)-N-isobutyl-4-(2-(isoxazol-5-yl)éthoxy)-3-(S-methyl-sulfonimidoyl)benzenesulfonamide |
| | Composé 38 |
| 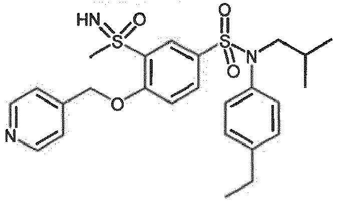 | N-(4-éthylphényl)-N-isobutyl-3-(S-méthyl-sulfonimidoyl)-4-(pyridin-4-ylméthoxy)-benzene-sulfonamide |
| | Composé 39 |
| 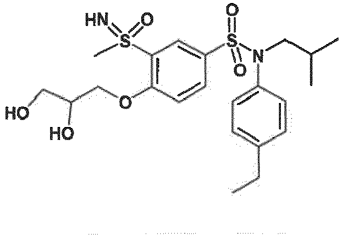 | 4-(2,3-dihydroxypropoxy)-N-(4-éthylphényl)-N-isobutyl-3-(S-methyl-sulfonimidoyl)benzènesulfonamide |
| | Composé 40 |
| 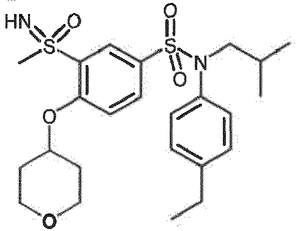 | N-(4-éthylphényl)-N-isobutyl-3-(S-méthylsulfonimidoyl)-4-((tétrahydro-2H-pyran-4-yl)oxy)benzenesulfonamide |
| | Composé 42 |
| 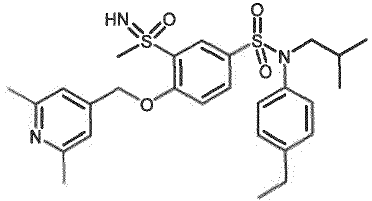 | 4-((2,6-dimethylpyridin-4-yl)méthoxy)-N-(4-éthylphenyl)-N-isobutyl-3-(S-méthyl-sulfonimidoyl)benzenesulfonamide |
| | Composé 43 |
| 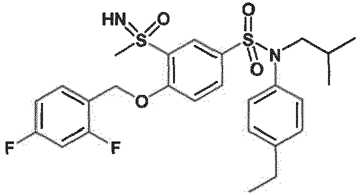 | 4-((2,4-difluorobenzyl)-oxy)-N-(4-ethylphenyl)-N-isobutyl-3 -(S-méthyl-sulfonimidoyl)benzenesulfonamide |
| | Composé 45 |
| 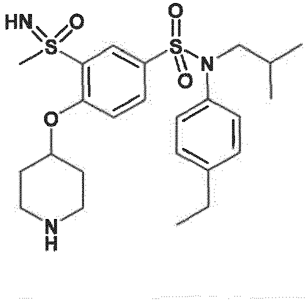 | N-(4-éthylphényl)-N-isobutyl-3-(S-méthyl-sulfonimidoyl)-4-(piperidin-4-ylméthoxy)-benzènesulfonamide N-(4-éthylphényl)-N-isobutyl-3-(S-méthy-lsulfonimidoyl)-4-(piperidin-4-ylméthoxy)-benzènesulfonamide |
| | Composé 46 |
| 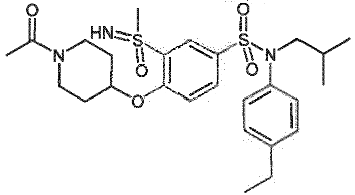 | 4-((1-acétylpiperidin-4-yl)oxy)-N-(4-éthylphényl)-N-isobutyl-3-(S-méthylsulfon-imidoyl)benzène sulfonamide |
| | Composé 47 |
| 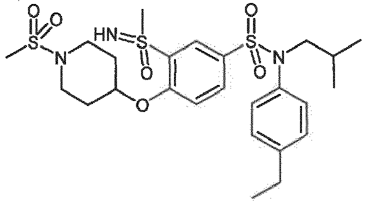 | N-(4-éthylphényl)-N-isobutyl-3-(S-méthyl-sulfonimidoyl)-4-((1-(méthylsulfonyl)-pipéridin-4-yl)oxy)benzène-sulfonamide |
| | Composé 48 |
| 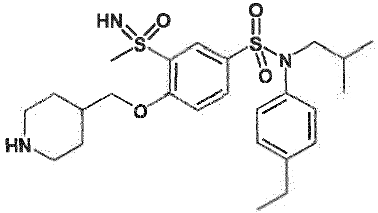 | N-(4-éthylphényl)-N-isobutyl-3-(S-méthyl-sulfonimidoyl)-4-(pipéridin-4-ylméthoxy)-benzenesulfonamide |
| | Composé 49 |
| 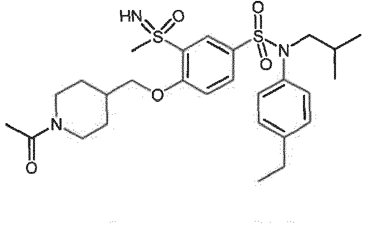 | 4-((1-acétylpiperidin-4-yl)méthoxy)-N-(4-ethyl-phényl)-N-isobutyl-3-(S-méthyl-sulfonimidoyl)-benzenesulfonamide |
| | Composé 50 |
| 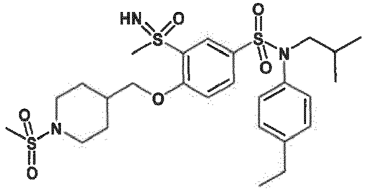 | N-(4-éthylphényl)-N-isobutyl-3-(S-méthyl-sulfonimidoyl)-4-((1-(methylsulfonyl-pipéridin-4-yl)methoxy)benzene-sulfonamide |
| | Composé 51 |
| 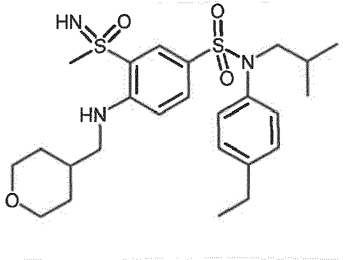 | N-(4-éthylphényl)-N-isobutyl-3-(S-méthyl-sulfonimidoyl)-4-[(tetrahydropyran-4-ylmethyl)-amino]-benzenesulfonamide |
| | Composé 52 |
| 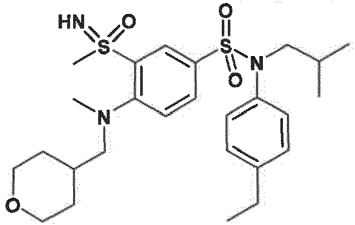 | N-(4-éthylphényl)-N-isobutyl-4-(méthyl-((tétrahydro-2H-pyran-4-yl)méthyl)amino)-3-(S-méthylsulfonimidoyl)-benzenesulfonamide |
| | Composé 53 |
| 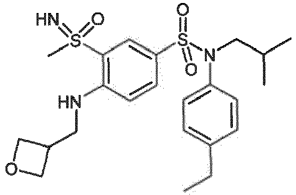 | N-(4-éthylphényl)-N-isobutyl-3-(S-methyl-sulfonimidoyl)-4-((oxetan-3-ylméthyl)amino)-benzenesulfonamide |
| | Composé 54 |
| 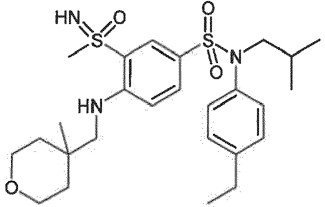 | N-(4-éthylphényl)-N-isobutyl-3-(S-méthyl-sulfonimidoyl)-4-(((4-méthyltetrahydro-2H-pyran-4-yl)methyl)amino)-benzenesulfonamide |
| | Composé 55 |
| 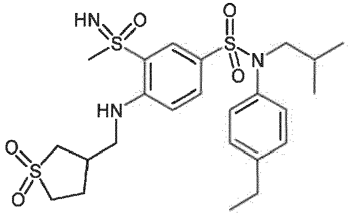 | 4-(((1,1-dioxidotétrahydro-thiophen-3-yl)-methyl)-amino)-N-(4-éthylphényl)-N-isobutyl-3 -(S-méthyl-sulfonimidoyl)benzene-sulfonamide |
| | Composé 56 |
| 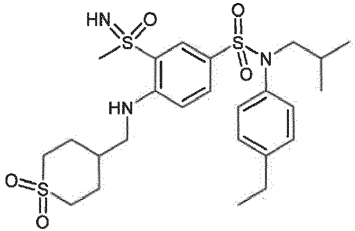 | 4-(((1,1-dioxidotetrahydro-2H-thiopyran-4-yl)-méthyl)amino)-N-(4-éthylphényl)-N-isobutyl-3 -(S-methylsulfonimidoyl)-benzenesulfonamide |
| | Composé 57 |
| 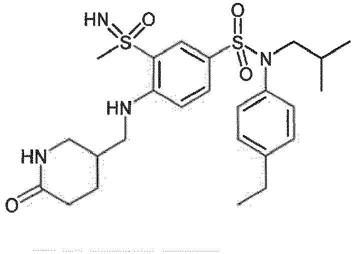 | N-(4-éthylphenyl)-N-isobutyl-3-(S-méthyl-sulfonimidoyl)-4-(((6-oxopiperidin-3-yl)-méthyl)-amino)benzene-sulfonamide |
| | Composé 58 |
| 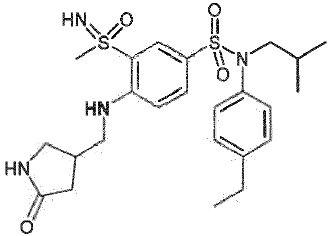 | N-(4-éthylphényl)-N-isobutyl-3-(S-méthyl-sulfonimidoyl)-4-(((5-oxopyrrolidin-3-yl)-methyl)amino)benzene-sulfonamide |
| | Composé 59 |
| 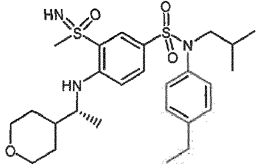 | N-(4-éthylphényl)-N-isobutyl-3-(S-méthyl-sulfonimidoyl)-4-(((R)-1-(tétrahydro-2H-pyran-4-yl)éthyl)amino)benzenesulfonamide |
| | Composé 60 |
| 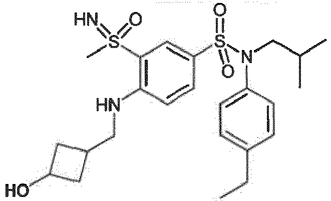 | N-(4-éthylphényl)-4-(((3-hydroxycyclobutyl)méthyl)amino)-N-isobutyl-3-(S-méthyl-sulfonimidoyl)-benzenesulfonamide |
| | Composé 61 |
| 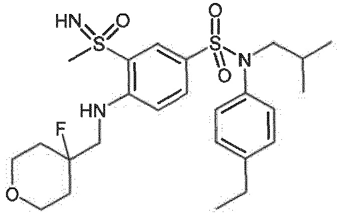 | N-(4-ethylphenyl)-4-(((4-fluorotetrahydro-2H-pyran-4-yl)methyl)amino)-N-isobutyl-3-(S-methyl-sulfonimidoyl)benzenesulfonamide |
| | Composé 62 |
| 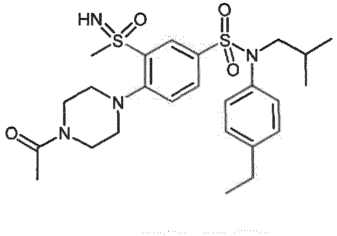 | :4-(4-acetylpiperazin-1-yl)-N-(4-ethylphenyl)-N-isobutyl-3-(S-methyl-sulfonimidoyl)-benzene-sulfonamide |
| | Composé 63 |
| 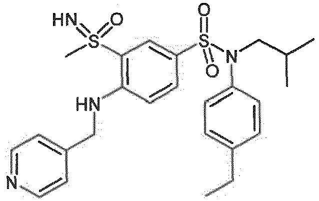 | N-(4-ethylphenyl)-N-isobutyl-3-(S-methyl-sulfonimidoyl)-4-((pyridin-4-ylméthyl)amino)-benzenesulfonamide |
| | Composé 64 |
| 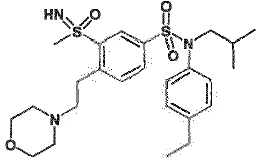 | N-(4-éthylphenyl)-N-isobutyl-3-(S-methy-lsulfonimidoyl)-4-(2-morpholinoethyl)-benzenesulfonamide |
| | Composé 65 |
| 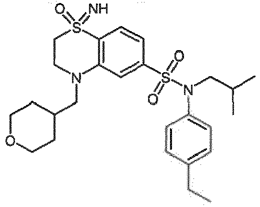 | Acide 1-imino-1-oxo-4-(tétrahydro-pyran-4-ylmethyl)-1,2,3,4-tetrahydro-1λ⁶-benzo-[1,4]thiazine-6-sulfonique (4-éthyl-phényl)-isobutyl-amide |
| | Composé 69 |
| 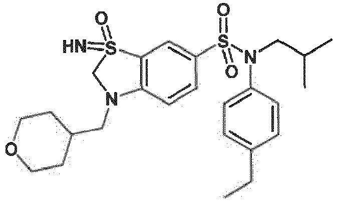 | Acide 1-Imino-1-oxo-3-(tetrahydro-pyran-4-ylmethyl)-2,3-dihydro-1H-1λ⁶-benzothiazole-6-sulfonique(4-éthyl-phényl)-isobutyl-amide |
| | Composé 76 |
| 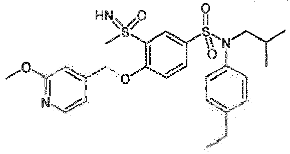 | N-(4-éthylphényl)-N-isobutyl-4-((2-méthoxy-pyridin-4-yl)methoxy)-3-(S-méthylsulfonimidoyl)-benzenesulfonamide |
| | Composé 77 |
| 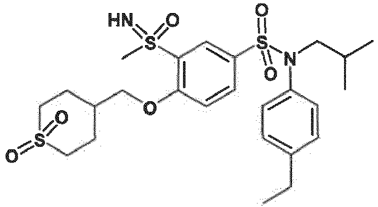 | N-(4-éthylphényl)-N-isobutyl-4-((2-méthoxy-pyridin-4-yl)méthoxy)-3-(S-méthylsulfonimidoyl)-benzenesulfonamide |
| | Composé 78 |
| 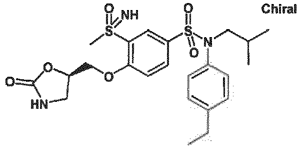 | N-(4-éthylphényl)-N-isobutyl-3-(S-méthyl-sulfonimidoyl)-4-(((R)-2-oxooxazolidin-5-yl)-méthoxy)benzenesulfonamide |
| | Composé 79 |
| 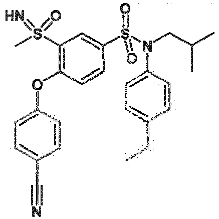 | 4-(4-cyanophénoxy)-N-(4-éthylphényl)-N-isobutyl-3 -(S-méthylsulfonimidoyl)-benzenesulfonamide |
| | Composé 80 |
| 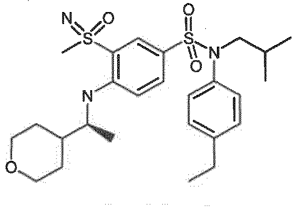 | N-(4-éthylphényl)-N-isobutyl-3-(S-méthyl-sulfonimidoyl)-4-(((S)-1-(tétrahydro-2H-pyran-4-yl)éthyl)amino)benzenesulfona mide |
| | Composé 81 |
| 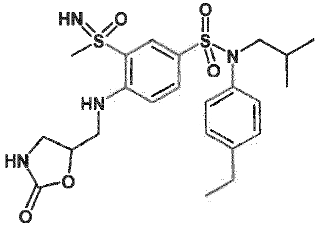 | N-(4-éthylphényl)-N-isobutyl-3-(S-methylsulfonimidoyl)-4-(((2-oxooxazolidin-5-yl)méthyl)amino)benzenesulfonam ide |
| | Composé 82 |
| 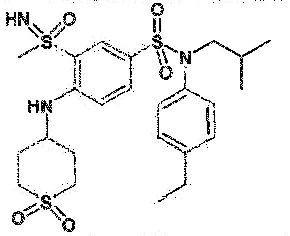 | 4-((1,1-dioxidotétrahydro-2H-thiopyran-4-yl)-amino)-N-(4-éthylphényl)-N-isobutyl-3-(S-méthylsulfonimidoyl)benzenesulfo namide |
| | Composé 83 |
| 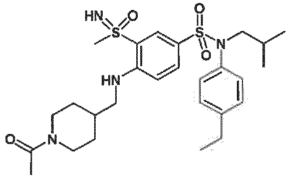 | 4-(((1-acétylpiperidin-4-yl)methyl)amino)-N-(4-éthylphényl)-N-isobutyl-3-(S-méthylsulfon-imidoyl)benzenesulfonamide |
| | Composé 84 |
| 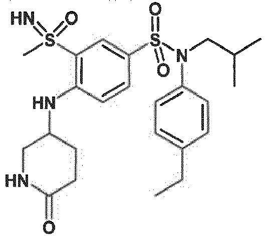 | N-(4-éthylphényl)-N-isobutyl-3-(S-méthyl-sulfonimidoyl)-4-((6-oxopiperidin-3-yl)-amino)benzenesulfonamide |
| | Composé 85 |
| 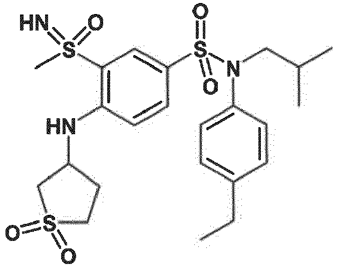 | 4-((1,1-dioxidotetrahydrothiophen-3-yl-)amino)-N-(4-éthylphényl)-N-isobutyl-3-(S-méthylsulfonimidoyl)benzenesu lfonamide |
| | Composé 86 |
| 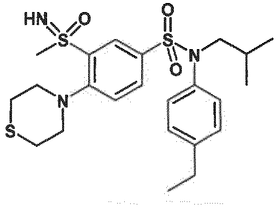 | N-(4-éthylphényl)-N-isobutyl-3-(S-méthyl-sulfonimidoyl)-4-thiomorpholinobenzene-sulfonamide |
| | Composé 87 |
| 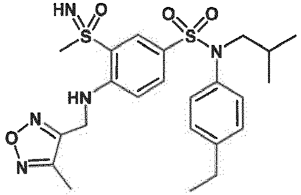 | N-(4-éthylphényl)-N-isobutyl-4-(((4-méthyl-1,2,5-oxadiazol-3-yl)méthyl)amino)-3-(S-méthylsulfonimidoyl)benzenesulfo namide |
| | Composé 88 |
| 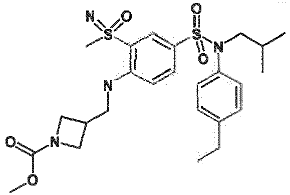 | 3-((4-(N-(4-éthylphényl)-N-isobutyl-sulfamoyl)-2-(S-méthylsulfonimidoyl)-phényl)amino)méthyl)azetidine-1 - carboxylate de méthyle |
| | Composé 89 |
| 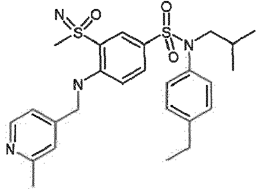 | N-(4-éthylphényl)-N-isobutyl-4-(((2-méthyl-pyridin-4-yl)méthyl)amino)-3-(S-méthyl-sulfonimidoyl)benzenesulfonamide |
| | Composé 90 |
| 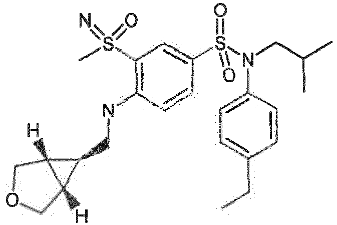 | 4-((((1R,5S,6s)-3-oxabicyclo[3.1.0]hexan-6-yl)méthyl)amino)-N-(4-éthylphényl)-N-isobutyl-3-(S-methylsulfonimidoyl)benzenesulfonamide |
| | Composé 91 |
| 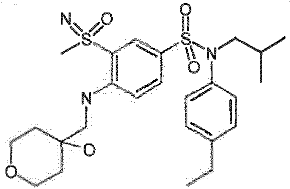 | N-(4-éthylphényl)-4-(((4-hydroxytétrahydro-2H-pyran-4-yl)méthyl)amino)-N-isobutyl-3-(S-méthylsulfonimidoyl)benzenesulfo namide |
| | Composé 92 |
| 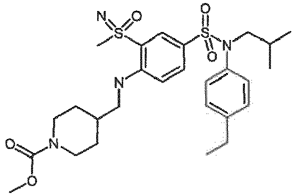 | 4-(((4-(N-(4-éthylphényl)-N-isobutyl-sulfamoyl)-2-(S-méthylsulfonimidoyl)phenyl)-amino-carboxylate de méthyle |
| | Composé 93 |
| 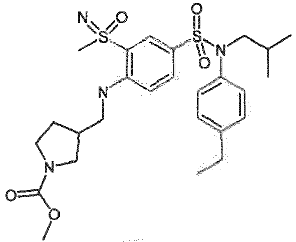 | 3-(((4-(N-(4-éthylphényl)-N-isobutyl-sulfamoyl)-2-(S-méthylsulfonimidoyl)phényl)-amino)méthyl)pyrrolidine-1-carboxylate de méthyle |
| | Composé 94 |
| 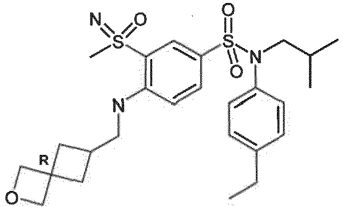 | 4-(((2-oxaspiro[3.3]heptan-6-yl)méthyl)amino)-N-(4-éthylphényl)-N-isobutyl-3-(S-méthyl-sulfonimidoyl)benzenesulfonamide |
| | Composé 95 |
| 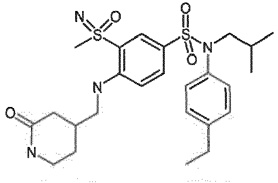 | 4-N-(4-éthylphényl)-N-isobutyl-3-(S-méthyl-sulfonimidoyl)-4-(((2-oxopiperidin-4-yl)-méthyl)amino)benzenesulfonamide |
| | Composé 96 |
| 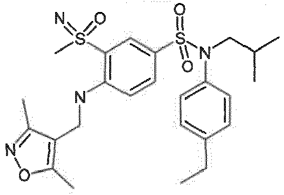 | 4-(((3,5-dimethylisoxazol-4-yl)methyl)amino)-N-(4-ethylphenyl)-N-isobutyl-3 -(S-methyl-sulfonimidoyl)benzenesulfonamide |
| | Composé 97 |
| 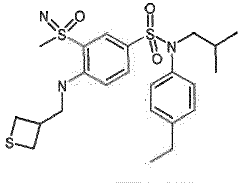 | N-(4-éthylphényl)-N-isobutyl-3-(S-méthy-sulfonimidoyl)-4-((thietan-3-ylmethyl)amino)-benzenesulfonamide |
| | Composé 99 |
| 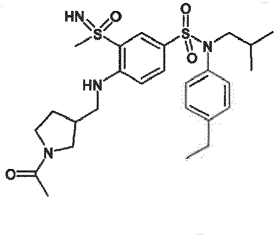 | 4-(((1-acétylpyrrolidin-3-yl)méthyl)amino)-N-(4-éthylphényl)-N-isobutyl-3-(S-méthyl-sulfonimidoyl)benzenesulfonamide |
| | Composé 100 |
| 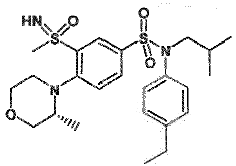 | N-(4-éthylphényl)-N-isobutyl-4-((R)-3-méthylmorpholino)-3-(S-méthyl-sulfonimidoyl)benzenesulfonamide |
| | Composé 103 |
| 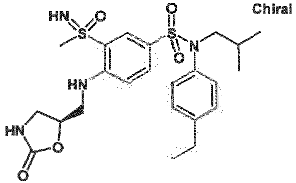 | N-(4-éthylphényl)-N-isobutyl-3-(S-methyl-sulfonimidoyl)-4-((((R)-2-oxooxazolidin-5-yl)méthyl)amino)benzenesulfonam ide |
| | Composé 105 |
| 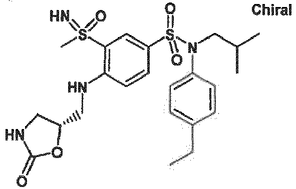 | N-(4-éthylphényl)-N-isobutyl-3-(S-méthyl-sulfonimidoyl)-4-(((S)-2-oxooxazolidin-5-yl)méthyl)amino)benzenesulfonam ide |
| | Composé 106 |
| 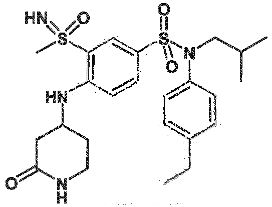 | N-(4-éthylphényl)-N-isobutyl-3-(S-méthyl-sulfonimidoyl)-4-((2-oxopiperidin-4-yl)amino)benzenesulfonamide |
| | Composé 107 |
| 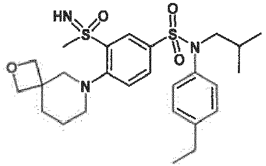 | N-(4-éthylphényl)-N-isobutyl-3-(S-méthyl-sulfonimidoyl)-4-(2-oxa-6-azaspiro [3 .5]nonan-6-yl)benzenesulfonamide |
| | Composé 109 |
| 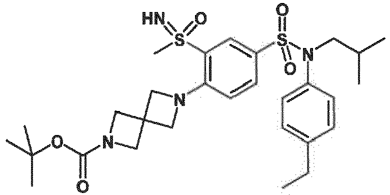 | tert-butyl 6-(4-(N-(4-ethylphenyl)-N-isobutyl-sulfamoyl)-2-(S-methylsulfonimidoyl)-phenyl)- 2,6-diazaspiro[3.3]heptane-2-carboxylate |
| | Composé 110 |
| 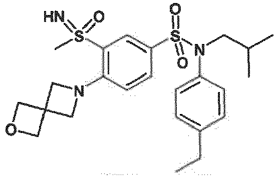 | N-(4-ethylphenyl)-N-isobutyl-3-(S-methyl-sulfonimidoyl)-4-(2-oxa-6-azaspiro-[3.3]heptan-6-yl)benzenesulfonamide |
| | Composé 111 |
| 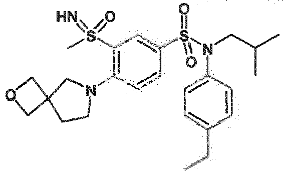 | N-(4-ethylphenyl)-N-isobutyl-3-(S-methyl-sulfonimidoyl)-4-(2-oxa-6-azaspiro[3.4]octan-6-yl)benzenesulfonamide |
| | Composé 112 |
| 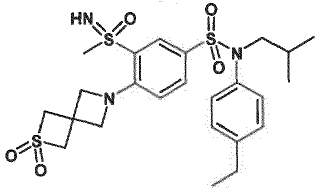 | 4-(2,2-dioxido-2-thia-6-azaspiro[3.3]heptan-6-yl)-N-(4-ethylphenyl)-N-isobutyl-3-(S-methyl-sulfonimidoyl)benzenesulfonamide |
| | Composé 113 |
| 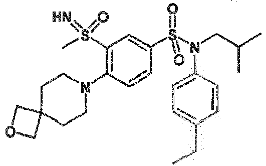 | N-(4-ethylphenyl)-N-isobutyl-3-(S-methyl-sulfonimidoyl)-4-(2-oxa-7-azaspiro[3.5]nonan-7-yl)benzenesulfonamide |
| | Composé 114 |
| 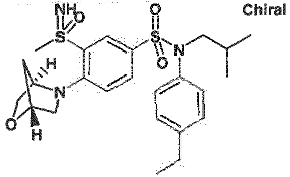 | 4-((1R,4R)-2-oxa-5-azabicyclo[2.2.1]heptan-5-yl)-N-(4-ethylphenyl)-N-isobutyl-3-(S-methyl-sulfonimidoyl)benzenesulfonamide |
| | Composé 115 |
| 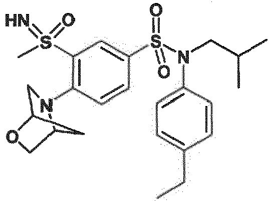 | 4-((1R,4R)-2-oxa-5-azabicyclo[2.2.1]heptan-5-yl)-N-(4-ethylphenyl)-N-isobutyl-3-(S-methyl-sulfonimidoyl)benzenesulfonamide |
| | Composé 117 |
| 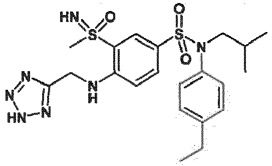 | 4-(((2H-tetrazol-5-yl)methyl)amino)-N-(4-ethylphenyl)-N-isobutyl-3-(S-methylsulfon-imidoyl)benzenesulfonamide |
| | Composé 118 |
| 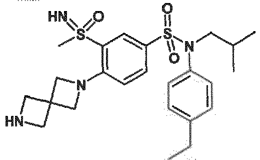 | -(4-éthylphényl)-N-isobutyl-3-(S-méthylsulfonimidoyl)-4-(2,6-diazaspiro[3.3]heptan-2-yl)benzenesulfonamide |
| | Composé 121 |
| 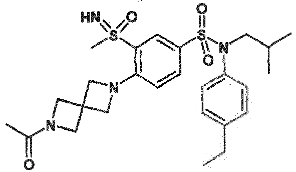 | 4-(6-acétyl-2,6-diazaspiro[3.3]heptan-2-yl)-N-(4-éthylphényl)-N-isobutyl-3-(S-méthylsulfonimidoyl)benzenesulfo namide |
| | Composé 122 |
| 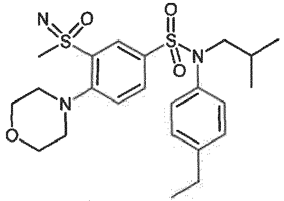 | N-(4-ethylphenyl)-N-isobutyl-3-(S-methyl-sulfonimidoyl)-4-morpholinobenzene-sulfonamide |
| | Composé 123 |
| 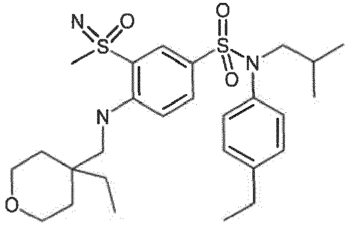 | N-(4-ethylphenyl)-4-(((4-ethyltetrahydro-2H-pyran-4-yl)methyl)amino)-N-isobutyl-3-(S-methylsulfonimidoyl)benzenesulfo namide |
| | Composé 124 |
| 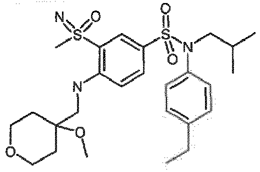 | N-(4-éthylphényl)-N-isobutyl-4-(((4-méthoxy-tétrahydro-2H-pyran-4-yl)méthyl)amino)-3-(S-méthylsulfonimidoyl)benzenesulfo namide |
| | Composé 125 |
| 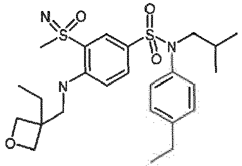 | 4-(((3-ethyloxetan-3 - yl)methyl)amino)-N-(4-ethylphenyl)-N-isobutyl-3 -(S-methylsulfon-imidoyl)benzenesulfonamide |
| | Composé 126 |
| 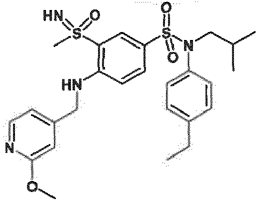 | N-(4-ethylphenyl)-N-isobutyl-4-(((2-methoxy-pyridin-4-yl)methyl)amino)-3-(S-methyl-sulfonimidoyl)benzenesulfonamide |
| | Composé 127 |
| 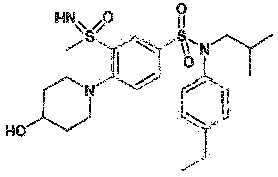 | N-(4-ethylphenyl)-4-(4-hydroxypiperidin-1-yl)-N-isobutyl-3-(S-methylsulfonimidoyl)benzenesulfonamide |
| | Composé 128 |
| 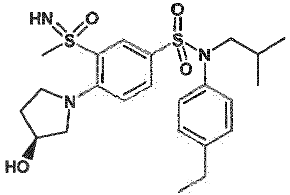 | N-(4-éthylphényl)-4-((S)-3-hydroxypyrrolidin-1-yl)-N-isobutyl-3-(S-méthylsulfonimidoyl)-benzenesulfonamide |
| | Composé 129 |
| 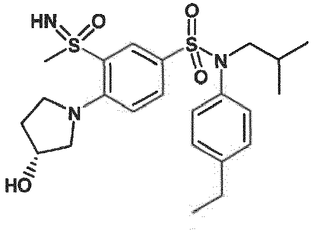 | N-(4-éthylphényl)-4-((R)-3-hydroxypyrrolidin-1-yl)-N-isobutyl-3-(S-methylsulfonimidoyl)-benzenesulfonamide |
| | Composé 130 |
| 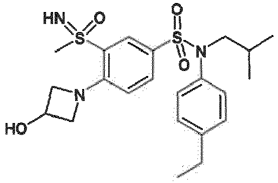 | N-(4-éthylphényl)-4-(3-hydroxyazetidin-1-yl)-N-isobutyl-3-(S-méthylsulfonimidoyl)-benzenesulfonamide |
| | Composé 131 |
| 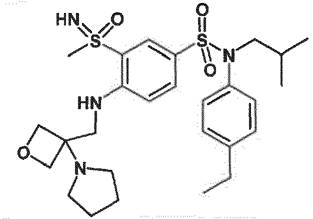 | N-(4-éthylphényl)-N-isobutyl-3-(S-méthyl-sulfonimidoyl)-4-(((3-(pyrrolidin-1-yl)oxetan-3-yl)méthyl)amino)benzenesulfonam ide |
| | Composé 132 |
| 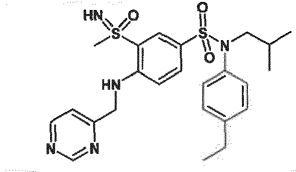 | N-(4-éthylphényl)-N-isobutyl-3-(S-méthylsulfonimidoyl)-4-((pyrimidin-4-ylméthyl)-amino)benzenesulfonamide |
| | Composé 133 |
| 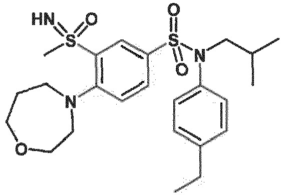 | N-(4-éthylphényl)-N-isobutyl-3-(S-méthyl-sulfonimidoyl)-4-(1,4-oxazepan-4-yl)benzenesulfonamide |
| | Composé 137 |
| 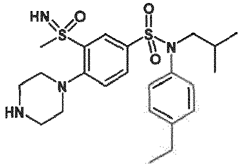 | N-(4-éthylphényl)-N-isobutyl-3-(S-méthylsulfonimidoyl)-4-(pipérazin-1-yl)benzenesulfonamide |
| | Composé 140 |
| 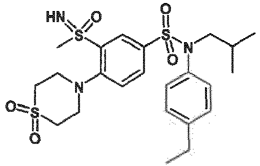 | N-(4-ethylphenyl)-4-(((3-hydroxycyclobutyl)methyl)amino)-N-isobutyl-3-(S-methyl-sulfonimidoyl)benzenesulfonamide |
| | Composé 141 |
| 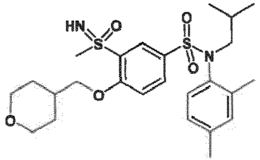 | N-(2,4-diméthylphényl)-N-isobutyl-3-(S-méthylsulfonimidoyl)-4-((tétrahydro-2H-pyran-4-yl)méthoxy)benzenesulfonamide |
| | Composé 142 |
| 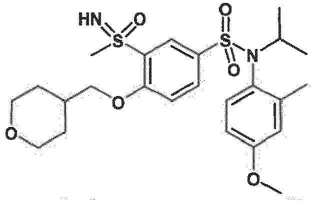 | N-isopropyl-N-(4-méthoxy-2-méthylphényl)-3-(S-méthylsulfonimidoyl)-4-((tétrahydro-2H-pyran-4-yl)méthoxy)benzenesulfonamide |
| | Composé 143 |

5. Composé selon l'une quelconque des revendications précédentes, **caractérisé en ce qu'**il est choisi parmi les composés suivants :
| | |
|---|---|
| 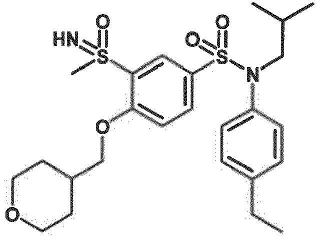 | N-(4-Ethyl-phenyl)-N-isobutyl-3-methane-sulfoximin-4-(tetrahydro-pyran-4-ylmethoxy)-benzenesulfonamide |
| | Composé 26 |
| 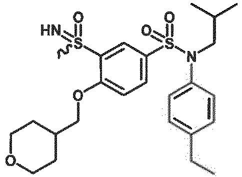 | énantiomère A du composé 7 |
| énantiomère A | |
| 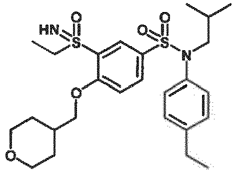 | N-(4-éthyl-phényl)-N-isobutyl-3-éthane-sulfoximin-4-(tétrahydro-pyran-4-ylméthoxy)-benzènesulfonamide |
| | Composé 18 |
| 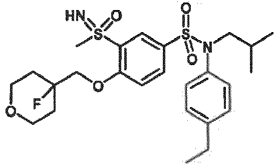 | N-(4-éthylphényl)-4-(4-fluorotétrahydro-2H-pyran-4-yl)méthoxy)-N-isobutyl-3-(S-méthyl-sulfonimidoyl)benzènesulfonamide |
| | Composé 30 |
| 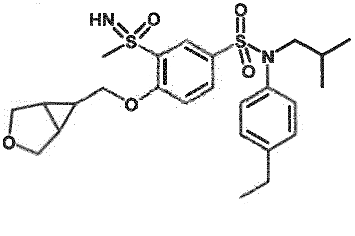 | 4-((3-oxabicyclo-[3.1.0]-hexan-6-yl)méthoxy)-N-(4-éthylphényl)-N-isobutyl- 3-(S-méthylsulfonimidoyl)benzene-sulfonamide |
| | Composé 31 |
| 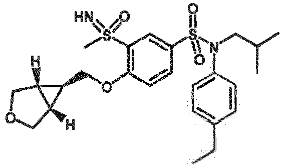 | 4-(((1R,5 S,6r)-3-oxabicyclo-[3.1.0]hexan-6-yl)méthoxy)-N-(4-éthylphényl)-N-isobutyl-3 -(S-méthylsulfonimidoyl)-benzenesulfonamide |
| | Composé 35 |
| 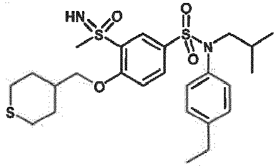 | N-(4-éthylphényl)-N-isobutyl-3-(S-méthyl-sulfonimidoyl)-4-(pyridin-4-ylmethoxy)-benzène-sulfonamide |
| | Composé 37 |
| 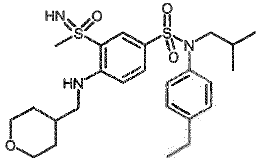 | N-(4-éthylphényl)-N-isobutyl-3-(S-methyl-sulfonimidoyl)-4-[(tetrahydropyran-4-ylmethyl)-amino]-benzenesulfonamide |
| | Composé 52 |
| 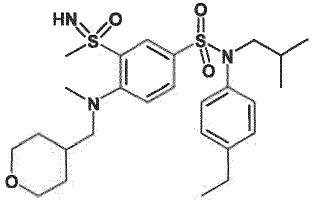 | N-(4-éthylphényl)-N-isobutyl-4-(méthyl-((tétrahydro-2H-pyran-4-yl)méthyl)amino)-3-(S-méthylsulfonimidoyl)-benzenesulfonamide |
| | Composé 53 |
| 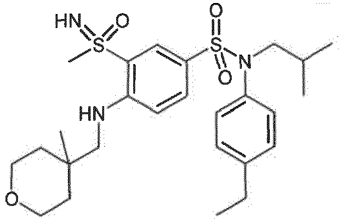 | N-(4-éthylphényl)-N-isobutyl-3-(S-méthyl-sulfonimidoyl)-4-(((4-methyltetrahydro-2H-pyran-4-yl)methyl)amino)-benzenesulfonamide |
| | Composé 55 |
| 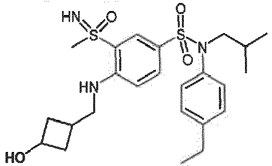 | N-(4-éthylphényl)-4-(((3-hydroxycyclobutyl)methyl)amino)-N-isobutyl-3-(S-methyl-sulfonimidoyl)-benzenesulfonamide |
| | Composé 61 |
| 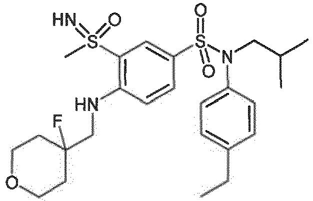 | N-(4-éthylphényl)-4-(((4-fluorotetrahydro-2H-pyran-4-yl)methyl)amino)-N-isobutyl-3-(S-methyl-sulfonimidoyl)benzene sulfonamide |
| | Composé 62 |
| 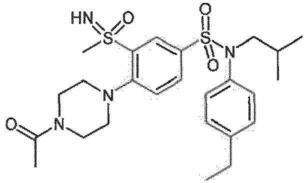 | :4-(4-acetylpiperazin-1-yl)-N-(4-ethylphenyl)-N-isobutyl-3-(S-methyl-sulfonimidoyl)-benzene-sulfonamide |
| | Composé 63 |
| 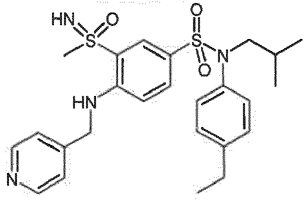 | N-(4-ethylphenyl)-N-isobutyl-3-(S-methyl-sulfonimidoyl)-4-((pyridin-4-ylmethyl)amino)-benzenesulfonamide |
| | Composé 64 |

6. Composé selon l'une quelconque des revendications précédentes, **caractérisé en ce qu'**il est choisi parmi les composés suivants:
| | |
|---|---|
| 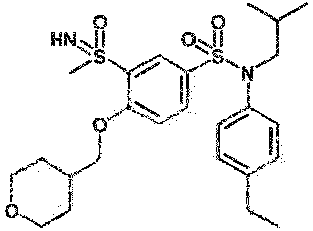 | N-(4-éthyl-phényl)-N-isobutyl-3-methane-sulfoximin-4-(tetrahydro-pyran-4-ylmethoxy)-benzenesulfonamide |
| | Composé 26 |
| 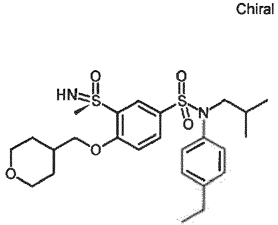 | N-(4-éthyl-phényl)-N-isobutyl-3-méthane-sulfoximin-4-(tétrahydro-pyran-4-ylméthoxy)-benzènesulfonamide |
| | composé 7 (énantiomère A) |
| 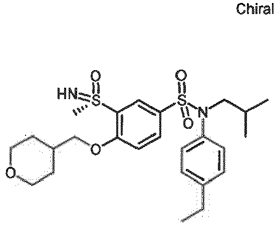 | N-(4-éthyl-phényl)-N-isobutyl-3-méthane-sulfoximin-4-(tétrahydro-pyran-4-ylméthoxy)-benzènesulfonamide |
| | composé 8 (énantiomère B) |
| 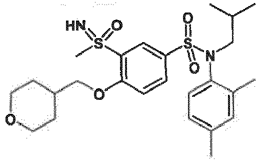 | N-(2,4-diméthylphényl)-N-isobutyl-3-(S-méthylsulfonimidoyl)-4-(tétrahydro-2H-pyran-4-yl)méthoxy)benzenesulfonamide |
| | Composé 142 |

7. Composé selon l'une quelconque des revendications précédentes pour son utilisation en tant que médicament.

8. Composé selon l'une quelconque des revendications 1 à 6, pour son utilisation dans le traitement des désordres inflammatoires et/ou des maladies auto-immunes médiées par le récepteur RORyt.

9. Composé pour son utilisation selon la revendication 8, dans le traitement de l'acné.

10. Composé pour son utilisation selon la revendication 8, dans le traitement du psoriasis.

11. Composition pharmaceutique comprenant un ou plusieurs composés tels que définis selon l'une quelconque des revendications 1 à 6.

12. Composition pharmaceutique selon la revendication 11 pour son utilisation pour le traitement des désordres inflammatoires et/ou des maladies auto-immunes médiées par le récepteur RORγt, notamment l'acné, la dermatite atopique et/ou le psoriasis.

## Patentansprüche

1. Verbindung der Formel (II), pharmazeutisch unbedenkliche Additionssalze davon, Hydrate davon und/oder Solvate davon: 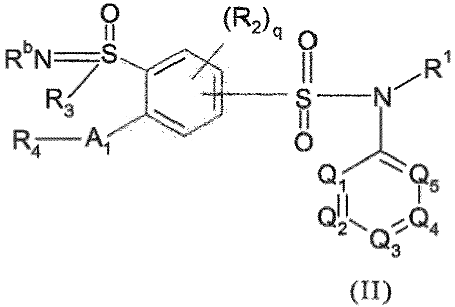 wobei in der Formel (II):
• q null oder eine natürliche ganze Zahl im Bereich von 1 bis 3 bedeutet,
• R¹ für einen linearen oder verzweigten C₃-C₅-Alkylrest, einen C₃-C₅-Cycloalkylrest, einen linearen oder verzweigten C₂-C₅-Alkenylrest, einen (C₁)Alkyl-C₃-C₅-cycloalkylrest, einen C₄-C₅-Heterocycloalkylrest oder einen (C₁)Alkyl-C₄-C₅-heterocycloalkylrest steht,
• R₂ für ein Wasserstoffatom oder ein Halogenatom, einen linearen oder verzweigten C₁-C₅-Alkylrest, einen linearen oder verzweigten C₂-C₄-Alkenylrest, einen C₁-C₄-Alkoxyrest oder eine Cyanogruppe -CN steht; wobei die Alkyl-, Alkenyl- und Alkoxyreste durch ein oder mehrere Halogenatome substituiert sein können;
• R³ für einen C₁-C₃-Alkylrest steht;
• R⁴ für ein Wasserstoffatom oder eine Gruppe (CHR⁵)ₙ-(Z)ₒ-(CHR'⁵)ₚ-R⁶ steht,
• n, o und p gleich oder verschieden sind und für null oder eine natürliche ganze Zahl im Bereich von 1 bis 3 stehen,
• Z für eine zweiwertige Gruppe steht, die aus -CH₂-, -NH- und -O- ausgewählt ist,
• R⁵ und R'⁵ gleich oder verschieden sind und für ein Wasserstoffatom, einen Methylrest -CH₃, einen Hydroxyrest -OH, einen Cᵢ-Hydroxyalkylrest oder einen Carboxylrest -COOH stehen,
• R⁶ für:
- ein Wasserstoffatom oder ein Halogenatom,
- einen Heterocycloalkylrest, der gegebenenfalls durch ein oder mehrere Halogenatome, eine oder mehrere lineare oder verzweigte C₁-C₃-Alkylgruppen, eine oder mehrere -OH-Gruppen, eine oder mehrere Carbonylfunktionen =O, eine oder mehrere lineare oder verzweigte C₁-C₄-Hydroxyalkylgruppen, einen Pyrrolidinring, eine oder mehrere Aminogruppen, eine oder mehrere Gruppen -C(=O)R⁷ oder eine oder mehrere Gruppen S(=O)₂R⁷ substituiert ist; wobei R⁷ für einen linearen oder verzweigten C₁-C₃-Alkylrest, einen Hydroxyrest -OH, einen linearen oder verzweigten C₁-C₄-Alkoxyrest oder einen Aminorest N(R^{7a}) (R^{7b}) steht; wobei R^{7a} und R^{7b} gleich oder verschieden sind und ein Wasserstoffatom, einen linearen oder verzweigten C₁-C₃-Alkylrest oder einen Cyclopropylrest bedeuten,
- einen C₃-C₆-Cycloalkylrest, der gegebenenfalls durch eine oder mehrere -OH-Gruppen substituiert ist,
- einen aromatischen oder heteroaromatischen Rest, der gegebenenfalls durch ein oder mehrere Halogenatome, eine oder mehrere C₁-C₃-Alkylgruppen, die gegebenenfalls durch ein oder mehrere Halogenatome substituiert sind, eine oder mehrere C₁-C₃-Alkoxygruppen, eine oder mehrere Aminogruppen -NR¹¹R¹², eine oder mehrere Gruppen -COR¹¹, eine Carbonylfunktion (=O), eine oder mehrere Gruppen -OR¹¹, eine oder mehrere C₁-C₄-Hydroxyalkylgruppen, eine oder mehrere Gruppen -COOR¹¹, eine oder mehrere Amidogruppen -CONR¹¹R¹², eine oder mehrere Gruppen -SOR¹¹, eine oder mehrere Gruppen -SO₂R¹¹, eine oder mehrere Gruppen -NHCOR¹¹, eine oder mehrere Gruppen -NHCOOR¹¹, eine oder mehrere Gruppen -SO₂NR¹¹R¹² oder eine oder mehrere -CN-Gruppen substiuiert ist; wobei R¹¹ und R¹² gleich oder verschieden sind und für ein Wasserstoffatom oder einen linearen oder verzweigten C₁-C₃-Alkylrest, der gegebenenfalls durch ein oder mehrere Halogenatome substituiert ist, stehen, steht,
• A₁ für eine zweiwertige Gruppe steht, die aus -NR^{a-}, -O-, -S-, -SO-, -SO₂-, -SO(=NH)-, -CH₂-, -C=C- und -CH(R^{a})- ausgewählt ist,
• R^{a} für ein Wasserstoffatom, einen linearen oder verzweigten C₁-C₃-Alkylrest oder einen Acetylrest -C(=O)CH₃ steht,
• R^{b} für ein Wasserstoffatom, einen linearen oder verzweigten C₁-C₃-Alkylrest oder eine Cyclopropylgruppe steht,
• Q₁, Q₂, Q₃, Q₄ und Q₅ gleich oder verschieden sind und für ein Stickstoffatom oder eine Gruppe -CR'₂ stehen,
• R'₂ für ein Wasserstoffatom oder einen linearen oder verzweigten C₁-C₅-Alkylrest steht,
• R^{a} und R³ zusammen mit den Kohlenstoffatomen, an die sie gebunden sind, eine Heterocycloalkylgruppe, die gegebenenfalls durch eine oder mehrere Carbonylfunktionen, einen oder mehrere C₁-C₃-Alkylreste substituiert sein kann, bilden können,
• dann, wenn A₁ für -NR^{a}- steht, R^{a} und R⁴ zusammen mit dem Stickstoffatom, an das sie gebunden sind, eine C₂-C₁₀-Heterocycloalkylgruppe, die gegebenenfalls ein bis drei Heteroatome, die aus einem Schwefelatom, dem Stickstoffatom und ein Sauerstoffatom ausgewählt sind, umfasst, bilden können, wobei die Heterocycloalkylgruppe gegebenenfalls durch mindestens einen Rest R¹⁴ substituiert ist,
• R¹⁴ für einen linearen oder verzweigten C₁-C₃-Alkylrest, einen linearen oder verzweigten C₁-C₃-Alkoxyrest, ein Halogenatom, eine Hydroxygruppe -OH, eine Cyanogruppe -CN, eine Gruppe -CONR¹⁵R¹⁶, eine Gruppe -SO₂R¹⁵, eine Gruppe -COR¹⁵ oder eine Aminogruppe -NR¹⁵R¹⁶ steht, wobei R¹⁵ und R¹⁶ gleich oder verschieden sind und für ein Wasserstoffatom oder einen linearen oder verzweigten C₁-C₃-Alkylrest stehen.

2. Verbindung der Formel (II) nach Anspruch 1, **dadurch gekennzeichnet, dass** R⁶ für einen heterocyclischen Rest steht, der aus den folgenden Heterocyclen ausgewählt ist: 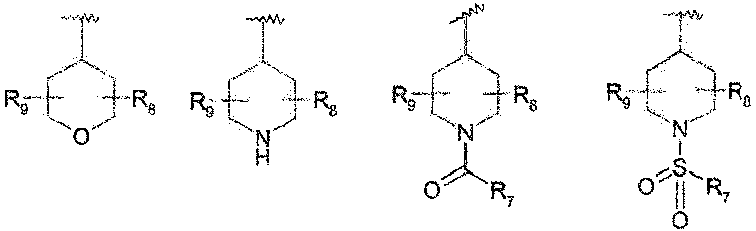 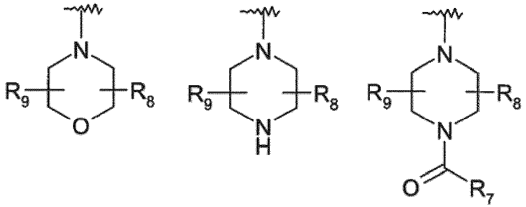 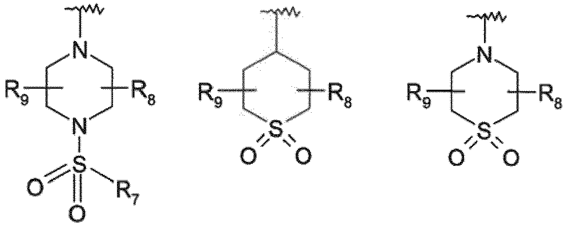 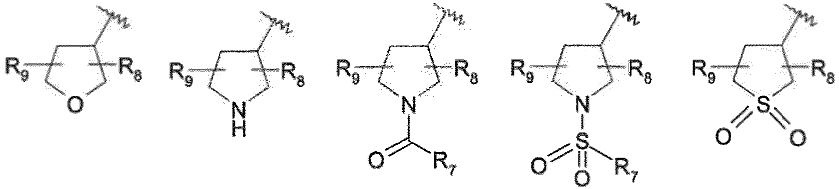 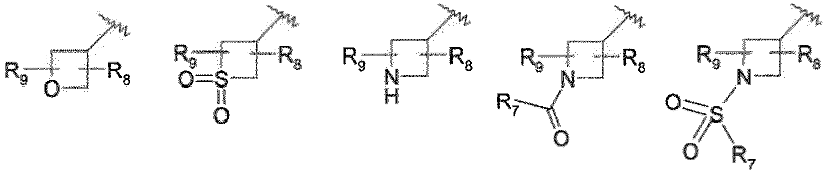 in denen:
- R₇ für einen linearen oder verzweigten C₁-C₃-Alkylrest, einen Hydroxyrest -OH, einen linearen oder verzweigten C₁-C₃-Alkoxyrest oder einen Aminorest N(R^{7a}) (R^{7b}) steht,
- R^{7a} und R^{7b} gleich oder verschieden sind und ein Wasserstoffatom, einen linearen oder verzweigten C₁-C₃-Alkylrest oder einen Cyclopropylrest bedeuten,
- R₈ und R₉ gleich oder verschieden sind und für ein Wasserstoffatom, einen linearen oder verzweigten C₁-C₃-Alkylrest, eine Hydroxygruppe -OH, eine Carbonylfunktion, einen C₁-Hydroxyalkylrest (-CH₂OH) oder eine Aminogruppe -NH₂ stehen,
- R₈ und R₉ zusammen mit den Kohlenstoffatomen, an die sie gebunden sind, einen 5- bis 7-gliedrigen heterocyclischen Ring bilden können.

3. Verbindung der Formel (II) nach Anspruch 1, **dadurch gekennzeichnet, dass** R⁶ für einen aromatischen oder heteroaromatischen Rest steht, der aus den folgenden Resten ausgewählt ist: 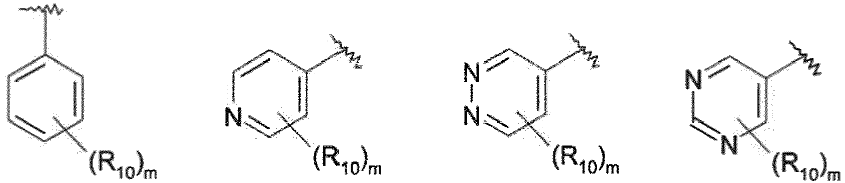 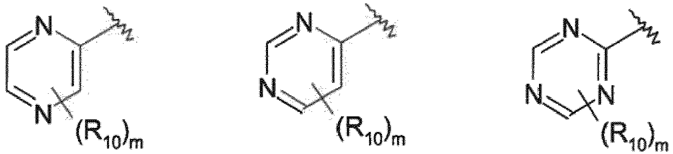 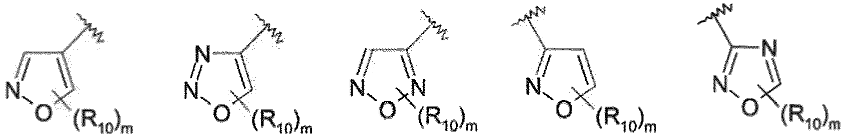 in denen:
- R₁₀ für ein Wasserstoffatom oder ein Halogenatom, eine C₁-C₃-Alkylgruppe, die gegebenenfalls durch ein oder mehrere Halogenatome substituiert ist, eine Carbonylfunktion (=0), eine Gruppe -OR¹¹, eine C₁-C₄-Hydroxyalkylgruppe, eine Aminogruppe -NR¹¹R¹², eine Gruppe -COR¹¹, eine Gruppe -COOR¹¹, eine Amidogruppe -CONR¹¹R¹², eine Gruppe -SOR¹¹, eine Gruppe -SO₂R¹¹, eine Gruppe -NHCOR¹¹, eine Gruppe -NHCOOR¹¹, eine Gruppe -SO₂NR¹¹R¹² oder eine Cyanogruppe -CN steht;
- R¹¹ und R¹² gleich oder verschieden sind und für ein Wasserstoffatom oder einen linearen oder verzweigten C₁-C₃-Alkylrest, der gegebenenfalls durch ein oder mehrere Halogenatome substituiert ist, stehen,
- m null oder eine natürliche ganze Zahl im Bereich von 1 bis 3 bedeutet.

4. Verbindung nach einem der vorhergehenden Ansprüche, **dadurch gekennzeichnet, dass** sie aus den folgenden Verbindungen sowie den pharmazeutisch unbedenklichen Additionssalzen davon, Hydraten davon und/oder Solvaten davon ausgewählt ist:
| | |
|---|---|
| 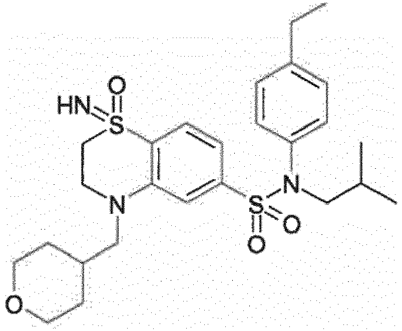 | Imino-1-oxo-4-(tetrahydropyran-4-ylmethyl)-1,2,3,4-tetrahydro-1λ⁶-benzo[1,4]thiazin-7-sulfonsäure(4-ethylphenyl)isobutylamid |
| | Verbindung 1 |
| 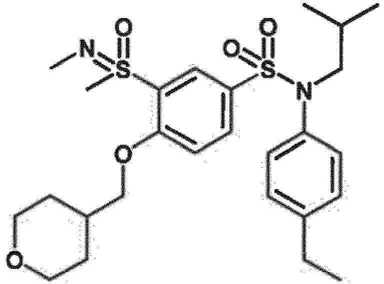 | N-(4-Ethylphenyl)-N-isobutyl-3-methansulfinyl-4-(tetrahydropyran-4-ylmethoxy)benzol-N-methylsulfoximin |
| | Verbindung 2 |
| 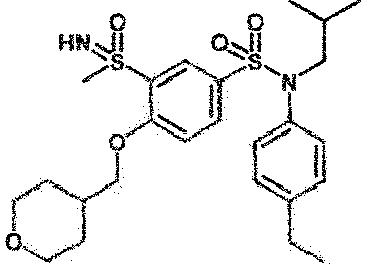 | N-(4-Ethylphenyl)-N-isobutyl-3-methansulfoximino-4-(tetrahydropyran-4-ylmethoxy)benzolsulfonamid |
| | Verbindung 26 |
| 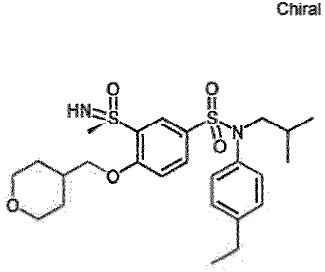 | N-(4-Ethylphenyl)-N-isobutyl-3-methansulfoximino-4-(tetrahydropyran-4-ylmethoxy)benzolsulfonamid |
| | Verbindung 7 (Enantiomer A) |
| 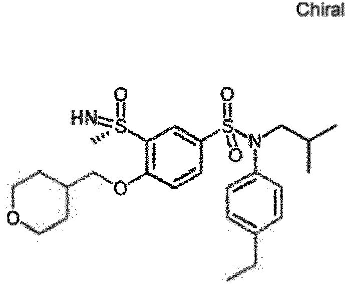 | N-(4-Ethylphenyl)-N-isobutyl-3-methansulfoximino-4-(tetrahydropyran-4-ylmethoxy)benzolsulfonamid |
| | Verbindung 8 (Enantiomer B) |
| 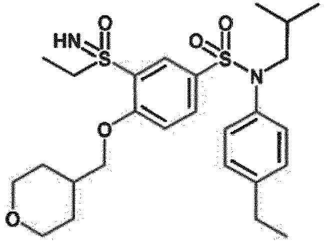 | N-(4-Ethylphenyl)-N-isobutyl-3-ethansulfoximino-4-(tetrahydropyran-4-ylmethoxy)benzolsulfonamid |
| | Verbindung 18 |
| 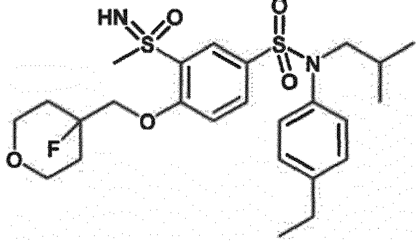 | N-(4-Ethylphenyl)-4-((4-fluortetrahydro-2H-pyran-4-yl)methoxy)-N-isobutyl-3-(S-methylsulfonimidoyl)benzolsulfonamid |
| | Verbindung 30 |
| 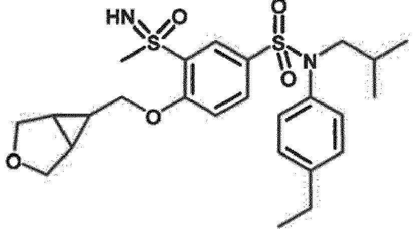 | 4-((3-Oxabicyclo[3.1.0]-hexan-6-yl)methoxy)-N-(4-ethylphenyl)-N-isobutyl-3-(S-methylsulfon-imidoyl)benzolsulfonamid |
| | Verbindung 31 |
| 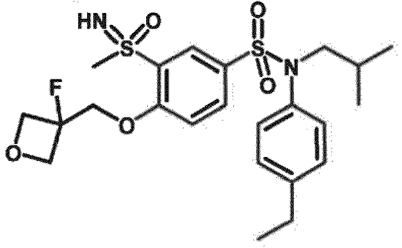 | N-(4-Ethylphenyl)-4-((3-fluoroxetan-3-yl)-methoxy)-N-isobutyl-3-(S-methylsulfonimidoyl)-benzolsulfonamid |
| | Verbindung 32 |
| 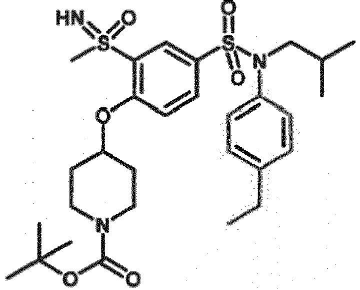 | 4-(4-(N-(4-Ethylphenyl)-N-isobutylsulfamoyl)-2-(S-methylsulfonimidoyl)-phenoxy)piperidin-1-carbonsäure-tert-butylester |
| | Verbindung 33 |
| 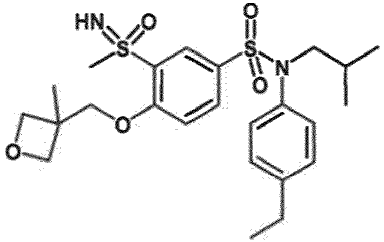 | N-(4-Ethylphenyl)-N-isobutyl-4-((3-methyloxetan-3-yl)methoxy)-3-(S-methylsulfonimidoyl)-benzolsulfonamid |
| | Verbindung 34 |
| 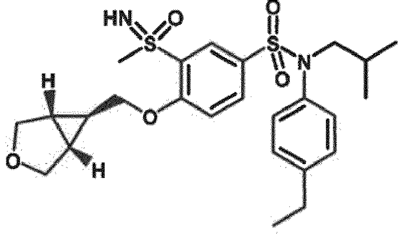 | 4-(((1R,5S,6R)-3-Oxabicyclo[3.1.0]hexan-6-yl)methoxy)-N-(4-ethylphenyl)-N-isobutyl-3-(S-methylsulfonimidoyl)benzolsulfonamid |
| | Verbindung 35 |
| 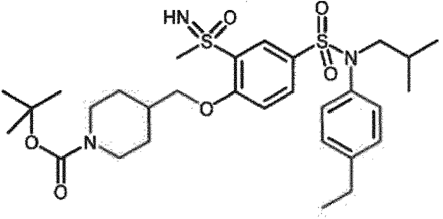 | 4-((4-(N-(4-ethylphenyl)-N-isobutylsulfamoyl)-2-(S-methylsulfonimidoyl)-phenoxy)methyl)piperidin-1-carbonsäure-tert-butylester |
| | Verbindung 36 |
| 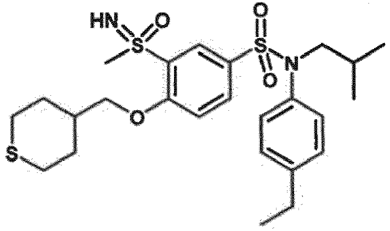 | N-(4-Ethylphenyl)-N-isobutyl-3-(S-methylsulfonimidoyl)-4-(pyridin-4-ylmethoxy)benzolsulfonamid |
| | Verbindung 37 |
| 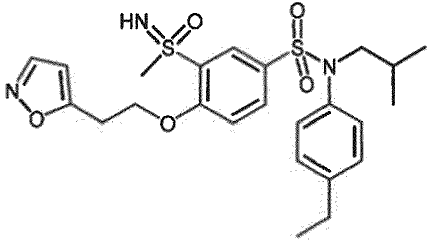 | N-(4-Ethylphenyl)-N-isobutyl-4-(2-(isoxazol-5-yl)ethoxy)-3-(S-methylsulfonimidoyl)-benzolsulfonamid |
| | Verbindung 38 |
| 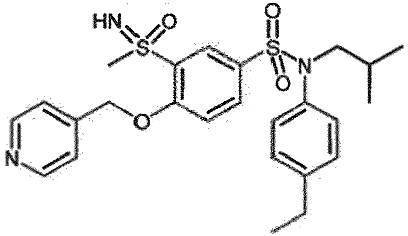 | N-(4-Ethylphenyl)-N-isobutyl-3-(S-methylsulfonimidoyl)-4-(pyridin-4-ylmethoxy)-benzolsulfonamid |
| | Verbindung 39 |
| 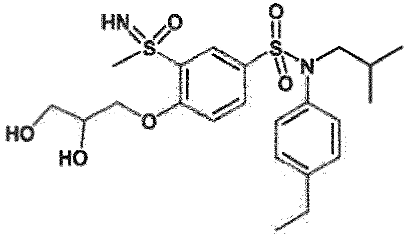 | 4-(2,3-Dihydroxypropoxy)-N-(4-ethylphenyl)-N-isobutyl-3-(S-methylsulfonimidoyl)benzolsulfonamid |
| | Verbindung 40 |
| 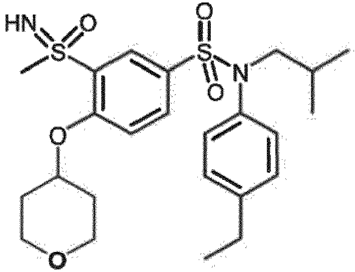 | N-(4-Ethylphenyl)-N-isobutyl-3-(S-methyl-sulfonimidoyl)-4-((tetrahydro-2H-pyran-4-yl)oxy)benzolsulfonamid |
| | Verbindung 42 |
| 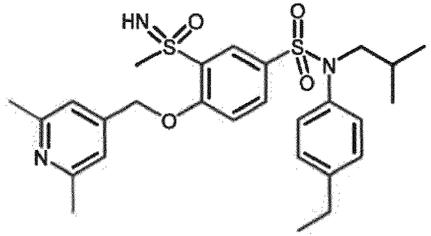 | 4-((2,6-Dimethylpyridin-4-yl)methoxy)-N-(4-ethylphenyl)-N-isobutyl-3-(S-methylsulfonimidoyl)benzolsulfonamid |
| | Verbindung 43 |
| 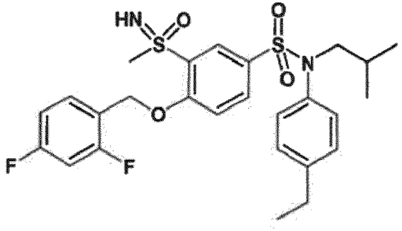 | 4-((2,4-Difluorbenzyl)-oxy)-N-(4-ethylphenyl)-N-isobutyl-3-(S-methyl-sulfonimidoyl)benzolsulfonamid |
| | Verbindung 45 |
| 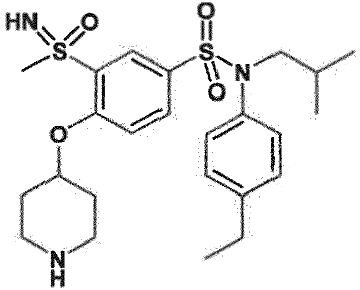 | N-(4-Ethylphenyl)-N-isobutyl-3-(S-methyl-sulfonimidoyl)-4-(piperidin-4-ylmethoxy)benzolsulfonamid N-(4-Ethylphenyl)-N-isobutyl-3-(S-methylsulfonimidoyl)-4-(piperidin-4-ylmethoxy)benzolsulfonamid |
| | Verbindung 46 |
| 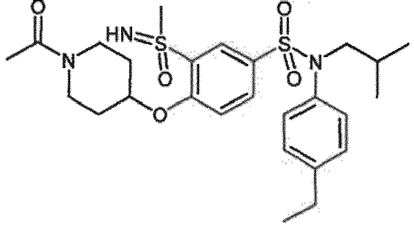 | 4-((1-Acetylpiperidin-4-yl)oxy)-N-(4-ethylphenyl)-N-isobutyl-3-(S-methylsulfonimidoyl)benzolsulfonamid |
| | Verbindung 47 |
| 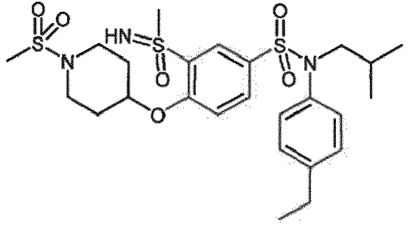 | N-(4-Ethylphenyl)-N-isobutyl-3-(S-methylsulfonimidoyl)-4-((1-(methylsulfonyl)-piperidin-4-yl)oxy)-benzol sulfonamid |
| | Verbindung 48 |
| 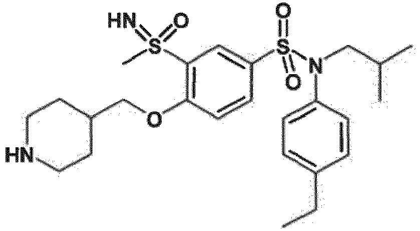 | N-(4-Ethylphenyl)-N-isobutyl-3-(S-methylsulfonimidoyl)-4-(piperidin-4-ylmethoxy)benzolsulfonamid |
| | Verbindung 49 |
| 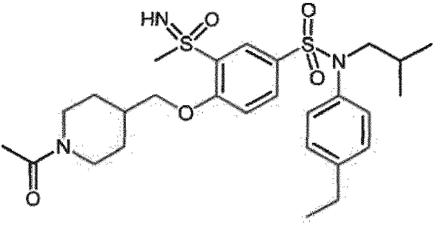 | 4-((1-Acetylpiperidin-4-yl)methoxy)-N-(4-ethylphenyl)-N-isobutyl-3-(S-methylsulfonimidoyl)benzolsulfonamid |
| | Verbindung 50 |
| 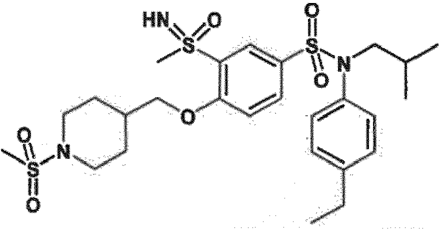 | N-(4-Ethylphenyl)-N-isobutyl-3-(S-methylsulfonimidoyl)-4-((1-(methylsulfonyl-piperidin-4-yl)methoxy)benzolsulfonamid |
| | Verbindung 51 |
| 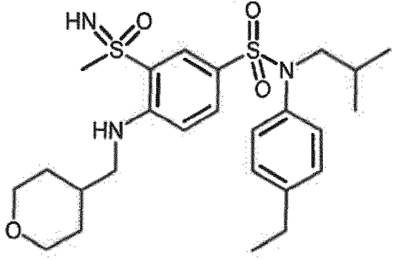 | N-(4-Ethylphenyl)-N-isobutyl-3-(S-methylsulfonimidoyl)-4-[(tetrahydropyran-4-ylmethyl)amino]benzolsulfonamid |
| | Verbindung 52 |
| 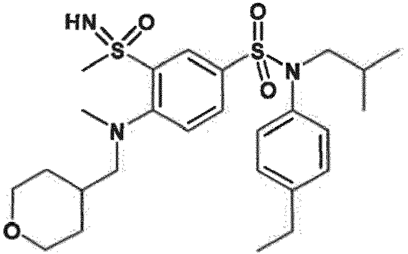 | N-(4-Ethylphenyl)-N-isobutyl-4-(methyl-((tetrahydro-2H-pyran-4-yl)methyl)amino)-3-(S-methylsulfonimidoyl)-benzol sulfonamid |
| | Verbindung 53 |
| 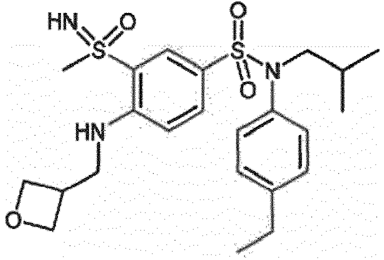 | N-(4-Ethylphenyl)-N-isobutyl-3-(S-methyl-sulfonimidoyl)-4-((oxetan-3-ylmethyl)-amino)benzolsulfonamid |
| | Verbindung 54 |
| 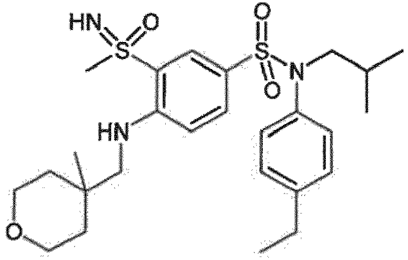 | N-(4-Ethylphenyl)-N-isobutyl-3-(S-methylsulfonimidoyl)-4-(((4-methyltetrahydro-2H-pyran-4-yl)methyl)amino)-benzolsulfonamid |
| | Verbindung 55 |
| 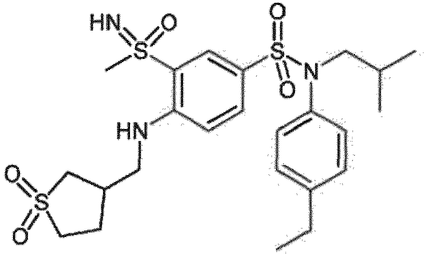 | 4-(((1,1-Dioxidotetrahydrothiophen -3-yl)methyl)amino)-N-(4-ethylphenyl)-N-isobutyl-3-(S-methylsulfonimidoyl)benzolsulfonamid |
| | Verbindung 56 |
| 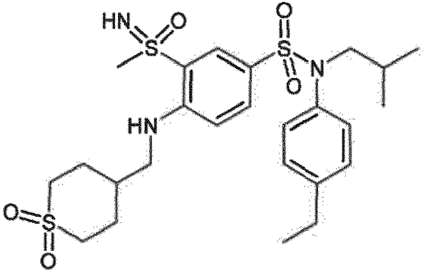 | 4-(((1,1-Dioxido-tetrahydro-2H-thiopyran-4-yl)methyl)amino)-N-(4-ethylphenyl)-N-isobutyl-3-(S-methylsulfonimidoyl)benzolsulfonamid |
| | Verbindung 57 |
| 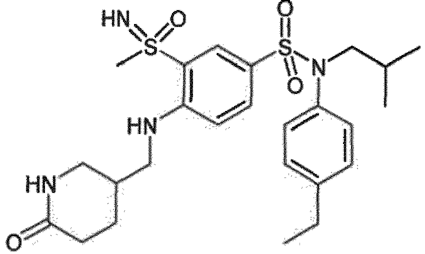 | N-(4-Ethylphenyl)-N-isobutyl-3-(S-methylsulfonimidoyl)-4-(((6-oxopiperidin-3-yl)-methyl)amino)benzolsulfonamid |
| | Verbindung 58 |
| 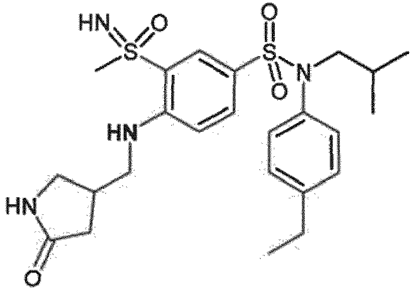 | N-(4-Ethylphenyl)-N-isobutyl-3-(S-methylsulfonimidoyl)-4-(((5-oxopyrrolidin-3-yl)methyl)amino)-benzolsulfonamid |
| | Verbindung 59 |
| 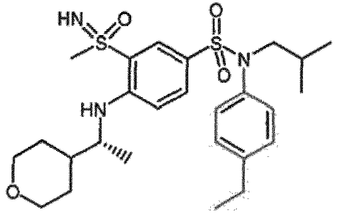 | N-(4-Ethylphenyl)-N-isobutyl-3-(S-methylsulfonimidoyl)-4-(((R)-1-(tetrahydro-2H-pyran-4-yl)ethyl)amino)-benzolsulfonamid |
| | Verbindung 60 |
| 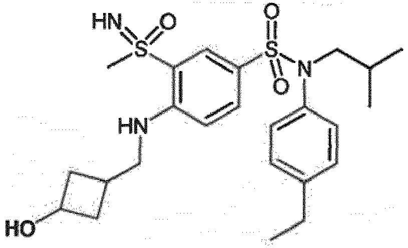 | N-(4-Ethylphenyl)-4-(((3-hydroxycyclobutyl) methyl) amino)-N-isobutyl-3-(S-methylsulfonimidoyl)-benzolsulfonamid |
| | Verbindung 61 |
| 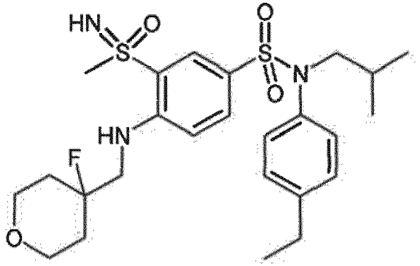 | N-(4-Ethylphenyl)-4-(((4-fluortetrahydro-2H-pyran-4-yl)methyl)amino)-N-isobutyl-3-(S-methylsulfonimidoyl)-benzolsulfonamid |
| | Verbindung 62 |
| 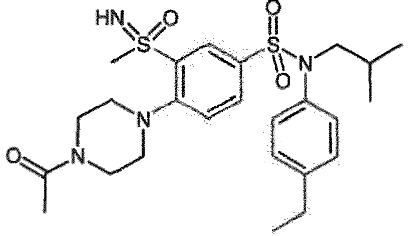 | 4-(4-Acetylpiperazin-1-yl)-N-(4-ethylphenyl)-N-isobutyl-3-(S-methylsulfonimidoyl)-benzol sulfonamid |
| | Verbindung 63 |
| 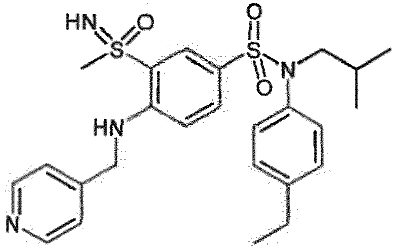 | N-(4-Ethylphenyl)-N-isobutyl-3-(S-methylsulfonimidoyl)-4-((pyridin-4-ylmethyl)-amino)benzolsulfonamid |
| | Verbindung 64 |
| 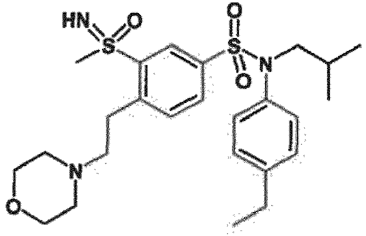 | N-(4-Ethylphenyl)-N-isobutyl-3-(S-methylsulfonimidoyl)-4-(2-morpholinoethyl)-benzol sulfonamid |
| | Verbindung 65 |
| 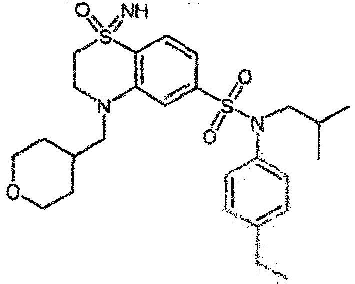 | 1-Imino-1-oxo-4-(tetrahydropyran-4-ylmethyl)-1,2,3,4-tetrahydro-1λ⁶-benzo[1,4]thiazin-6-sulfonsäure(4-ethylphenyl)isobutylamid |
| | Verbindung 69 |
| 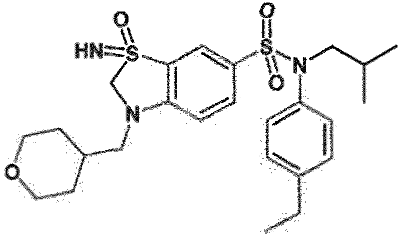 | 1-Imino-1-oxo-3-(tetrahydropyran-4-ylmethyl)-2,3-dihydro-1H-1λ⁶-benzothiazol-6-sulfonsäure(4-ethylphenyl)isobutylamid |
| | Verbindung 76 |
| 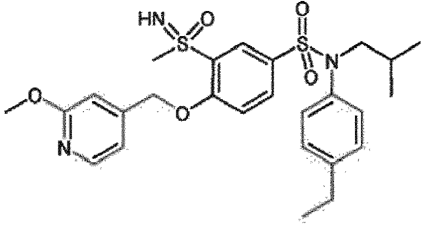 | N-(4-Ethylphenyl)-N-isobutyl-4-((2-methoxypyridin-4-yl)methoxy)-3-(S-methylsulfonimidoyl)-benzolsulfonamid |
| | Verbindung 77 |
| 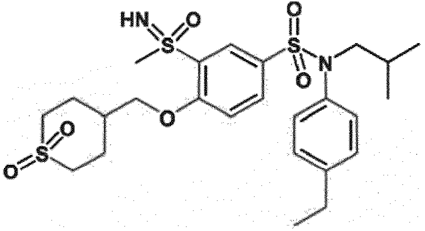 | N-(4-Ethylphenyl)-N-isobutyl-4-((2-methoxypyridin-4-yl)methoxy)-3-(S-methylsulfonimidoyl)-benzolsulfonamid |
| | Verbindung 78 |
| 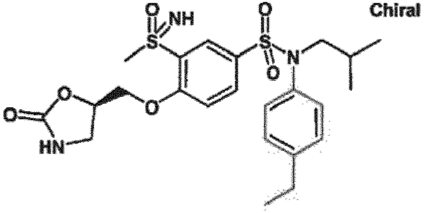 | N-(4-Ethylphenyl)-N-isobutyl-3-(S-methylsulfonimidoyl)-4-(((R)-2-oxooxazolidin-5-yl)methoxy)-benzolsulfonamid |
| | Verbindung 79 |
| 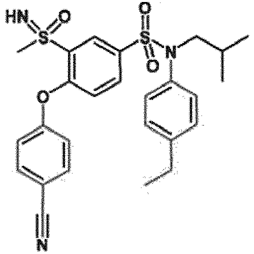 | 4-(4-Cyanophenoxy)-N-(4-ethylphenyl)-N-isobutyl-3-(S-methylsulfonimidoyl)-benzolsulfonamid |
| | Verbindung 80 |
| 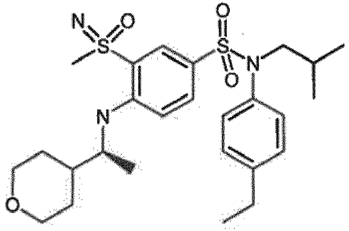 | N-(4-Ethylphenyl)-N-isobutyl-3-(S-methylsulfonimidoyl)-4-(((S)-1-(tetrahydro-2H-pyran-4-yl)ethyl)amino)-benzolsulfonamid |
| | Verbindung 81 |
| 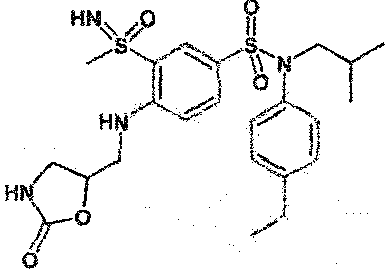 | N-(4-Ethylphenyl)-N-isobutyl-3-(S-methylsulfonimidoyl)-4-(((2-oxooxazolidin-5-yl)methyl)amino)-benzolsulfonamid |
| | Verbindung 82 |
| 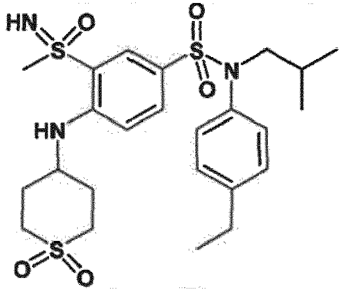 | 4-((1,1-Dioxido-tetrahydro-2H-thiopyran-4-yl)amino)-N-(4-ethylphenyl)-N-isobutyl-3-(S-methylsulfonimidoyl)-benzolsulfonamid |
| | Verbindung 83 |
| 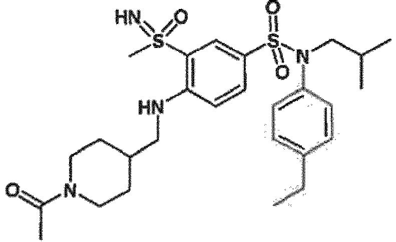 | 4-(((1-Acetyl-piperidin-4-yl)methyl)amino)-N-(4-ethylphenyl)-N-isobutyl-3-(S-methylsulfonimidoyl)-benzolsulfonamid |
| | Verbindung 84 |
| 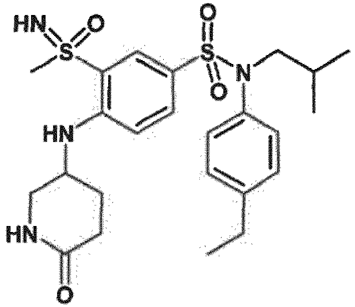 | N-(4-Ethylphenyl)-N-isobutyl-3-(S-methylsulfonimidoyl)-4-((6-oxopiperidin-3-yl)amino)benzolsulfonamid |
| | Verbindung 85 |
| 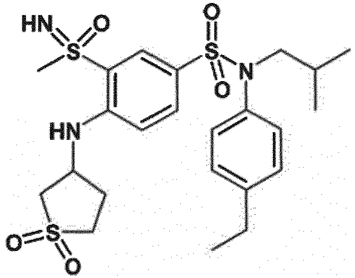 | 4-((1,1-Dioxido-tetrahydrothiophen-3-yl)amino)-N-(4-ethylphenyl)-N-isobutyl-3-(S-methylsulfonimidoyl)-benzolsulfonamid |
| | Verbindung 86 |
| 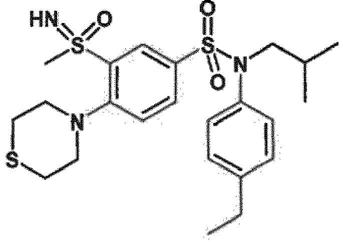 | N-(4-Ethylphenyl)-N-isobutyl-3-(S-methylsulfonimidoyl)-4-thiomorpholinobenzol-sulfonamid |
| | Verbindung 87 |
| 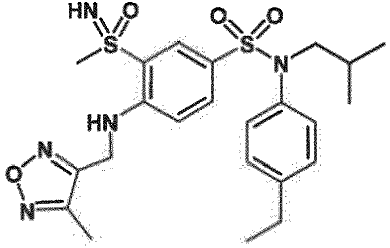 | N-(4-Ethylphenyl)-N-isobutyl-4-(((4-methyl-1,2,5-oxadiazol-3-yl)methyl)amino)-3-(S-methylsulfonimidoyl)-benzolsulfonamid |
| | Verbindung 88 |
| 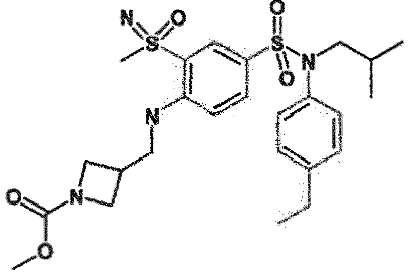 | 3-(((4-(N-(4-Ethylphenyl)-N-isobutylsulfamoyl)-2-(S-methylsulfonimidoyl)phenyl)amino)-methyl)azetidin-1-carbonsäuremethylester |
| | Verbindung 89 |
| 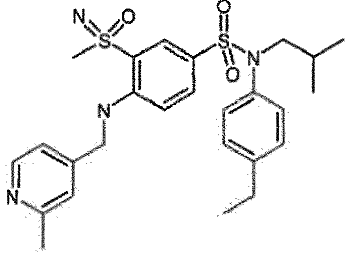 | N-(4-Ethylphenyl)-N-isobutyl-4-(((2-methylpyridin-4-yl)methyl)amino)-3-(S-methylsulfonimidoyl)-benzolsulfonamid |
| | Verbindung 90 |
| 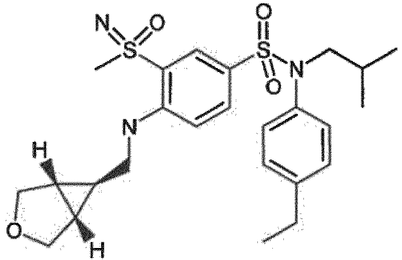 | 4-((((1R,5S,6S)-3-Oxabicyclo[3.1.0]hexan-6-yl)methyl)amino)-N-(4-ethylphenyl)-N-isobutyl-3-(S-methylsulfonimidoyl)-benzolsulfonamid |
| | Verbindung 91 |
| 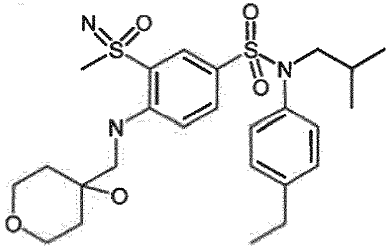 | N-(4-Ethylphenyl)-4-(((4-hydroxytetrahydro-2H-pyran-4-yl)methyl)-amino)-N-isobutyl-3-(S-methylsulfonimidoyl)-benzolsulfonamid |
| | Verbindung 92 |
| 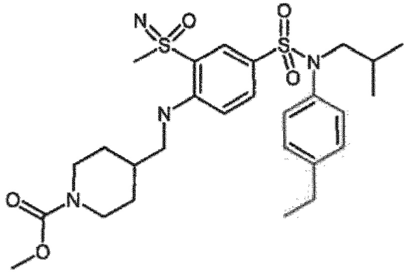 | 4-(((4-(N-(4-Ethyl-phenyl)-N-isobutyl-sulfamoyl)-2-(S-methylsulfonimidoyl)-phenyl)amino)methyl)-piperidin-1-carbonsäuremethylester |
| | Verbindung 93 |
| 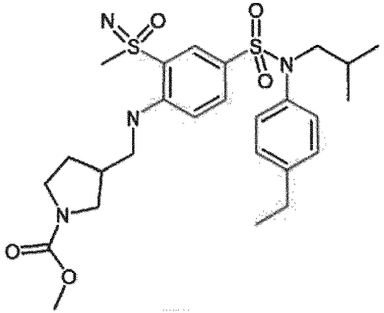 | 3-(((4-(N-(4-Ethylphenyl)-N-isobutylsulfamoyl)-2-(S-methylsulfonimidoyl)phenyl)amino)-methyl)pyrrolidin-1-carbonsäuremethylester |
| | Verbindung 94 |
| 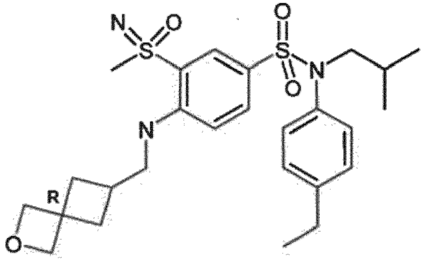 | 4-(((2-Oxaspiro[3.3]-heptan-6-yl)methyl)-amino)-N-(4-ethyl-phenyl)-N-isobutyl-3-(S-methylsulfonimidoyl)benzolsulfonamid |
| | Verbindung 95 |
| 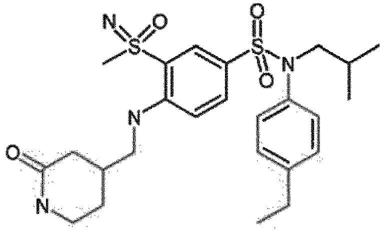 | 4-N-(4-Ethylphenyl)-N-isobutyl-3-(S-methylsulfonimidoyl)-4-(((2-oxopiperidin-4-yl)methyl)amino)benzolsulfonamid |
| | Verbindung 96 |
| 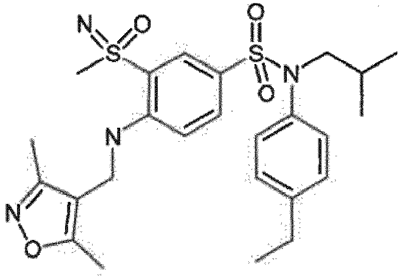 | 4-(((3,5-Dimethyl-isoxazol-4-yl)methyl)-amino)-N-(4-ethylphenyl)-N-isobutyl-3-(S-methylsulfonimidoyl)benzolsulfonamid |
| | Verbindung 97 |
| 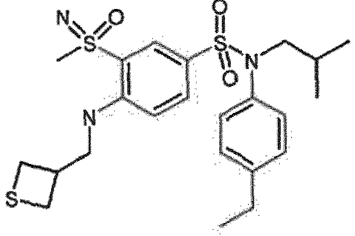 | N-(4-Ethylphenyl)-N-isobutyl-3-(S-methylsulfonimidoyl)-4-((thietan-3-ylmethyl)amino)-benzol sulfonamid |
| | Verbindung 99 |
| 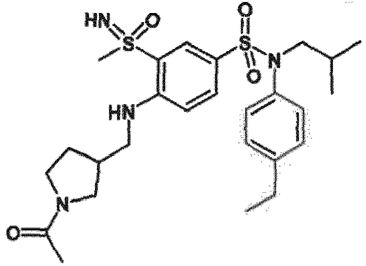 | 4-(((1-Acetyl-pyrrolidin-3-yl)-methyl)amino)-N-(4-ethylphenyl)-N-isobutyl-3-(S-methylsulfonimidoyl)-benzolsulfonamid |
| | Verbindung 100 |
| 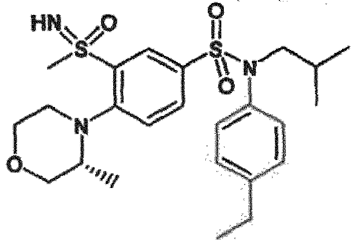 | N-(4-Ethylphenyl)-N-isobutyl-4-((R)-3-methylmorpholino)-3-(S-methylsulfonimidoyl)-benzolsulfonamid |
| | Verbindung 103 |
| 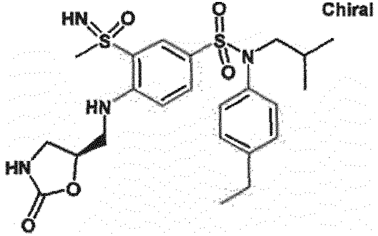 | N-(4-Ethylphenyl)-N-isobutyl-3-(S-methylsulfonimidoyl)-4-((((R)-2-oxooxazolidin-5-yl)methyl)amino)-benzolsulfonamid |
| | Verbindung 105 |
| 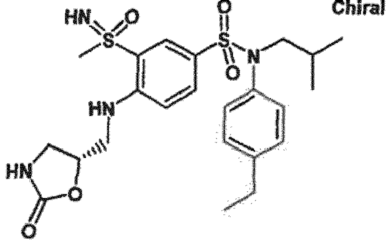 | N-(4-Ethylphenyl)-N-isobutyl-3-(S-methyl-sulfonimidoyl)-4-((((S)-2-oxooxazolidin-5-yl)methyl)amino)-benzolsulfonamid |
| | Verbindung 106 |
| 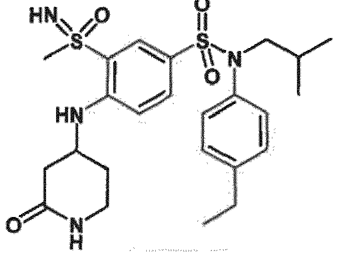 | N-(4-Ethylphenyl)-N-isobutyl-3-(S-methylsulfonimidoyl)-4-((2-oxopiperidin-4-yl)-amino)benzolsulfonamid |
| | Verbindung 107 |
| 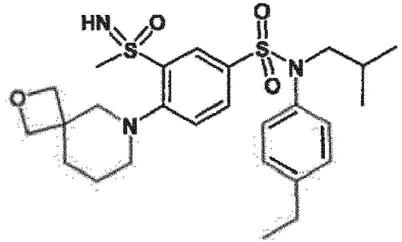 | N-(4-Ethylphenyl)-N-isobutyl-3-(S-methylsulfonimidoyl)-4-(2-oxa-6-azaspiro[3.5]-nonan-6-yl)benzolsulfonamid |
| | Verbindung 109 |
| 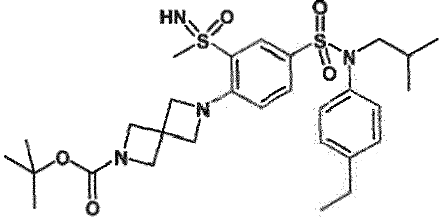 | 6-(4-(N-(4-Ethylphenyl)-N-isobutyl-sulfamoyl)-2-(S-methylsulfonimidoyl)-phenyl)-2,6-diazaspiro[3.3]heptan-2-carbonsäure-tert-butylester |
| | Verbindung 110 |
| 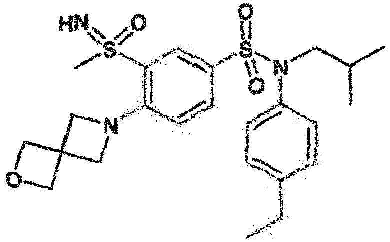 | N-(4-Ethylphenyl)-N-isobutyl-3-(S-methylsulfonimidoyl)-4-(2-oxa-6-azaspiro-[3.3]heptan-6-yl)benzolsulfonamid |
| | Verbindung 111 |
| 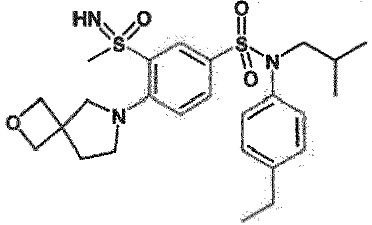 | N-(4-Ethylphenyl)-N-isobutyl-3-(S-methylsulfonimidoyl)-4-(2-oxa-6-azaspiro-[3.4]octan-6-yl)-benzolsulfonamid |
| | Verbindung 112 |
| 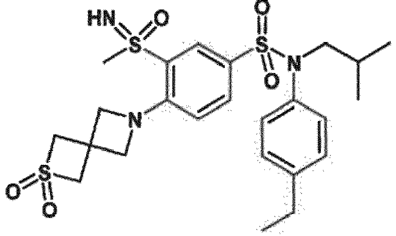 | 4-(2,2-Dioxido-2-thia-6-azaspiro[3.3]heptan-6-yl)-N-(4-ethylphenyl)-N-isobutyl-3-(S-methylsulfonimidoyl)benzolsulfonamid |
| | Verbindung 113 |
| 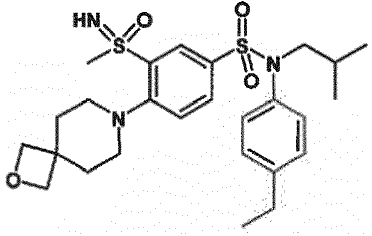 | N-(4-Ethylphenyl)-N-isobutyl-3-(S-methylsulfonimidoyl)-4-(2-oxa-7-azaspiro[3.5]-nonan-7-yl)benzolsulfonamid |
| | Verbindung 114 |
| 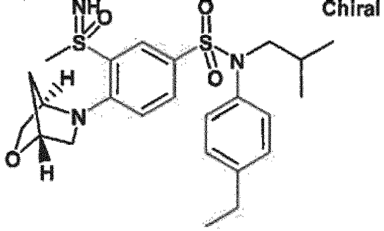 | 4-((1R,4R)-2-Oxa-5-azabicyclo[2.2.1]-heptan-5-yl)-N-(4-ethylphenyl)-N-isobutyl-3-(S-methylsulfonimidoyl)-benzolsulfonamid |
| | Verbindung 115 |
| 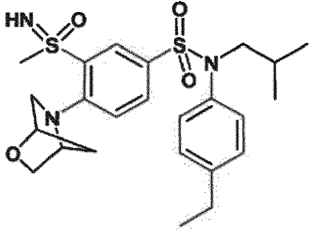 | 4-((1R,4R)-2-Oxa-5-azabicyclo[2.2.1]heptan -5-yl)-N-(4-ethylphenyl)-N-isobutyl-3-(S-methylsulfonimidoyl)benzolsulfonamid |
| | Verbindung 117 |
| 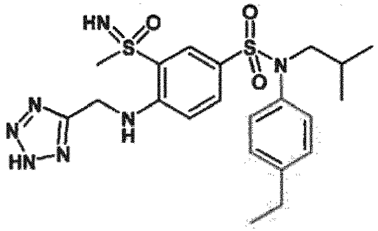 | 4-(((2H-Tetrazol-5-yl)methyl)amino)-N-(4-ethylphenyl)-N-isobutyl-3-(S-methylsulfonimidoyl)benzolsulfonamid |
| | Verbindung 118 |
| 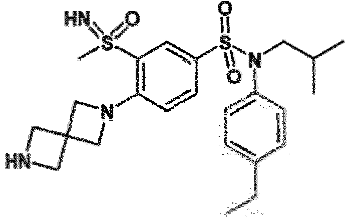 | -(4-Ethylphenyl)-N-isobutyl-3-(S-methylsulfonimidoyl)-4-(2,6-diazaspiro[3.3]heptan-2-yl)benzolsulfonamid |
| | Verbindung 121 |
| 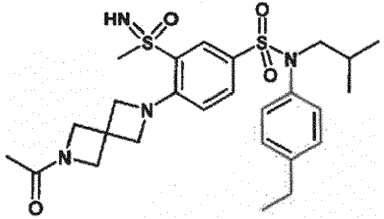 | 4-(6-Acetyl-2,6-diazaspiro[3.3]heptan-2-yl)-N-(4-ethylphenyl)-N-isobutyl-3-(S-methylsulfonimidoyl)benzolsulfonamid |
| | Verbindung 122 |
| 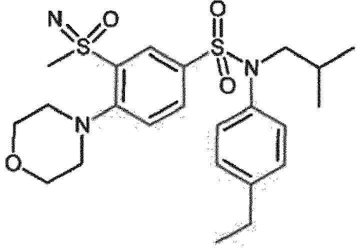 | N-(4-Ethylphenyl)-N-isobutyl-3-(S-methylsulfonimidoyl)-4-morpholinobenzolsulfonamid |
| | Verbindung 123 |
| 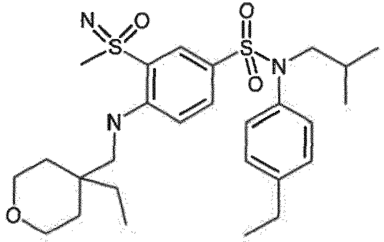 | N-(4-Ethylphenyl)-4-(((4-ethyltetrahydro-2H-pyran-4-yl)methyl)-amino)-N-isobutyl-3-(S-methylsulfonimidoyl)-benzolsulfonamid |
| | Verbindung 124 |
| 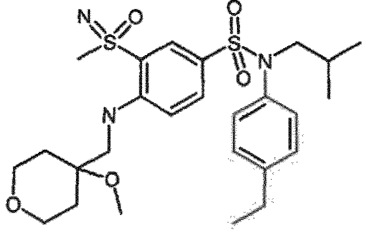 | N-(4-Ethylphenyl)-N-isobutyl-4-(((4-methoxytetrahydro-2H-pyran-4-yl)methyl)-amino)-3-(S-methylsulfonimidoyl)benzolsulfonamid |
| | Verbindung 125 |
| 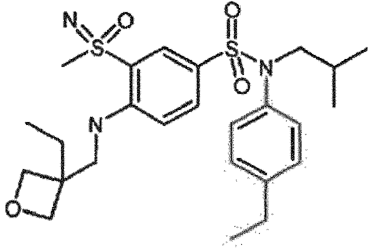 | 4-(((3-Ethyloxetan-3-yl)methyl)amino)-N-(4-ethylphenyl)-N-isobutyl-3-(S-methylsulfonimidoyl)benzolsulfonamid |
| | Verbindung 126 |
| 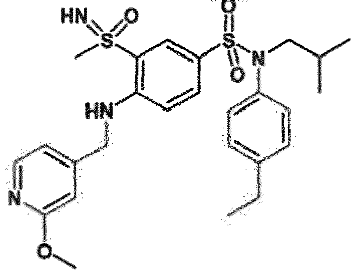 | N-(4-Ethylphenyl)-N-isobutyl-4-(((2-methoxypyridin-4-yl)methyl)amino)-3-(S-methylsulfonimidoyl)-benzolsulfonamid |
| | Verbindung 127 |
| 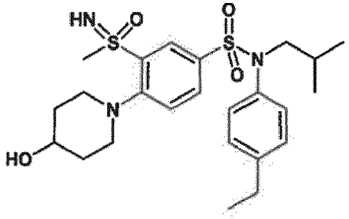 | N-(4-Ethylphenyl)-4-(4-hydroxypiperidin-1-yl)-N-isobutyl-3-(S-methylsulfonimidoyl)-benzolsulfonamid |
| | Verbindung 128 |
| 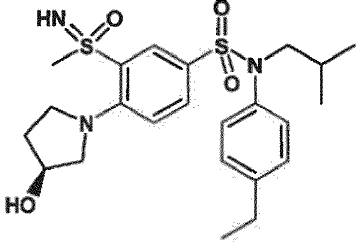 | N-(4-Ethylphenyl)-4-((S)-3-hydroxy-pyrrolidin-1-yl)-N-isobutyl-3-(S-methylsulfonimidoyl)benzolsulfonamid |
| | Verbindung 129 |
| 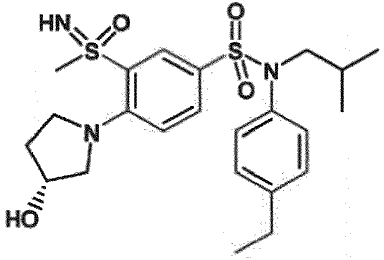 | N-(4-Ethylphenyl)-4-((R)-3-hydroxy-pyrrolidin-1-yl)-N-isobutyl-3-(S-methylsulfonimidoyl)-benzolsulfonamid |
| | Verbindung 130 |
| 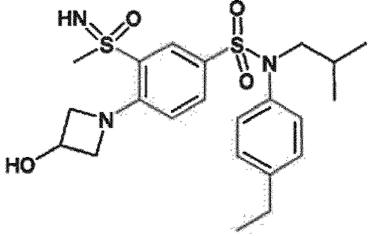 | N-(4-Ethylphenyl)-4-(3-hydroxyazetidin-1-yl)-N-isobutyl-3-(S-methylsulfonimidoyl)-benzolsulfonamid |
| | Verbindung 131 |
| 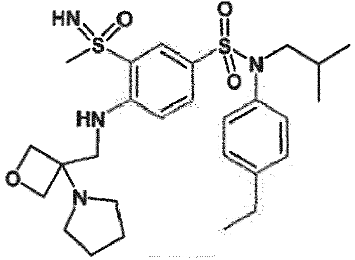 | N-(4-Ethylphenyl)-N-isobutyl-3-(S-methylsulfonimidoyl)-4-(((3-(pyrrolidin-1-yl)oxetan-3-yl)methyl)-amino)benzolsulfonamid |
| | Verbindung 132 |
| 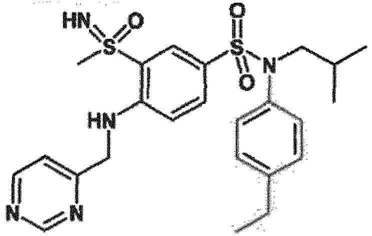 | N-(4-Ethylphenyl)-N-isobutyl-3-(S-methylsulfonimidoyl)-4-((pyrimidin-4-yl-methyl)amino)benzolsulfonamid |
| | Verbindung 133 |
| 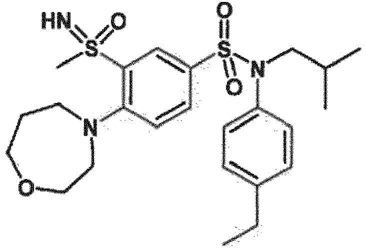 | N-(4-Ethylphenyl)-N-isobutyl-3-(S-methylsulfonimidoyl)-4-(1,4-oxazepan-4-yl)benzolsulfonamid |
| | Verbindung 137 |
| 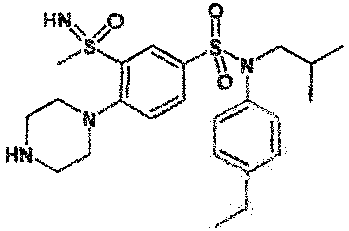 | N-(4-Ethylphenyl)-N-isobutyl-3-(S-methylsulfonimidoyl)-4-(piperazin-1-yl)benzolsulfonamid |
| | Verbindung 140 |
| 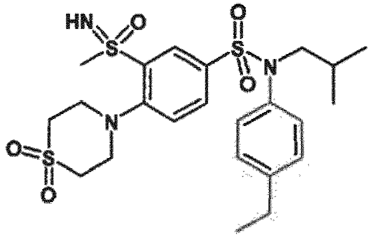 | N-(4-Ethylphenyl)-4-(((3-hydroxycyclo-butyl)methyl)amino)-N-isobutyl-3-(S-methylsulfonimidoyl)benzolsulfonamid |
| | Verbindung 141 |
| 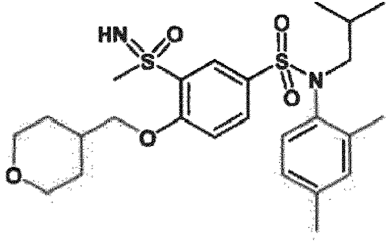 | N-(2,4-Dimethylphenyl)-N-isobutyl-3-(S-methylsulfonimidoyl)-4-((tetrahydro-2H-pyran-4-yl)methoxy)benzolsulfonamid |
| | Verbindung 142 |
| 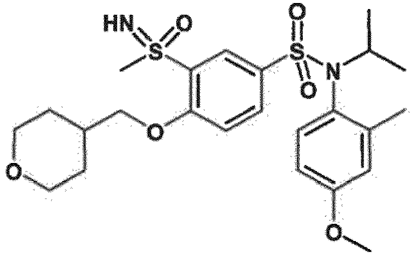 | N-Isopropyl-N-(4-methoxy-2-methylphenyl)-3-(S-methylsulfonimidoyl)-4-((tetrahydro-2H-pyran-4-yl)methoxy)benzolsulfonamid |
| | Verbindung 143 |

5. Verbindung nach einem der vorhergehenden Ansprüche, **dadurch gekennzeichnet, dass** sie aus den folgenden Verbindungen ausgewählt ist:
| | |
|---|---|
| 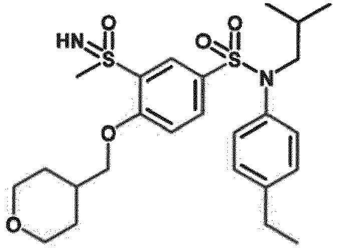 | N-(4-Ethylphenyl)-N-isobutyl-3-methansulfoximino-4-(tetrahydropyran-4-ylmethoxy)-benzol sulfonamid |
| | Verbindung 26 |
| 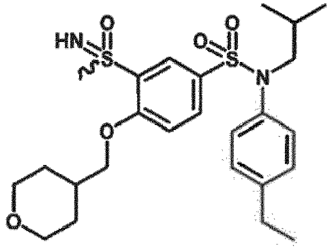 | Enantiomer A von Verbindung 7 |
| Enantiomer A | |
| 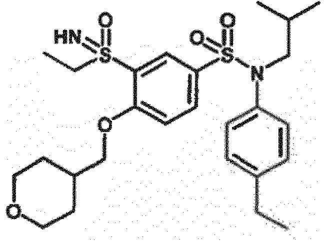 | N-(4-Ethylphenyl)-N-isobutyl-3-ethansulfoximino-4-(tetrahydropyran-4-ylmethoxy)benzolsulfonamid |
| | Verbindung 18 |
| 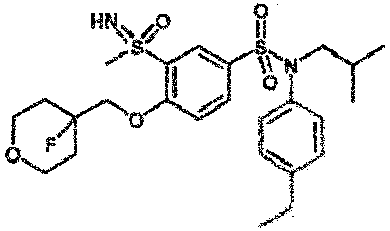 | N-(4-Ethylphenyl)-4-((4-fluortetrahydro-2H-pyran-4-yl)methoxy)-N-isobutyl-3-(S-methylsulfonimidoyl)benzolsulfonamid |
| | Verbindung 30 |
| 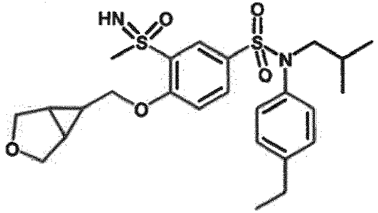 | 4-((3-Oxabicyclo[3.1.0]-hexan-6-yl)methoxy)-N-(4-ethylphenyl)-N-isobutyl-3-(S-methylsulfonimidoyl)benzolsulfonamid |
| | Verbindung 31 |
| 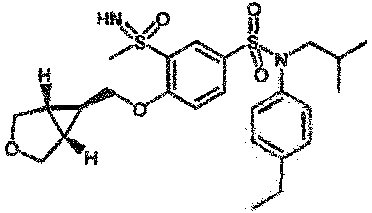 | 4-(((1R,5S,6R)-3-Oxabicyclo[3.1.0]hexan-6-yl)methoxy)-N-(4-ethylphenyl)-N-isobutyl-3-(S-methylsulfonimidoyl)benzolsulfonamid |
| | Verbindung 35 |
| 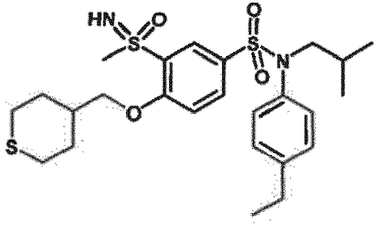 | N-(4-Ethylphenyl)-N-isobutyl-3-(S-methylsulfonimidoyl)-4-(pyridin-4-ylmethoxy)benzolsulfonamid |
| | Verbindung 37 |
| 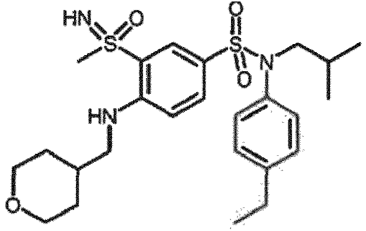 | N-(4-Ethylphenyl)-N-isobutyl-3-(S-methylsulfonimidoyl)-4-[(tetrahydropyran-4-ylmethyl)amino]benzolsulfonamid |
| | Verbindung 52 |
| 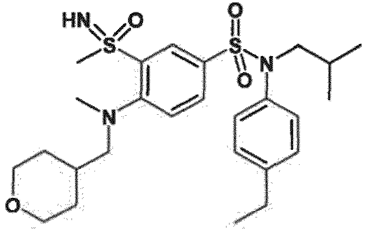 | N-(4-Ethylphenyl)-N-isobutyl-4-(methyl-((tetrahydro-2H-pyran-4-yl)methyl)amino)-3-(S-methylsulfonimidoyl)-benzolsulfonamid |
| | Verbindung 53 |
| 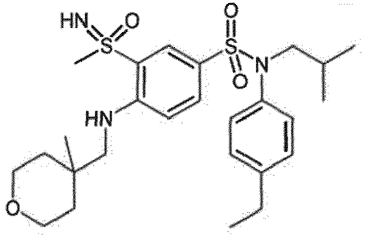 | N-(4-Ethylphenyl)-N-isobutyl-3-(S-methylsulfonimidoyl)-4-(((4-methyltetrahydro-2H-pyran-4-yl)methyl)amino)-benzolsulfonamid |
| | Verbindung 55 |
| 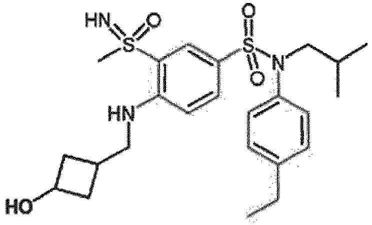 | N-(4-Ethylphenyl)-4-(((3-hydroxycyclobutyl) methyl) amino)-N-isobutyl-3-(S-methylsulfonimidoyl)-benzolsulfonamid |
| | Verbindung 61 |
| 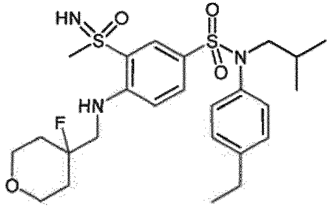 | N-(4-Ethylphenyl)-4-(((4-fluortetrahydro-2H-pyran-4-yl)methyl)amino)-N-isobutyl-3-(S-methylsulfonimidoyl)-benzolsulfonamid |
| | Verbindung 62 |
| 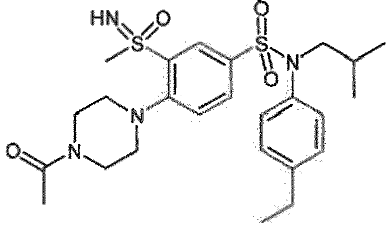 | 4-(4-Acetylpiperazin-1-yl)-N-(4-ethylphenyl)-N-isobutyl-3-(S-methylsulfonimidoyl)-benzol sulfonamid |
| | Verbindung 63 |
| 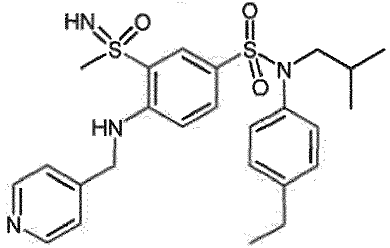 | N-(4-Ethylphenyl)-N-isobutyl-3-(S-methylsulfonimidoyl)-4-((pyridin-4-ylmethyl)-amino)benzolsulfonamid |
| | Verbindung 64 |

6. Verbindung nach einem der vorhergehenden Ansprüche, **dadurch gekennzeichnet, dass** sie aus den folgenden Verbindungen ausgewählt ist:
| | |
|---|---|
| 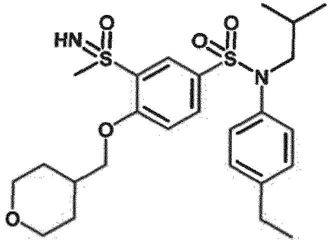 | N-(4-Ethylphenyl)-N-isobutyl-3-methansulfoximino-4-(tetrahydropyran-4-ylmethoxy)-benzolsulfonamid |
| | Verbindung 26 |
| 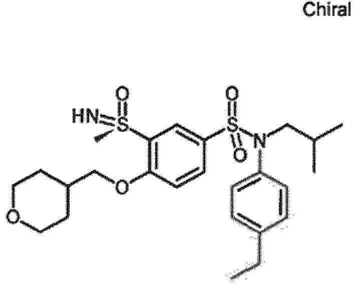 | N-(4-Ethylphenyl)-N-isobutyl-3-methansulfoximino-4-(tetrahydropyran-4-ylmethoxy)benzolsulfonamid |
| | Verbindung 7 (Enantiomer A) |
| 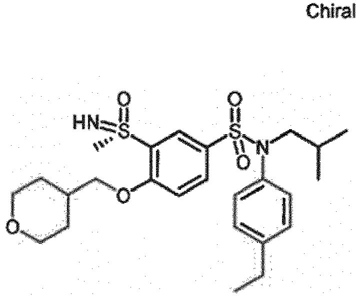 | N-(4-Ethylphenyl)-N-isobutyl-3-methansulfoximino-4-(tetrahydropyran-4-ylmethoxy)benzolsulfonamid |
| | Verbindung 8 (Enantiomer B) |
| 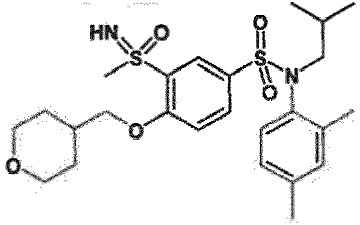 | N-(2,4-Dimethylphenyl)-N-isobutyl-3-(S-methylsulfonimidoyl)-4-((tetrahydro-2H-pyran-4-yl)methoxy)benzolsulfonamid |
| | Verbindung 142 |

7. Verbindung nach einem der vorhergehenden Ansprüche zur Verwendung als Medikament.

8. Verbindung nach einem der Ansprüche 1 bis 6 bei der Behandlung von entzündlichen Störungen und/oder Autoimmunerkrankungen, die durch den RORγt-Rezeptor vermittelt werden.

9. Verbindung zur Verwendung nach Anspruch 8 bei der Behandlung von Akne.

10. Verbindung zur Verwendung nach Anspruch 8 bei der Behandlung von Psoriasis.

11. Pharmazeutische Zusammensetzung, umfassend eine oder mehrere Verbindungen gemäß einem der Ansprüche 1 bis 6.

12. Pharmazeutische Zusammensetzung nach Anspruch 11 zur Verwendung bei der Behandlung von entzündlichen Störungen und/oder Autoimmunerkrankungen, die durch den RORγt-Rezeptor vermittelt werden, insbesondere Akne, atopischer Dermatitis und/oder Psoriasis.

## Claims

1. Compound of Formula (II) and also pharmaceutically acceptable addition salts thereof, hydrates thereof and/or solvates thereof 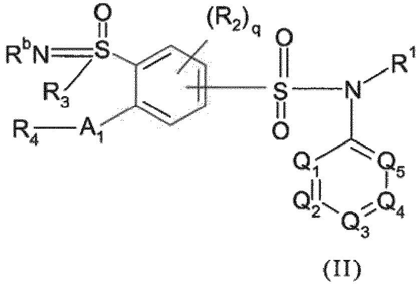 in which Formula (II):
• q denotes zero or a natural integer ranging from 1 to 3,
• R¹ represents a linear or branched C₃-C₅ alkyl radical, a C₃-C₅ cycloalkyl radical, a linear or branched C₂-C₅ alkenyl radical, a (C₁)alkyl (C₃-C₅) cycloalkyl radical, a C₄-C₅ heterocycloalkyl radical, or a (C₁) alkyl (C₄-C₅) heterocycloalkyl radical,
• R₂ represents a hydrogen atom or a halogen atom, a linear or branched C₁-C₅ alkyl radical, a linear or branched C₂-C₄ alkenyl radical, a C₁-C₄ alkoxy radical, or a cyano group -CN; it being possible for the alkyl, alkenyl and alkoxy radicals to be substituted with one or more halogen atoms,
• R³ represents a C₁-C₃ alkyl radical,
• R⁴ represents a hydrogen atom or a (CHR⁵)ₙ-(Z)ₒ-(CHR'⁵)ₚ-R⁶ group,
• n, o and p, which may be identical or different, denote zero or a natural integer ranging from 1 to 3,
• Z represents a divalent group chosen from -CH₂-, -NH- and -O-,
• R⁵ and R'⁵, which may be identical or different, represent a hydrogen atom, a methyl radical -CH₃, a hydroxyl radical -OH, a C₁ hydroxyalkyl radical or a carboxylic radical -COOH,
• R⁶ represents:
- a hydrogen atom or a halogen atom,
- a heterocycloalkyl radical optionally substituted with one or more halogen atoms, one or more linear or branched C₁-C₃ alkyl groups, one or more -OH groups, one or more carbonyl functions =O, one or more linear or branched C₁-C₄ hydroxyalkyl groups, a pyrrolidine ring, one or more amino groups, one or more -C(=O)R⁷ groups, or one or more S(=O)₂R⁷ groups; R⁷ representing a linear or branched C₁-C₃ alkyl radical, a hydroxyl radical -OH, a linear or branched C₁-C₄ alkoxy radical, or an amino radical N(R^{7a})(R^{7b}); with R^{7a} and R^{7b}, which may be identical or different, denoting a hydrogen atom, a linear or branched C₁-C₃ alkyl radical or a cyclopropyl radical,
- a C₃-C₆ cycloalkyl radical optionally substituted with one or more -OH groups, or
- an aromatic or heteroaromatic radical optionally substituted with one or more halogen atoms, one or more linear or branched C₁-C₃ alkyl groups optionally substituted with one or more halogen atoms, one or more C₁-C₃ alkoxy groups, one or more amino groups -NR¹¹R¹², one or more -COR¹¹ groups, a carbonyl function (=O), one or more -OR¹¹ groups, one or more C₁-C₄ hydroxyalkyl groups, one or more -COOR¹¹ groups, one or more amido groups -CONR¹¹R¹², one or more -SOR¹¹ groups, one or more -SO₂R¹¹ groups, one or more -NHCOR¹¹ groups, one or more -NHCOOR¹¹ groups, one or more -SO₂NR¹¹R¹² groups or one or more -CN groups; R¹¹ and R¹², which may be identical or different, representing a hydrogen atom or a linear or branched C₁-C₃ alkyl radical optionally substituted with one or more halogen atoms;
• A₁ represents a divalent group chosen from -NR^{a}-, -O-, -S-, -SO-, -SO₂-, -SO(=NH)-, -CH₂-, -C=C- and -CH(R^{a})-;
• R^{a} represents a hydrogen atom, a linear or branched C₁-C₃ alkyl radical or an acetyl radical -C(=O)CH₃,
• R^{b} represents a hydrogen atom, a linear or branched C₁-C₃ alkyl radical or a cyclopropyl group,
• Q₁, Q₂, Q₃, Q₄ and Q₅, which may be identical or different, represent a nitrogen atom or a -CR'₂ group,
• R'₂ represents a hydrogen atom or a linear or branched C₁-C₅ alkyl radical,
• R^{a} and R³ can form, together with the carbon atoms to which they are attached, a heterocycloalkyl group which can be optionally substituted with one or more carbonyl functions, or one or more C₁-C₃ alkyl radicals,
• when A₁ represents -NR^{a}-, then R^{a} and R⁴ can form, together with the nitrogen atom to which they are attached, a C₂-C₁₀ heterocycloalkyl group optionally comprising one to three heteroatoms chosen from a sulfur atom, a nitrogen atom and an oxygen atom; said heterocycloalkyl group being optionally substituted with at least one R¹⁴ radical,
• R¹⁴ represents a linear or branched C₁-C₃ alkyl radical, a linear or branched C₁-C₃ alkoxy radical, a halogen atom, a hydroxyl group -OH, a cyano group -CN, a -CONR¹⁵R¹⁶ group, a -SO₂R¹⁵ group, a -COR¹⁵ group or an amino group -NR¹⁵R¹⁶; R¹⁵ and R¹⁶, which may be identical or different, represent a hydrogen atom or a linear or branched C₁-C₃ alkyl radical.

2. Compound of Formula (II) according to Claim 1, **characterized in that** R⁶ represents a heterocyclic radical chosen from the following heterocycles: 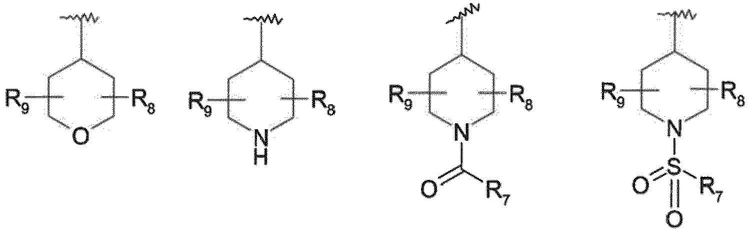 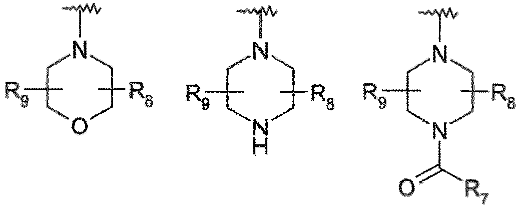 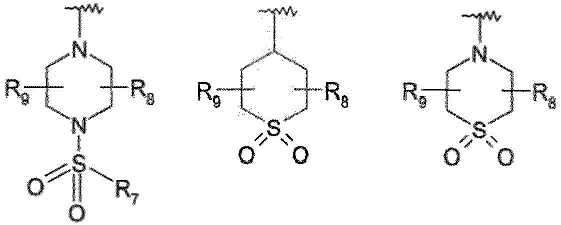 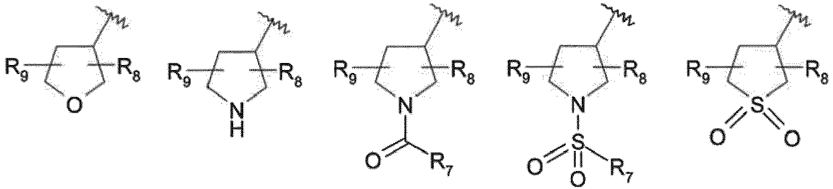 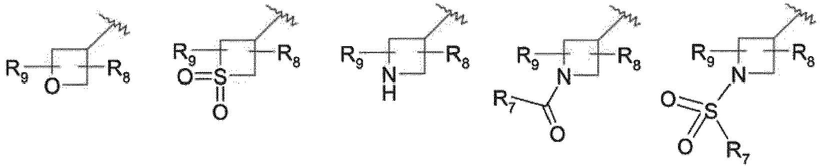 in which:
- R₇ represents a linear or branched C₁-C₃ alkyl radical, a hydroxyl radical -OH, a C₁-C₃ alkoxy radical or an amino radical N(R^{7a})(R^{7b}),
- R^{7a} and R^{7b}, which may be identical or different, denote a hydrogen atom, a linear or branched C₁-C₃ alkyl radical or a cyclopropyl radical,
- R₈ and R₉, which may be identical or different, represent a hydrogen atom, a linear or branched C₁-C₃ alkyl radical, a hydroxyl group -OH, a carbonyl group, a C₁ hydroxyalkyl radical (CH₂OH) or an amino group -NH₂,
- R₈ and R₉ can form, together with the carbon atoms to which they are attached, a carbocyclic ring comprising from 5 to 7 ring members.

3. Compound of Formula (II) according to Claim 1, **characterized in that** R⁶ represents an aromatic or heteroaromatic radical chosen from: 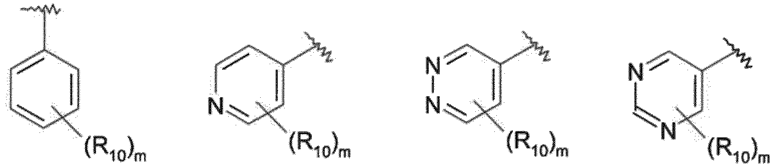 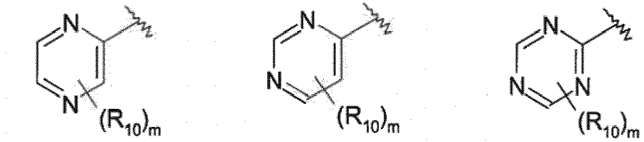 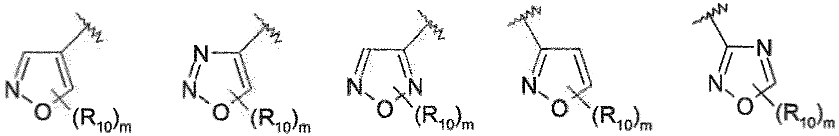 in which:
- R₁₀ represents a hydrogen atom or a halogen atom; a linear or branched C₁-C₃ alkyl radical optionally substituted with one or more halogen atoms; a carbonyl function (=O), an OR¹¹ group, a C₁-C₄ hydroxyalkyl group, an amino group -NR¹¹R¹², a -COR¹¹ group, a -COOR¹¹ group, an amido group -CONR¹¹R¹², an -SOR¹¹ group, an -SO₂R¹¹ group, an -NHCOR¹¹ group, an -NHCOOR¹¹ group, an -SO₂NR¹¹R¹² group or a cyano group -CN,
- R¹¹ and R¹², which may be identical or different, represent a hydrogen atom or a linear or branched C₁-C₃ alkyl radical optionally substituted with one or more halogen atoms,
- m denotes zero or a natural integer ranging from 1 to 3.

4. Compound according to any one of the preceding claims, **characterized in that** it is chosen from the following compounds and also pharmaceutically acceptable addition salts thereof, hydrates thereof and/or solvates thereof:
| | |
|---|---|
| 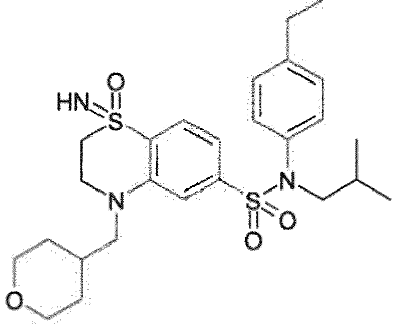 | Imino-1-oxo-4-(tetrahydropyran-4-ylmethyl)-1,2,3,4-tetrahydro-λ⁶-benzo[1,4]thiazine-7-sulfonic acid (4-ethylphenyl)isobutylamide |
| | Compound 1 |
| 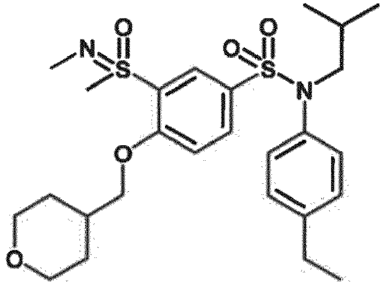 | N-(4-Ethylphenyl)-N-isobutyl-3-methanesulfinyl-4-(tetrahydropyran-4-ylmethoxy)benzene-N-methylsulfoximine |
| | Compound 2 |
| 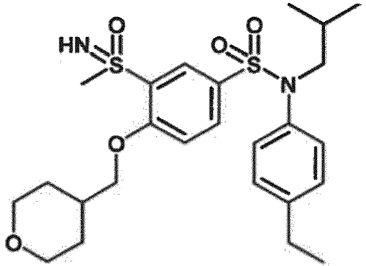 | N-(4-Ethylphenl)-N-isobutyl-3-methanesulfoximine-4-(tetrahydropyran-4-ylmethoxy)benzenesulfonamide |
| | Compound 26 |
| 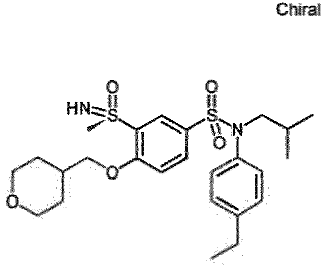 | N-(4-Ethylphenyl)-N-isobutyl-3-methanesulfoximine-4-(tetrahydropyran-4-ylmethoxy)benzenesulfonamide |
| | Compound 7 (enantiomer A) |
| 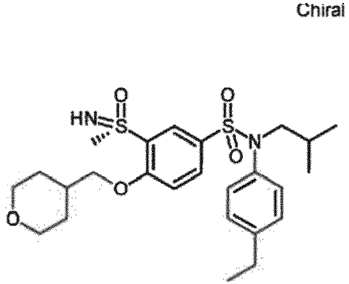 | N-(4-Ethylphenyl)-N-isobutyl-3-methanesulfoximine-4-(tetrahydropyran-4-ylmethoxy)benzenesulfonamide |
| | Compound 8 (enantiomer B) |
| 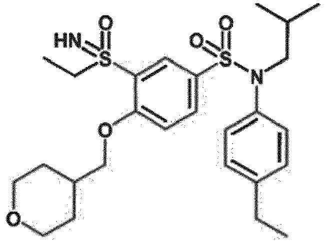 | N-(4-Ethylphenyl)-N-isobutyl-3-ethanesulfoximine-4-(tetrahydropyran-4-ylmethoxy)benzenesulfonamide |
| | Compound 18 |
| 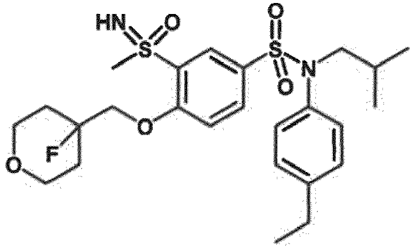 | N-(4-Ethylphenyl)-4-((4-fluorotetrahydro-2H-pyran-4-yl)methoxy)-N-isobutyl-3-(S-methylsulfonimidoyl) benzenesulfonamide |
| | Compound 30 |
| 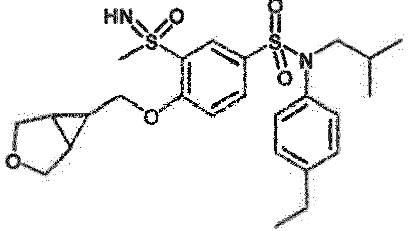 | 4-((3-Oxabicyclo-[3.1.0]-hexan-6-yl)methoxy)-N-(4-ethylphenyl)-N-isobutyl-3-(S-methylsulfonimidoyl)-benzenesulfonamide |
| | Compound 31 |
| 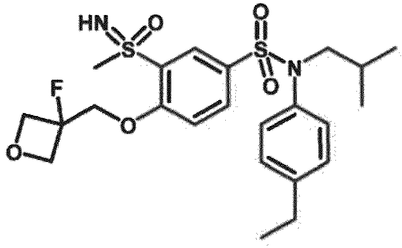 | N-(4-Ethylphenyl)-4-((3-fluorooxetan-3-yl)methoxy)-N-isobutyl-3-(S-methylsulfonimidoyl)-benzenesulfonamide |
| | Compound 32 |
| 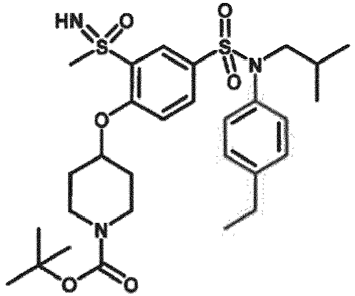 | Tert-butyl 4-(4-(N-(4-ethylphenyl)-N-isobutylsulfamoyl)-2-(S-methylsulfonimidoyl)-phenoxy)piperidine-1-carboxylate |
| | Compound 33 |
| 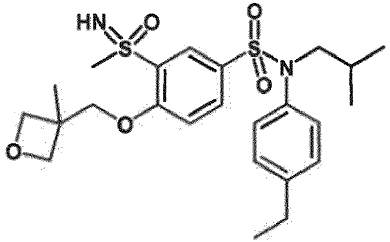 | N-(4-Ethylphenyl)-N-isobutyl-4-((3-methyloxetan-3-yl)methoxy)-3-(S-methylsulfonimidoyl)-benzenesulfonamide |
| | Compound 34 |
| 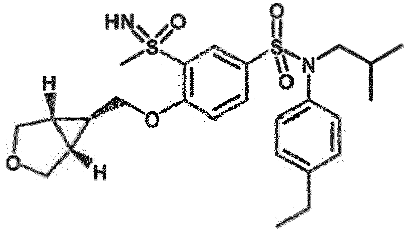 | 4-(((1R,5S,6r)-3-Oxabicyclo[3.1.0]hexan-6-yl)methoxy)-N-(4-ethylphenyl)-N-isobutyl-3-(S-methylsulfonimidoyl)benzenesulfonamide |
| | Compound 35 |
| 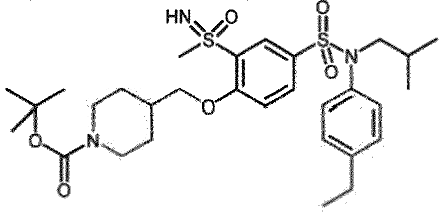 | Tert-butyl 4-((4-(N-(4-ethylphenyl)-N-isobutylsulfamoyl)-2-(S-methylsulfonimidoyl)-phenoxy) methyl)piperidine-1-carboxylate |
| | Compound 36 |
| 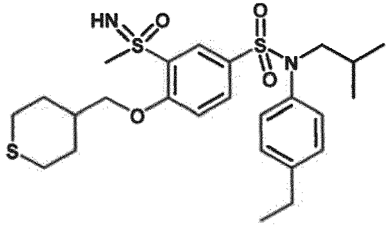 | N-(4-Ethylphenyl)-N-isobutyl-3-(S-methylsulfonimidoyl)-4-(pyridin-4-ylmethoxy)benzenesulfonamide |
| | Compound 37 |
| 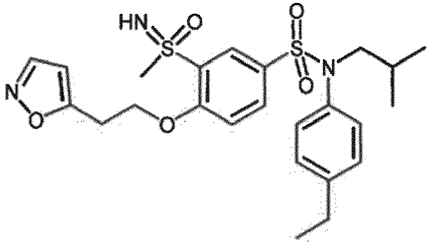 | N-(4-Ethylphenyl)-N-isobutyl-4-(2-(isoxazol-5-yl)ethoxy)-3-(S-methylsulfonimidoyl)-benzenesulfonamide |
| | Compound 38 |
| 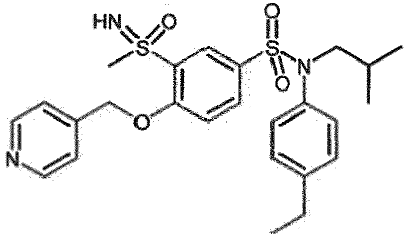 | N-(4-Ethylphenyl)-N-isobutyl-3-(S-methylsulfonimidoyl)-4-(pyridin-4-ylmethoxy)benzenesulfonamide |
| | Compound 39 |
| 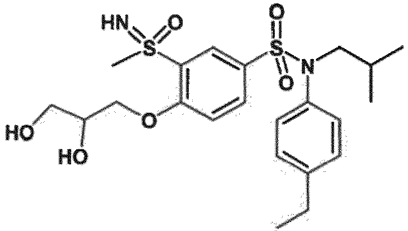 | 4-(2,3-Dihydroxypropoxy)-N-(4-ethylphenyl)-N-isobutyl-3-(S-methylsulfonimidoyl)benzenesulfonamide |
| | Compound 40 |
| 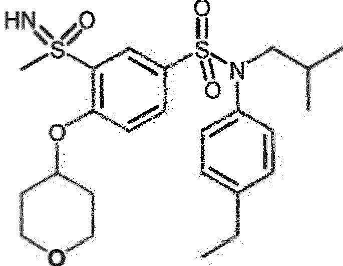 | N-(4-Ethylphenyl)-N-isobutyl-3-(S-methylsulfonimidoyl)-4-((tetrahydro-2H-pyran-4-yl)oxy)benzenesulfonamide |
| | Compound 42 |
| 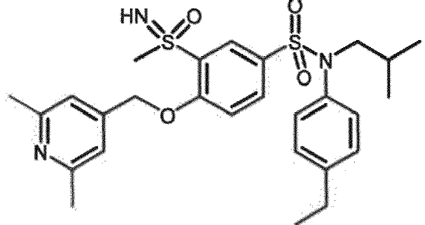 | 4-((2,6-Dimethylpyridin-4-yl)methoxy)-N-(4-ethylphenyl)-N-isobutyl-3-(S-methylsulfonimidoyl)benzenesulfonamide |
| | Compound 43 |
| 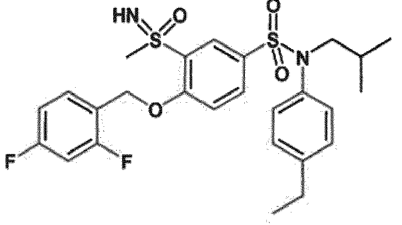 | 4-((2,4-Difluorobenzyl)oxy)-N-(4-ethylphenyl)-N-isobutyl-3-(S-methylsulfonimidoyl)-benzenesulfonamid |
| | Compound 45 |
| 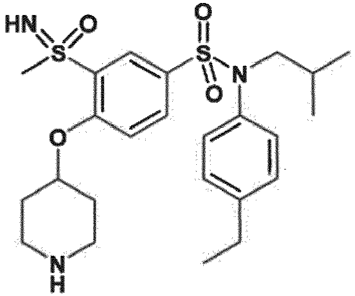 | N-(4-Ethylphenyl)-N-isobutyl-3-(S-methylsulfonimidoyl)-4-(piperidin-4-ylmethoxy)-benzenesulfonamide N-(4-Ethylphenyl)-N-isobutyl-3-(S-methylsulfonimidoyl)-4-(piperidin-4-ylmethoxy)-benzenesulfonamide |
| | Compound 46 |
| 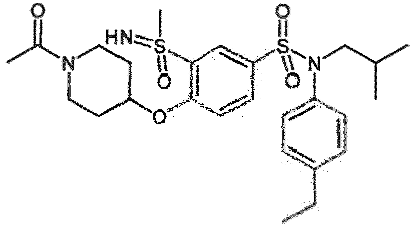 | 4-((1-Acetylpiperidin-4-yl)oxy)-N-(4-ethylphenyl)-N-isobutyl-3-(S-methylsulfonimidoyl)benzenesulfonamide |
| | Compound 47 |
| 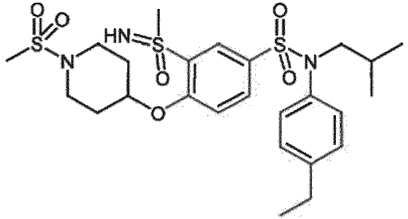 | N-(4-Ethylphenyl)-N-isobutyl-3-(S-methylsulfonimidoyl)-4-((1-(methylsulfonyl)piperidin-4-yl)oxy)benzenesulfonamide |
| | Compound 48 |
| 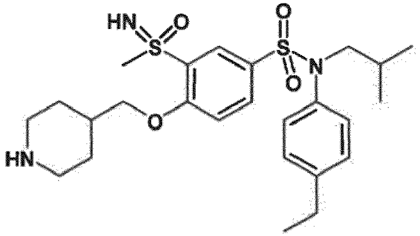 | N-(4-Ethylphenyl)-N-isobutyl-3-(S-methylsulfonimidoyl)-4-(piperidin-4-ylmethoxy)-benzenesulfonamide |
| | Compound 49 |
| 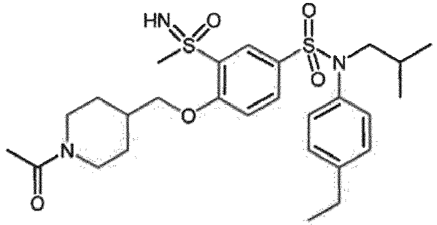 | 4-((1-Acetylpiperidin-4-yl)methoxy)-N-(4-ethylphenyl)-N-isobutyl-3-(S-methylsulfonimidoyl)benzenesulfonamide |
| | Compound 50 |
| 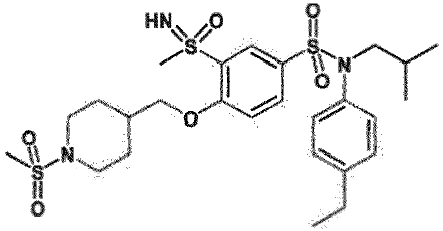 | N-(4-Ethylphenyl)-N-isobutyl-3-(S-methylsulfonimidoyl)-4-((1-(methylsulfonylpiperidin-4-yl)methoxy)benzenesulfonamide |
| | Compound 51 |
| 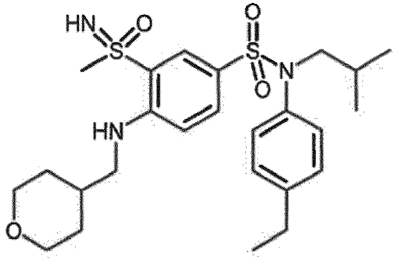 | N-(4-Ethylphenyl)-N-isobutyl-3-(S-methylsulfonimidoyl)-4-[(tetrahydropyran-4-ylmethyl)amino]benzenesulfonamide |
| | Compound 52 |
| 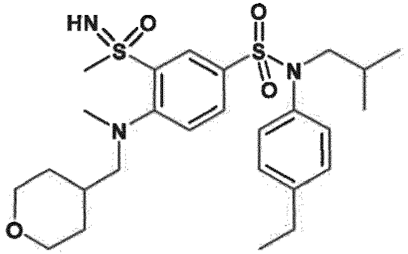 | N-(4-Ethylphenyl)-N-isobutyl-4-(methyl((tetrahydro-2H-pyran-4-yl)methyl)amino)-3-(S-methylsulfonimidoyl)-benzenesulfonamide |
| | Compound 53 |
| 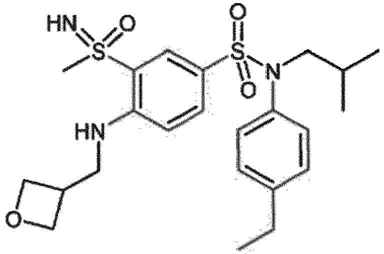 | N-(4-Ethylphenyl)-N-isobutyl-3-(S-methylsulfonimidoyl)-4-((oxetan-3-ylmethyl)-amino)benzenesulfonamide |
| | Compound 54 |
| 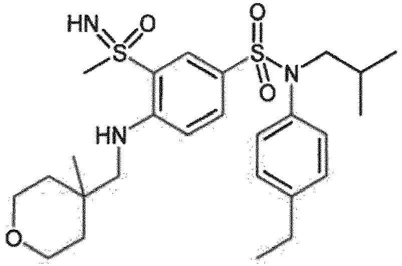 | N-(4-Ethylphenyl)-N-isobutyl-3-(S-methylsulfonimidoyl)-4-(((4-methyltetrahydro-2H-pyran-4-yl)methyl)amino)-benzenesulfonamide |
| | Compound 55 |
| 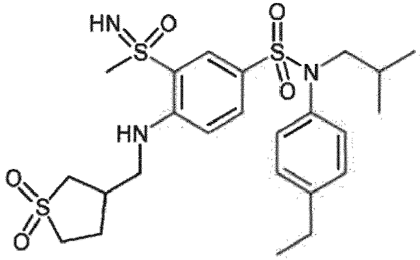 | 4-(((1,1-Dioxidotetra-hydrothiophen-3-yl)methyl)amino)-N-(4-ethylphenyl)-N-isobutyl-3-(S-methylsulfonimidoyl)-benzenesulfonamide |
| | Compound 56 |
| 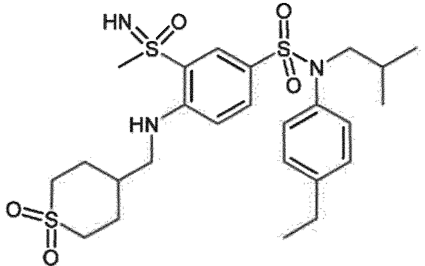 | 4-(((1,1-Dioxidotetrahydro-2H-thiopyran-4-yl)methyl)amino)-N-(4-ethylphenyl)-N-isobutyl-3-(S-methylsulfonimidoyl)-benzenesulfonamide |
| | Compound 57 |
| 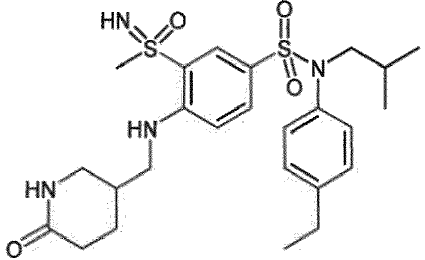 | N-(4-Ethylphenyl)-N-isobutyl-3-(S-methylsulfonimidoyl)-4-(((6-oxopiperidin-3-yl)-methyl)amino)-benzenesulfonamide |
| | Compound 58 |
| 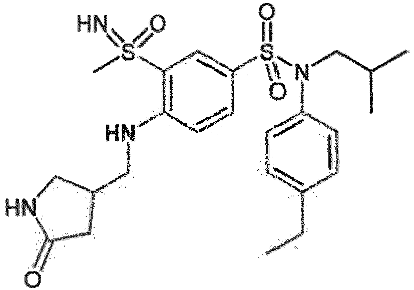 | N-(4-Ethylphenyl)-N-isobutyl-3-(S-methylsulfonimidoyl)-4-(((5-oxopyrrolidin-3-yl)methyl)amino)-benzenesulfonamide |
| | Compound 59 |
| 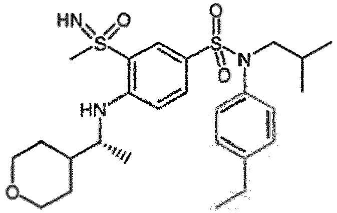 | N-(4-Ethylphenyl)-N-isobutyl-3-(S-methylsulfonimidoyl)-4-(((R)-1-(tetrahydro-2H-pyran-4-yl)ethyl)amino)-benzenesulfonamide |
| | Compound 60 |
| 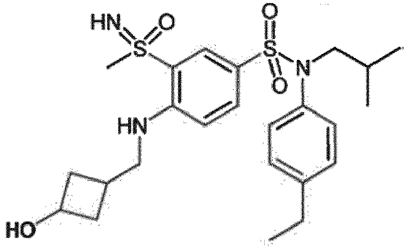 | N-(4-Ethylphenyl)-4-(((3-hydroxycyclobutyl)methyl)-amino)-N-isobutyl-3-(S-methylsulfonimidoyl)-benzenesulfonamide |
| | Compound 61 |
| 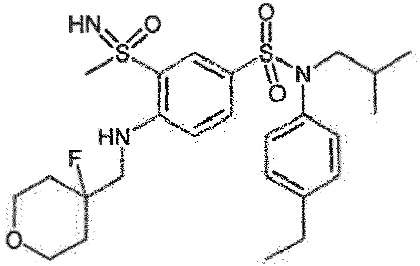 | N-(4-Ethylphenyl)-4-(((4-fluortetrahydro-2H-pyran-4-yl)methyl)amino)-N-isobutyl-3-(S-methylsulfonimidoyl)-benzenesulfonamide |
| | Compound 62 |
| 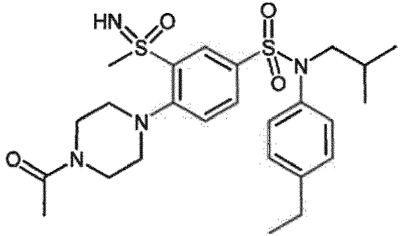 | 4-(4-Acetylpiperazin-1-yl)-N-(4-ethylphenyl)-N-isobutyl-3-(S-methylsulfonimidoyl)-benzenesulfonamide |
| | Compound 63 |
| 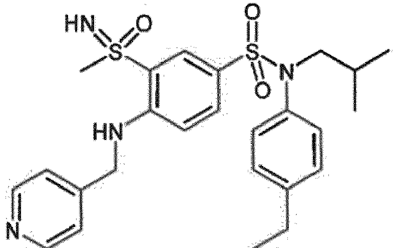 | N-(4-Ethylphenyl)-N-isobutyl-3-(S-methylsulfonimidoyl)-4-((pyridin-4-ylmethyl)-amino)benzenesulfonamide |
| | Compound 64 |
| 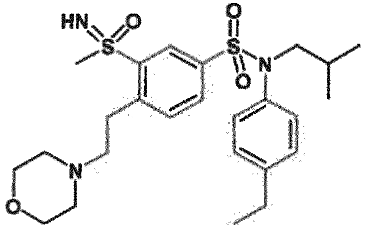 | N-(4-Ethylphenyl)-N-isobutyl-3-(S-methylsulfonimidoyl)-4-(2-morpholinoethyl)-benzenesulfonamide |
| | Compound 65 |
| 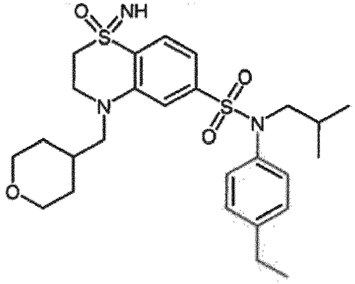 | 1-Imino-1-oxo-4-(tetrahydropyran-4-ylmethyl)-1,2,3,4-tetrahydro-1λ⁶-benzo[1,4]thiazine-6-sulfonic acid (4-ethylphenyl)-isobutylamide |
| | Compound 69 |
| 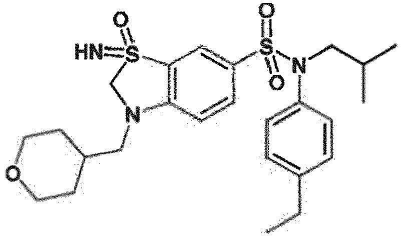 | 1-Imino-1-oxo-3-(tetrahydropyran-4-ylmethyl)-2,3-dihydro-1H-1λ⁶-benzothiazol-6-sulfonic acid (4-ethylphenyl)isobutylamide |
| | Compound 76 |
| 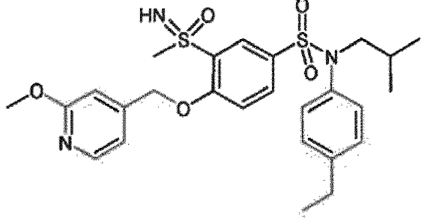 | N-(4-Ethylphenyl)-N-isobutyl-4-((2-methoxypyridin-4-yl)methoxy)-3-(S-methylsulfonimidoyl)-benzenesulfonamide |
| | Compound 77 |
| 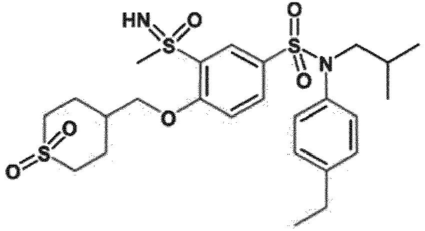 | N-(4-Ethylphenyl)-N-isobutyl-4-((2-methoxypyridin-4-yl)methoxy)-3-(S-methylsulfonimidoyl)-benzenesulfonamide |
| | Compound 78 |
| 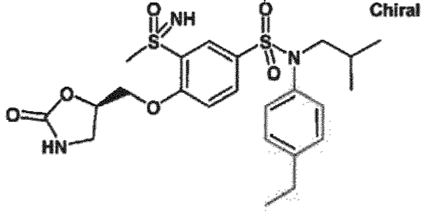 | N-(4-Ethylphenyl)-N-isobutyl-3-(S-methylsulfonimidoyl)-4-(((R)-2-oxooxazolidin-5-yl)methoxy)-benzenesulfonamide |
| | Compound 79 |
| 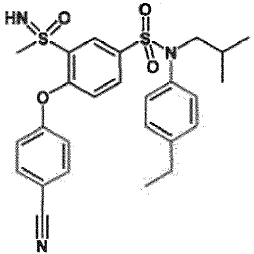 | 4-(4-Cyanophenoxy)-N-(4-ethylphenyl)-N-isobutyl-3-(S-methylsulfonimidoyl)-benzenesulfonamide |
| | Compound 80 |
| 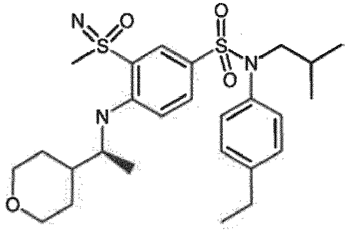 | N-(4-Ethylphenyl)-N-isobutyl-3-(S-methylsulfonimidoyl)-4-(((S)-1-(tetrahydro-2H-pyran-4-yl)ethyl)amino)-benzenesulfonamide |
| | Compound 81 |
| 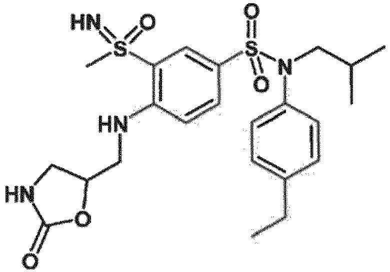 | N-(4-Ethylphenyl)-N-isobutyl-3-(S-methylsulfonimidoyl)-4-(((2-oxooxazolidin-5-yl)methyl)amino)-benzenesulfonamide |
| | Compound 82 |
| 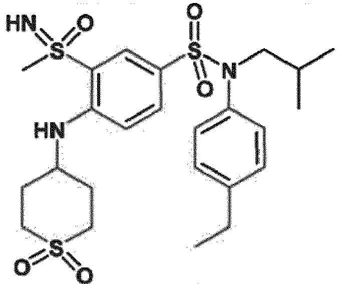 | 4-((1,1-Dioxidotetrahydro-2H-thiopyran-4-yl)amino)-N-(4-ethylphenyl)-N-isobutyl-3-(S-methylsulfonimidoyl)-benzenesulfonamide |
| | Compound 83 |
| 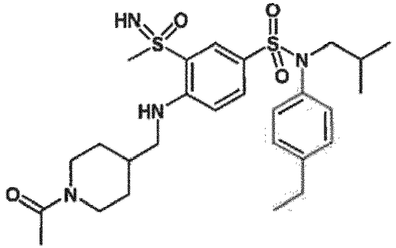 | 4-(((1-Acetylpiperidin-4-yl)methyl)amino)-N-(4-ethylphenyl)-N-isobutyl-3-(S-methylsulfonimidoyl)-benzenesulfonamide |
| | Compound 84 |
| 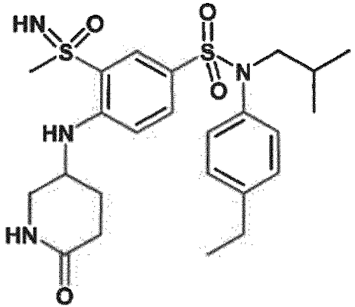 | N-(4-Ethylphenyl)-N-isobutyl-3-(S-methylsulfonimidoyl)-4-((6-oxopiperidin-3-yl)amino)benzenesulfonamide |
| | Compound 85 |
| 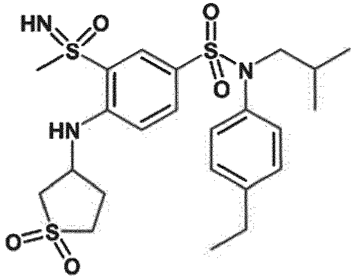 | 4-((1,1-Dioxido-tetrahydrothiophen-3-yl)amino)-N-(4-ethylphenyl)-N-isobutyl-3-(S-methylsulfonimidoyl)-benzenesulfonamide |
| | Compound 86 |
| 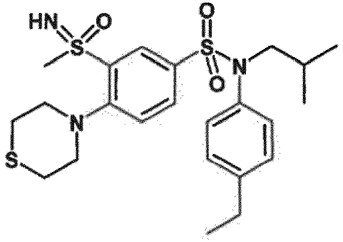 | N-(4-Ethylphenyl)-N-isobutyl-3-(S-methylsulfonimidoyl)-4-thiomorpholinobenzene-sulfonamide |
| | Compound 87 |
| 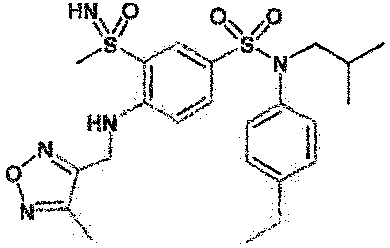 | N-(4-Ethylphenyl)-N-isobutyl-4-(((4-methyl-1,2,5-oxadiazol-3-yl)methyl)amino)-3-(S-methylsulfonimidoyl)-benzenesulfonamide |
| | Compound 88 |
| 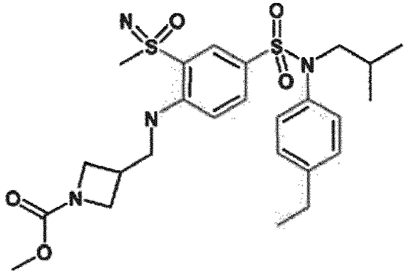 | 3-(((4-(N-(4-Ethylphenyl)-N-isobutylsulfamoyl)-2-(S-methylsulfonimidoyl)phenyl)-amino)methyl)azetidine-1-methyl carboxylate |
| | Compound 89 |
| 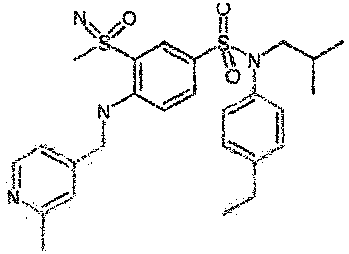 | N-(4-Ethylphenyl)-N-isobutyl-4-(((2-methylpyridin-4-yl)methyl)amino)-3-(S-methylsulfonimidoyl)-benzenesulfonamide |
| | Compound 90 |
| 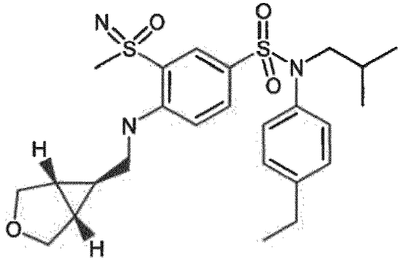 | 4-((((1R,5S,6S)-3-Oxabicyclo[3.1.0]hexan-6-yl)methyl)amino)-N-(4-ethylphenyl)-N-isobutyl-3-(S-methylsulfonimidoyl)-benzenesulfonamide |
| | Compound 91 |
| 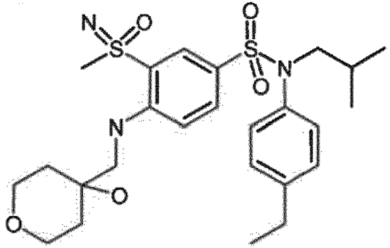 | N-(4-Ethylphenyl)-4-(((4-hydroxytetrahydro-2H-pyran-4-yl)methyl)amino)-N-isobutyl-3-(S-methylsulfonimidoyl)-benzenesulfonamide |
| | Compound 92 |
| 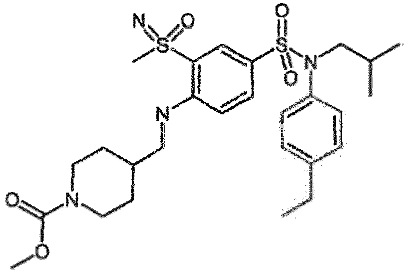 | 4-(((4-(N-(4-Ethylphenyl)-N-isobutylsulfamoyl)-2-(S-methylsulfonimidoyl)-phenyl)amino)methyl)-piperidine-1-methyl carboxylate |
| | Compound 93 |
| 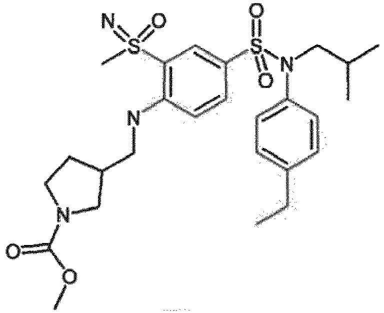 | 3-(((4-(N-(4-Ethylphenyl)-N-isobutylsulfamoyl)-2-(S-methylsulfonimidoyl)phenyl)-amino)methyl)pyrrolidin-1-methyl carboxylate |
| | Compound 94 |
| 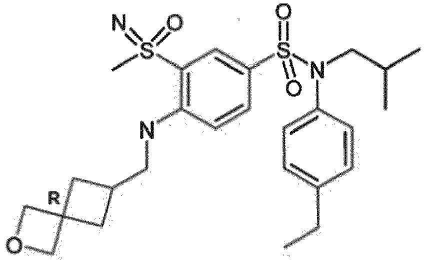 | 4-(((2-Oxaspiro[3.3]heptan-6-yl)methyl)amino)-N-(4-ethylphenyl)-N-isobutyl-3-(S-methylsulfonimidoyl)-benzenesulfonamide |
| | Compound 95 |
| 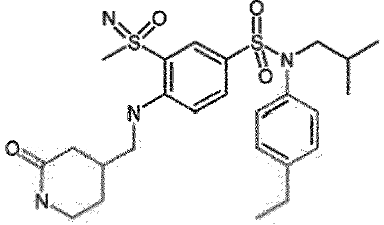 | 4-N-(4-Ethylphenyl)-N-isobutyl-3-(S-methylsulfonimidoyl)-4-(((2-oxopiperidin-4-yl)methyl)amino)-benzenesulfonamide |
| | Compound 96 |
| 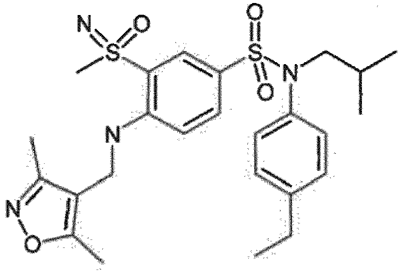 | 4-(((3,5-Dimethylisoxazol-4-yl)methyl)amino)-N-(4-ethylphenyl)-N-isobutyl-3-(S-methylsulfonimidoyl)-benzenesulfonamide |
| | Compound 97 |
| 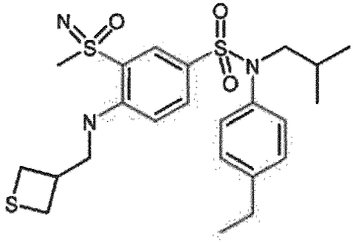 | N-(4-Ethylphenyl)-N-isobutyl-3-(S-methylsulfonimidoyl)-4-((thietan-3-ylmethyl)amino)-benzenesulfonamide |
| | Compound 99 |
| 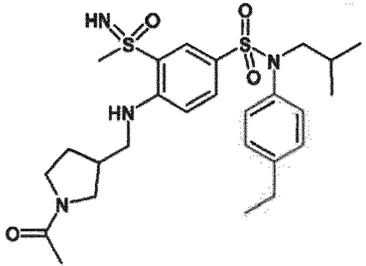 | 4-(((1-Acetylpyrrolidin-3-yl)methyl)amino)-N-(4-ethylphenyl)-N-isobutyl-3-(S-methylsulfonimidoyl)-benzenesulfonamide |
| | Compound 100 |
| 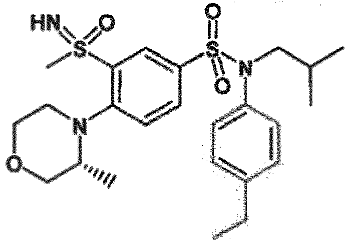 | N-(4-Ethylphenyl)-N-isobutyl-4-((R)-3-methylmorpholino)-3-(S-methylsulfonimidoyl)-benzenesulfonamide |
| | Compound 103 |
| 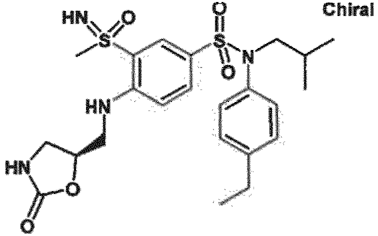 | N-(4-Ethylphenyl)-N-isobutyl-3-(S-methylsulfonimidoyl)-4-((((R)-2-oxooxazolidin-5-yl)methyl)amino)-benzenesulfonamide |
| | Compound 105 |
| 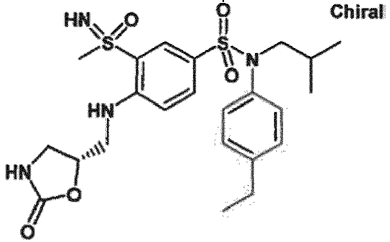 | N-(4-Ethylphenyl)-N-isobutyl-3-(S-methyl-sulfonimidoyl)-4-((((S)-2-oxooxazolidin-5-yl)methyl)amino)-benzenesulfonamide |
| | Compound 106 |
| 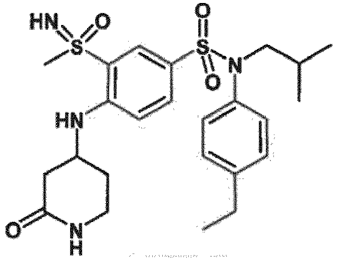 | N-(4-Ethylphenyl)-N-isobutyl-3-(S-methyl-sulfonimidoyl)-4-((2-oxopiperidin-4-yl)amino)-benzenesulfonamide |
| | Compound 107 |
| 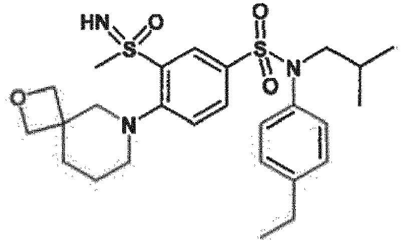 | N-(4-Ethylphenyl)-N-isobutyl-3-(S-methyl-sulfonimidoyl)-4-(2-oxa-6-azaspiro[3.5]nonan-6-yl)benzenesulfonamide |
| | Compound 109 |
| 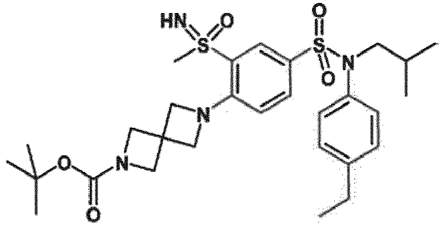 | Tert-butyl 6-(4-(N-(4-Ethylphenyl)-N-isobutyl-sulfamoyl)-2-(S-methylsulfonimidoyl)phenyl)-2,6-diazaspiro[3.3]heptan-2-carboxylate |
| | Compound 110 |
| 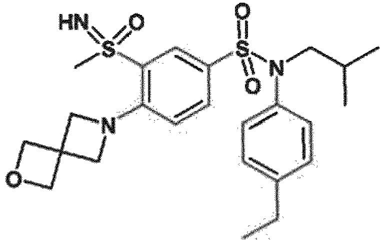 | N-(4-Ethylphenyl)-N-isobutyl-3-(S-methylsulfonimidoyl)-4-(2-oxa-6-azaspiro[3.3]heptan-6-yl)benzenesulfonamide |
| | Compound 111 |
| 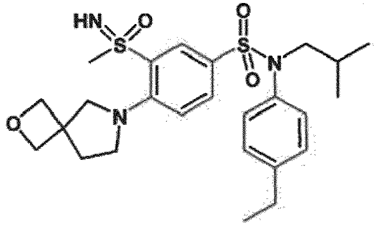 | N-(4-Ethylphenyl)-N-isobutyl-3-(S-methylsulfonimidoyl)-4-(2-oxa-6-azaspiro[3.4]octan-6-yl)benzenesulfonamide |
| | Compound 112 |
| 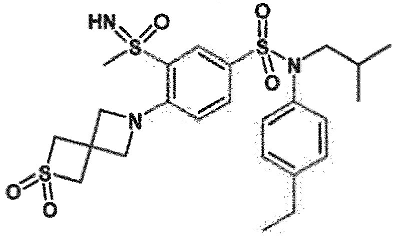 | 4-(2,2-Dioxido-2-thia-6-azaspiro[3.3]heptan-6-yl)-N-(4-ethylphenyl)-N-isobutyl-3-(S-methylsulfonimidoyl)benzenesulfonamide |
| | Compound 113 |
| 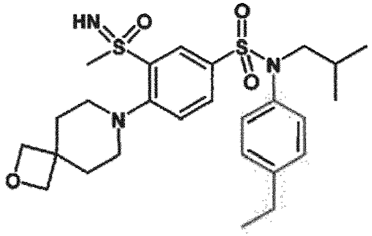 | N-(4-Ethylphenyl)-N-isobutyl-3-(S-methylsulfonimidoyl)-4-(2-oxa-7-azaspiro[3.5]nonan-7-yl)benzenesulfonamide |
| | Compound 114 |
| 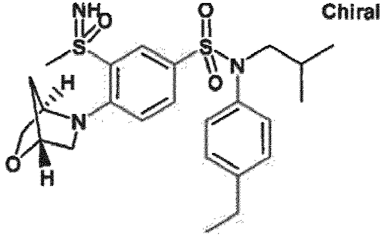 | 4-((1R,4R)-2-Oxa-5-azabicyclo[2.2.1]heptan-5-yl)-N-(4-ethylphenyl)-N-isobutyl-3-(S-methylsulfonimidoyl)-benzenesulfonamide |
| | Compound 115 |
| 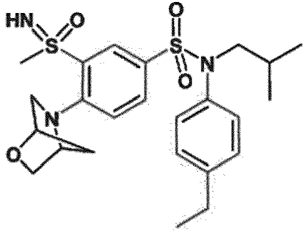 | 4-((1R,4R)-2-Oxa-5-azabicyclo[2.2.1]heptan-5-yl)-N-(4-ethylphenyl)-N-isobutyl-3-(S-methylsulfonimidoyl)benzenesulfonamide |
| | Compound 117 |
| 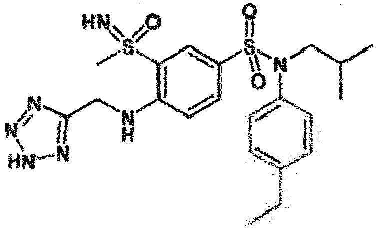 | 4-(((2H-Tetrazol-5-yl)methyl)amino)-N-(4-ethylphenyl)-N-isobutyl-3-(S-methylsulfonimidoyl)-benzenesulfonamide |
| | Compound 118 |
| 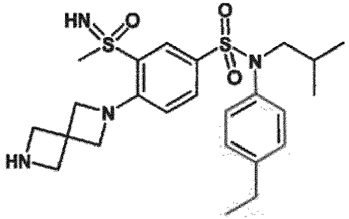 | -(4-Ethylphenyl)-N-isobutyl-3-(S-methylsulfonimidoyl)-4-(2,6-diazaspiro[3.3]heptan-2-yl)benzenesulfonamide |
| | Compound 121 |
| 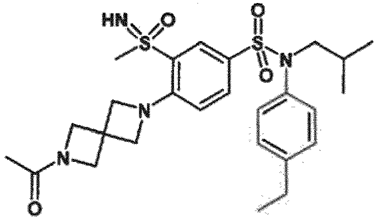 | 4-(6-Acetyl-2,6-diazaspiro[3.3]heptan-2-yl)-N-(4-ethylphenyl)-N-isobutyl-3-(S-methylsulfonimidoyl)benzenesulfonamide |
| | Compound 122 |
| 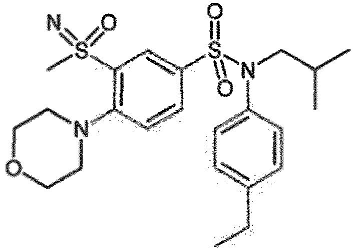 | N-(4-Ethylphenyl)-N-isobutyl-3-(S-methylsulfonimidoyl)-4-morpholinobenzenesulfonamide |
| | Compound 123 |
| 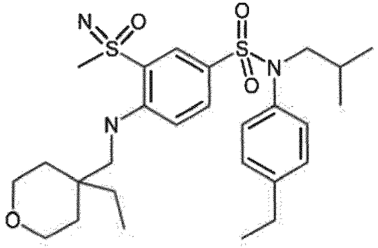 | N-(4-Ethylphenyl)-4-(((4-ethyltetrahydro-2H-pyran-4-yl)methyl)amino)-N-isobutyl-3-(S-methylsulfonimidoyl)-benzenesulfonamide |
| | Compound 124 |
| 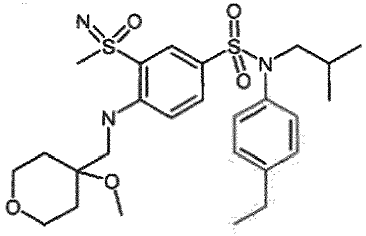 | N-(4-Ethylphenyl)-N-isobutyl-4-(((4-methoxytetrahydro-2H-pyran-4-yl)methyl)amino)-3-(S-methylsulfonimidoyl)-benzenesulfonamide |
| | Compound 125 |
| 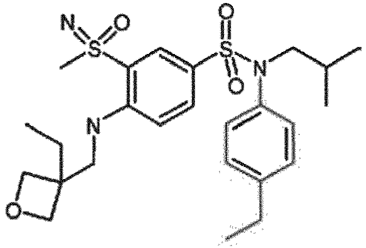 | 4-(((3-Ethyloxetan-3-yl)methyl)amino)-N-(4-ethylphenyl)-N-isobutyl-3-(S-methylsulfonimidoyl)-benzenesulfonamide |
| | Compound 126 |
| 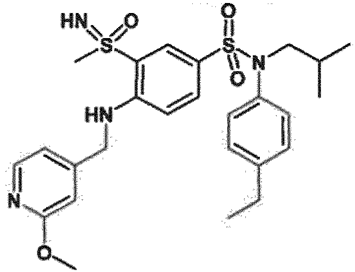 | N-(4-Ethylphenyl)-N-isobutyl-4-(((2-methoxypyridin-4-yl)methyl)amino)-3-(S-methylsulfonimidoyl)-benzenesulfonamide |
| | Compound 127 |
| 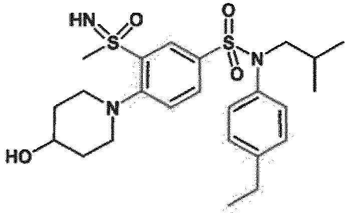 | N-(4-Ethylphenyl)-4-(4-hydroxypiperidin-1-yl)-N-isobutyl-3-(S-methylsulfonimidoyl)-benzenesulfonamide |
| | Compound 128 |
| 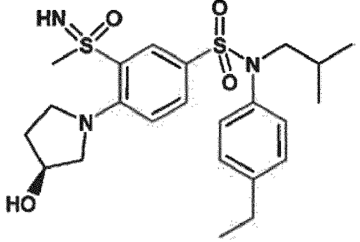 | N-(4-Ethylphenyl)-4-((S)-3-hydroxypyrrolidin-1-yl)-N-isobutyl-3-(S-methylsulfonimidoyl)benzenesulfonamide |
| | Compound 129 |
| 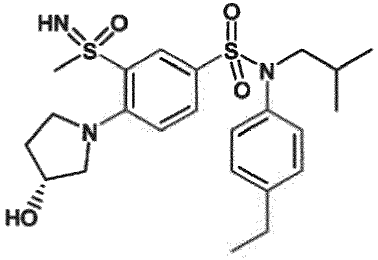 | N-(4-Ethylphenyl)-4-((R)-3-hydroxypyrrolidin-1-yl)-N-isobutyl-3-(S-methylsulfonimidoyl)-benzenesulfonamide |
| | Compound 130 |
| 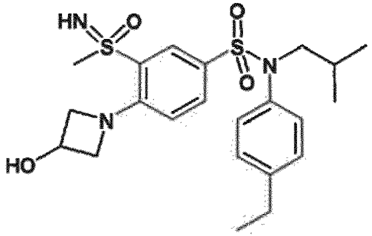 | N-(4-Ethylphenyl)-4-(3-hydroxyazetidin-1-yl)-N-isobutyl-3-(S-methylsulfonimidoyl)-benzenesulfonamide |
| | Compound 131 |
| 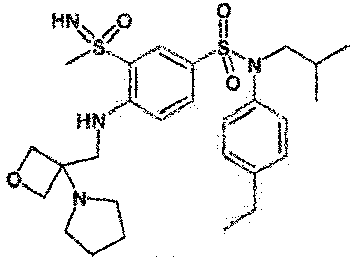 | N-(4-Ethylphenyl)-N-isobutyl-3-(S-methylsulfonimidoyl)-4-(((3-(pyrrolidin-1-yl)oxetan-3-yl)methyl)amino)-benzenesulfonamide |
| | Compound 132 |
| 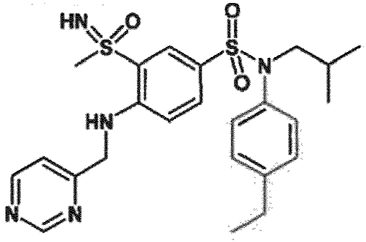 | N-(4-Ethylphenyl)-N-isobutyl-3-(S-methylsulfonimidoyl)-4-((pyrimidin-4-ylmethyl)-amino)benzenesulfonamide |
| | Compound 133 |
| 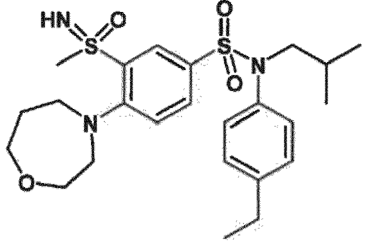 | N-(4-Ethylphenyl)-N-isobutyl-3-(S-methylsulfonimidoyl)-4-(1,4-oxazepan-4-yl)benzenesulfonamide |
| | Compound 137 |
| 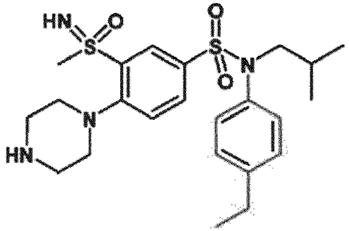 | N-(4-Ethylphenyl)-N-isobutyl-3-(S-methylsulfonimidoyl)-4-(piperazin-1-yl)benzenesulfonamide |
| | Compound 140 |
| 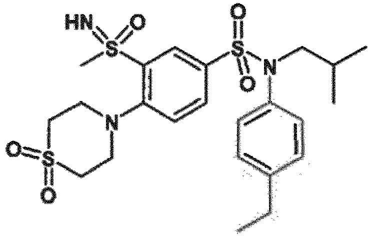 | N-(4-Ethylphenyl)-4-(((3-hydroxycyclobutyl)-methyl)amino)-N-isobutyl-3-(S-methylsulfonimidoyl)-benzenesulfonamide |
| | Compound 141 |
| 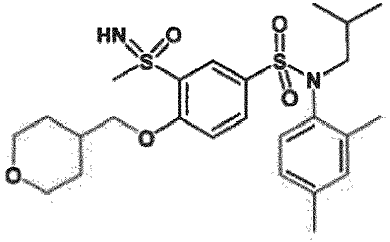 | N-(2,4-Dimethylphenyl)-N-isobutyl-3-(S-methylsulfonimidoyl)-4-((tetrahydro-2H-pyran-4-yl)methoxy)benzenesulfonamide |
| | Compound 142 |
| 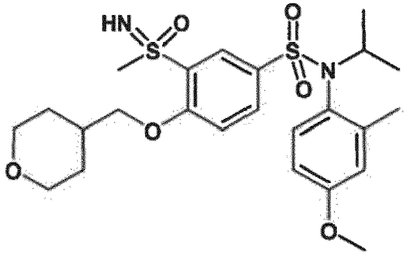 | N-Isopropyl-N-(4-methoxy-2-methylphenyl)-3-(S-methylsulfonimidoyl)-4-((tetrahydro-2H-pyran-4-yl)methoxy)benzenesulfonamide |
| | Compound 143 |

5. Compound according to any one of the preceding claims, **characterized in that** it is chosen from the following compounds:
| | |
|---|---|
| 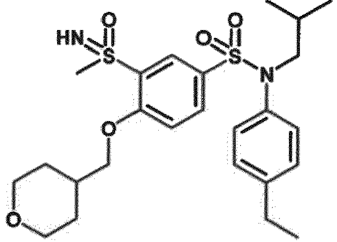 | N-(4-Ethylphenyl)-N-isobutyl-3-methansulfoximino-4-(tetrahydropyran-4-ylmethoxy)-benzenesulfonamide |
| | Compound 26 |
| 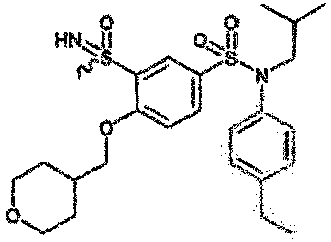 | Enantiomer A of Compound 7 |
| | |
| 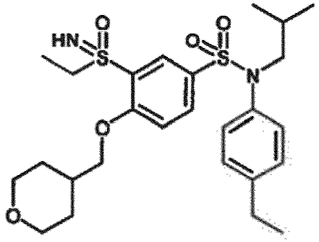 | N-(4-Ethylphenyl)-N-isobutyl-3-ethanesulfoximine-4-(tetrahydropyran-4-ylmethoxy)benzenesulfonamide |
| | Compound 18 |
| 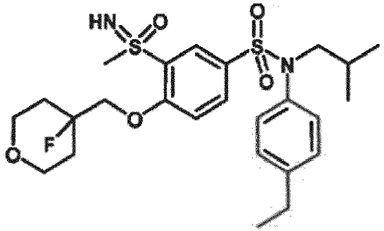 | N-(4-Ethylphenyl)-4-((4-fluorotetrahydro-2H-pyran-4-yl)methoxy)-N-isobutyl-3-(S-methylsulfonimidoyl)-benzenesulfonamide |
| | Compound 30 |
| 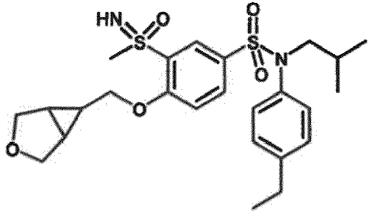 | 4-((3-Oxabicyclo[3.1.0]hexan-6-yl)methoxy)-N-(4-ethylphenyl)-N-isobutyl-3-(S-methylsulfonimidoyl)-benzenesulfonamide |
| | Compound 31 |
| 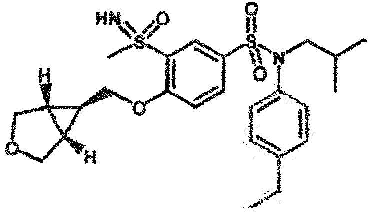 | 4-(((1R,5S,6R)-3-Oxabicyclo[3.1.0]hexan-6-yl)methoxy)-N-(4-ethylphenyl)-N-isobutyl-3-(S-methylsulfonimidoyl)benzenesulfonamide |
| | Compound 35 |
| 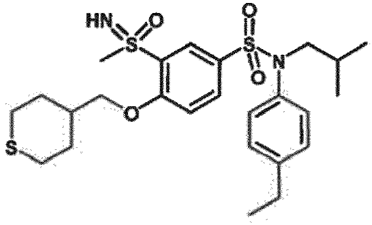 | N-(4-Ethylphenyl)-N-isobutyl-3-(S-methylsulfonimidoyl)-4-(pyridin-4-ylmethoxy) benzenesulfonamide |
| | Compound 37 |
| 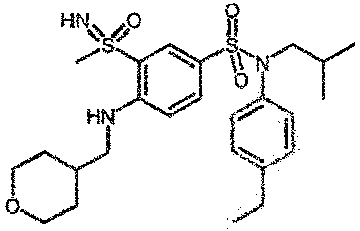 | N-(4-Ethylphenyl)-N-isobutyl-3-(S-methylsulfonimidoyl)-4-[(tetrahydropyran-4-ylmethyl)amino]benzenesulfonamide |
| | Compound 52 |
| 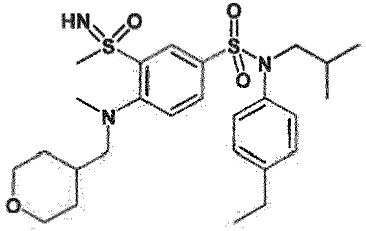 | N-(4-Ethylphenyl)-N-isobutyl-4-(methyl((tetrahydro-2H-pyran-4-yl)methyl)amino)-3-(S-methylsulfonimidoyl)-benzenesulfonamide |
| | Compound 53 |
| 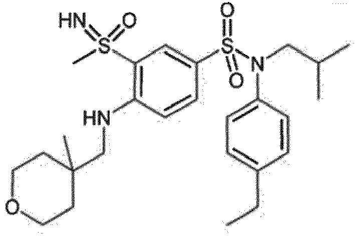 | N-(4-Ethylphenyl)-N-isobutyl-3-(S-methylsulfonimidoyl)-4-(((4-methyltetrahydro-2H-pyran-4-yl)methyl)amino)-benzenesulfonamide |
| | Compound 55 |
| 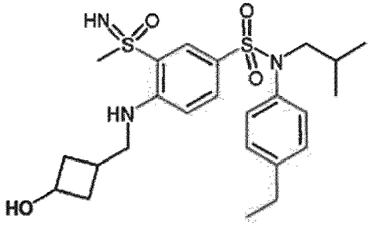 | N-(4-Ethylphenyl)-4-(((3-hydroxycyclobutyl)methyl)-amino)-N-isobutyl-3-(S-methylsulfonimidoyl)-benzenesulfonamide |
| | Compound 61 |
| 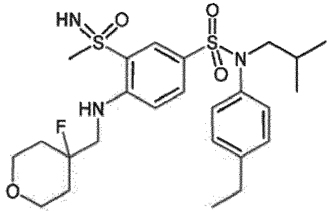 | N-(4-Ethylphenyl)-4-(((4-fluortetrahydro-2H-pyran-4-yl)methyl)amino)-N-isobutyl-3-(S-methylsulfonimidoyl)-benzenesulfonamide |
| | Compound 62 |
| 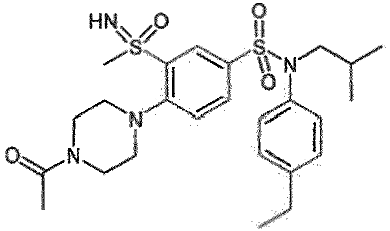 | 4-(4-Acetylpiperazin-1-yl)-N-(4-ethylphenyl)-N-isobutyl-3-(S-methylsulfonimidoyl)-benzenesulfonamide |
| | Compound 63 |
| 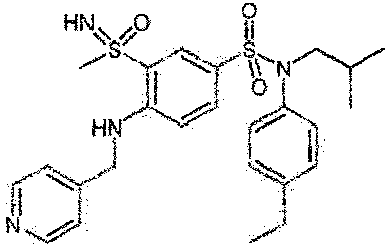 | N-(4-Ethylphenyl)-N-isobutyl-3-(S-methylsulfonimidoyl)-4-((pyridin-4-ylmethyl)-amino)benzenesulfonamide |
| | Compound 64 |

6. Compound according to any one of the preceding claims, **characterized in that** it is chosen from the following compounds:
| | |
|---|---|
| 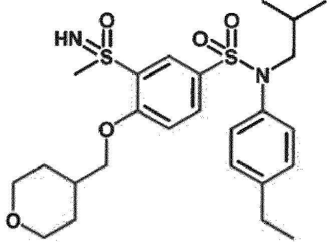 | N-(4-Ethylphenyl)-N-isobutyl-3-methansulfoximino-4-(tetrahydropyran-4-ylmethoxy)-benzenesulfonamide |
| | Compound 26 |
| 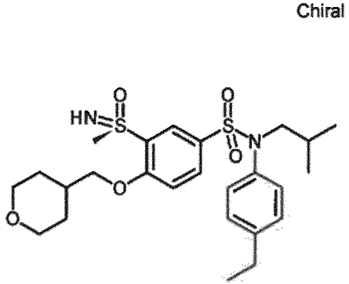 | N-(4-Ethylphenyl)-N-isobutyl-3-methansulfoximino-4-(tetrahydropyran-4-ylmethoxy)benzenesulfonamide |
| | Compound 7 (enantiomer A) |
| 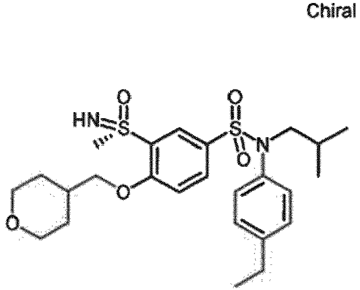 | N-(4-Ethylphenyl)-N-isobutyl-3-methanesulfoximine-4-(tetrahydropyran-4-ylmethoxy)benzenesulfonamide |
| | Compound 8 (enantiomer B) |
| 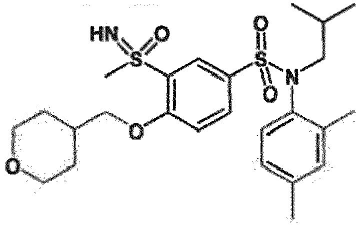 | N-(2,4-Dimethylphenyl)-N-isobutyl-3-(S-methylsulfonimidoyl)-4-((tetrahydro-2H-pyran-4-yl)methoxy)benzenesulfonamide |
| | Compound 142 |

7. Compound according to any one of the preceding claims, for use thereof as a medicament.

8. Compound according to any one of Claims 1 to 6, for use thereof in the treatment of inflammatory disorders and/or autoimmune diseases mediated by the receptor RORγt.

9. Compound for use thereof according to Claim 8, in the treatment of acne.

10. Compound for use thereof according to Claim 8, in the treatment of psoriasis.

11. Pharmaceutical composition comprising one or more compounds as defined according to any one of Claims 1 to 6.

12. Pharmaceutical composition according to Claim 11, for use thereof for the treatment of inflammatory disorders and/or autoimmune diseases mediated by the receptor RORγt, in particular acne, atopic dermatitis and/or psoriasis.
